# EUROPEAN PATENT APPLICATION

(11) **EP 4 428 130 A1**
(43) Date of publication of application: **11.09.2024**
(21) Application number: 22890463.7
(22) Date of filing: 04.11.2022
(51) Int. Cl.: C07D 401/14, C07D 471/04, A61K 31/506

(54) **COMPOUND HAVING BTK PROTEIN DEGRADATION ACTIVITY, AND MEDICAL USES THEREOF**

(30) Priority: 05.11.2021 KR 20210151162; 18.03.2022 KR 20220034349
(71) Applicant: Ubix Therapeutics, Inc., Seoul 05836 (KR)
(72) Inventor: LEE, Song Hee, Seoul 05823 (KR); RYU, Je Ho, Seongnam-si, Gyeonggi-do 13470 (KR); AHN, Jung Min, Incheon 21984 (KR); MOON, Hee Jung, Incheon 22389 (KR); LEE, Ho Hyun, Siheung-si, Gyeonggi-do 14999 (KR); JANG, Mi Young, Ansan-si, Gyeonggi-do 15509 (KR); YUN, Whee Sahng, Seoul 05841 (KR); KIM, Ye Eun, Incheon 21984 (KR); YOO, Sun Mi, Seoul 05855 (KR); LIM, Ye Seul, Seoul 05384 (KR); JEONG, Na Rae, Seoul 02118 (KR); KIM, So Hyuk, Seoul 05841 (KR); CHOI, Ae Ran, Seoul 05855 (KR); KIM, Han Wool, Ansan-si, Gyeonggi-do 15294 (KR)
(74) Representative: Engelhard, Markus
(86) International application number: PCT/KR2022/017291
(87) International publication number: WO 2023/080732

(57) **Abstract**

The present disclosure provides: a compound of a specific chemical structure, having excellent activity with respect to BTK degradation; or a pharmaceutically acceptable salt thereof. The present disclosure also provides a composition comprising the compound or pharmaceutically acceptable salts thereof. The present disclosure also provides are pharmaceutical use for treating or preventing BTK-associated diseases (for example, autoimmune diseases or cancer) of the compound, the salt thereof, and the composition comprising same according to the present disclosure. The present disclosure also provides a method for treating or preventing BTK-associated diseases (for example, autoimmune diseases or cancer), comprising administering, to a subject requiring treatment, an effective amount of the compound, the salt thereof, or the composition comprising same according to the present disclosure.

## Description

### [Technical Field]

The present disclosure relates to a group of compounds having BTK proteolytic activity. In particular, the present disclosure relates to a group of compounds having a specific structure and having very excellent activity of degrading BTK protein. The present disclosure also relates to useful methods of treating diseases related to BTK protein using such compounds. That is, the present disclosure relates to the medical use of the compounds according to the present disclosure for treating or preventing BTK protein related diseases.

### [Background Art]

Bruton's tyrosine kinase (BTK) is a member of the Tec family of tyrosine kinases. It affects early B-cell development and maturation, B-cell activation, and is a regulatory factor that regulates B-cell signaling and survival. BTK is activated through the B-cell receptor and plays an important role in signaling and development of B cells by regulating various major signaling pathways within the cell and inducing signaling essential for cell survival.

However, when BTK is overactivated or overexpressed due to abnormal signal transduction in B cells, it causes abnormal B-cell proliferation and the formation of pathological autoantibodies, resulting in autoimmune diseases such as systemic lupus erythematosus and rheumatoid arthritis, cancer, B-cell malignancy, inflammatory diseases, etc. (see U.S. Patent No. 9,624,224).

Additionally, BTK is known to be overexpressed in B-lineage malignant lymphoid tumors such as B-cell precursor-acute lymphocytic leukemia, chronic lymphocytic leukemia, and non-Hodgkin's lymphoma. Inhibiting BTK in the proliferation of abnormal B-cells can block B-cell-mediated diseases by blocking signal transduction by B-cell receptors. As a result, the use of BTK inhibitors may be a useful approach for treating B-cell mediated diseases. Significant medicinal effects have been demonstrated through BTK inhibition in experimental animal models for autoimmune diseases or B-cell malignancies (see U.S. Patent Application Publication No. 2021-0002285).

Currently, the correlation between BTK inhibitors and many diseases has been well proven, and clinical trials for leukemia (e.g., lymphocytic leukemia, B-cell leukemia, prolymphocytic leukemia, myeloid leukemia, hairy cell leukemia), lymphoma (e.g., B-cell lymphoma, CNS (central nervous system) lymphoma, follicular lymphoma, non-Hodgkin lymphoma, Hodgkin lymphoma, lymphocytic lymphoma, mantle cell lymphoma, Burkitt lymphoma, B-cell diffuse lymphoma, marginal zone lymphoma, small-cell lymphoma, lymphoplasmacytoid lymphoma, Richter's transformation (Richter's syndrome)), Waldenstrom macroglobulinemia, ependymoma, medulloblastoma, glioblastoma, primitive neuroectodermal tumor, non-small cell lung cancer, breast cancer, pancreatic cancer, gastroesophageal cancer, B-cell malignancy, recurrent mature B-cell neoplasm, multiple myeloma, metastatic pancreatic adenocarcinoma, multiple sclerosis, autoimmune diseases (e.g., rheumatoid arthritis, autoimmune hemolytic anemia, warm antibody autoimmune hemolytic anemia, chronic spontaneous urticaria (CSU), food allergy, immune thrombocytopenia, Sjögren syndrome, systemic lupus erythematosus), graft-versus-host-disease, agammaglobulinemia, hepatitis, neuromyelitis optica, acute inflammatory disease, etc. are being conducted using various BTK inhibitors (see https://www.clinicaltrials.gov/).

### [Disclosure]

### [Technical Problem]

Deletion of BTK protein may provide an alternative and more effective strategy for inhibiting BTK activity. Therefore, a problem to be solved by the present invention is to provide a compound having Bruton's tyrosine kinase (BTK)-degrading activity, a pharmaceutical composition comprising the same as an active ingredient, and a medical use for the treatment or prevention of BTK-related diseases (preferably, autoimmune diseases or cancers).

Another problem to be solved by the present invention is to provide a method for treating or alleviating BTK-related diseases (preferably, autoimmune diseases or cancers), characterized in that it degrades BTK and consequently lowers BTK activity, and it comprises administering to a patient in need of treatment, improvement or prevention of BTK related diseases the compound according to the present invention.

### [Technical Solution]

### Compounds of the present invention

In order to solve the above problem, one embodiment of the present invention provides a compound represented by the following Chemical Formula 1 or a pharmaceutically acceptable salt thereof.

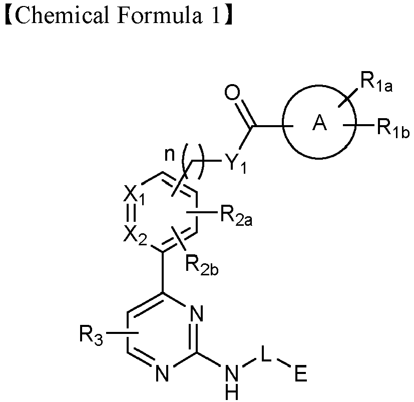

In the Chemical Formula 1,
A is carbocycle, heterocycle, aryl, or heteroaryl,
R₁ₐ and R_{1b} are each independently H, halogen, C₁₋₆ alkyl, C₁₋₆ alkoxy, or carbocycle, wherein one or more hydrogens in the alkyl, alkoxy, or carbocycle are optionally substituted with one or more selected from the group consisting of halogen, C₁₋₆ alkyl, C₁₋₆ haloalkyl, hydroxy, and C₁₋₆ hydroxyalkyl,
R₂ₐ and R_{2b} are each independently H, halogen, C₁₋₆ alkyl, C₁₋₆ haloalkyl, C₁₋₆ alkoxy, C₁₋₆ haloalkoxy, hydroxy, or C₁₋₆ hydroxyalkyl,
R₃ is H, halogen, C₁₋₆ alkyl, C₁₋₆ haloalkyl, or -NH₂,
X₁ and X₂ are each independently CH or N,
Y₁ is NH or O,
n is an integer of 0, 1, or 2,
L is the following Chemical Formula 2, 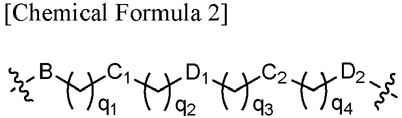
in the Chemical Formula 2,
B is aryl or heteroaryl, wherein one or more hydrogens in the aryl or heteroaryl are optionally substituted with one or more selected from the group consisting of C₁₋₆ alkyl, halogen, and C₁₋₆ haloalkyl,
C₁ and C₂ are each independently direct bond, carbocycle, or heterocycle, wherein one or more hydrogens in the carbocycle or heterocycle are optionally substituted with one or more selected from the group consisting of C₁₋₆ alkyl, halogen, C₁₋₆ haloalkyl, and hydroxy,
D₁ and D₂ are each independently direct bond, -O-, -N(R₄)- -C(O)-, -CC-, -C(O)NH-, or -NHC(O)-, wherein R₄ is H, C₁₋₆ alkyl, or C₁₋₆ haloalkyl,
q₁, q₂, q₃ and q₄ are each independently an integer of from 0 to 3,
E is the following Chemical Formula 3 or Chemical Formula 4, 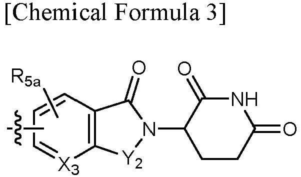 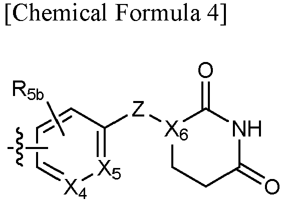
in the Chemical Formula 3 and 4,
X₃, X₄, X₅, and X₆ are each independently CH or N,
Y₂ is -C(R₆ₐ)(R_{6b})-, -C(O)-, -C(R₆ₐ)(R_{6b})-C(R₆ₐ)(R_{6b})-, -C(R₆ₐ)=C(R_{6b})-, -C(R₆ₐ)=N-, - N=C(R₆ₐ)-, or -N=N-,
Z is direct bond, -C(R₆ₐ)(R_{6b})-, -N(R₆ₐ)-, -O-, or -C(O)NH-,
R₅ₐ and R_{5b} are each independently H, halogen, C₁₋₆ alkyl, C₁₋₆ alkoxy, C₁₋₆ haloalkyl, or C₁₋₆ haloalkoxy, and
R₆ₐ and R_{6b} are each independently H, halogen, C₁₋₆ alkyl, C₁₋₆ haloalkyl, C₁₋₆ alkoxy, or C₁₋₆ haloalkoxy.

The compounds according to the present invention are PROTAC (Proteolysis-targeting chimera) compounds of'BTK binding ligand - linker - E3 ligase ligand' ('Moiety located to the left of L - linker (L) - Moiety located to the right of L' in Chemical Formula 1). The present inventors evaluated various substituents. In the case of the structure such as Chemical Formula 1 of the present disclosure, particularly, in the case of the preferred structure to be described later, excellent compounds in various aspects including BTK degradation activity could be obtained. That is, the present invention provides novel compounds having excellent BTK degradation activity, (metabolism) stability, etc., and excellent physicochemical properties (cLogP value, water solubility, cell membrane permeability) as an active ingredient.

As used herein, the terms "substituent", "radical", "group", "moiety", and "fragment" may be used interchangeably.

If a substituent is described as "optionally substituted", the substituent may be unsubstituted or substituted with one or more of the defined substituents. If the substitutable position is unsubstituted, the default substituent is hydrogen.

As used herein, the term "alkyl" means a saturated straight chain or branched non-cyclic hydrocarbon, unless the context clearly dictates otherwise, having from 1 to 10 carbon atoms. "Lower alkyl" means alkyl having from 1 to 4 carbon atoms. Representative saturated straight chain alkyls include -methyl, -ethyl, -n-propyl, -n-butyl, -n-pentyl, -n-hexyl, -n-heptyl, -n-octyl, - n-nonyl and -n-decyl, while saturated branched alkyls include -isopropyl, - sec-butyl, -isobutyl, - tert-butyl, -isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like. In a preferred embodiment of the present invention, alkyl is methyl, ethyl, isopropyl, or t-butyl.

As used herein, the term "alkoxy" means -O-(alkyl), including -OCH₃, -OCH₂CH₃, - O(CH₂)₂CH₃, -OC(CH₃)₂H, -OC(CH₃)₃, and the like, wherein the alkyl is as defined above.

As used herein, if the term "C₁₋₆", "C1-6", or "C1-C6" is used, it means the number of carbon atoms is from 1 to 6. For example, C₁₋₆alkyl means an alkyl which carbon number is any integer of from 1 to 6.

As used herein, the terms "halogen" and "halo" mean fluorine, chlorine, bromine or iodine. In a preferred embodiment of the present invention, the halogen is fluorine.

As used herein, the term "haloalkyl" or "haloalkoxy" means an alkyl or alkoxy group, wherein one or more hydrogen atoms are substituted with halogen atoms. For example, the haloalkyl includes -CF₃, -CHF₂, -CH₂F, -CBr₃, -CHBr₂, -CH₂Br, -CCl₃, -CHCl₂, -CH₂CI, -CI₃, - CHI₂, -CH₂I, -CH₂-CF₃, -CH₂-CHF₂, -CH₂-CH₂F, -CH₂-CBr₃, -CH₂-CHBr₂, -CH₂-CH₂Br, -CH₂-CC1₃, -CH₂-CHC1₂, -CH₂-CH₂CI, -CH₂-CI₃, -CH₂-CHI₂, -CH₂-CH₂I, and the like, wherein alkyl, alkoxy and halogen are as described above. In a preferred embodiment of the present invention, haloalkyl is -CF₃.

As used herein, the term "hydroxyalkyl" means linear or branched C₁₋₁₀alkyl substituted with one or more hydroxy groups. Examples of hydroxyalkyl group include hydroxymethyl, hydroxyethyl, hydroxy(iso)propyl and hydroxybutyl, but is not limited thereto.

As used herein, the term "carbocycle" or "cycloalkyl" means a monocyclic or polycyclic saturated ring having carbon and hydrogen atoms and having no carbon-carbon multiple bonds. Examples of monocyclic rings include, but are not limited to, (C₃-C₇)cycloalkyl groups, including cyclopropyl, cyclobutyl, cyclopentyl, cyclohexyl, and cycloheptyl. Examples of polycyclic rings include, but are not limited to, fused bicyclic rings such as octahydropentalene and decahydronaphthalene; spiro rings such as spiro[3.3]heptane, spiro[3.4]octane, spiro[3.5]nonane, spiro[4.4]nonane, spiro[4.5]decane, and spiro[5.5]undecane; and bridged bicycle rings such as bicyclo[2.1.1]hexane, bicyclo[2.2.1]heptane, and bicyclo[2.2.2]octane. A cycloalkyl group can be unsubstituted or optionally substituted. In an embodiment of the present invention, cycloalkyl is monocyclic ring.

The term "heterocycle" or "heterocycloalkyl" means a 5- to 7-membered monocyclic, or 7- to 12-membered bicyclic, saturated heterocyclic ring which contains from 1 to 4 heteroatoms independently selected from nitrogen, oxygen and sulfur, and wherein the nitrogen and sulfur heteroatoms can be optionally oxidized, and the nitrogen heteroatom can be optionally quaternized. Heterocycles include heteroaryls as defined above. Representative heterocycles include oxiran, oxetan, tetrahydrofuran, tetrahydropyran, 1,4-dioxane, aziridine, azetidine, pyrrolidine, piperidine, piperazine, pyrrolidinone, hydantoine, valerolactam, thiirane, thietane, tetrahydrothiophene, tetrahydrothiopyran, morpholine, tetrahydropyridine, and tetrahydropyrimidine. Heterocycles include a bicyclic ring in which part of the heterocycle is fused to a benzene or cyclopenta-1,3-diene ring. The heterocycle can be attached via any heteroatom or carbon atom. In addition, heterocycles include fused bicyclic rings, spiro rings and bridged bicyclic rings in which one or more carbon atoms of the aforementioned polycyclic rings are replaced with nitrogen, oxygen or sulfur atoms. For example, when the heteroatom is nitrogen, these include, but are limited to, fused heterobicyclic rings such as octahydrocyclopenta[c]pyrrole, octahydropyrrolo[3,4-c]pyrrole, decahydroisoquinoline, and decahydro-2,6-naphthyridine; spiro rings such as 2-azaspiro[3.3]heptane, 2,6-diazaspiro[3.3]heptane, 2-azaspiro[3.4]octane, 2,6-diazaspiro[3.4]octane, 2-azaspiro[3.5]nonane, 2,7-diazaspiro[3.5]nonane, 2-azaspiro[4.4]nonane, 2,7-diazaspiro[4.4]nonane, 8-azaspiro[4.5]decane, 2,8-diazaspiro[4.5]decane, 3-azaspiro[5.5]undecane, and 3,9-diazaspiro[5.5]undecane; and bridged heterobicyclic rings such as 2-azabicyclo[2.1.1]hexane, 2-azabicyclo[2.2.1]heptane, 2,5-diazabicyclo[2.2.1]heptane, 2-azabicyclo[2.2.2]octane, and 2,5-diazabicyclo[2.2.2]octane.

As used herein, the term "aryl" means a carbocyclic aromatic group containing from 5 to 10 atoms. Representative examples include, but are not limited to, phenyl (benzene), tolyl, xylyl, naphthyl, tetrahydronaphthyl, anthracenyl, fluorenyl, indenyl, and azulenyl. A carbocyclic aromatic group can be unsubstituted or optionally substituted.

As used herein, the term "heteroaryl" means an aromatic heterocycle ring of 5- to 10 members and having at least one heteroatom selected from nitrogen, oxygen and sulfur, and containing at least 1 carbon atom, including both mono- and bicyclic ring systems. Representative heteroaryls are furan, 4H-pyran, pyrrole, imidazole, pyrazole, triazole, tetrazole, pyridine, pyrimidine, pyridazine, pyrazine, triazine, thiophene, ozaxole, isoxazole, thiazole, isothiazole, oxadiazole, benzofuran, benzothiophene, quinoline, dihydroquinoline, isoquinoline, dihydroisoquinoline, indole, benzoxazole, benzimidazole, benzothiazole, cinnoline, phthalazine, quinazoline, 1H-azepine, thiadiazole, tetrahydroisoquinoline, tetrahydropyrazolopyrazine, etc.

In this specification, * or 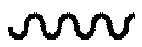 means connected to another moiety.

Preferably, in various aspects such as BTK degradation activity, (metabolism) stability, physicochemical properties, and the like, a preferred embodiment of the present invention provides a compound represented by the Chemical Formula 1 or a pharmaceutically acceptable salt thereof, wherein
A is carbocycle, heterocycle, aryl, or heteroaryl,
R₁ₐ and R_{1b} are each independently H, halogen, C₁₋₆ alkyl, or C₁₋₆ alkoxy, wherein one or more hydrogens in the alkyl or alkoxy are optionally substituted with one or more selected from the group consisting of halogen, C₁₋₃ alkyl, C₁₋₃ haloalkyl, hydroxy, and C₁₋₃ hydroxyalkyl,
R₂ₐ and R_{2b} are each independently H, halogen, C₁₋₆ alkyl, C₁₋₆ haloalkyl, C₁₋₆ alkoxy, C₁₋₆ haloalkoxy, hydroxy, or C₁₋₆ hydroxyalkyl,
R₃ is H, halogen, C₁₋₃ alkyl, C₁₋₃ haloalkyl, or -NH₂,
X₁ and X₂ are each independently CH or N,
Y₁ is NH or O,
n is an integer of 0, 1, or 2,
L is the following Chemical Formula 2, 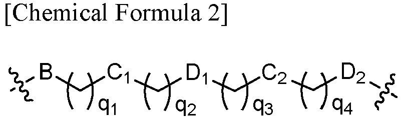
in the Chemical Formula 2,
B is aryl or heteroaryl, wherein one or more hydrogens in the aryl or heteroaryl are optionally substituted with one or more selected from the group consisting of C₁₋₃ alkyl, halogen, and C₁₋₃ haloalkyl,
C₁ and C₂ are each independently direct bond, carbocycle, or heterocycle, wherein one or more hydrogens in the carbocycle or heterocycle are optionally substituted with one or more selected from the group consisting of C₁₋₃ alkyl, halogen, and hydroxy,
D₁ and D₂ are each independently direct bond, -O-, -N(R₄)- -C(O)-, -CC-, -C(O)NH-, or -NHC(O)-, wherein R₄ is H, C₁₋₃ alkyl, or C₁₋₃ haloalkyl,
q₁, q₂, q₃ and q₄ are each independently an integer of from 0 to 3,
E is the following Chemical Formula 3 or Chemical Formula 4, 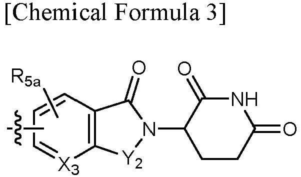 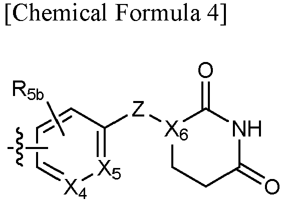
in the Chemical Formula 3 and 4,
X₃, X₄, X₅, and X₆ are each independently CH or N,
Y₂ is -C(R₆ₐ)(R_{6b})-, -C(O)-, -C(R₆ₐ)=C(R_{6b})-, -C(R₆ₐ)=N-, -N=C(R₆ₐ)-, or -N=N-,
Z is direct bond, -C(R₆ₐ)(R_{6b})-, -N(R₆ₐ)-, -O-, or -C(O)NH-,
R₅ₐ and R_{5b} are each independently H, halogen, C₁₋₃ alkyl, C₁₋₃ alkoxy, C₁₋₃ haloalkyl, or C₁₋₆ haloalkoxy, and
R₆ₐ and R_{6b} are each independently H, halogen, C₁₋₃ alkyl, or C₁₋₃ haloalkyl.

More preferably, in various aspects such as BTK degradation activity, (metabolism) stability, physicochemical properties, and the like, particularly in terms of BTK degradation activity, a more preferred embodiment of the present invention provides a compound represented by the Chemical Formula 1 or a pharmaceutically acceptable salt thereof, wherein
A is azetidine, pyrrolidine, piperidine, piperazine, benzene, pyridine, pyrimidine, pyrazole, triazole, oxazole, thiazole, oxadiazole, or thiadiazole,
R₁ₐ and R_{1b} are each independently H, halogen, C₁₋₆ alkyl, or C₁₋₆ alkoxy, wherein one or more hydrogens in the alkyl or alkoxy are optionally substituted with one or more selected from the group consisting of halogen, C₁₋₃ alkyl, and hydroxy,
R₂ₐ and R_{2b} are each independently H, halogen, C₁₋₃ alkyl, C₁₋₃ haloalkyl, or C₁₋₃ hydroxyalkyl,
R₃ is H or halogen,
X₁ is CH or N,
X₂ is CH,
Y₁ is NH or O,
n is an integer of 0, 1, or 2,
L is the following Chemical Formula 2, 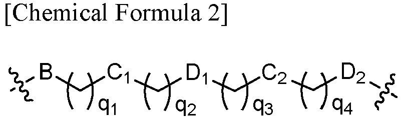
in the Chemical Formula 2,
B is benzene, pyridine, pyrimidine, pyrazine, pyridazine, tetrahydroisoquinoline, pyrazole, triazole, or tetrahydropyrazolopyrazine,
C₁ and C₂ are each independently direct bond, cyclobutane, cyclohexane, azetidine, pyrrolidine, piperidine, or piperazine, wherein one or more hydrogens in the cyclobutane, cyclohexane, azetidine, pyrrolidine, piperidine, or piperazine are optionally substituted with one or more selected from the group consisting of C₁₋₃ alkyl, halogen, and hydroxy,
D₁ and D₂ are each independently direct bond, -O-, -NH-, -C(O)-, -C(O)NH-, or - NHC(O)-,
q₁, q₂, q₃ and q₄ are each independently an integer of from 0 to 3,
E is the following Chemical Formula 3 or Chemical Formula 4, 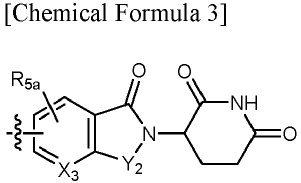 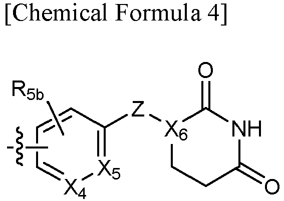
in the Chemical Formula 3 and 4,
X₃, X₄, X₅, and X₆ are each independently CH or N,
Y₂ is -CH₂- or -C(O)-,
Z is direct bond, -NH-, or -O-, and
R₅ₐ and R_{5b} are each independently H, halogen, C₁₋₃ alkyl, or C₁₋₃ alkoxy.

Preferably, in the BTK PROTAC compound according to the present invention, the moiety (BTK ligand) on the left of the linker (L) is one of the following structures. When it has the structure below, BTK degradation activity is excellent and it is more suitable for various purposes of the present invention.

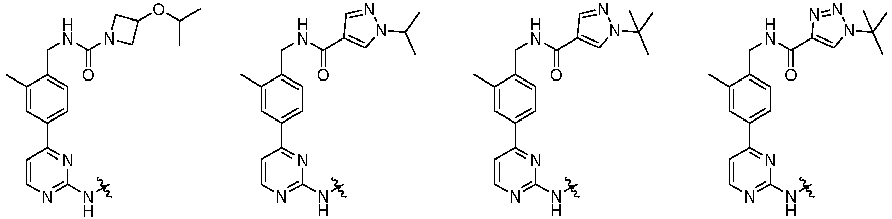

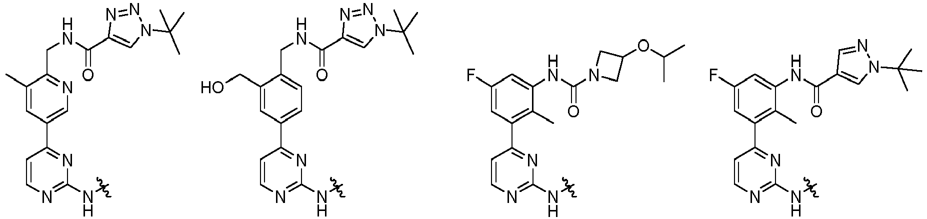

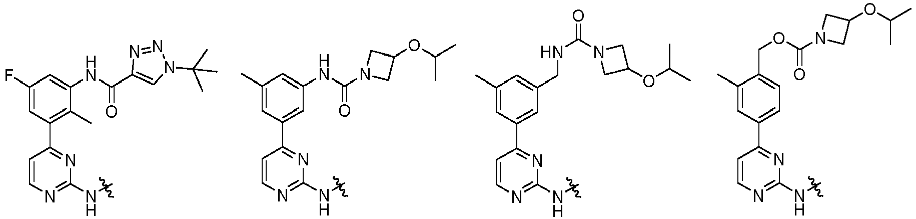

Preferably, in the BTK PROTAC compounds according to the present invention, the linker (L) is any one of the following linkers. When it has the following linker, BTK degradation activity is excellent, and it is more suitable for various purposes of the present invention.

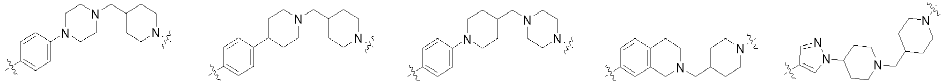

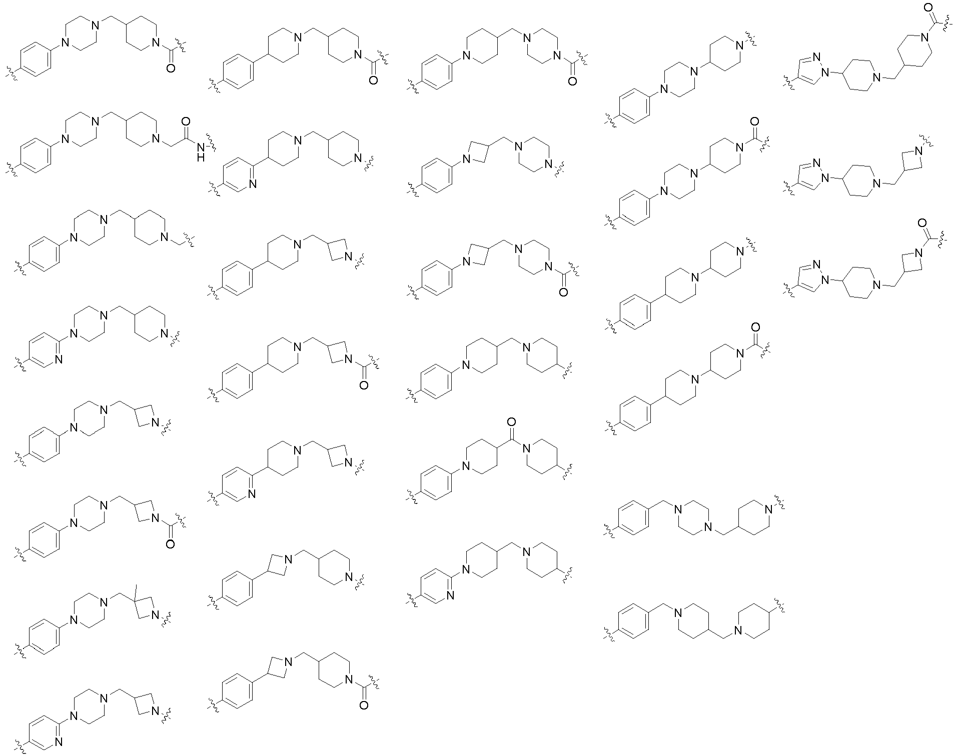

Preferably, in the BTK PROTAC compound according to the present invention, the moiety (CRBN ligand) on the right side of the linker (L) is one of the following structures. When it has the structure below, BTK degradation activity is excellent and it is more suitable for various purposes of the present invention.

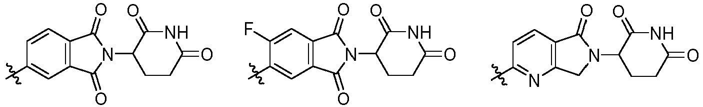

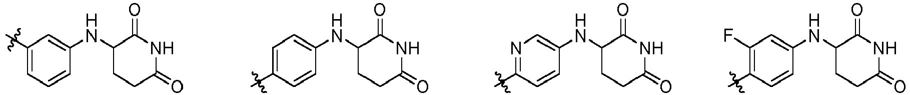

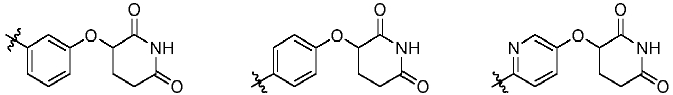

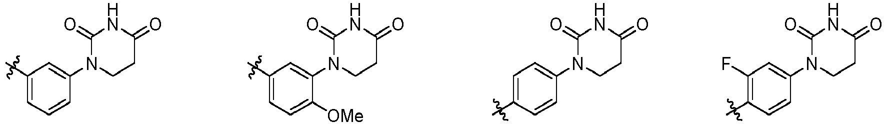

Non-limiting examples of the compound of Chemical Formula 1 according to the present disclosure are the compounds prepared in the Examples described below. Each example number corresponds to each compound number. For example, the number of the final compound prepared in Example 10 is Compound 10.

Among the compounds, the compounds of Table 1 below were particularly preferable in terms of BTK degradation activity.

**[Table 1]**

| Comp ound no. | IUPAC Name |
|---|---|
| 1 | N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-3-isopropoxyazetidine-1-carboxamide |
| 2 | 1-(tert-butyl)-N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-1H-pyrazole-4-carboxamide |
| 3 | 1-(tert-butyl)-N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-1H-1,2,3-triazole-4-carboxamide |
| 7 | N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-3-isopropoxyazetidine-1-carboxamide |
| 8 | 1-(tert-butyl)-N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1H-pyrazole-4-carboxamide |
| 9 | 1-(tert-butyl)-N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1H-1,2,3-triazole-4-carboxamide |
| 10 | N-(4-(2-((4-(4-((1-(6-(2,6-dioxopiperidin-3-yl)-5-oxo-6,7-dihydro-5H-pyrrolo[3,4-b]pyridin-2-yl)piperidin-4-yl)methyl)piperazin-1-yl)phenyl)amino)pyrimidin-4-yl)-2-methylbenzyl)-3-isopropoxyazetidine-1-carboxamide |
| 11 | N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-3-isopropoxyazetidine-1-carboxamide |
| 12 | N-(4-(2-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)pyrimidin-4-yl)-2-methylbenzyl)-3-isopropoxyazetidine-1-carboxamide |
| 13 | N-(4-(2-((4-(4-((1-(2-(2,6-dioxopiperidin-3-yl)-6-fluoro-1,3-dioxoisoindolin-5-yl)azetidin-3-yl)methyl)piperazin-1-yl)phenyl)amino)pyrimidin-4-yl)-2-methylbenzyl)-3-isopropoxyazetidine-1-carboxamide |
| 14 | N-(4-(2-((4-(4-((1-(2-(2,6-dioxopiperidin-3-yl)-1,3-dioxoisoindolin-5-yl)-3-methylazetidin-3-yl)methyl)piperazin-1-yl)phenyl)amino)pyrimidin-4-yl)-2-methylbenzyl)-3-isopropoxyazetidine-1-carboxamide |
| 15 | N-(4-(2-((4-(1-((1-(2-(2,6-dioxopiperidin-3-yl)-1,3-dioxoisoindolin-5-yl)piperidin-4-yl)methyl)piperidin-4-yl)phenyl)amino)pyrimidin-4-yl)-2-methylbenzyl)-3-isopropoxyazetidine-1-carboxamide |
| 16 | N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-3-isopropoxyazetidine-1-carboxamide |
| 17 | N-(4-(2-((6-(4-((1-(2-(2,6-dioxopiperidin-3-yl)-1,3-dioxoisoindolin-5-yl)piperidin-4-yl)methyl)piperazin-1-yl)pyridin-3-yl)amino)pyrimidin-4-yl)-2-methylbenzyl)-3-isopropoxyazetidine-1-carboxamide |
| 18 | N-(4-(2-((6-(4-((1-(2-(2,6-dioxopiperidin-3-yl)-1,3-dioxoisoindolin-5-yl)azetidin-3-yl)methyl)piperazin-1-yl)pyridin-3-yl)amino)pyrimidin-4-yl)-2-methylbenzyl)-3-isopropoxyazetidine-1-carboxamide |
| 19 | N-(4-(2-((6-(1-((1-(2-(2,6-dioxopiperidin-3-yl)-1,3-dioxoisoindolin-5-yl)piperidin-4-yl)methyl)piperidin-4-yl)pyridin-3-yl)amino)pyrimidin-4-yl)-2-methylbenzyl)-3-isopropoxyazetidine-1-carboxamide |
| 20 | N-(4-(2-((6-(1-((1-(2-(2,6-dioxopiperidin-3-yl)-1,3-dioxoisoindolin-5-yl)azetidin-3-yl)methyl)piperidin-4-yl)pyridin-3-yl)amino)pyrimidin-4-yl)-2-methylbenzyl)-3-isopropoxyazetidine-1-carboxamide |
| 21 | N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-3-isopropoxyazetidine-1-carboxamide |
| 22 | N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-3-isopropoxyazetidine-1-carboxamide |
| 23 | N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-fluoro-2-methylphenyl)-3-isopropoxyazetidine-1-carboxamide |
| 26 | N-(3-(2-((4-(4-((1-(6-(2,6-dioxopiperidin-3-yl)-5-oxo-6,7-dihydro-5H-pyrrolo[3,4-b]pyridin-2-yl)piperidin-4-yl)methyl)piperazin-1-yl)phenyl)amino)pyrimidin-4-yl)-5-fluoro-2-methylphenyl)-3-isopropoxyazetidine-1-carboxamide |
| 27 | N-(3-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-5-fluoro-2-methylphenyl)-3-isopropoxyazetidine-1-carboxamide |
| 31 | 1-(tert-butyl)-N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1H-pyrazole-4-carboxamide |
| 32 | 1-(tert-butyl)-N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1H-1,2,3-triazole-4-carboxamide |
| 33 | N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1-isopropyl-1H-pyrazole-4-carboxamide |
| 34 | N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1-isopropyl-1H-pyrazole-4-carboxamide |
| 35 | N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1-isopropyl-1H-pyrazole-4-carboxamide |
| 36 | N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1-isopropyl-1H-pyrazole-4-carboxamide |
| 37 | 1-(tert-butyl)-N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1H-pyrazole-4-carboxamide |
| 38 | 1-(tert-butyl)-N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1H-pyrazole-4-carboxamide |
| 39 | 1-(tert-butyl)-N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1H-1,2,3-triazole-4-carboxamide |
| 40 | 1-(tert-butyl)-N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1H-1,2,3-triazole-4-carboxamide |
| 41 | N-(4-(2-((4-(1-((1-(2-(2,6-dioxopiperidin-3-yl)-1,3-dioxoisoindolin-5-yl)piperidin-4-yl)methyl)azetidin-3-yl)phenyl)amino)pyrimidin-4-yl)-2-methylbenzyl)-3-isopropoxyazetidine-1-carboxamide |
| 42 | N-(4-(2-((4-(4-((1-(3-((2,6-dioxopiperidin-3-yl)oxy)phenyl)piperidin-4-yl)methyl)piperazin-1-yl)phenyl)amino)pyrimidin-4-yl)-2-methylbenzyl)-3-isopropoxyazetidine-1-carboxamide |
| 43 | N-(4-(2-((4-(4-((1-(4-((2,6-dioxopiperidin-3-yl)oxy)phenyl)piperidin-4-yl)methyl)piperazin-1-yl)phenyl)amino)pyrimidin-4-yl)-2-methylbenzyl)-3-isopropoxyazetidine-1-carboxamide |
| 44 | N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1-isopropyl-1H-pyrazole-4-carboxamide |
| 45 | 1-(tert-butyl)-N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1H-pyrazole-4-carboxamide |
| 46 | 1-(tert-butyl)-N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1H-1,2,3-triazole-4-carboxamide |
| 47 | N-(4-(2-((4-(1-((1-(4-((2,6-dioxopiperidin-3-yl)oxy)phenyl)piperidin-4-yl)methyl)piperidin-4-yl)phenyl)amino)pyrimidin-4-yl)-2-methylbenzyl)-3-isopropoxyazetidine-1-carboxamide |
| 48 | N-(4-(2-((4-(4-((1-(4-((2,6-dioxopiperidin-3-yl)oxy)phenyl)piperidin-4-yl)methyl)piperazin-1-yl)phenyl)amino)pyrimidin-4-yl)-2-methylbenzyl)-1-isopropyl-1H-pyrazole-4-carboxamide |
| 49 | N-(4-(2-((4-(1-((1-(4-((2,6-dioxopiperidin-3-yl)oxy)phenyl)piperidin-4-yl)methyl)piperidin-4-yl)phenyl)amino)pyrimidin-4-yl)-2-methylbenzyl)-1-isopropyl-1H-pyrazole-4-carboxamide |
| 50 | N-(4-(2-((4-(4-((1-(5-((2,6-dioxopiperidin-3-yl)oxy)pyridin-2-yl)piperidin-4-yl)methyl)piperazin-1-yl)phenyl)amino)pyrimidin-4-yl)-2-methylbenzyl)-3-isopropoxyazetidine-1-carboxamide |
| 51 | N-(4-(2-((4-(1-((1-(5-((2,6-dioxopiperidin-3-yl)oxy)pyridin-2-yl)piperidin-4-yl)methyl)piperidin-4-yl)phenyl)amino)pyrimidin-4-yl)-2-methylbenzyl)-3-isopropoxyazetidine-1-carboxamide |
| 52 | N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-3-isopropoxyazetidine-1-carboxamide |
| 53 | N-(4-(2-((4-(1-((1-(5-((2,6-dioxopiperidin-3-yl)amino)pyridin-2-yl)piperidin-4-yl)methyl)piperidin-4-yl)phenyl)amino)pyrimidin-4-yl)-2-methylbenzyl)-3-isopropoxyazetidine-1-carboxamide |
| 54 | N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-3-isopropoxyazetidine-1-carboxamide |
| 55 | N-(4-(2-((4-(4-((1-(4-((2,6-dioxopiperidin-3-yl)amino)phenyl)piperidin-4-yl)methyl)piperazin-1-yl)phenyl)amino)pyrimidin-4-yl)-2-methylbenzyl)-3-isopropoxyazetidine-1-carboxamide |
| 56 | N-(4-(2-((4-(1-((1-(4-((2,6-dioxopiperidin-3-yl)amino)phenyl)piperidin-4-yl)methyl)piperidin-4-yl)phenyl)amino)pyrimidin-4-yl)-2-methylbenzyl)-3-isopropoxyazetidine-1-carboxamide |
| 57 | N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1-isopropyl-1H-pyrazole-4-carboxamide |
| 58 | N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1-isopropyl-1H-pyrazole-4-carboxamide |
| 59 | 1-(tert-butyl)-N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1H-pyrazole-4-carboxamide |
| 60 | 1-(tert-butyl)-N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1H-pyrazole-4-carboxamide |
| 61 | 1-(tert-butyl)-N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1H-1,2,3-triazole-4-carboxamide |
| 62 | 1-(tert-butyl)-N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1H-1,2,3-triazole-4-carboxamide |
| 63 | N-(4-(2-((4-((4-((1-(3-((2,6-dioxopiperidin-3-yl)oxy)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 64 | N-(4-(2-((4-((4-((1-(3-((2,6-dioxopiperidin-3-yl)amino)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 65 | N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-3-isopropoxyazetidine-1-carboxamide |
| 66 | N-(4-(2-((4-(4-((1-(4-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-3-isopropoxyazetidine-1-carboxamide |
| 67 | N-(4-(2-((4-((4-((1-(3-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 68 | N-(4-(2-((4-((4-((1-(4-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 69 | 1-(tert-butyl)-N-(4-(2-((4-((4-((1-(3-((2,6-dioxopiperidin-3-yl)amino)phenyl)piperidin-4-yl)methyl)piperazin-1-yl)methyl)phenyl)amino)pyrimidin-4-yl)-2-methylbenzyl)-1H-1,2,3-triazole-4-carboxamide |
| 70 | 1-(tert-butyl)-N-(4-(2-((4-(4-((1-(3-(2,4-dioxotetrahydropyrimidin-1 (2H)-yl)phenyl)piperidin-4-yl)methyl)piperazin-1-yl)phenyl)amino)pyrimidin-4-yl)-2-methylbenzyl)-1H-1,2,3-triazole-4-carboxamide |
| 71 | 1-(tert-butyl)-N-(4-(2-((4-(4-((1-(4-(2,4-dioxotetrahydropyrimidin-1 (2H)-yl)phenyl)piperidin-4-yl)methyl)piperazin-1-yl)phenyl)amino)pyrimidin-4-yl)-2-methylbenzyl)-1H-1,2,3-triazole-4-carboxamide |
| 72 | 1-(tert-butyl)-N-(4-(2-((4-(4-((1-(4-((2,6-dioxopiperidin-3-yl)amino)-2-fluorophenyl)piperidin-4-yl)methyl)piperazin-1-yl)phenyl)amino)pyrimidin-4-yl)-2-methylbenzyl)-1H-1,2,3-triazole-4-carboxamide |
| 73 | 1-(tert-butyl)-N-(4-(2-((4-(4-((1-(4-(2,4-dioxotetrahydropyrimidin-1(2H)-yl)-2-fluorophenyl)piperidin-4-yl)methyl)piperazin-1-yl)phenyl)amino)pyrimidin-4-yl)-2-methylbenzyl)-1H-1,2,3-triazole-4-carboxamide |
| 74 | 1-(tert-butyl)-N-(4-(2-((4-((4-((1-(3-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-1H-1,2,3-triazole-4-carboxamide |
| 75 | 1-(tert-butyl)-N-(4-(2-((4-((4-((1-(4-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-1H-1,2,3-triazole-4-carboxamide |
| 76 | 1-(tert-butyl)-N-(4-(2-((6-(4-((1-(3-(2,4-dioxotetrahydropyrimidin-1 (2H)-yl)phenyl)piperidin-4-yl)methyl)piperazin-1-yl)pyridin-3-yl)amino)pyrimidin-4-yl)-2-methylbenzyl)-1H-1,2,3-triazole-4-carboxamide |
| 77 | 1-(tert-butyl)-N-(4-(2-((6-(4-((1-(4-(2,4-dioxotetrahydropyrimidin-1 (2H)-yl)phenyl)piperidin-4-yl)methyl)piperazin-1-yl)pyridin-3-yl)amino)pyrimidin-4-yl)-2-methylbenzyl)-1H-1,2,3-triazole-4-carboxamide |
| 78 | 1-(tert-butyl)-N-(4-(2-((4-(1-((1-(3-(2,4-dioxotetrahydropyrimidin-1 (2H)-yl)phenyl)piperidin-4-yl)methyl)piperidin-4-yl)phenyl)amino)pyrimidin-4-yl)-2-methylbenzyl)-1H-1,2,3-triazole-4-carboxamide |
| 79 | 1-(tert-butyl)-N-(4-(2-((4-(1-((1-(4-(2,4-dioxotetrahydropyrimidin-1 (2H)-yl)phenyl)piperidin-4-yl)methyl)piperidin-4-yl)phenyl)amino)pyrimidin-4-yl)-2-methylbenzyl)-1H-1,2,3-triazole-4-carboxamide |
| 80 | N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1-isopropyl-1H-pyrazole-4-carboxamide |
| 81 | 1-(tert-butyl)-N-(4-(2-((4-(4-((1-(3-(2,4-dioxotetrahydropyrimidin-1 (2H)-yl)phenyl)piperidin-4-yl)methyl)piperazin-1-yl)phenyl)amino)pyrimidin-4-yl)-2-methylbenzyl)-1H-pyrazole-4-carboxamide |
| 82 | N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1-isopropyl-1H-pyrazole-4-carboxamide |
| 83 | 1-(tert-butyl)-N-(4-(2-((6-(4-((1-(3-(2,4-dioxotetrahydropyrimidin-1 (2H)-yl)phenyl)piperidin-4-yl)methyl)piperazin-1-yl)pyridin-3-yl)amino)pyrimidin-4-yl)-2-methylbenzyl)-1H-pyrazole-4-carboxamide |
| 84 | N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1-isopropyl-1H-pyrazole-4-carboxamide |
| 85 | 1-(tert-butyl)-N-(4-(2-((4-(1-((1-(3-(2,4-dioxotetrahydropyrimidin-1 (2H)-yl)phenyl)piperidin-4-yl)methyl)piperidin-4-yl)phenyl)amino)pyrimidin-4-yl)-2-methylbenzyl)-1H-pyrazole-4-carboxamide |
| 86 | N-(4-(2-((4-(4-((1-(3-(2,4-dioxotetrahydropyrimidin-1(2H)-yl)-4-methoxybenzoyl)piperidin-4-yl)methyl)piperazin-1-yl)phenyl)amino)pyrimidin-4-yl)-2-methylbenzyl)-3-isopropoxyazetidine-1-carboxamide |
| 87 | N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-3-isopropoxyazetidine-1-carboxamide |
| 88 | N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-3-isopropoxyazetidine-1-carboxamide |
| 91 | N-(4-(2-((1-(1-((1-(3-((2,6-dioxopiperidin-3-yl)amino)benzoyl)piperidin-4-yl)methyl)piperidin-4-yl)-1H-pyrazol-4-yl)amino)pyrimidin-4-yl)-2-methylbenzyl)-3-isopropoxyazetidine-1-carboxamide |
| 93 | N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1-isopropyl-1H-pyrazole-4-carboxamide |
| 94 | N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1-isopropyl-1H-pyrazole-4-carboxamide |
| 95 | 1-(tert-butyl)-N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1H-pyrazole-4-carboxamide |
| 96 | 1-(tert-butyl)-N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1H-pyrazole-4-carboxamide |
| 97 | 1-(tert-butyl)-N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1H-1,2,3-triazole-4-carboxamide |
| 98 | 1-(tert-butyl)-N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1H-1,2,3-triazole-4-carboxamide |
| 99 | N-(4-(2-((4-(1-((1-(3-((2,6-dioxopiperidin-3-yl)amino)benzoyl)piperidin-4-yl)methyl)piperidin-4-yl)phenyl)amino)pyrimidin-4-yl)-2-methylbenzyl)-3-isopropoxyazetidine-1-carboxamide |
| 100 | N-(4-(2-((4-(1-((1-(3-((2,6-dioxopiperidin-3-yl)amino)benzoyl)azetidin-3-yl)methyl)piperidin-4-yl)phenyl)amino)pyrimidin-4-yl)-2-methylbenzyl)-3-isopropoxyazetidine-1-carboxamide |
| 101 | N-(4-(2-((4-(1-((1-(3-((2,6-dioxopiperidin-3-yl)amino)benzoyl)piperidin-4-yl)methyl)azetidin-3-yl)phenyl)amino)pyrimidin-4-yl)-2-methylbenzyl)-3-isopropoxyazetidine-1-carboxamide |
| 102 | N-(4-(2-((4-(4-((1-(3-((2,6-dioxopiperidin-3-yl)oxy)benzoyl)piperidin-4-yl)methyl)piperazin-1-yl)phenyl)amino)pyrimidin-4-yl)-2-methylbenzyl)-3-isopropoxyazetidine-1-carboxamide |
| 103 | N-(4-(2-((4-(4-(1-(3-((2,6-dioxopiperidin-3-yl)amino)benzoyl)piperidin-4-yl)piperazin-1-yl)phenyl)amino)pyrimidin-4-yl)-2-methylbenzyl)-3-isopropoxyazetidine-1-carboxamide |
| 105 | N-(4-(2-((4-(4-((1-(2-((3-((2,6-dioxopiperidin-3-yl)amino)phenyl)amino)-2-oxoethyl)piperidin-4-yl)methyl)piperazin-1-yl)phenyl)amino)pyrimidin-4-yl)-2-methylbenzyl)-3-isopropoxyazetidine-1-carboxamide |
| 107 | N-(4-(2-((4-(4-((1-(6-(2,6-dioxopiperidin-3-yl)-5-oxo-6,7-dihydro-5H-pyrrolo[3,4-b]pyridin-2-yl)azetidin-3-yl)methyl)piperazin-1-yl)phenyl)amino)pyrimidin-4-yl)-2-methylbenzyl)-3-isopropoxyazetidine-1-carboxamide |
| 108 | N-(3-(2-((4-(4-((1-(6-(2,6-dioxopiperidin-3-yl)-5-oxo-6,7-dihydro-5H-pyrrolo[3,4-b]pyridin-2-yl)azetidin-3-yl)methyl)piperazin-1-yl)phenyl)amino)pyrimidin-4-yl)-5-fluoro-2-methylphenyl)-3-isopropoxyazetidine-1-carboxamide |
| 109 | N-(4-(2-((4-(4-(1-(2-(2,6-dioxopiperidin-3-yl)-1,3-dioxoisoindolin-5-yl)piperidin-4-yl)piperazin-1-yl)phenyl)amino)pyrimidin-4-yl)-2-methylbenzyl)-3-isopropoxyazetidine-1-carboxamide |
| 110 | N-(4-(2-((4-(1'-(2-(2,6-dioxopiperidin-3-yl)-1,3-dioxoisoindolin-5-yl)-[1,4'-bipiperidin]-4-yl)phenyl)amino)pyrimidin-4-yl)-2-methylbenzyl)-3-isopropoxyazetidine-1-carboxamide |
| 111 | N-(4-(2-((4-(4-((4-(2-(2,6-dioxopiperidin-3-yl)-1,3-dioxoisoindolin-5-yl)piperazin-1-yl)methyl)piperidin-1-yl)phenyl)amino)pyrimidin-4-yl)-2-methylbenzyl)-3-isopropoxyazetidine-1-carboxamide |
| 112 | N-(4-(2-((4-(3-((4-(2-(2,6-dioxopiperidin-3-yl)-1,3-dioxoisoindolin-5-yl)piperazin-1-yl)methyl)azetidin-1-yl)phenyl)amino)pyrimidin-4-yl)-2-methylbenzyl)-3-isopropoxyazetidine-1-carboxamide |
| 113 | N-(4-(2-((4-(3-((4-(3-((2,6-dioxopiperidin-3-yl)amino)benzoyl)piperazin-1-yl)methyl)azetidin-1-yl)phenyl)amino)pyrimidin-4-yl)-2-methylbenzyl)-3-isopropoxyazetidine-1-carboxamide |
| 114 | N-(4-(2-((4-(4-((4-(3-((2,6-dioxopiperidin-3-yl)amino)benzoyl)piperazin-1-yl)methyl)piperidin-1-yl)phenyl)amino)pyrimidin-4-yl)-2-methylbenzyl)-3-isopropoxyazetidine-1-carboxamide |
| 115 | N-(4-(2-((4-(4-((1-(3-((2,6-dioxopiperidin-3-yl)amino)benzyl)piperidin-4-yl)methyl)piperazin-1-yl)phenyl)amino)pyrimidin-4-yl)-2-methylbenzyl)-3-isopropoxyazetidine-1-carboxamide |
| 116 | N-(4-(2-((4-(4-(4-(3-((2,6-dioxopiperidin-3-yl)amino)phenyl)piperidine-1-carbonyl)piperidin-1-yl)phenyl)amino)pyrimidin-4-yl)-2-methylbenzyl)-3-isopropoxyazetidine-1-carboxamide |
| 117 | N-(4-(2-((4-(4-(4-(4-((2,6-dioxopiperidin-3-yl)amino)phenyl)piperidine-1-carbonyl)piperidin-1-yl)phenyl)amino)pyrimidin-4-yl)-2-methylbenzyl)-3-isopropoxyazetidine-1-carboxamide |
| 118 | N-(4-(2-((4-(4-(4-(4-((2,6-dioxopiperidin-3-yl)oxy)phenyl)piperidine-1-carbonyl)piperidin-1-yl)phenyl)amino)pyrimidin-4-yl)-2-methylbenzyl)-3-isopropoxyazetidine-1-carboxamide |
| 119 | N-(4-(2-((4-(4-((4-(4-((2,6-dioxopiperidin-3-yl)oxy)phenyl)piperidin-1-yl)methyl)piperidin-1-yl)phenyl)amino)pyrimidin-4-yl)-2-methylbenzyl)-3-isopropoxyazetidine-1-carboxamide |
| 120 | N-(4-(2-((4-(4-((4-(3-((2,6-dioxopiperidin-3-yl)amino)phenyl)piperazin-1-yl)methyl)piperidin-1-yl)phenyl)amino)pyrimidin-4-yl)-2-methylbenzyl)-3-isopropoxyazetidine-1-carboxamide |
| 121 | N-(4-(2-((4-(4-((4-(4-((2,6-dioxopiperidin-3-yl)amino)phenyl)piperazin-1-yl)methyl)piperidin-1-yl)phenyl)amino)pyrimidin-4-yl)-2-methylbenzyl)-3-isopropoxyazetidine-1-carboxamide |
| 122 | N-(4-(2-((4-(4-((4-(3-((2,6-dioxopiperidin-3-yl)amino)phenyl)piperidin-1-yl)methyl)piperidin-1-yl)phenyl)amino)pyrimidin-4-yl)-2-methylbenzyl)-3-isopropoxyazetidine-1-carboxamide |
| 123 | N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-3-isopropoxyazetidine-1-carboxamide |
| 124 | N-(4-(2-((4-(4-((4-(5-((2,6-dioxopiperidin-3-yl)oxy)pyridin-2-yl)piperidin-1-yl)methyl)piperidin-1-yl)phenyl)amino)pyrimidin-4-yl)-2-methylbenzyl)-3-isopropoxyazetidine-1-carboxamide |
| 125 | N-(4-(2-((4-(4-((4-(5-((2,6-dioxopiperidin-3-yl)oxy)pyridin-2-yl)piperazin-1-yl)methyl)piperidin-1-yl)phenyl)amino)pyrimidin-4-yl)-2-methylbenzyl)-3-isopropoxyazetidine-1-carboxamide |
| 126 | N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-3-isopropoxyazetidine-1-carboxamide |
| 127 | N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-3-isopropoxyazetidine-1-carboxamide |
| 128 | N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1-isopropyl-1H-pyrazole-4-carboxamide |
| 129 | N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1-isopropyl-1H-pyrazole-4-carboxamide |
| 130 | 1-(tert-butyl)-N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1H-pyrazole-4-carboxamide |
| 131 | 1-(tert-butyl)-N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1H-pyrazole-4-carboxamide |
| 132 | 1-(tert-butyl)-N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1H-1,2,3-triazole-4-carboxamide |
| 133 | 1-(tert-butyl)-N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1H-1,2,3-triazole-4-carboxamide |
| 134 | N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1-isopropyl-1H-pyrazole-4-carboxamide |
| 135 | 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1H-pyrazole-4-carboxamide |
| 136 | 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1H-1,2,3-triazole-4-carboxamide |
| 137 | 1-(tert-butyl)-N-((5-(2-((4-(4-((4-(4-((2,6-dioxopiperidin-3-yl)amino)phenyl)piperidin-1-yl)methyl)piperidin-1-yl)phenyl)amino)pyrimidin-4-yl)-3-methylpyridin-2-yl)methyl)-1H-1,2,3-triazole-4-carboxamide |
| 138 | 1-(tert-butyl)-N-(4-(2-((4-(4-((4-(3-(2,4-dioxotetrahydropyrimidin-1(2H)-yl)phenyl)piperidin-1-yl)methyl)piperidin-1-yl)phenyl)amino)pyrimidin-4-yl)-2-methylbenzyl)-1H-1,2,3-triazole-4-carboxamide |
| 139 | 1-(tert-butyl)-N-(4-(2-((4-(4-((4-(4-(2,4-dioxotetrahydropyrimidin-1(2H)-yl)phenyl)piperidin-1-yl)methyl)piperidin-1-yl)phenyl)amino)pyrimidin-4-yl)-2-methylbenzyl)-1H-1,2,3-triazole-4-carboxamide |
| 141 | 1-(tert-butyl)-N-(4-(2-((4-((4-((4-(4-(2,4-dioxotetrahydropyrimidin-1(2H)-yl)phenyl)piperidin-1-yl)methyl)piperidin-1-yl)methyl)phenyl)amino)pyrimidin-4-yl)-2-methylbenzyl)-1H-1,2,3-triazole-4-carboxamide |
| 142 | 1-(tert-butyl)-N-(4-(2-((4-((4-((4-(4-((2,6-dioxopiperidin-3-yl)amino)phenyl)piperidin-1-yl)methyl)piperidin-1-yl)methyl)phenyl)amino)pyrimidin-4-yl)-2-methylbenzyl)-1H-1,2,3-triazole-4-carboxamide |
| 143 | 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-(hydroxymethyl)benzyl)-1H-1,2,3-triazole-4-carboxamide |
| 144 | N-(4-(2-((6-(4-((4-(4-((2,6-dioxopiperidin-3-yl)oxy)phenyl)piperidin-1-yl)methyl)piperidin-1-yl)pyridin-3-yl)amino)pyrimidin-4-yl)-2-methylbenzyl)-3-isopropoxyazetidine-1-carboxamide |
| 145 | N-(4-(2-((6-(4-((4-(4-((2,6-dioxopiperidin-3-yl)amino)phenyl)piperidin-1-yl)methyl)piperidin-1-yl)pyridin-3-yl)amino)pyrimidin-4-yl)-2-methylbenzyl)-3-isopropoxyazetidine-1-carboxamide |
| 146 | 1-(tert-butyl)-N-(4-(2-((6-(4-((4-(4-((2,6-dioxopiperidin-3-yl)amino)phenyl)piperidin-1-yl)methyl)piperidin-1-yl)pyridin-3-yl)amino)pyrimidin-4-yl)-2-methylbenzyl)-1H-1,2,3-triazole-4-carboxamide |

As used herein, the term "pharmaceutically acceptable salt(s)" refers to a salt prepared from active compounds according to the present disclosure with relatively non-toxic acids or bases, depending on the particular substituents of those compounds. When the compounds have a relatively acidic group, base-added salts can be obtained by contacting the neutral compounds with a sufficient amount of the desired base and a pure or inert solvent. Suitable pharmaceutically acceptable base addition salts include, but are not limited to sodium, potassium, calcium, ammonium, organic amino, magnesium salts and the like. When the compounds have a relatively basic group, acid-added salts can be obtained by contacting the neutral compounds with a sufficient amount of the desired acid and pure or inert solvent. Suitable pharmaceutically acceptable acid addition salts include salts derived from non-toxic organic acids including, but are not limited to, acetic acid, propionic acid, isobutyl acid, oxalic acid, maleic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, and the like, and non-toxic inorganic acids including, but are not limited to, hydrochloric acid, hydrobromic acid, nitric acid, carbonic acid, monohydrogencarbonic acid, phosphoric acid, monohydrogenphosphoric acid, dihydrogenphosphoric acid, sulfuric acid, monohydrogensulfuric acid, hydrogen iodide, phosphorous acid and the like. Also it includes a salt of amino acid such as arginate or its analogues, and it also includes analogues of organic acid such as glucuronic or galacturonic acid. Some specific compounds of this disclosure have both basic and acidic functionality for the conversion of compounds with a basic or acidic portion (addition) salts. Other examples of salts are well known through literature known in the art to which the present invention pertains.

As used herein, the phrase "compound(s) of this/the invention" includes any compound(s) of Chemical Formula 1, as well as isotopic variants, clathrates, hydrates, solvates, or polymorphs thereof. And, even if the term "compound(s) of the invention" does not mention its pharmaceutically acceptable sat, the term includes salts thereof. In one embodiment, the compounds of this disclosure include stereo-chemically pure compounds, e.g., those substantially free (e.g., greater than 85% ee, greater than 90% ee, greater than 95% ee, greater than 97% ee, or greater than 99% ee) of other stereoisomers. That is, if the compounds of Chemical Formula 1 according to the present disclosure or salts thereof are tautomeric isomers and/or stereoisomers (e.g., geometrical isomers and conformational isomers), such isolated isomers and their mixtures also are included in the scope of this disclosure. If the compounds of the present disclosure or salts thereof have an asymmetric carbon in their structures, their active optical isomers and their racemic mixtures also are included in the scope of this disclosure.

As used herein, the term "isotopic variant" refers to a compound that contains unusual ratios of isotopes at one or more atoms that make up the compound. For example, isotopic variants of a compound may be radiolabeled. For example, the hydrogen atom may be selected from hydrogen, deuterium and tritium, and isotopic variants may contain carbon-13 (¹³C), nitrogen-15 (¹⁵N), etc.

As used herein, the term "polymorph" refers to solid crystalline forms of a compound of this disclosure or complex thereof. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

As used herein, the term "solvate" means a compound or its salt according to this disclosure that further includes a stoichiometric or non-stoichiometric amount of a solvent bound by non-covalent intermolecular forces. Preferred solvents are volatile, non-toxic, and acceptable for administration to humans in trace amounts.

As used herein, the term "hydrate" means a compound or its salt according to this disclosure that further includes a stoichiometric or non-stoichiometric amount of water bound by non-covalent intermolecular forces.

As used herein, the term "clathrate" means a compound or its salt in the form of a crystal lattice that contains spaces (e.g., channels) that have a guest molecule (e.g., a solvent or water) trapped within.

As used herein, the term "purified" means that when isolated, the isolate is greater than 90% pure, in one embodiment greater than 95% pure, in another embodiment greater than 99% pure and in another embodiment greater than 99.9% pure.

### Medical uses and methods of treatment of the compounds according to the present invention

The present invention further provides methods for treating a disease or condition in a subject having or susceptible to having such a disease or condition, by administering to the subject a therapeutically-effective amount of one or more compounds as described above. In one embodiment, the treatment is preventative treatment. In another embodiment, the treatment is palliative treatment. In another embodiment, the treatment is restorative treatment.

### 1. Diseases or conditions

The compounds of the present invention for degrading BTK are useful for various therapeutic or prophylactic uses (e.g., autoimmune diseases or cancers). These compounds can be used to degrade BTK to lower BTK activity, and can also be used to treat BTK-related diseases or to prevent exacerbation of these diseases. Accordingly, the present invention provides a method for degrading BTK in a cell. In this method the cells are contacted with an effective amount of a compound of the invention. In one embodiment, the cell is present in a subject. The method of the present invention comprises administering to a subject in need of treatment or prevention a pharmaceutical composition comprising a therapeutically or prophylactically effective amount of a compound according to the present invention.

In one embodiment, the present invention provides a method of degrading BTK in a cell of a BTK-associated disease. For example, the present invention can be used to degrade BTK in cells of a subject having a BTK-related disease, which will be described later, and consequently lower BTK activity. In another embodiment of the present invention, the present invention can be used to degrade BTK in cells of autoimmune diseases, cancers and so on.

In another embodiment, the present invention provides a method of treating a BTK-related disease, comprising administering to a subject a therapeutically effective amount of a compound of Chemical Formula 1 or a pharmaceutically acceptable salt thereof. Such a method comprises administering to a subject in need of treatment an amount of a compound of the invention sufficient to degrade BTK, i.e., a therapeutically effective amount. In such a method, a compound of the present invention may be administered to the subject in the form of a pharmaceutical composition described herein.

In one embodiment of the present invention, the BTK-related disease is cancer. The cancer refers to solid cancer or blood cancer. The solid cancer includes, but is not particularly limited to, brain tumor, benign astrocytoma, malignant astrocytoma, pituitary adenoma, meningioma, brain lymphoma, oligodendroglioma, intracranial tumor, ependymoma, brainstem tumor, head and neck tumor, laryngeal cancer, oropharyngeal cancer, nasal cavity cancer, nasopharyngeal cancer, salivary gland cancer, hypopharyngeal cancer, thyroid cancer, oral cancer, thoracic tumor, small cell lung cancer, non-small cell lung cancer, thymic cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colon cancer, anal cancer, bladder cancer, kidney cancer, male genital cancer, penile cancer, prostate cancer, female genital cancer, cervical cancer, endometrial cancer, ovarian cancer, uterine sarcoma, vaginal cancer, female external genital cancer, female urethra cancer, or skin cancer. Additionally, the blood cancer may include, but is not particularly limited to, leukemia, malignant lymphoma, multiple myeloma, or aplastic anemia.

In another embodiment of the present invention, the BTK-related disease is an autoimmune disease. The autoimmune disease includes, but is not particularly limited to, rheumatoid arthritis, psoriatic arthritis, osteoarthritis, Still's disease, juvenile arthritis, lupus, diabetes, myasthenia gravis, Hashimoto's thyroiditis, Ord's thyroiditis, Graves' disease, Sjögren's syndrome, multiple sclerosis, Guillain-Barré syndrome, acute disseminated encephalomyelitis, Addison's disease, ocular clonus-myoclonus syndrome, ankylosing myelitis, antiphospholipid antibody syndrome, aplastic anemia, autoimmune hepatitis, chronic digestive disorder, goodpasture syndrome, idiopathic thrombocytopenia purpura, optic neuritis, scleroderma, primary biliary cirrhosis, Takayasu's arteritis, temporal arteritis, autoimmune hemolytic anemia, Wegener's granulomatosis, psoriasis, alopecia universalis, Behcet's disease, chronic fatigue, dysautonomia, endometriosis, interstitial cystitis, neuromuscular dystonia, and vulvar pain. It may be selected from the group consisting of pain, but is not particularly limited thereto.

In the preferable one embodiment of the present invention, the BTK-related disease is leukemia (e.g., lymphocytic leukemia, B-cell leukemia, prolymphocytic leukemia, myeloid leukemia, hairy cell leukemia, acute myeloid leukemia (AML), chronic myeloid leukemia (CML), chronic lymphocytic leukemia (CLL), acute lymphoblastic leukemia (ALL), T-cell leukemia, T-cell acute lymphoblastic leukemia (T-ALL), myeloproliferative diseases), lymphoma (e.g., B-cell lymphoma, central nervous system lymphoma, follicular lymphoma, non-Hodgkin lymphoma, Hodgkin lymphoma, lymphocytic lymphoma, mantle cell lymphoma, Burkitt lymphoma, B-Cell diffuse lymphoma, marginal zone lymphoma, small-cell lymphoma, lymphoplasmacytoid lymphoma, Richter's transformation (Richter's syndrome), small lymphocytic lymphoma (SLL), diffuse large B-cell lymphoma (DLBCL), immunoblastic large cell lymphoma, precursor B-lymphoblastic lymphoma (B-LBL), lymphoblasmacytic lymphoma, primary mediastinal large B-cell lymphoma (PMBCL), intravascular large B-cell lymphoma (IVLBCL), primary effusion lymphoma, T-cell lymphoma, lymphomatoid granulomatosis (LYG), post-transplant lymphoproliferative disorder (PTLD)), solid cancer (e.g., ependymoma, medulloblastoma, glioblastoma, primitive neuroectodermal tumor, non-small cell lung cancer, breast cancer, pancreatic cancer, gastroesophageal cancer, metastatic pancreatic adenocarcinoma, central nervous system cancer, brain cancer, lung cancer, head and neck cancer, esophageal and esophagogastric junction cancer, gastric cancer, colorectal cancer, rectal cancer, anal cancer, hepatobiliary cancer, non-melanoma skin cancer, melanoma, renal cancer, prostate cancer, bladder cancer, uterine cancer, cervical cancer, ovarian cancer, bone cancer, neuroendocrine cancer, mesothelioma cancer, testicular cancer, thymoma and thymic carcinoma, thyroid cancer), autoimmune disease (e.g., rheumatoid arthritis, autoimmune hemolytic anemia, warm antibody autoimmune hemolytic anemia, chronic spontaneous urticaria (CSU), food allergy, immune thrombocytopenia, Sjögren syndrome, systemic lupus erythematosus, allergic disease, anaphylaxis, systemic onset juvenile idiopathic arthritis (SOJIA), pemphigus vulgaris, immune thrombocytopenic purpura, myasthenia gravis, anti-neutrophil cytoplasmic antibodies (ANCA)-associated vasculitides, cryoglobulinemia, chronic autoimmune urticarial, atopic dermatitis, contact dermatitis, allergic rhinitis, ulcerative colitis, morbus crohn, goodpasture's syndrome, anti-GBM/anti-TBM nephritis, Hashimoto's thyroiditis, Grave's disease, achalasia, Addison's disease, adult Sill's disease, alopecia areata, amyloidosis, ankylosing spondylitis, antiphospholipid syndrome, autoimmune angioedema, autoimmune dysautonomia, autoimmune encephalomyelitis, autoimmune hepatitis, autoimmune inner ear disease (AIED), autoimmune myocarditis, autoimmune oophoritis, autoimmune orchitis, autoimmune pancreatitis, autoimmune retinopathy, axonal and neuronal neuropathy (AMAN), Balo disease, Behcet's disease, benign mucosal pemphigoid, bullous pemphigoid, Castleman disease (CD), celiac disease, chronic inflammatory demyelinating polyneuropathy (CIDP), chronic recurrent multifocal osteomyelitis (CRMO), Churg-Strauss Syndrome (CSS), eosinophilic granulomatosis (EGPA), cicatricial pemphigoid, Cogan's syndrome, cold agglutinin disease, congenital heart block, coxsackie myocarditis, CREST syndrome, Crohn's disease, dermatitis herpetiformis, dermatomyositis, Devic's disease, neuromyelitis optica, discoid lupus, Dressier's syndrome, endometriosis, eosinophilic esophagitis (EoE), eosinophilic fasciitis, erythema nodosum, essential mixed cryoglobulinemia, evans syndrome, fibromyalgia, fibrosing alveolitis, giant cell arteritis, temporal arteritis, giant cell myocarditis, glomerulonephritis, granulomatosis with poly angiitis, Guillain-Barre syndrome, Henoch-Schonlein purpura (HSP), herpes gestationis, pemphigoid gestationis (PG), acne inversa, hypogammalglobulinemia, IgA nephropathy, IgG4-related sclerosing disease, inclusion body myositis (IBM), interstitial cystitis (IC), juvenile arthritis, juvenile diabetes, juvenile myositis (JM), Kawasaki disease, Lambert-Eaton syndrome, leukocytoclastic vasculitis, lichen planus, lichen sclerosus, ligneous conjunctivitis, linear IgA disease (LAD), lupus, Meniere's disease, microscopic poly angiitis (MPA), mixed connective tissue disease (MCTD), Mooren's ulcer, Mucha-Habermann disease, multifocal motor neuropathy (MMN), myositis, narcolepsy, neonatal lupus, neutropenia, ocular cicatricial pemphigoid, optic neuritis, palindromic rheumatism (PR), paraneoplastic cerebellar degeneration (PCD), paroxysmal nocturnal hemoglobinuria (PNH), Parry Romberg syndrome, peripheral uveitis, Parsonnage-Tumer syndrome, pemphigus, peripheral neuropathy, perivenous encephalomyelitis, pernicious anemia (PA), polyarteritis nodosa, polyglandular syndromes, polymyalgia rheumatica, polymyositis, postmyocardial infarction syndrome, postpericardiotomy syndrome, primary biliary cirrhosis, primary sclerosing cholangitis, progesterone dermatitis, psoriasis, psoriatic arthritis, pure red cell aplasia (PRCA), pyoderma gangrenosum, Raynaud's phenomenon, reactive arthritis, reflex sympathetic dystrophy, relapsing polychondritis, restless legs syndrome (RLS), retroperitoneal fibrosis, rheumatic fever, sarcoidosis, Schmidt syndrome, scleritis, scleroderma, sperm and testicular autoimmunity, stiff person syndrome (SPS), subacute bacterial endocarditis (SBE), Susac's syndrome, sympathetic ophthalmia (SO), Takayasu's arteritis, thrombocytopenic purpura (TTP), Tolosa-Hunt syndrome (THS), transverse myelitis, type 1 diabetes, undifferentiated connective tissue disease (UCTD), uveitis, vasculitis, vitiligo, granulomatosis with polyangiitis (GPA), systemic sclerosis), Waldenstrom macroglobulinemia, B-cell malignancy, recurrent mature B-cell neoplasm, multiple myeloma, multiple sclerosis, graft-versus-host-disease, agammaglobulinemia, hepatitis, neuromyelitis optica, myelodysplastic syndrome (MDS), plasmacytoma, asthma, chronic obstructive pulmonary disease (COPD), transplant rejection, gout, atherosclerosis, inflammatory bowel disease, pancreatitis, B-cell-mediated hyperacute disease, thromboembolic disorders, pulmonary embolism, polycythemia vera, essential thrombocythemia, myelofibrosis with myeloid metaplasia, or acute inflammatory disease.

That is, the present invention provides a medical use of the compound of Chemical Formula 1 or a pharmaceutically acceptable salt thereof for treating or preventing the above diseases.

### 2. Subjects

Suitable subjects to be treated according to the present invention include mammalian subjects. Mammals according to the present disclosure include, but are not limited to, human, canine, feline, bovine, caprine, equine, ovine, porcine, rodents, lagomorphs, primates, and the like, and encompass mammals *in utero.*

In one embodiment, the suitable subject to be treated according to the present invention is human.

### 3. Administration and Dosing

The compounds of the present invention are generally administered in a therapeutically effective amount.

As used herein, "effective amount" refers to an amount of a compound of the invention sufficient to slow or minimize the progression of a BTK-related disease or to provide a therapeutic benefit in the treatment or management of a BTK-related disease. "Effective amount" also refers to an amount sufficient to inhibit or reduce BTK activity, either *in vitro* or *in vivo.*

The compounds of the present invention can be administered by any suitable route in the form of a pharmaceutical composition adapted to such a route, and in a dose effective for the treatment intended. An effective dosage is typically in the range of about 0.001 to about 100 mg per kg body weight per day, preferably about 0.01 to about 50 mg/kg/day, in single or divided doses. Depending on age, species and disease or condition being treated, dosage levels below the lower limit of this range may be suitable. In other cases, still larger doses may be used without harmful side effects. Larger doses may also be divided into several smaller doses, for administration throughout the day.

### Pharmaceutical compositions of the compounds of the present invention

In another embodiment, the present invention provides a pharmaceutical composition comprising the compound of Chemical Formula 1 or a pharmaceutically acceptable salt thereof, and a pharmaceutically acceptable carrier or excipient. In one embodiment of the present invention, the pharmaceutical composition is used for the treatment or prevention of BTK-related diseases, preferably autoimmune diseases or cancers, which is described above.

The term "pharmaceutically acceptable" means suitable for use as a pharmaceutical preparation, and generally considered safe for such use. The term also means that it has been officially approved by the governing body of a country for this use, or is listed in the Korean Pharmacopoeia or the United States Pharmacopoeia.

### Pharmaceutical compositions, dosage forms and administration routes

For the treatment of the diseases or conditions referred to above, the compounds described herein or pharmaceutically acceptable salts thereof can be administered as follows:

### Oral administration

The compounds of the present invention may be administered orally, including by swallowing, so that the compound enters the gastrointestinal tract, or absorbed into the blood stream directly from the mouth (e.g., buccal or sublingual administration).

Suitable compositions for oral administration include solid, liquid, gel or powder formulations, and have a dosage form such as tablet, lozenge, capsule, granule or powder.

Compositions for oral administration may optionally be enteric coated and may exhibit delayed or sustained release through the enteric coating. That is, the composition for oral administration according to the present invention may be a formulation having an immediate or modified release pattern.

Liquid formulations can include solutions, syrups and suspensions, which can be used in soft or hard capsules. Such formulations may include a pharmaceutically acceptable carrier, for example, water, ethanol, polyethylene glycol, cellulose, or an oil. The formulation may also include one or more emulsifying agents and/or suspending agents.

In a tablet dosage form the amount of drug, active ingredient, present may be from about 0.05% to about 95% by weight, more typically from about 2% to about 50% by weight of the dosage form. In addition, tablets may contain a disintegrant, comprising from about 0.5% to about 35% by weight, more typically from about 2% to about 25% of the dosage form. Examples of disintegrants include, but are not limited to, lactose, starch, sodium starch glycolate, crospovidone, croscarmellose sodium, maltodextrin, or mixtures thereof.

Suitable lubricants, for use in a tablet, may be present in amounts from about 0.1% to about 5% by weight, and include, but are not limited to, talc, silicon dioxide, stearic acid, calcium, zinc or magnesium stearate, sodium stearyl fumarate and the like.

Suitable binders, for use in a tablet, include, but are not limited to, gelatin, polyethylene glycol, sugars, gums, starch, polyvinyl pyrrolidone, hydroxypropyl cellulose, hydroxypropylmethyl cellulose and the like. Suitable diluents, for use in a tablet, include, but are not limited to, mannitol, xylitol, lactose, dextrose, sucrose, sorbitol, microcrystalline cellulose and starch.

Suitable solubilizers, for use in a tablet, may be present in amounts from about 0.1% to about 3% by weight, and include, but are not limited to, polysorbates, sodium lauryl sulfate, sodium dodecyl sulfate, propylene carbonate, diethyleneglycol monoethyl ether, dimethyl isosorbide, polyethylene glycol (natural or hydrogenated) castor oil, HCOR^{™} (Nikkol), oleyl ester, Gelucire^{™}, caprylic/caprylic acid mono/diglyceride, sorbitan fatty acid esters, and Solutol HS^{™}.

### Parenteral Administration

Compounds of the present disclosure may be administered directly into the blood stream, muscle, or internal organs. Suitable means for parenteral administration include intravenous, intramuscular, subcutaneous intraarterial, intraperitoneal, intrathecal, intracranial, and the like. Suitable devices for parenteral administration include injectors (including needle and needle-free injectors) and infusion methods.

Compositions for parenteral administration may be formulated as immediate or modified release, including delayed or sustained release.

Most parenteral formulations are liquid compositions, and the liquid composition is an aqueous solution containing the active ingredient according to the present invention, a salt, a buffering agent, an isotonic agent, and the like.

Parenteral formulations may also be prepared in a dehydrated form (e.g., by lyophilization) or as sterile non-aqueous solutions. These formulations can be used with a suitable vehicle, such as sterile water. Solubility-enhancing agents may also be used in preparation of parenteral solutions.

### Topical Administration

Compounds of the present invention may be administered topically to the skin or transdermally. Formulations for this topical administration can include lotions, solutions, creams, gels, hydrogels, ointments, foams, implants, patches and the like. Pharmaceutically acceptable carriers for topical administration formulations can include water, alcohol, mineral oil, glycerin, polyethylene glycol and the like. Topical administration can also be performed by electroporation, iontophoresis, phonophoresis and the like.

Compositions for topical administration may be formulated as immediate or modified release, including delayed or sustained release.

### [Advantageous Effects]

The present disclosure provides compounds capable of exhibiting various pharmacological activities due to very good BTK degradation activity, pharmaceutical compositions comprising the compound as an active ingredient, their medical uses (especially autoimmune diseases or cancers), and methods of treatment or prevention comprising administering the compound to a subject in need of such treatment or prevention. The compound according to the present invention or a pharmaceutically acceptable salt thereof is excellent in various aspects such as BTK degradation activity, (metabolism) stability, physicochemical properties, etc., and in particular, BTK degradation activity is much superior to that of compounds having a similar structure.

### [Mode for Invention]

Hereinafter, the present invention is described in considerable detail with examples to help those skilled in the art understand the present invention. However, the following examples are offered by way of illustration and are not intended to limit the scope of the invention. It is apparent that various changes may be made without departing from the spirit and scope of the invention or sacrificing all of its material advantages.

### Preparation of compounds of the present invention

Hereinafter, the synthesis process of some compounds of the present invention will be described, and the other compounds not mentioned below can be prepared by substituting starting materials, intermediates and/or reactants in a similar manner.

### Intermediate 1-1: N-(4-(2-chloropyrimidin-4-yl)-2-methylbenzyl)-3-isopropoxyazetidine-1-carboxamide

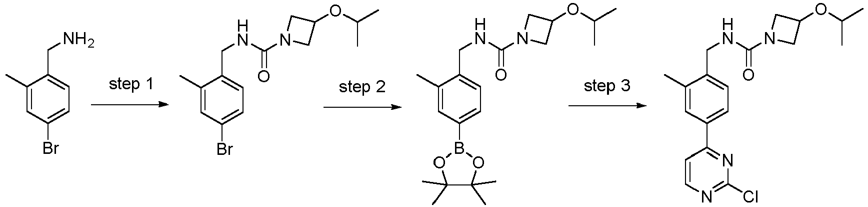

### Step 1: Synthesis of N-(4-bromo-2-methylbenzyl)-3-isopropoxyazetidine-1-carboxamide

Triphosgene (0.78 g, 2.63 mmol) and (4-bromo-2-methylphenyl)methanamine (1.50 g, 7.50 mmol) were suspended in DCM (60 mL) and stirred at 0 °C. After adding triethylamine (2.60 mL, 18.75 mmol) to the reaction solution, 3-isopropoxyazetidine hydrochloride (1.36 g, 9.0 mmol) was diluted in DCM (20 mL) and slowly added thereto. After stirring at room temperature for 1 hour, distilled water (50 mL) was added and extracted with DCM (30 mL x 3). The organic layer was dried over anhydrous sodium sulfate, filtered, and concentrated under reduced pressure. The resulting residue was subjected to MPLC (1-3% MeOH/DCM) to obtain 1.86 g (76%) of a colorless liquid.

### Step 2: Synthesis of 3-isopropoxy-N-(2-methyl-4-(4,4,5,5-tetramethyl-1,3,2-dioxaborolan-2-yl)benzyl)azetidine-1 -carboxamide

N-(4-bromo-2-methylbenzyl)-3-isopropoxyazetidine-1-carboxamide (1.86 g, 5.45 mmol), 4,4,4',4',5,5,5',5'-octamethyl-2 ,2'-bi(1,3,2-dioxaborolane) (1.66 g, 6.54 mmol), Pd(dppf)Cl₂ (0.08 g, 0.11 mmol), and KOAc (1.60 g, 16.35 mmol) were suspended in dioxane (20 mL) and stirred at 100 °C for 16 hours. Distilled water (50 mL) was added to the reaction solution, and then extracted with EtOAc (30 mL x 3). The organic layer was dried over anhydrous sodium sulfate, filtered, and concentrated under reduced pressure. The resulting residue was subjected to MPLC (20-50% EtOAc/Hexane) to obtain 1.43 g (68%) of a colorless liquid.

### Step 3: Synthesis of N-(4-(2-chloropyrimidin-4-yl)-2-methylbenzyl)-3-isopropoxyazetidine-1 -carboxamide

3-isopropoxy-N-(2-methyl-4-(4,4,5,5-tetramethyl-1,3,2-dioxaborolan-2-yl)benzyl)azetidine-1-carboxamide (1.43 g, 3.68 mmol), 2,4-dichloropyrimidine (0.66 g, 4.42 mmol), Pd(dppf)Cl₂ (54 mg, 2 mol%), and K₂CO₃ (1.53 g, 11.04 mmol) were suspended in dioxane (24 mL) and H₂O (6 mL), and stirred at 90 °C for 2 hours. Distilled water (50 mL) was added to the reaction solution, and then extracted with EtOAc (30 mL x 3). The organic layer was dried over anhydrous sodium sulfate, filtered, and concentrated under reduced pressure. The resulting residue was subjected to MPLC (50-90% EtOAc/Hexane) to obtain 1.12 g (81%) of a white solid.

### Intermediates 1-2 and 1-3 were synthesized using the same method as the synthesis of Intermediate 1-1.

| | |
|---|---|
| Intermediate 1-2: N-(3-(2-chloropyrimidin-4-yl)-5-methylbenzyl)-3-isopropoxyazetidine-1-carboxamide | 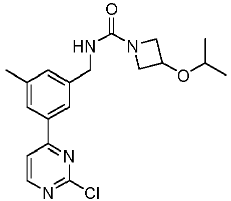 |
| Intermediate 1-3: N-(3-(2-chloropyrimidin-4-yl)-5-methylphenyl)-3-isopropoxyazetidine-1-carboxamide | 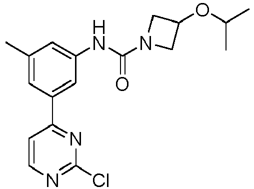 |

### Intermediate 1-4: 1-(tert-butyl)-N-(4-(2-chloropyrimidin-4-yl)-2-methylbenzyl)- 1H-pyrazole-4-carboxamide

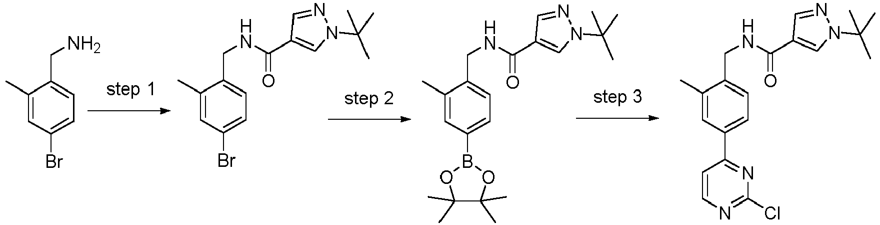

### Step 1: Synthesis of N-(4-bromo-2-methylbenzyl)-1-(tert-butyl)-1H-pyrazole-4-carboxamide

1-(tert-butyl)-1H-pyrazole-4-carboxylic acid (0.46 g, 2.75 mmol) was suspended in DMF (8 mL). Then, HATU (1.14 g, 3.00 mmol), DIPEA (1.3 mL, 7.50 mmol), and (4-bromo-2-methylphenyl)methanamine (0.5 g, 2.50 mmol) were added and stirred at room temperature for 16 hours. NHCl₄ aqueous solution (80 mL) was added to the reaction solution, and then extracted with EtOAc (20 mL x 3). The organic layer was dried over anhydrous sodium sulfate, filtered, and concentrated under reduced pressure. The resulting residue was subjected to MPLC (20-50% EtOAc/Hexane) to obtain 0.72 g (82%) of a white solid.

### Step 2: Synthesis of 1-(tert-butyl)-N-(2-methyl-4-(4,4,5,5-tetramethyl-1,3,2-dioxaborolan-2-yl)benzyl)-1H-pyrazole-4-carboxamide

N-(4-bromo-2-methylbenzyl)-1-(tert-butyl)-1H-pyrazole-4-carboxamide (0.40 g, 1.14 mmol), 4,4,4',4',5,5,5',5'-octamethyl-2,2'-bi(1,3,2-dioxaborolane) (0.29 g, 1.14 mmol), Pd(dppf)Cl₂ (0.08 g, 0.11 mmol), and KOAc (0.34 g, 3.43 mmol) were suspended in dioxane (12 mL) and stirred at 100 °C for 16 hours. Distilled water (50 mL) was added to the reaction solution and extracted with EtOAc (20 mL x 3). The organic layer was dried over anhydrous sodium sulfate, filtered, and concentrated under reduced pressure. The resulting residue was subjected to MPLC (20-40% EtOAc/Hexane) to obtain 0.33 g (73%) of a colorless liquid.

### Step 3: Synthesis of 1-(tert-butyl)-N-(4-(2-chloropyrimidin-4-yl)-2-methylbenzyl)-1H-pyrazole-4-carboxamide

1-(tert-butyl)-N-(2-methyl-4-(4,4,5,5-tetramethyl-1,3,2-dioxaborolan-2-yl)benzyl)-1H-pyrazole-4-carboxamide (0.33 g, 0.83 mmol), 2,4-dichloropyrimidine (0.15 g, 1.00 mmol), Pd(dppf)Cl₂ (12 mg, 2 mol%), and K₂CO₃ (0.34 g, 2.49 mmol) were suspended in dioxane (8 mL) and H₂O (2 mL), and stirred at 90 °C for 2 hours. Distilled water (50 mL) was added to the reaction solution, extracted with EtOAc (20 mL x 3), and the organic layer was dried over anhydrous sodium sulfate, filtered, and concentrated under reduced pressure. The obtained residue was subjected to MPLC (50-80% EtOAc/Hexane) to obtain 0.15 g (48%) of a bright yellow solid.

### Intermediates 1-5 to 1-7 were synthesized using the same method as the synthesis of Intermediate 1-4.

| | |
|---|---|
| Intermediate 1-5: 1-(tert-butyl)-N-(4-(2-chloropyrimidin-4-yl)-2-methylbenzyl)-1H-1,2,3-triazole-4-carboxamide | 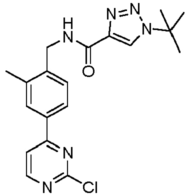 |
| Intermediate 1-6: N-(4-(2-chloropyrimidin-4-yl)-2-methylbenzyl)-1-isopropyl-1H-pyrazole-4-carboxamide | 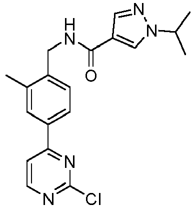 |
| Intermediate 1-7: 1-(tert-butyl)-N-((5-(2-chloropyrimidin-4-yl)-3-methylpyridin-2-yl)methyl)-1H-1,2,3-triazole-4-carboxamide | 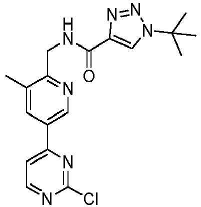 |

### Intermediate 1-8: N-(3-(2-chloropyrimidin-4-yl)-5-fluoro-2-methylphenyl)-3-isopropoxyazetidine-1 -carboxamide

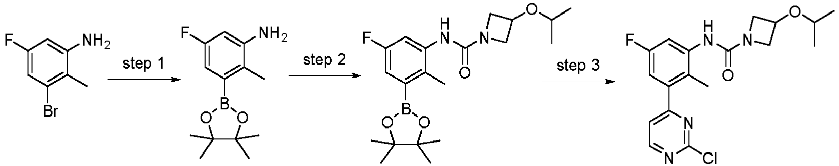

### Step 1: Synthesis of 5-fluoro-2-methyl-3-(4,4,5,5-tetramethyl-1,3,2-dioxaborolan-2-yl)aniline

3-bromo-5-fluoro-2-methylaniline (1.00 g, 4.90 mmol), 4,4,4',4',5,5,5',5'-octamethyl-2,2'-bi(1,3,2-dioxaborolane) (1.49 g, 5.88 mmol), KOAc (1.44 g, 14.70 mmol), and Pd(dppf)Cl₂ (36 mg, 0.05 mmol) were suspended in 1,4-dioxane (15 mL) and stirred at 100 °C for 16 hours. Brine (130 mL) was added to the reaction solution and extracted with EtOAc (40 mL x 3). The organic layer was dried over anhydrous sodium sulfate, filtered, and concentrated under reduced pressure. The resulting residue was subjected to MPLC (25% EtOAc/Hexane) to obtain 840 mg (68%) of a white solid.

### Step 2: Synthesis of N-(5-fluoro-2-methyl-3-(4,4,5,5-tetramethyl-1,3,2-dioxaborolan-2-yl)phenyl)-3-isopropoxyazetidine-1-carboxamide

Triphosgene (351 mg, 1.18 mmol) was suspended in DCM (10 mL). 5-fluoro-2-methyl-3-(4,4,5,5-tetramethyl-1,3,2-dioxaborolan-2-yl)aniline (850 mg, 3.38 mmol) and TEA (0.47 mL, 3.38 mmol) were dissolved in DCM (9.5 mL) and added slowly at 0 °C. 3-isopropoxyazetidine hydrochloride (513.26 mg, 3.38 mmol) and TEA (0.47 mL, 3.38 mmol) were dissolved in DCM (19.5 mL) and slowly added to the reaction solution at 0 °C, followed by stirring at room temperature for 3 hours. Distilled water (30 mL) was added to the reaction solution and extracted with DCM (30 mL x 3). The organic layer was dried over anhydrous sodium sulfate, filtered, and concentrated under reduced pressure. The resulting residue was subjected to MPLC (20% EtOAc/Hexane) to obtain 560 mg (42%) of a white solid.

### Step 3: Synthesis of N-(3-(2-chloropyrimidin-4-yl)-5-fluoro-2-methylphenyl)-3-isopropoxyazetidine-1 -carboxamide

N-(5-fluoro-2-methyl-3-(4,4,5, 5-tetramethyl-1,3,2-dioxaborolan-2-yl)phenyl)-3-isopropoxyazetidine-1-carboxamide (560 mg, 1.42 mmol), 2,4-dichloropyrimidine (255 mg, 1.71 mmol), Pd(dppf)Cl₂ (102 mg, 0.14 mmol), and K₂CO₃ (592 mg, 4.28 mmol) were suspended in 1,4-dioxane:H₂O=4:1 (4.4 mL) and stirred at 90 °C for 3 hours. The reaction solution was filtered and concentrated under reduced pressure, and the resulting residue was subjected to MPLC (50-100% EtOAc/Hexane) to obtain 170 mg (31%) of a white solid.

### Intermediate 1-9: 1-(tert-butyl)-N-(3-(2-chloropyrimidin-4-yl)-5-fluoro-2-methylphenyl)-1H-pyrazole-4-carboxamide

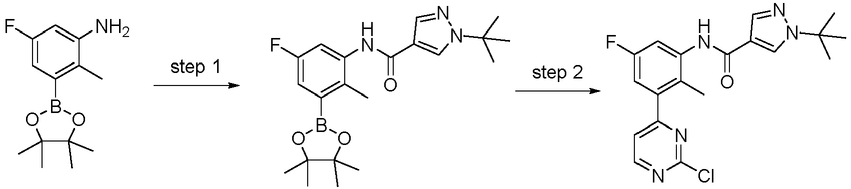

### Step 1: Synthesis of 1-(tert-butyl)-N-(5-fluoro-2-methyl-3-(4,4,5,5-tetramethyl-1,3,2-dioxaborolan-2-yl)phenyl)-1H-pyrazole-4-carboxamide

5-fluoro-2-methyl-3-(4,4,5,5-tetramethyl-1,3,2-dioxaborolan-2-yl)aniline (685.5 mg, 2.73 mmol) and TEA (0.54 mL, 4.0 mmol) were suspended in DCM (8.6 mL). Then, 1-(tert-butyl)-1H-pyrazole-4-carbonyl chloride (442 mg, 2.60 mmol) was slowly added at 0 °C and stirred at room temperature for 4 hours. Distilled water (20 mL) was added to the reaction solution and extracted with DCM (30 mL x 2). The organic layer was dried over anhydrous sodium sulfate, filtered, and concentrated under reduced pressure. The resulting residue was subjected to MPLC (0-25% EtOAc/Hexane) to obtain 720 mg (66%) of a white solid.

### Step 2: Synthesis of 1-(tert-butyl)-N-(3-(2-chloropyrimidin-4-yl)-5-fluoro-2-methylphenyl)-1H-pyrazole-4-carboxamide

1-(tert-butyl)-N-(5-fluoro-2-methyl-3-(4,4,5,5-tetramethyl-1,3,2-dioxaborolan-2-yl)phenyl)-1H-pyrazole-4-carboxamide (700 mg, 1.74 mmol), 2,4-dichloropyrimidine (300 mg, 2.01 mmol), Pd(dppf)Cl₂ (124.4 mg, 0.17 mmol), and K₂CO₃ (723 mg, 5.23 mmol) were suspended in 1,4-dioxane:H₂O=4:1 (5.4 mL) and stirred at 90 °C for 16 hours. Distilled water (40 mL) was added to the reaction solution, extracted with EtOAc (40 mL x 2), and the organic layer was dried over anhydrous sodium sulfate, filtered, and concentrated under reduced pressure. The obtained residue was subjected to MPLC (50-70% EtOAc/Hexane) to obtain 560 mg (83%) of a white solid.

### Intermediate 1-10: 1-(tert-butyl)-N-(3-(2-chloropyrimidin-4-yl)-5-fluoro-2-methylphenyl)-1H-1,2,3-triazole-4-carboxamide

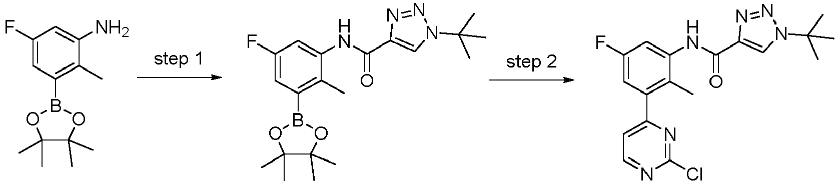

### Step 1: Synthesis of 1-(tert-butyl)-N-(5-fluoro-2-methyl-3-(4,4,5,5-tetramethyl-1,3,2-dioxaborolan-2-yl)phenyl)-1H-1,2,3-triazole-4-carboxamide

5-fluoro-2-methyl-3-(4,4,5,5-tetramethyl-1,3,2-dioxaborolan-2-yl)aniline (480 mg, 1.90 mmol), 1-(tert-butyl)-1H-1,2,3-triazole-4-carboxylic acid (320 mg, 1.90 mmol), HATU (1.08 g, 2.86 mmol), and DIPEA (1.0 mL, 5.73 mmol) were suspended in DMF (9.5 mL) and stirred at room temperature for 16 hours. Distilled water (20 mL) was added to the reaction solution, and then extracted with EtOAc (20 mL x 2). The organic layer was dried over anhydrous sodium sulfate, filtered, and concentrated under reduced pressure. The resulting residue was subjected to MPLC (50% EtOAc/Hexane) to obtain 450 mg (59%) of a white solid.

### Step 2: Synthesis of 1-(tert-butyl)-N-(3-(2-chloropyrimidin-4-yl)-5-fluoro-2-methylphenyl)-1H-1,2,3-triazole-4-carboxamide

1-(tert-butyl)-N-(5-fluoro-2-methyl-3-(4,4,5,5-tetramethyl-1,3,2-dioxaborolan-2-yl)phenyl)-1H-1,2,3-triazole-4-carboxamide (450 mg, 1.12 mmol), 2,4-dichloropyrimidine (200 mg, 1.34 mmol), Pd(dppf)Cl₂ (80.5 mg, 0.11 mmol), and K₂CO₃ (464.4 mg, 3.36 mmol) were suspended in 1,4-dioxane:H₂O=4:1 (3.5 mL) and stirred at 90 °C for 2 hours. The reaction solution was filtered and concentrated under reduced pressure, and the resulting residue was subjected to MPLC (25-50% EtOAc/Hexane) to obtain 205 mg (47%) of a yellow solid.

### Intermediate 1-11: 4-(2-chloropyrimidin-4-yl)-2-methylbenzyl 3-isopropoxyazetidine-1-carboxylate

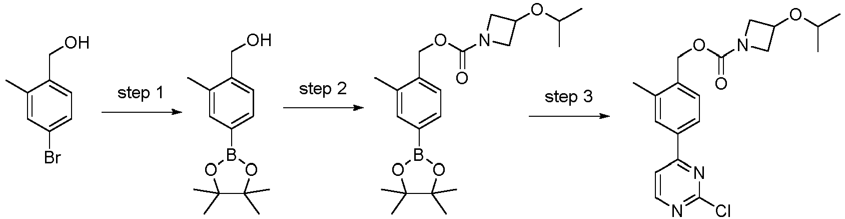

### Step 1: Synthesis of (2-methyl-4-(4,4,5,5-tetramethyl-1,3,2-dioxaborolan-2-yl)phenyl)methanol

(4-bromo-2-methylphenyl)methanol (400 mg, 2.00 mmol), 4,4,4',4',5,5,5',5'-octamethyl-2,2'-bi(1,3,2-dioxaborolane) (610 mg, 2.40 mmol), KOAc (586 mg, 6.00 mmol), and Pd(dppf)Cl₂ (88 mg, 0.12 mmol) were suspended in 1,4-dioxane (6 mL) and then heated and refluxed for 4 hours. Distilled water (30 mL) was added to the reaction solution, and then extracted with EtOAc (30 mL x 3). The organic layer was dried over anhydrous sodium sulfate, filtered, and concentrated under reduced pressure. The resulting residue was subjected to MPLC (10-15% EtOAc/Hexane) to obtain 365 mg (74%) of a colorless oil.

### Step 2: Synthesis of 2-methyl-4-(4,4,5,5-tetramethyl-1,3,2-dioxaborolan-2-yl)benzyl 3-isopropoxyazetidine-1 -carboxylate

(2-methyl-4-(4,4,5,5-tetramethyl-1,3,2-dioxaborolan-2-yl)phenyl)methanol (365 mg, 1.47 mmol) and CDI (215 mg, 1.32 mmol) were suspended in THF (2 mL) and stirred at room temperature for 16 hours. 3-isopropoxyazetidine hydrochloride (178 mg, 1.17 mmol) and TEA (0.20 mL, 1.47 mmol) were dissolved in THF (1 mL) and added to the reaction solution, followed by stirring at room temperature for 4 hours. Distilled water (30 mL) was added to the reaction solution and extracted with DCM (40 mL x 2). The organic layer was dried over anhydrous sodium sulfate, filtered, and concentrated under reduced pressure. The resulting residue was subjected to MPLC (35% EtOAc/Hexane) to obtain 330 mg (58%) of a colorless oil.

### Step 3: Synthesis of 4-(2-chloropyrimidin-4-yl)-2-methylbenzyl 3-isopropoxyazetidine-1-carboxylate

2-methyl-4-(4,4,5,5-tetramethyl-1,35-tetramethyl-1,3,2-dioxaborolan-2-yl)benzyl 3-isopropoxyazetidine-1-carboxylate (320 mg, 0.82 mmol), 2,4-dichloropyrimidine (146 mg, 0.98 mmol), Pd(dppf)Cl₂ (59 mg, 0.08 mmol), and K₂CO₃ (340 mg, 2.46 mmol) were suspended in 1,4-dioxane:H₂O=4:1 (2.56 mL) and stirred at 90 °C for 1.5 hours. Distilled water (30 mL) was added to the reaction solution and extracted with EtOAc (30 mL x 2). The organic layer was dried over anhydrous sodium sulfate, filtered, and concentrated under reduced pressure. The resulting residue was subjected to MPLC (50-100% EtOAc/Hexane) to obtain 250 mg (81%) of a colorless oil.

Intermediate 1-12: 1-(tert-butyl)-N-(4-(2-chloropyrimidin-4-yl)-2-(hydroxymethyl)benzyl)-1H-1,2,3-triazole-4-carboxamide

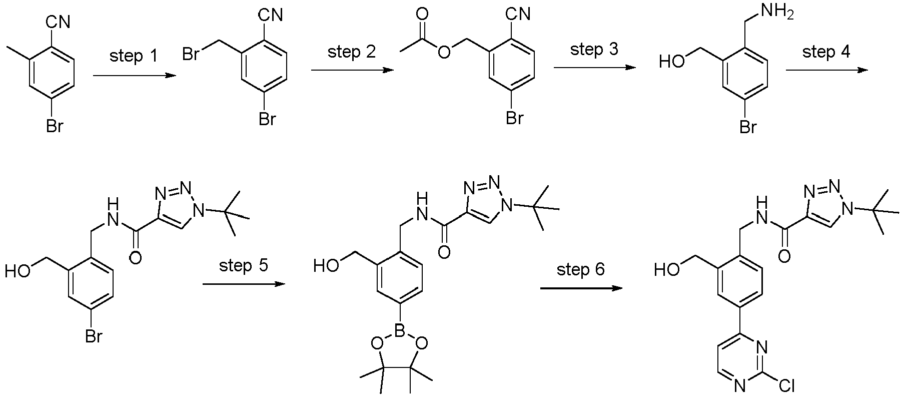

### Step 1: Synthesis of 4-bromo-2-(bromomethyl)benzonitrile

4-bromo-2-methylbenzonitrile (3.0 g, 15.30 mmol), N-bromosuccinimide (2.90 g, 16.83 mmol), and ACHN (374 mg, 1.53 mmol) were suspended in ACN (40 mL) and then heated and refluxed for 16 hours. Distilled water (30 mL) was added to the reaction solution, and then extracted with EtOAc (30 mL x 3). The organic layer was dried over anhydrous sodium sulfate, filtered, and concentrated under reduced pressure. The resulting residue was subjected to MPLC (0-3% EtOAc/Hexane) to obtain 3.0 g of the mixture.

### Step 2: Synthesis of 5-bromo-2-cyanobenzyl acetate

4-bromo-2-(bromomethyl)benzonitrile (3.0 g, 10.91 mmol) and KOAc (1.31 g, 13.33 mmol) were suspended in DMF (55 mL) and stirred at 80 °C for 2 hours. Brine (100 mL) was added to the reaction solution and extracted with EtOAc (30 mL x 4). The organic layer was dried over anhydrous sodium sulfate, filtered, and concentrated under reduced pressure. The resulting residue was subjected to MPLC (0-15% EtOAc/Hexane) to obtain 1.8 g (65%, 2 steps yield) of a white solid.

### Step 3: Synthesis of (2-(aminomethyl)-5-bromophenyl)methanol

5-bromo-2-cyanobenzyl acetate (1.20 g, 4.70 mmol) was suspended in THF (23 mL). Then, 2M LAH in THF (8 mL, 16.0 mmol) was slowly added thereto at 0 °C and stirred for 6 hours. Potassium sodium L-(+)-tartrate tetrahydrate solution (40 mL) was slowly added to the reaction solution, and then extracted with EtOAc (30 mL x 4). The organic layer was dried over anhydrous sodium sulfate, filtered, and concentrated under reduced pressure to obtain 700 mg of the mixture.

### Step 4: Synthesis of N-(4-bromo-2-(hydroxymethyl)benzyl)-1-(tert-butyl)-1H-1,2,3-triazole-4-carboxamide

(2-(aminomethyl)-5-bromophenyl)methanol (800 mg, 3.70 mmol), 1-(tert-butyl)-1H-1,2,3-triazole-4-carboxylic acid (751 mg, 4.44 mmol), HOBT (750 mg, 5.55 mmol), TBTU (1.70 g, 5.55 mmol), and DIPEA (1.9 mL, 11.10 mmol) were suspended in DCM (20.0 mL) and stirred at room temperature for 4 hours. Distilled water (30 mL) was added to the reaction solution, and then extracted with EtOAc (30 mL x 3). The organic layer was dried over anhydrous sodium sulfate, filtered, and concentrated under reduced pressure. The resulting residue was subjected to MPLC (0-40% EtOAc/Hexane) to obtain 650 mg of the mixture.

### Step 5: Synthesis of 1-(tert-butyl)-N-(2-(hydroxymethyl)-4-(4,4,5,5-tetramethyl-1,3,2-dioxaborolan-2-yl)benzyl)-1H-1,2,3-triazole-4-carboxamide

N-(4-bromo-2-(hydroxymethyl)benzyl)-1-(tert-butyl)-1H-1,2,3-triazole-4-carboxamide (300 mg, 0.81 mmol), 4,4,4',4',5,5,5',5'-octamethyl-2,2'-bi(1,3,2-dioxaborolane) (249 mg, 0.98 mmol), Pd(dppf)Cl₂ (40 mg, 5.55 mmol), and KOAc (238 mg, 2.43 mmol) were suspended in 1,4-dioxane (4 mL) and stirred at 100 °C for 16 hours. The reaction solution was filtered through Celite and concentrated under reduced pressure. The resulting residue was subjected to MPLC (0-100% EtOAc/Hexane) to obtain 340 mg of the mixture.

### Step 6: Synthesis of 1-(tert-butyl)-N-(4-(2-chloropyrimidin-4-yl)-2-(hydroxymethyl)benzyl)-1H-1,2,3-triazole-4-carboxamide

1-(tert-butyl)-N-(2-(hydroxymethyl)-4-(4,4,5,5-tetramethyl-1,3,2-dioxaborolan-2-yl)benzyl)-1H-1,2,3-triazole-4-carboxamide (280 mg, 0.67 mmol), 2,4-dichloropyrimidine (110 mg, 0.74 mmol), Pd(dppf)Cl₂ (24 mg, 0.03 mmol), and K₂CO₃ (185 mg, 1.34 mmol) were suspended in 1,4-dioxane : H₂O=5 : 1 (3.35 mL) and stirred at 90 °C for 1 hour. The reaction solution was filtered through Celite and concentrated under reduced pressure. The resulting residue was subjected to MPLC (0-60% EtOAc/Hexane) to obtain 100 mg (6.0%, 4 steps yield) of a bright yellow solid.

### Intermediate 2-1: tert-butyl 4-(4-aminophenyl)piperazine-1-carboxylate

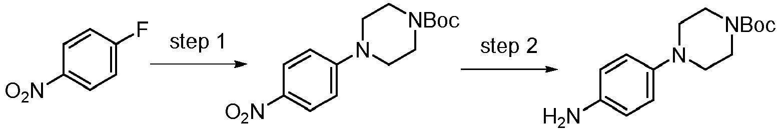

### Step 1: Synthesis of tert-butyl 4-(4-nitrophenyl)piperazine-1-carboxylate

1-fluoro-4-nitrobenzene (3.00 g, 21.26 mmol) was suspended in ACN (100 mL). Then, 1-Boc-piperazine (4.36 g, 23.39 mmol) and DIPEA (11.10 mL, 63.78 mmol) were added and stirred at 85 °C for 16 hours. The reaction solution was concentrated under reduced pressure, distilled water (100 mL) was added, and extracted with EtOAc (30 mL x 3). The organic layer was dried over anhydrous sodium sulfate, filtered, and concentrated under reduced pressure. The resulting residue was subjected to MPLC (2% MeOH/DCM) to obtain 4.53 g (77%) of a yellow solid.

### Step 2: Synthesis of tert-butyl 4-(4-aminophenyl)piperazine-1-carboxylate

Tert-butyl 4-(4-nitrophenyl)piperazine-1-carboxylate (2.52 g, 8.20 mmol) was suspended in MeOH (40 mL). Then, 10% Pd/C (0.44 g, 0.41 mmol) was added and stirred for 2 hours under a hydrogen stream. The reaction solution was filtered and concentrated under reduced pressure, and the resulting residue was subjected to MPLC (1-3% MeOH/DCM) to obtain 2.22 g (98%) of a gray solid.

### Intermediates 2-2 to 2-4 were synthesized using the same method as the synthesis of Intermediate 2-1.

| | |
|---|---|
| Intermediate 2-2: tert-butyl 4-(5-aminopyridin-2-yl)piperazine-1-carboxylate | 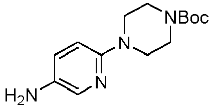 |
| Intermediate 2-3: (1-(4-aminophenyl)piperidin-4-yl)methanol | 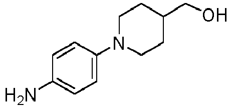 |
| Intermediate 2-4: (1-(5-aminopyridin-2-yl)piperidin-4-yl)methanol | 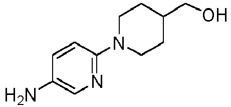 |

### Intermediate 2-5: tert-butyl 4-(4-aminophenyl)piperidine-1-carboxylate

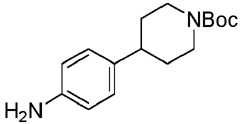

Tert-butyl 4-(4-nitrophenyl)piperidine-1-carboxylate (0.15 g, 0.49 mmol) was suspended in MeOH. Then, 10% Pd/C (26 mg) was added and stirred for 2 hours at room temperature under a hydrogen stream. The reaction solution was filtered and concentrated under reduced pressure to obtain 0.13 g (97%) of a gray solid.

### Intermediate 2-6: tert-butyl 4-(5-aminopyridin-2-yl)piperidine-1-carboxylate

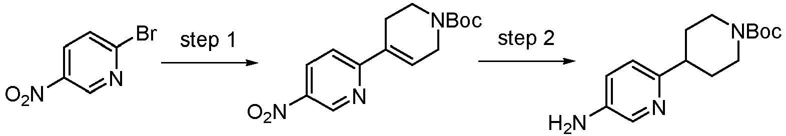

### Step 1: Synthesis of tert-butyl 5-nitro-3',6'-dihydro-[2,4'-bipyridine]-1'(2'H)-carboxylate

2-bromo-5-nitropyridine (500 mg, 2.46 mmol), tert-butyl 4-(4,4,5,5-tetramethyl-1,3,2-dioxaborolan-2-yl)-3,6-dihydropyridine-1(2H)-carboxylate (761 mg, 2.46 mmol), PdCl₂(PPh₃)₂ (86 mg, 0.12 mmol), and 3M Na₂CO₃ (2.4 mL, 7.4 mmol) were suspended in 1,4-dioxane (16 mL) and stirred at 100 °C for 16 hours. Distilled water (40 mL) was added to the reaction solution, and then extracted with EtOAc (40 mL). The organic layer was dried over anhydrous sodium sulfate, filtered, and concentrated under reduced pressure. The resulting residue was subjected to MPLC (15% EtOAc/Hexane) to obtain 295 mg (40%) of a yellow solid.

### Step 2: Synthesis of tert-butyl 4-(5-aminopyridin-2-yl)piperidine-1-carboxylate

Tert-butyl 5-nitro-3',6'-dihydro-[2,4'-bipyridine]-1'(2'H)-carboxylate (290 mg, 0.95 mmol) was dissolved in MeOH (160 mL) and DCM (1 mL). Then, 10% Pd/C (140 mg) was added and stirred for 15 hours at room temperature under a hydrogen stream. The reaction solution was filtered and concentrated under reduced pressure to obtain 262 mg (99%) of a white solid.

### Intermediate 2-7: tert-butyl 4-(4-amino-1H-pyrazol-1-yl)piperidine-1-carboxylate

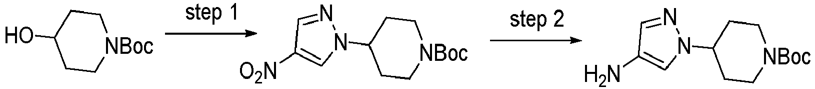

### Step 1: Synthesis of tert-butyl 4-(4-nitro-1H-pyrazol-1-yl)piperidine-1-carboxylate

4-nitro-1H-pyrazole (500 mg, 4.40 mmol), tert-butyl 4-hydroxypiperidine-1-carboxylate (590 mg, 3.00 mmol), diethyl azodicarboxylate (0.76 mL, 4.4 mmol), and triphenylphosphine (1.15 g, 4.4 mmol) were suspended in THF (8.8 mL) at 0 °C and stirred for 10 minutes. Then, it was stirred at room temperature for 3 days. Distilled water (30 mL) was added to the reaction solution, and then extracted with EtOAc (30 mL). The organic layer was dried over anhydrous sodium sulfate, filtered, and concentrated under reduced pressure. The resulting residue was subjected to MPLC (10% EtOAc/Hexane) to obtain 474 mg (54%) of a colorless oil.

### Step 2: Synthesis of tert-butyl 4-(4-amino-1H-pyrazol-1-yl)piperidine-1-carboxylate

Tert-butyl 4-(4-nitro-1H-pyrazol-1-yl)piperidine-1-carboxylate (470 mg, 1.59 mmol) was dissolved in MeOH (16mL). Then, 10% Pd/C (250 mg) was added and stirred for 16 hours at room temperature under a hydrogen stream. The reaction solution was filtered and concentrated under reduced pressure to obtain 418 mg (99%) of purple oil.

### Intermediate 2-8: tert-butyl 4-((1-(4-aminophenyl)piperidin-4-yl)methyl)piperazine-1-carboxylate

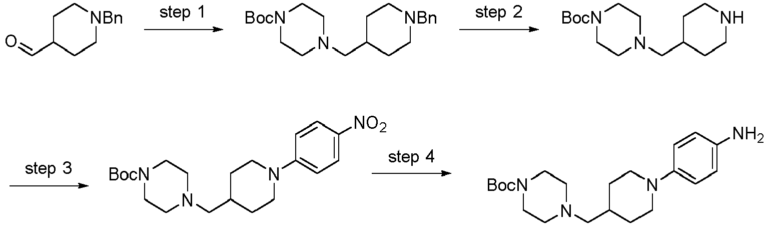

### Step 1: Synthesis of tert-butyl 4-((1-benzylpiperidin-4-yl)methyl)piperazine-1-carboxylate

1-benzyl-4-piperidinecarboxaldehyde (2.0 g, 9.84 mmol) and 1-Boc piperazine (2.20 g, 1.18 mmol) were suspended in ACN (20 mL) and stirred at room temperature for 30 minutes. NaBH(OAc)₃ (4.17 g, 19.70 mmol) was added and stirred at room temperature for 16 hours. The reaction solution was concentrated under reduced pressure, NaHCO₃ aqueous solution was added, and extracted with EtOAc (40 mL x 2). The organic layer was dried over anhydrous sodium sulfate, filtered, and concentrated under reduced pressure. The resulting residue was subjected to MPLC (50% EtOAc/Hexane) to obtain 3.06 g (83%) of a white solid.

### Step 2: Synthesis of tert-butyl 4-(piperidin-4-ylmethyl)piperazine-1-carboxylate

Tert-butyl 4-((1-benzylpiperidin-4-yl)methyl)piperazine-1-carboxylate (2.52 g, 6.74 mmol) was dissolved in MeOH (30 mL). Then, 10% Pd/C (504 mg) was added and stirred for 6 hours at room temperature under a hydrogen stream. The reaction solution was filtered and concentrated under reduced pressure to obtain 1.89 g (99%) of a white solid.

### Step 3: Synthesis of tert-butyl 4-((1-(4-nitrophenyl)piperidin-4-yl)methyl)piperazine-1-carboxylate

Tert-butyl 4-(piperidin-4-ylmethyl)piperazine-1-carboxylate (300 mg, 1.05 mmol) and 1-fluoro-4-nitrobenzene (124 mg, 0.88 mmol) were suspended in ACN (2 mL). Then, DIPEA (0.45 mL, 2.64 mmol) was added and stirred at 100 °C for 16 hours. The reaction solution was concentrated under reduced pressure, distilled water (30 mL) was added, and extracted with EtOAc (30 mL x 2). The organic layer was dried over anhydrous sodium sulfate, filtered, and concentrated under reduced pressure. The resulting residue was triturated (EtOAc/Hexane) to obtain 295 mg (69%) of a yellow solid.

### Step 4: Synthesis of tert-butyl 4-((1-(4-aminophenyl)piperidin-4-yl)methyl)piperazine-1-carboxylate

Tert-butyl 4-((1-(4-nitrophenyl)piperidin-4-yl)methyl)piperazine-1-carboxylate (290 mg, 0.71 mmol) was dissolved in MeOH (3.6 mL). Then, 10% Pd/C (30 mg) was added and stirred for 3 hours at room temperature under a hydrogen stream. The reaction solution was filtered and concentrated under reduced pressure to obtain 224 mg (83.4%) of a gray solid.

### Intermediate 2-9: tert-butyl 4-((1-(4-aminophenyl)azetidin-3-yl)methyl)piperazine-1-carboxylate

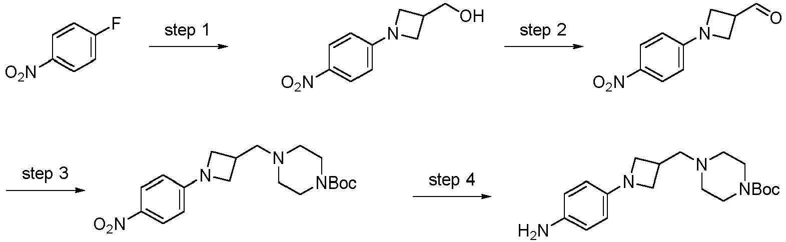

### Step 1: Synthesis of (1-(4-nitrophenyl)azetidin-3-yl)methanol

1-fluoro-4-nitrobenzene (350 mg, 1.68 mmol), azetidin-3-ylmethanol hydrochloride (482 mg, 3.90 mmol), and DIPEA (1.85 mL, 10.63 mmol) were suspended in DMSO (5 mL) and stirred at 100 °C for 16 hours. Distilled water (50 mL) was added to the reaction solution and extracted with EtOAc (20 mL x 3). The organic layer was dried over anhydrous sodium sulfate, filtered, and concentrated under reduced pressure. The resulting residue was subjected to MPLC (2-4% MeOH/DCM) to obtain 730 mg (90%) of an orange solid.

### Step 2: Synthesis of 1-(4-nitrophenyl)azetidine-3-carbaldehyde

(1-(4-nitrophenyl)azetidin-3-yl)methanol (350 mg, 1.68 mmol) was suspended in DCM (20 mL). Then, DMP (1.40 g, 3.36 mmol) was added and stirred at room temperature for 2 hours. Na₂S₂O₃ aqueous solution (15 mL) was added to the reaction solution, and then extracted with DCM (20 mL x 3). The organic layer was dried over anhydrous sodium sulfate, filtered, and concentrated under reduced pressure. The resulting residue was subjected to MPLC (1-6% MeOH/DCM) to obtain 475 mg (crude) of a yellow solid.

### Step 3: Synthesis of tert-butyl 4-((1-(4-nitrophenyl)azetidin-3-yl)methyl)piperazine-1-carboxylate

1-(4-nitrophenyl)azetidine-3-carbaldehyde (200 mg, 0.97 mmol) and tert-butyl piperazine-1-carboxylate (298 mg, 1.60 mmol) were suspended in DCM (8 mL). Then, NaBH(OAc)₃ (564 mg, 2.66 mmol) was added and stirred at room temperature for 2 hours. NaHCO₃ aqueous solution (20 mL) was added to the reaction solution and extracted with DCM (10 mL x 3). The organic layer was dried over anhydrous sodium sulfate, filtered, and concentrated under reduced pressure. The resulting residue was subjected to MPLC (1-3% MeOH/DCM) to obtain 193 mg (53%) of a yellow solid.

### Step 4: Synthesis of tert-butyl 4-((1-(4-aminophenyl)azetidin-3-yl)methyl)piperazine-1-carboxylate

Tert-butyl 4-((1-(4-nitrophenyl)azetidin-3-yl)methyl)piperazine-1-carboxylate (193 mg, 0.51 mmol) was suspended in MeOH (3 mL). Then, 10% Pd/C (27 mg) was added and stirred for 2 hours at room temperature under a hydrogen stream. The reaction solution was filtered through Celite and concentrated under reduced pressure to obtain 169 mg (95%) of a yellow solid.

### Intermediate 2-10: tert-butyl 1-(4-aminophenyl)piperidine-4-carboxylate

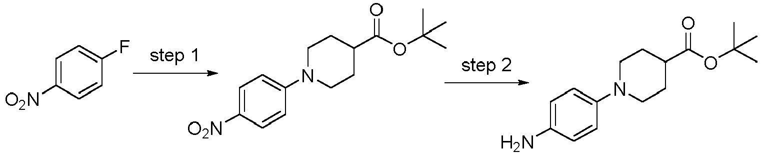

### Step 1: Synthesis of tert-butyl 1-(4-nitrophenyl)piperidine-4-carboxylate

1-fluoro-4-nitrobenzene (3.0 g, 21.26 mmol), tert-butyl piperidine-4-carboxylate (4.72 g, 25.51 mmol), and DIPEA (11 mL, 63.78 mmol) were suspended in ACN (38 mL) and stirred at 100 °C for 16 hours. Distilled water (40 mL) was added to the reaction solution and extracted with DCM (30 mL x 3). The organic layer was dried over anhydrous sodium sulfate, filtered, and concentrated under reduced pressure. The resulting residue was triturated (EtOAc/Hexane) to obtain 5.64 g (87%) of a yellow solid.

### Step 2: Synthesis of tert-butyl 1-(4-aminophenyl)piperidine-4-carboxylate

Tert-butyl 1-(4-nitrophenyl)piperidine-4-carboxylate (1.0 g, 3.26 mmol) was dissolved in MeOH (16 mL). Then, 10% Pd/C (100 mg) was added and stirred for 2 hours at room temperature under a hydrogen stream. The reaction solution was filtered through Celite and concentrated to obtain 800 mg (89%) of a bright pink solid.

### Intermediate 2-11: 4-(4-(1,3-dioxolan-2-yl)piperidin-1-yl)aniline

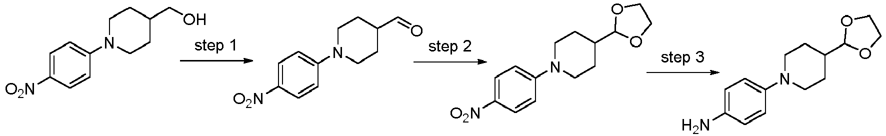

### Step 1: Synthesis of 1-(4-nitrophenyl)piperidine-4-carbaldehyde

(1-(4-nitrophenyl)piperidin-4-yl)methanol (0.5 g, 2.12 mmol) was suspended in DCM (80 mL). Then, DMP (1.79 g, 4.23 mmol) was added and stirred at room temperature for 16 hours. Na₂S₂O₃ aqueous solution (100 mL) was added to the reaction solution, and then extracted with DCM (20 mL x 3). The organic layer was dried over anhydrous sodium sulfate, filtered, and concentrated under reduced pressure. The resulting residue was subjected to MPLC (20-50% EtOAc/Hexane) to obtain 0.41 g (83%) of a yellow solid.

### Step 2: Synthesis of 4-(1,3-dioxolan-2-yl)-1-(4-nitrophenyl)piperidine

1-(4-nitrophenyl)piperidine-4-carbaldehyde (413 mg, 1.76 mmol) was suspended in toluene (20 mL). Then, ethylene glycol (219 mg, 3.53 mmol) and PPTS (44 mg, 0.18 mmol) were added and stirred at 115 °C for 3 hours. Distilled water (50 mL) was added to the reaction solution and extracted with EtOAc (20 mL x 3). The organic layer was dried over anhydrous sodium sulfate, filtered, and concentrated under reduced pressure. The resulting residue was subjected to MPLC (1-4% MeOH/DCM) to obtain 303 mg (62%) of a yellow solid.

### Step 3: Synthesis of 4-(4-(1,3-dioxolan-2-yl)piperidin-1-yl)aniline

4-(1,3-dioxolan-2-yl)-1-(4-nitrophenyl)piperidine (303 mg, 1.09 mmol) was suspended in MeOH (10 mL). Then, 10% Pd/C (58 mg) was added and stirred for 2 hours at room temperature under a hydrogen stream. The reaction solution was filtered through Celite and concentrated to obtain 190 mg (70%) of a yellow solid.

### Intermediate 2-12 was synthesized using the same method as that of Intermediate 2-11.

| | |
|---|---|
| Intermediate 2-12: 6-(4-(1,3-dioxolan-2-yl)piperidin-1-yl)pyridin-3-amine | 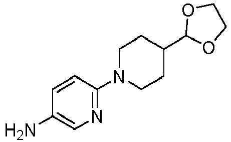 |

### Intermediate 2-13: tert-butyl 4-(4-aminobenzyl)piperazine-1-carboxylate

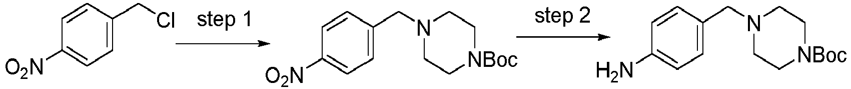

### Step 1: Synthesis of tert-butyl 4-(4-nitrobenzyl)piperazine-1-carboxylate

1-(chloromethyl)-4-nitrobenzene (887 mg, 5.17 mmol), tert-butyl piperazine-1-carboxylate (1.07 g, 5.74 mmol), and TEA (0.78 mL, 5.58 mmol) were suspended in ACN (26 mL) and stirred at room temperature for 16 hours. Distilled water (30 mL) was added to the reaction solution, and then extracted with EtOAc (30 mL x 3). The organic layer was dried over anhydrous sodium sulfate, filtered, and concentrated under reduced pressure. The resulting residue was subjected to MPLC (0-40% EtOAc/Hexane) to obtain 1.3 g (78%) of a white solid.

### Step 2: Synthesis of tert-butyl 4-(4-aminobenzyl)piperazine-1-carboxylate

Tert-butyl 4-(4-nitrobenzyl)piperazine-1-carboxylate (1.34 g, 4.16 mmol) was dissolved in MeOH (20 mL). Then, 10% Pd/C (134 mg) was added and stirred for 16 hours at room temperature under a hydrogen stream. The reaction solution was filtered through Celite and concentrated to obtain 190 mg (16%) of a light yellow solid.

### Intermediate 2-14: 4-((4-(1,3-dioxolan-2-yl)piperidin-1-yl)methyl)aniline

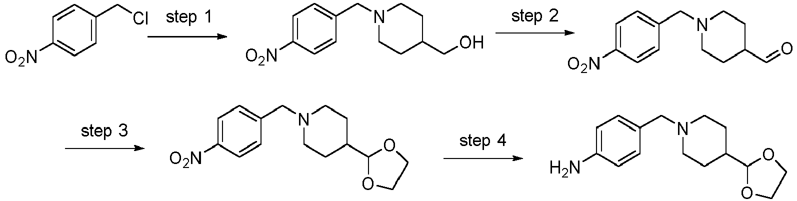

### Step 1: Synthesis of (1-(4-nitrobenzyl)piperidin-4-yl)methanol

1-(chloromethyl)-4-nitrobenzene (3.0 g, 17.48 mmol), piperidin-4-ylmethanol (2.0 g, 17.48 mmol), and TEA (4.90 mL, 35.0 mmol) were suspended in ACN (87 mL) and stirred at room temperature for 16 hours. Distilled water (60 mL) was added to the reaction solution, and then extracted with EtOAc (30 mL x 3). The organic layer was dried over anhydrous sodium sulfate, filtered, and concentrated under reduced pressure. The resulting residue was subjected to MPLC (0-4% MeOH/DCM) to obtain 2.45 g (56%) of a bright yellow solid.

### Step 2: Synthesis of 1-(4-nitrobenzyl)piperidine-4-carbaldehyde

Oxalyl chloride (0.87 mL, 10.18 mmol) was dissolved in DCM (20.0 mL). Then, DMSO (0.75 mL, 10.66 mmol) was diluted in DCM (4 mL) and added slowly at -78 °C. (1-(4-nitrobenzyl)piperidin-4-yl)methanol (1.70 g, 6.79 mmol) was diluted in DCM (4 mL) and slowly added, and the reaction solution was stirred at -78 °C for 1 hour. TEA (2.83 mL, 20.37 mmol) was diluted in DCM (4 mL) and added slowly at -78 °C, and the reaction solution was stirred at room temperature for 0.5 hours. 1N NaOH (50 mL) was added to the reaction solution and extracted with DCM (30 mL x 3). The organic layer was dried over anhydrous sodium sulfate, filtered, and concentrated under reduced pressure to obtain 1.56 g (92.5%) of a yellow liquid.

### Step 3: Synthesis of 4-(1,3-dioxolan-2-yl)-1-(4-nitrobenzyl)piperidine

1-(4-nitrobenzyl)piperidine-4-carbaldehyde (1.56 g, 6.28 mmol), PPTS (158 mg, 0.63 mmol), and ethylene glycol (780 mg, 12.56 mmol) were suspended in toluene (26 mL) and heated and refluxed for 16 hours. The residue obtained by concentrating the reaction solution was subjected to MPLC (0-50% EtOAc/Hexane) to obtain 635 mg (35%) of a yellow solid.

### Step 4: Synthesis of 4-((4-(1,3-dioxolan-2-yl)piperidin-1-yl)methyl)aniline

4-(1,3-dioxolan-2-yl)-1-(4-nitrobenzyl)piperidine (635 mg, 2.17 mmol) was suspended in EtOH (10 mL). Then, SnCl₂ (2.06 g, 10.86 mmol) was added and stirred at 80 °C for 3 hours. NaHCO₃ aqueous solution (40 mL) was added to the reaction solution, and then extracted with EtOAc (30 mL x 4). The organic layer was dried over anhydrous sodium sulfate, filtered, and concentrated under reduced pressure to obtain 530 mg (93%) of a yellow solid.

### Intermediate 3-1: 1-(2-(2,6-dioxopiperidin-3-yl)-1,3-dioxoisoindolin-5-yl)piperidine-4-carbaldehyde

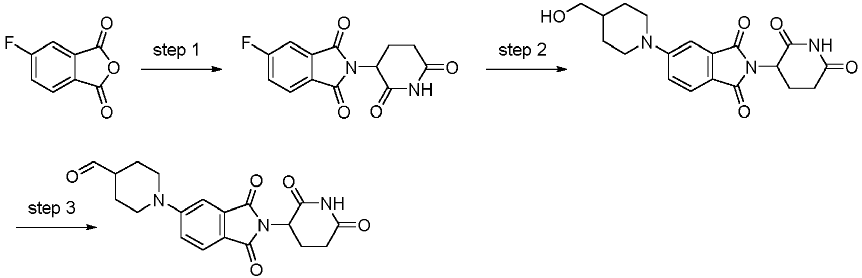

### Step 1: Synthesis of 2-(2,6-dioxopiperidin-3-yl)-5-fluoroisoindoline-1,3-dione

5-fluoroisobenzofuran-1,3-dione (5.00 g, 30.10 mmol), 3-aminopiperidine-2,6-dione hydrochloride (4.95 g, 30.10 mmol), and sodium acetate (4.94 mg, 60.20 mmol) were suspended in AcOH (50 mL) and stirred at 120 °C for 24 hours. The reaction solution was concentrated under reduced pressure, distilled water (30 mL) was added, and the resulting solid was filtered and dried to obtain 7.55 g (90%) of a purple solid.

### Step 2: Synthesis of 2-(2,6-dioxopiperidin-3-yl)-5-(4-(hydroxymethyl)piperidin-1-yl)isoindoline-1,3-dione

2-(2,6-dioxopiperidin-3-yl)-5-fluoroisoindoline-1,3-dione (300 mg, 1.09 mmol), piperidin-4-ylmethanol (149 mg, 1.30 mmol), and DIPEA(0.29 mL, 1.64 mmol) were suspended in DMSO (5.0 mL) and stirred at 100 °C for 16 hours. Distilled water (30 mL) was added to the reaction solution, and then extracted with EtOAc (25 mL x 3). The organic layer was dried over anhydrous sodium sulfate, filtered, and concentrated under reduced pressure. The resulting residue was subjected to MPLC (50% EtOAc/Hexane) to obtain 332 mg (82%) of a yellow solid.

### Step 3: Synthesis of 1-(2-(2,6-dioxopiperidin-3-yl)-1,3-dioxoisoindolin-5-yl)piperidine-4-carbaldehyde

2-(2,6-dioxopiperidin-3-yl)-5-(4-(hydroxymethyl)piperidin-1-yl)isoindoline-1,3-dione (332 mg, 0.89 mmol) was suspended in DCM (5.0 mL). Then, DMP (569 mg, 1.34 mmol) was added and stirred at room temperature for 2 hours. Na₂S₂O₃ aqueous solution (10 mL) was added to the reaction solution, and then extracted with DCM (25 mL x 2). The organic layer was dried over anhydrous sodium sulfate, filtered, and concentrated under reduced pressure. The resulting residue was subjected to MPLC (50% EtOAc/DCM) to obtain 303 mg (92%) of a yellow solid.

### Intermediates 3-2 to 3-5 were synthesized using the same method as the synthesis of Intermediate 3-1.

| | |
|---|---|
| Intermediate 3-2: 1-(2-(2,6-dioxopiperidin-3-yl)-1,3-dioxoisoindolin-5-yl)azetidine-3-carbaldehyde | 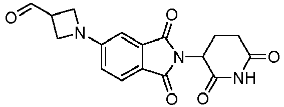 |
| Intermediate 3-3: 1-(2-(2,6-dioxopiperidin-3-yl)-1,3-dioxoisoindolin-5-yl)-3-methylazetidine-3-carbaldehyde | 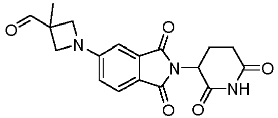 |
| Intermediate 3-4: 1-(2-(2,6-dioxopiperidin-3-yl)-6-fluoro-1,3-dioxoisoindolin-5-yl)piperidine-4-carbaldehyde | 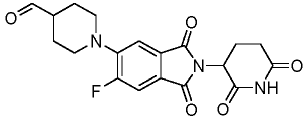 |
| Intermediate 3-5: 1-(2-(2,6-dioxopiperidin-3-yl)-6-fluoro-1,3-dioxoisoindolin-5-yl)azetidine-3-carbaldehyde | 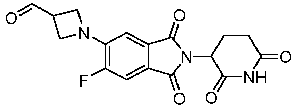 |

### Intermediate 3-6: 1-(6-(2,6-dioxopiperidin-3-yl)-5-oxo-6,7-dihydro-5H-pyrrolo[3,4-b]pyridin-2-yl)piperidine-4-carbaldehyde

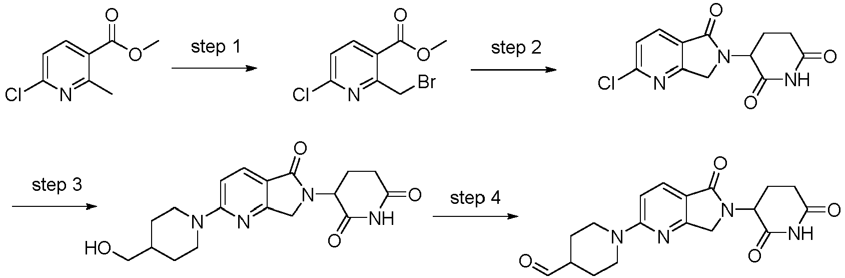

### Step 1: Synthesis of methyl 2-(bromomethyl)-6-chloronicotinate

Methyl 6-chloro-2-methylnicotinate (1.00 g, 5.38 mmol), *N*-bromosuccinimide (1.44 g, 8.08 mmol), and AHCN (130 mg, 0.05 mmol) were suspended in ACN (10 mL) and stirred in a microwave at 110 °C for 4 hours. Distilled water (10 mL) was added to the reaction solution and extracted with EtOAc (10 mL x 2). The organic layer was washed with brine (10 mL x 2), dried over anhydrous sodium sulfate, filtered, and concentrated under reduced pressure. The obtained residue was subjected to MPLC (10% EtOAc/Hexane) to obtain 980 mg of a white solid. m/z 263.99 [M+H]⁺.

### Step 2: Synthesis of 3-(2-chloro-5-oxo-5,7-dihydro-6H-pyrrolo[3,4-b]pyridin-6-yl)piperidine-2,6-dione

Methyl 2-(bromomethyl)-6-chloronicotinate (980 mg), 3-aminopiperidine-2,6-dione hydrochloride (686 mg, 4.17 mmol), and DIPEA (1.05 mL, 10.40 mmol) were suspended in ACN (10 mL) and stirred at 110 °C for 2 hours. Distilled water (30 mL) was added to the reaction solution, and then extracted with EtOAc (25 mL x 3). The organic layer was dried over anhydrous sodium sulfate, filtered, and concentrated under reduced pressure. The resulting residue was recrystallized (MeOH) to obtain 244 mg (16%, 2 steps yield) of an off-white solid. m/z 280.09 [M+H]⁺.

### Step 3: Synthesis of 3-(2-(4-(hydroxymethyl)piperidin-1-yl)-5-oxo-5,7-dihydro-6H-pyrrolo[3,4-b]pyridin-6-yl)piperidine-2,6-dione

3-(2-chloro-5-oxo-5,7-dihydro-6H-pyrrolo[3,4-b]pyridin-6-yl)piperidine-2,6-dione (0.77 g, 2.75 mmol), piperidin-4-ylmethanol (0.38 g, 3.30 mmol), and DIPEA (0.96 mL, 5.51 mmol) were suspended in DMSO (10 mL) and stirred at 100 °C for 16 hours. Distilled water (50 mL) was added to the reaction solution, and then extracted with EtOAc (20 mL x 3) and DCM (10 mL x 12). The organic layer was dried over anhydrous sodium sulfate, filtered, and concentrated under reduced pressure. The obtained residue was suspended in DCM (10 mL), filtered, and dried to obtain 0.43 g (44%) of a bright yellow solid.

### Step 4: Synthesis of 1-(6-(2,6-dioxopiperidin-3-yl)-5-oxo-6,7-dihydro-5H-pyrrolo[3,4-b]pyridin-2-yl)piperidine-4-carbaldehyde

3-(2-(4-(hydroxymethyl)piperidin-1-yl)-5-oxo-5,7-dihydro-6H-pyrrolo[3,4-b]pyridin-6-yl)piperidine-2,6-dione (0.43 g, 1.21 mmol) was suspended in DCM (40 mL). Then, DMP (1.02 g, 2.41 mmol) was added and stirred at room temperature for 2 hours. Na₂S₂O₃ aqueous solution (20 mL) was added to the reaction solution, and then extracted with DCM (20 mL x 3). The organic layer was dried over anhydrous sodium sulfate, filtered, and concentrated under reduced pressure. The resulting residue was subjected to MPLC (3-8% MeOH/DCM) to obtain 0.36 mg (83%) of a yellow solid.

### Intermediate 3-7: 3-((3-aminophenyl)amino)piperidine-2,6-dione

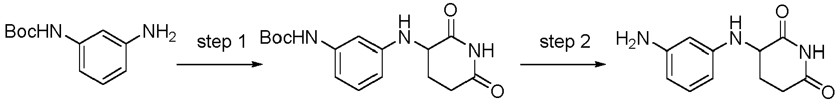

### Step 1: Synthesis of tert-butyl (3-((2,6-dioxopiperidin-3-yl)amino)phenyl)carbamate

Tert-butyl (3-aminophenyl)carbamate (300 mg, 1.44 mmol) and 3-bromopiperidine-2,6-dione (331 mg, 1.73 mmol) were suspended in DMF (5.0 mL). Then, NaHCO₃ (241 mg, 2.88 mmol) was added and stirred at 50 °C for 16 hours. Distilled water (30 mL) was added to the reaction solution, and the resulting solid was filtered and dried to obtain 323 mg (70%) of a green solid. m/z 342.20 [M+Na]⁺.

### Step 2: Synthesis of 3-((3-aminophenyl)amino)piperidine-2,6-dione

Tert-butyl (3-((2,6-dioxopiperidin-3-yl)amino)phenyl)carbamate (100 mg, 0.31 mmol) was suspended in DCM (1 mL). Then, 4M HCl in dioxane (0.39 mL, 1.57 mmol) was added and stirred at room temperature for 1 hour. The reaction solution was concentrated, NaHCO₃ aqueous solution (15 mL) was added, and extracted with DCM (20 mL x 2). The organic layer was dried over anhydrous sodium sulfate, filtered, and concentrated under reduced pressure to obtain 34 mg (51%) of a brown solid. m/z 220.20 [M+H]⁺.

### Intermediate 3-8: 3-((2,6-dioxopiperidin-3-yl)amino)benzoic acid

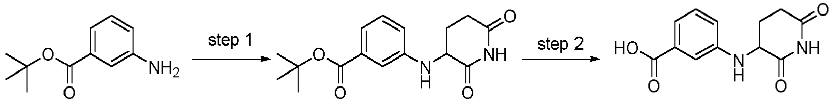

### Step 1: Synthesis of tert-butyl 3-((2,6-dioxopiperidin-3-yl)amino)benzoate

Tert-butyl 3-aminobenzoate (0.50 g, 2.59 mmol) and 3-bromopiperidine-2,6-dione (0.60 g, 3.10 mmol) were suspended in DMF (10 mL). Then, NaHCO₃ (0.44 g, 5.17 mmol) was added and stirred at 80 °C for 16 hours. NH₄Cl aqueous solution (50 mL) was added to the reaction solution, and then extracted with EtOAc (30 mL x 4). The organic layer was dried over anhydrous sodium sulfate, filtered, and concentrated under reduced pressure. The resulting residue was subjected to MPLC (20-50% EtOAc/Hexane) to obtain 0.22 g (28%) of green oil.

### Step 2: Synthesis of 3-((2,6-dioxopiperidin-3-yl)amino)benzoic acid

Tert-butyl 3-((2,6-dioxopiperidin-3-yl)amino)benzoate (0.22 g, 0.73 mmol) was suspended in DCM (2.0 mL). Then, 4M HCl in dioxane (2 mL, 8.03 mmol) was added and stirred at room temperature for 8 hours. The reaction solution was concentrated under reduced pressure and dried to obtain 0.18 g (73%) of a gray solid.

### Intermediate 3-9: 3-((4-(piperidin-4-yl)phenyl)amino)piperidine-2,6-dione hydrochloride

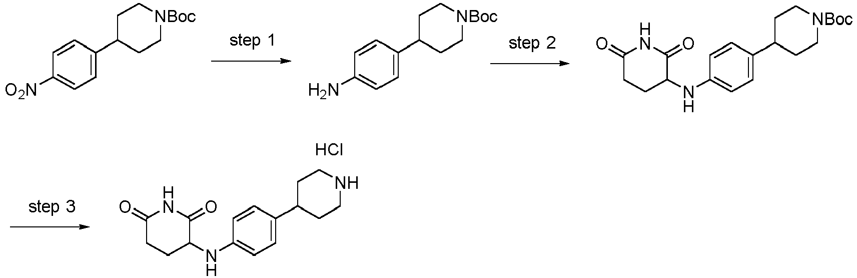

### Step 1: Synthesis of tert-butyl 4-(4-aminophenyl)piperidine-1-carboxylate

Tert-butyl 4-(4-nitrophenyl)piperidine-1-carboxylate (3.08 g, 12.4 mmol) was suspended in MeOH (80 mL). Then, 10% Pd/C (0.66 g) was added and stirred for 4 hours at room temperature under a hydrogen stream. The reaction solution was filtered through Celite and concentrated under reduced pressure to obtain 3.70 g (108%) of a light gray solid.

### Step 2: Synthesis of tert-butyl 4-(4-((2,6-dioxopiperidin-3-yl)amino)phenyl)piperidine-1-carboxylate

Tert-butyl 4-(4-aminophenyl)piperidine-1-carboxylate (3.7 g, 13.39 mmol) was suspended in DMF (30 mL). Then, 3-bromopiperidine-2,6-dione (3.08 g, 16.06 mmol) and DIPEA (4.77 mL, 26.77 mmol) were added and stirred at 100 °C for 16 hours. Distilled water (150 mL) was added to the reaction solution, and then extracted with EtOAc (30 mL x 3). The organic layer was dried over anhydrous sodium sulfate, filtered, and concentrated under reduced pressure. The resulting residue was subjected to MPLC (20-50% EtOAc/Hexane) to obtain 1.22 g (25%, 2 steps yield) of a green solid.

### Step 3: Synthesis of 3-((4-(piperidin-4-yl)phenyl)amino)piperidine-2,6-dione hydrochloride

Tert-butyl 4-(4-((2,6-dioxopiperidin-3-yl)amino)phenyl)piperidine-1-carboxylate (1.22 g, 3.15 mmol) was suspended in DCM (30 mL). Then, 4M HCl in dioxane (5 mL) was added and stirred at room temperature for 16 hours. The reaction solution was concentrated under reduced pressure and then triturated (DCM/diethyl ether) to obtain 1.06 g (104%) of a green solid.

### Intermediate 3-10 was synthesized using the same method as that of Intermediate 3-9.

| | |
|---|---|
| Intermediate 3-10: 3-((3-(piperidin-4-yl)phenyl)amino)piperidine-2,6-dione hydrochloride | 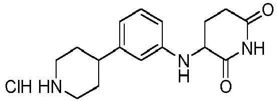 |

### Intermediate 3-11: 3-(2,4-dioxotetrahydropyrimidin-1(2H)-yl)benzoic acid

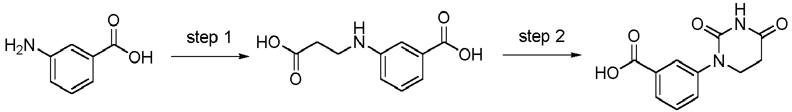

### Step 1: Synthesis of 3-((2-carboxyethyl)amino)benzoic acid

3-aminobenzoic acid (400 mg, 2.92 mmol) was suspended in toluene (8 mL). Then, acrylic acid (0.26 mL, 3.79 mmol) was added and stirred at 120 °C for 19 hours. After cooling the reaction solution to room temperature, the precipitated solid was filtered, washed with toluene, and dried to obtain 501 mg (82%) of a white solid.

### Step 2: Synthesis of 3-(2,4-dioxotetrahydropyrimidin-1(2H)-yl)benzoic acid

3-((2-carboxyethyl)amino)benzoic acid (405 mg, 1.94 mmol) and urea (349 mg, 5.81 mmol) were suspended in AcOH (6.5 mL) and stirred at 120 °C for 18 hours. The reaction solution was concentrated under reduced pressure, an excess of water was added, and the resulting solid was filtered, washed with water, and dried to obtain 254 mg (56%) of a light brown solid.

### Intermediate 3-12 was synthesized using the same method as that of Intermediate 3-11.

| | |
|---|---|
| Intermediate 3-12: 3-(2,4-dioxotetrahydropyrimidin-1(2H)-yl)-4-methoxybenzoic acid | 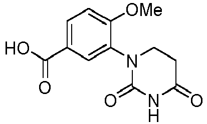 |

### Intermediate 3-13: 3-((3-(bromomethyl)phenyl)amino)piperidine-2,6-dione

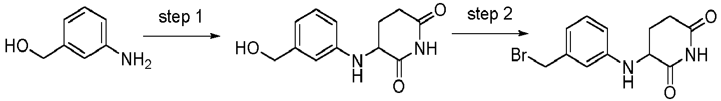

### Step 1: Synthesis of 3-((3-(hydroxymethyl)phenyl)amino)piperidine-2,6-dione

(3-aminophenyl)methanol (300 mg, 2.44 mmol) and 3-bromopiperidine-2,6-dione (515 mg, 2.68 mmol) were suspended in DMF (5.0 mL). Then, NaHCO₃ (409 mg, 4.87 mmol) was added and stirred at 80 °C for 16 hours. Distilled water (50 mL) was added to the reaction solution, and then extracted with EtOAc (20 mL x 6). The organic layer was dried over anhydrous sodium sulfate, filtered, and concentrated under reduced pressure. The resulting residue was subjected to MPLC (2-8% MeOH/DCM) to obtain 258 mg (45%) of a yellow solid.

### Step 2: Synthesis of 3-((3-(bromomethyl)phenyl)amino)piperidine-2,6-dione

3-((3-(hydroxymethyl)phenyl)amino)piperidine-2,6-dione (14 mg, 0.06 mmol) was suspended in 30% HBr in acetic acid (0.5 mL) and stirred at 90 °C for 10 minutes. Distilled water (10 mL) was added to the reaction solution and extracted with EtOAc (10 mL x 3). The organic layer was dried over anhydrous sodium sulfate, filtered, and concentrated under reduced pressure. The resulting residue was subjected to MPLC (2-4% MeOH/DCM) to obtain 14 mg (crude) of a yellow solid.

### Intermediate 3-14: 3-((2,6-dioxopiperidin-3-yl)oxy)benzoic acid

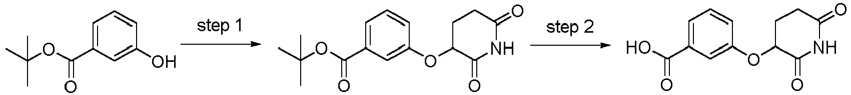

### Step 1: Synthesis of tert-butyl 3-((2,6-dioxopiperidin-3-yl)oxy)benzoate

Tert-butyl 3-hydroxybenzoate (300 mg, 1.54 mmol) was suspended in THF (10 mL). Then, sodium hydride (60%, dispersion in paraffin liquid, 185 mg, 4.63 mmol) was slowly added and stirred at 60 °C for 30 minutes. 3-bromopiperidine-2,6-dione (445 mg, 2.3 mmol) was added to the reaction solution and stirred at 60 °C for 50 minutes. Distilled water (50 mL) was added to the reaction solution, and then extracted with EtOAc (50 mL). The organic layer was dried over anhydrous sodium sulfate, filtered, and concentrated under reduced pressure. The resulting residue was subjected to MPLC (30% EtOAc/Hexane) to obtain 214 mg (46%) of a white solid.

### Step 2: Synthesis of 3-((2,6-dioxopiperidin-3-yl)oxy)benzoic acid

Tert-butyl 3-((2,6-dioxopiperidin-3-yl)oxy)benzoate (212 mg, 0.7 mmol) was suspended in DCM (5 mL). 4M HCl in dioxane (3.5 mL) was then added and stirred at room temperature for 4 hours. The reaction solution was concentrated under reduced pressure to obtain 186 mg (93%) of a white solid.

### Intermediate 3-15: 1-(4-((2,6-dioxopiperidin-3-yl)oxy)phenyl)piperidine-4-carbaldehyde

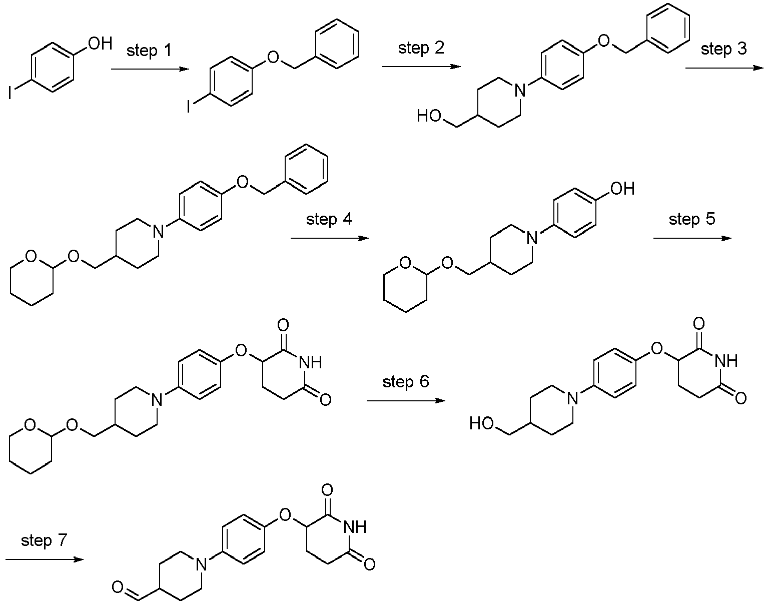

### Step 1: Synthesis of 1-(benzyloxy)-4-iodobenzene

4-iodophenol (1.0 g, 4.54 mmol), benzyl bromide (778 mg, 4.54 mmol), and K₂CO₃ (1.25 g, 9.08 mmol) were suspended in acetonitrile (10 mL) and stirred at 60 °C for 15 hours. Distilled water (40 mL) was added to the reaction solution, and then extracted with EtOAc (40 mL). The organic layer was dried over anhydrous sodium sulfate, filtered, and concentrated under reduced pressure. The resulting residue was subjected to MPLC (1% EtOAc/Hexane) to obtain 1.38 g (98%) of a white solid.

### Step 2: Synthesis of (1-(4-(benzyloxy)phenyl)piperidin-4-yl)methanol

1-(benzyloxy)-4-iodobenzene (1.38 g, 4.47 mmol), piperidin-4-ylmethanol (1.0 g, 8.94 mmol), CuI (170 mg, 0.89 mmol), 2-acetylcyclohexanone (131 mg, 0.89 mmol), and Cs₂CO₃ (2.9 g, 8.94 mmol) were suspended in DMF (15 mL) and stirred at 50 °C for 24 hours. Distilled water (100 mL) was added to the reaction solution, and then extracted with EtOAc (100 mL). The organic layer was dried with anhydrous magnesium sulfate, filtered, and concentrated under reduced pressure. The resulting residue was subjected to MPLC (40% EtOAc/Hexane) to obtain 567 mg (43%) of a white solid.

### Step 3: Synthesis of 1-(4-(benzyloxy)phenyl)-4-(((tetrahydro-2H-pyran-2-yl)oxy)methyl)piperidine

(1-(4-(benzyloxy)phenyl)piperidin-4-yl)methanol (567 mg, 1.91 mmol), 3,4-dihydro-2H-pyran (481 mg, 5.7 mmol), andpTSA (33 mg, 0.19 mmol) were suspended in 1,4-dioxane (18 mL) and stirred at 100 °C for 2 hours. Distilled water (40 mL) was added to the reaction solution, and then extracted with EtOAc (40 mL). The organic layer was dried with anhydrous magnesium sulfate, filtered, and concentrated under reduced pressure. The resulting residue was subjected to MPLC (10% EtOAc/Hexane) to obtain 602 mg (83%) of a pink solid.

### Step 4: Synthesis of 4-(4-(((tetrahydro-2H-pyran-2-yl)oxy)methyl)piperidin-1-yl)phenol

1-(4-(benzyloxy)phenyl)-4-(((tetrahydro-2H-pyran-2-yl)oxy)methyl)piperidine (602 mg, 1.58 mmol) was suspended in MeOH (2 mL) and DCM (2 mL). Then, palladium (10 wt. % on activated carbon, 300 mg) was added and stirred at room temperature for 14 hours under a hydrogen stream. The reaction solution was filtered through Celite and concentrated under reduced pressure. The resulting residue was subjected to MPLC (30% EtOAc/Hexane) to obtain 448 mg (97%) of clear oil.

### Step 5: Synthesis of 3-(4-(4-(((tetrahydro-2H-pyran-2-yl)oxy)methyl)piperidin-1-yl)phenoxy)piperidine-2,6-dione

4-(4-(((tetrahydro-2H-pyran-2-yl)oxy)methyl)piperidin-1-yl)phenol (448 mg, 1.54 mmol) was suspended in THF (10 mL). Then, sodium hydride (60%, dispersion in paraffin liquid, 185 mg, 4.63 mmol) was slowly added and stirred at 60 °C for 30 minutes. 3-bromopiperidine-2,6-dione (443 mg, 2.3 mmol) was added to the reaction solution and stirred at 60 °C for 30 minutes. Distilled water (50 mL) was added to the reaction solution, and then extracted with EtOAc (50 mL). The organic layer was dried over anhydrous sodium sulfate, filtered, and concentrated under reduced pressure. The resulting residue was subjected to MPLC (40% EtOAc/Hexane) to obtain 268 mg (43%) of a white solid.

### Step 6: Synthesis of 3-(4-(4-(hydroxymethyl)piperidin-1-yl)phenoxy)piperidine-2,6-dione

3-(4-(4-(((tetrahydro-2H-pyran-2-yl)oxy)methyl)piperidin-1-yl)phenoxy)piperidine-2,6-dione (266 mg, 0.66 mmol) was suspended in DCM (5 mL). Then, 4M HCl in dioxane (1.6 mL) was added and stirred at room temperature for 20 minutes. NaHCO₃ aqueous solution (20 mL) was added to the reaction solution, and then extracted with EtOAc (20 mL x 2). The organic layer was dried over anhydrous magnesium sulfate, filtered, and concentrated under reduced pressure to obtain 208 mg (99%) of a gray solid.

### Step 7: Synthesis of 1-(4-((2,6-dioxopiperidin-3-yl)oxy)phenyl)piperidine-4-carbaldehyde

3-(4-(4-(hydroxymethyl)piperidin-1-yl)phenoxy)piperidine-2,6-dione (30 mg, 0.094 mmol) was suspended in DCM (1 mL). Then, DMP (48 mg, 0.11 mmol) was added and stirred at room temperature for 20 minutes. NaHCO₃ aqueous solution (10 mL) was added to the reaction solution and extracted with DCM (10 mL x 2). The organic layer was dried over anhydrous magnesium sulfate, filtered, and concentrated under reduced pressure. The resulting residue was subjected to MPLC (3% MeOH/DCM) to obtain 17 mg (58%) of a purple solid.

### Intermediate 3-16 was synthesized using the same method as that of Intermediate 3-15.

| | |
|---|---|
| Intermediate 3-16: 1-(3-((2,6-dioxopiperidin-3-yl)oxy)phenyl)piperidine-4-carbaldehyde | 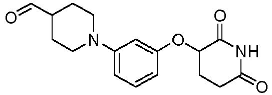 |

### Intermediate 3-17: 3-(4-(piperidin-4-yl)phenoxy)piperidine-2,6-dione hydrochloride

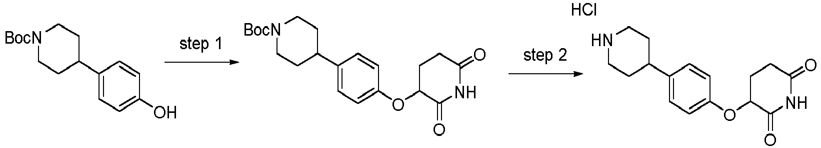

### Step 1: Synthesis of tert-butyl 4-(4-((2,6-dioxopiperidin-3-yl)oxy)phenyl)piperidine-1-carboxylate

Tert-butyl 4-(4-hydroxyphenyl)piperidine-1-carboxylate (400 mg, 1.44 mmol) was suspended in DMF (5 mL). Then, NaH (173 mg, 4.33 mmol) was added and stirred for 10 minutes. 3-bromopiperidine-2,6-dione (554 mg, 2.88 mmol) was added and stirred at 90 °C for 16 hours. Distilled water (50 mL) was added to the reaction solution and extracted with EtOAc (20 mL x 3). The organic layer was dried over anhydrous sodium sulfate, filtered, and concentrated under reduced pressure. The resulting residue was subjected to MPLC (NH silica, 1-2% MeOH/DCM) to obtain 239 mg (43%) of a pink solid.

### Step 2: Synthesis of 3-(4-(piperidin-4-yl)phenoxy)piperidine-2,6-dione hydrochloride

Tert-butyl 4-(4-((2,6-dioxopiperidin-3-yl)oxy)phenyl)piperidine-1-carboxylate (239 mg, 0.62 mmol) was suspended in DCM (2 mL). Then, 4M HCl in dioxane (0.5 mL) was added and stirred at room temperature for 2 hours. The reaction solution was concentrated under reduced pressure to obtain 198 mg (99%) of a pink solid.

### Intermediate 3-18: 1-(5-((2,6-dioxopiperidin-3-yl)oxy)pyridin-2-yl)piperidine-4-carbaldehyde

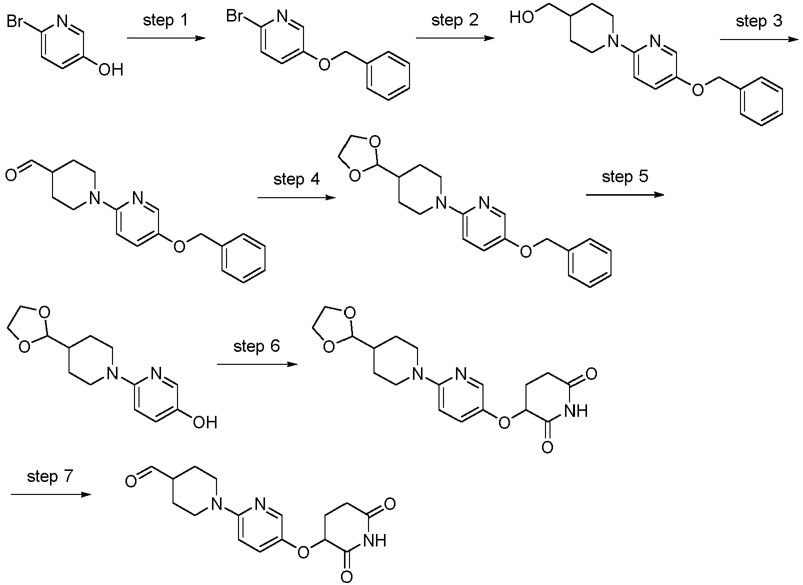

### Step 1: Synthesis of 5-(benzyloxy)-2-bromopyridine

6-bromopyridin-3-ol (2 g, 11.49 mmol), benzyl bromide (1.5 mL, 12.64 mmol), and K₂CO₃ (3.18 g, 22.98 mmol) were suspended in MeCN (50 mL) and stirred at 60 °C for 16 hours. The reaction solution was concentrated under reduced pressure, distilled water (80 mL) was added, and extracted with EtOAc (20 mL x 3). The organic layer was dried over anhydrous sodium sulfate, filtered, and concentrated under reduced pressure. The resulting residue was subjected to MPLC (10-20% EtOAc/Hexane) to obtain 2.48 g (82%) of a bright yellow solid.

### Step 2: Synthesis of (1-(5-(benzyloxy)pyridin-2-yl)piperidin-4-yl)methanol

5-(benzyloxy)-2-bromopyridine (1 g, 3.79 mmol), piperidin-4-ylmethanol (0.52 g, 4.54 mmol), Pd₂(dba)₃ (0.69 g, 0.76 mmol), BINAP (0.47 g, 0.76 mmol), and sodium tert-butoxide (0.73 g, 7.57 mmol) were suspended in dioxane (30 mL) and stirred at 105 °C for 16 hours. Distilled water (80 mL) was added to the reaction solution and extracted with EtOAc (20 mL x 3). The organic layer was dried over anhydrous sodium sulfate, filtered, and concentrated under reduced pressure. The resulting residue was subjected to MPLC (50-100% EtOAc/Hexane) to obtain 0.51 g (45%) of an orange solid.

### Step 3: Synthesis of 1-(5-(benzyloxy)pyridin-2-yl)piperidine-4-carbaldehyde

DMSO (0.22 mL, 3.11 mmol) was diluted in DCM (20mL), and oxalyl chloride (0.2 mL, 2.33 mmol) was added thereto at 0 °C. (1-(5-(benzyloxy)pyridin-2-yl)piperidin-4-yl)methanol (464 mg, 1.56 mmol) was added to the reaction solution, and triethylamine (0.65 mL, 4.67 mmol) diluted in DCM (10 mL) was slowly added and stirred at room temperature for 1 hour. Distilled water (50 mL) was added to the reaction solution and extracted with EtOAc (20 mL x 3). The organic layer was dried over anhydrous sodium sulfate, filtered, and concentrated under reduced pressure. The resulting residue was subjected to MPLC (20-50% EtOAc/Hexane) to obtain 341 mg (74%) of an orange liquid.

### Step 4: Synthesis of 2-(4-(1,3-dioxolan-2-yl)piperidin-1-yl)-5-(benzyloxy)pyridine

1-(5-(benzyloxy)pyridin-2-yl)piperidine-4-carbaldehyde (341 mg, 1.15 mmol) was suspended in toluene (15 mL). Then, ethylene glycol (143 mg, 2.30 mmol) and PPTS (29 mg, 0.12 mmol) were added and stirred at 115 °C for 1 hour. Distilled water (50 mL) was added to the reaction solution and extracted with EtOAc (20 mL x 3). The organic layer was dried over anhydrous sodium sulfate, filtered, and concentrated under reduced pressure. The resulting residue was subjected to MPLC (1-2% MeOH/DCM) to obtain 382 mg (98%) of a yellow solid.

### Step 5: Synthesis of 6-(4-(1,3-dioxolan-2-yl)piperidin-1-yl)pyridin-3-ol

2-(4-(1,3-dioxolan-2-yl)piperidin-1-yl)-5-(benzyloxy)pyridine (382 mg, 1.12 mmol) was suspended in MeOH (10 mL). Then, 10% Pd/C (60 mg) was added and stirred for 6 hours at room temperature under a hydrogen stream. The reaction solution was filtered through Celite and concentrated under reduced pressure. The resulting residue was subjected to MPLC (1-3% MeOH/DCM) to obtain 155 mg (55%) of a bright yellow solid.

### Step 6: Synthesis of (3-((6-(4-(1,3-dioxolan-2-yl)piperidin-1-yl)pyridin-3-yl)oxy)piperidine-2,6-dione

6-(4-(1,3-dioxolan-2-yl)piperidin-1-yl)pyridin-3-ol (155 mg, 0.62 mmol) was suspended in THF (10 mL). Then, NaH (74 mg, 1.86 mmol) was added and stirred for 5 minutes. 3-bromopiperidine-2,6-dione (179 mg, 0.93 mmol) was added and stirred at 65 °C for 2 hours. Distilled water (50 mL) was added to the reaction solution and extracted with EtOAc (10 mL x 3). The organic layer was dried over anhydrous sodium sulfate, filtered, and concentrated under reduced pressure. The resulting residue was subjected to MPLC (60-80% EtOAc/Hexane) to obtain 48 mg (21%) of a bright yellow solid.

### Step 7: Synthesis of (1-(5-((2,6-dioxopiperidin-3-yl)oxy)pyridin-2-yl)piperidine-4-carbaldehyde

(3-((6-(4-(1,3-dioxolan-2-yl)piperidin-1-yl)pyridin-3-yl)oxy)piperidine-2,6-dione (48 mg, 0.13 mmol) was suspended in DCM (2 mL). Then, TFA (0.5 mL) was added and stirred at 50 °C for 16 hours. NaHCO₃ aqueous solution (30 mL) was added to the reaction solution and extracted with DCM (10 mL x 3). The organic layer was dried over anhydrous sodium sulfate, filtered, and concentrated under reduced pressure to obtain 29 mg (69%) of a bright yellow solid.

### Intermediate 3-19: 3-((6-(piperidin-4-yl)pyridin-3-yl)amino)piperidine-2,6-dione hydrochloride

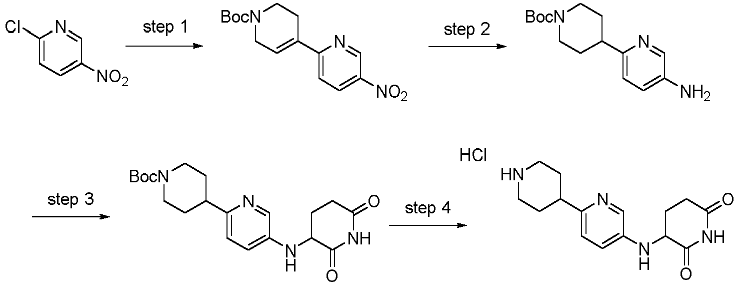

### Step 1: Synthesis of tert-butyl 5-nitro-3',6'-dihydro-[2,4'-bipyridine]-1'(2'H)-carboxylate

2-chloro-5-nitropyridine (1.20 g, 7.56 mmol), tert-butyl 4-(4,4,5,5-tetramethyl-1,3,2-dioxaborolan-2-yl)-3,6-dihydropyridine-1(2H)-carboxylate (2.52 g, 8.28 mmol), Pd(dppf)Cl₂ (302 mg, 0.37 mmol), and K₂CO₃ (2.09 g, 15.12 mmol) were suspended in 1,4-dioxane : H₂O=5 : 1 (24 mL) and stirred at 100 °C for 16 hours. The reaction solution was filtered through Celite and concentrated under reduced pressure. The resulting residue was subjected to MPLC (0-60% EtOAc/Hexane) to obtain 1.53 g (66%) of a white solid.

### Step 2: Synthesis of tert-butyl 4-(5-aminopyridin-2-yl)piperidine-1-carboxylate

Tert-butyl 5-nitro-3',6'-dihydro-[2,4'-bipyridine]-1'(2'H)-carboxylate (1.53 g, 5.01 mmol) was dissolved inMeOH (25 mL). Then, 10% Pd/C (153 mg) was added and stirred at room temperature under a hydrogen stream for 4 hours. The reaction solution was filtered through Celite and concentrated under reduced pressure to obtain 1.39 g (100%) of a bright white solid.

### Step 3: Synthesis of tert-butyl 4-(5-((2,6-dioxopiperidin-3-yl)amino)pyridin-2-yl)piperidine-1-carboxylate

Tert-butyl 4-(5-aminopyridin-2-yl)piperidine-1-carboxylate (1.39 g, 5.01 mmol), 3-bromopiperidine-2,6-dione (1.15 g, 6.01 mmol), and NaHCO₃ (840 mg, 10.0 mmol) were suspended in DMF (25 mL) and stirred at 60 °C for 16 hours. Distilled water (30 mL) was added to the reaction solution, and then extracted with EtOAc (30 mL x 3). The organic layer was dried over anhydrous sodium sulfate, filtered, and concentrated under reduced pressure. The resulting residue was subjected to MPLC (0-100% EtOAc/Hexane) to obtain 373 mg (20.0%) of a green solid.

### Step 4: Synthesis of 3-((6-(piperidin-4-yl)pyridin-3-yl)amino)piperidine-2,6-dione hydrochloride

Tert-butyl 4-(5-((2, 6-dioxopiperidin-3-yl)amino)pyridin-2-yl)piperidine-1-carboxylate (373 mg, 0.96 mmol) and 4N HCl in dioxane (1.5 mL) were suspended in DCM (3 mL) and stirred at room temperature for 6 hours. The reaction solution was concentrated under reduced pressure to obtain 350 mg (112.0%) of a gray solid.

### Intermediate 3-20: 3-((6-(piperazin-1-yl)pyridin-3-yl)amino)piperidine-2,6-dione hydrochloride

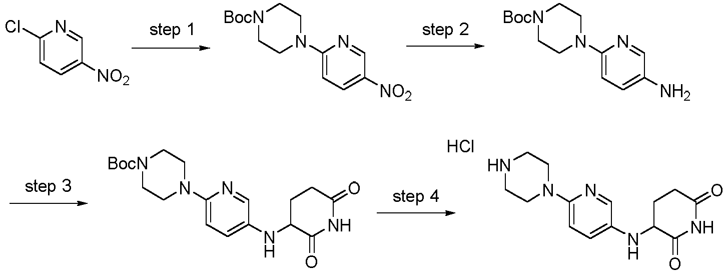

### Step 1: Synthesis of tert-butyl 4-(5-nitropyridin-2-yl)piperazine-1-carboxylate

2-chloro-5-nitropyridine (1.0 g, 6.30 mmol), tert-butyl piperazine-1-carboxylate (2.90 g, 9.40 mmol), and DIPEA (3.3 mL, 19.0 mmol) were suspended in DMF (30 mL) and stirred at 100 °C for 16 hours. Brine (50 mL) was added to the reaction solution and extracted with EtOAc (30 mL x 4). The organic layer was dried over anhydrous sodium sulfate, filtered, and concentrated under reduced pressure. The resulting residue was triturated (EtOAc/Hexane) to obtain 1.86 g (96%) of a bright yellow solid.

### Step 2: Synthesis of tert-butyl 4-(5-aminopyridin-2-yl)piperazine-1-carboxylate

Tert-butyl 4-(5-nitropyridin-2-yl)piperazine-1-carboxylate (1.86 g, 6.03 mmol) was dissolved in MeOH (30 mL). Then, 10% Pd/C (186 mg) was added and stirred for 4 hours at room temperature under a hydrogen stream. The reaction solution was filtered through Celite and concentrated under reduced pressure to obtain 1.58 g (94%) of a brown solid.

### Step 3: Synthesis of tert-butyl 4-(5-((2,6-dioxopiperidin-3-yl)amino)pyridin-2-yl)piperazine-1-carboxylate

Tert-butyl 4-(5-aminopyridin-2-yl)piperazine-1-carboxylate (1.58 g, 5.67 mmol), 3-bromopiperidine-2,6-dione (1.30 g, 6.80 mmol), and NaHCO₃ (954 mg, 11.35 mmol) were suspended in DMF (28 mL) and stirred at 60 °C for 16 hours. Distilled water (30 mL) was added to the reaction solution, and then extracted with EtOAc (30 mL x 3). The organic layer was dried over anhydrous sodium sulfate, filtered, and concentrated under reduced pressure. The resulting residue was subjected to MPLC (0-100% EtOAc/Hexane) to obtain 723 mg (33%) of a brown solid.

### Step 4: Synthesis of 3-((6-(piperazin-1-yl)pyridin-3-yl)amino)piperidine-2,6-dione hydrochloride

Tert-butyl 4-(5-((2, 6-dioxopiperidin-3-yl)amino)pyridin-2-yl)piperazine-1-carboxylate (723 mg, 1.85 mmol) and 4N HCl in dioxane (3 mL) were suspended in DCM (6 mL) and stirred at room temperature for 6 hours. The reaction solution was concentrated under reduced pressure to obtain 667 mg (110%) of a gray solid.

### Intermediates 3-21 to 3-22 were synthesized using the same method as the synthesis of Intermediate 3-20.

| | |
|---|---|
| Intermediate 3-21: 3-((3-(piperazin-1-yl)phenyl)amino)piperidine-2,6-dione hydrochloride | 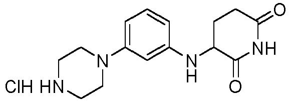 |
| Intermediate 3-22: 3-((4-(piperazin-1-yl)phenyl)amino)piperidine-2,6-dione hydrochloride | 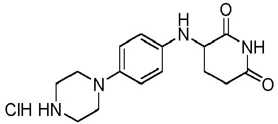 |

### Intermediate 3-23: 1-(5-((2,6-dioxopiperidin-3-yl)amino)pyridin-2-yl)piperidine-4-carbaldehyde

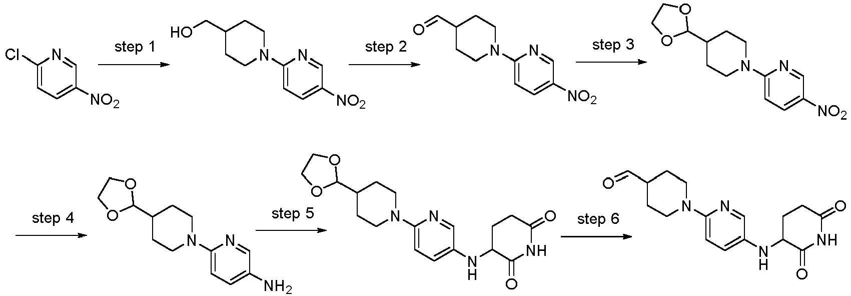

### Step 1: Synthesis of (1-(5-nitropyridin-2-yl)piperidin-4-yl)methanol

2-chloro-5-nitropyridine (500 mg, 3.15 mmol) and piperidin-4-ylmethanol (363 mg, 3.15 mmol) were suspended in EtOH (15 mL) and stirred at 80 °C for 3 hours. The reaction solution was concentrated under reduced pressure, and the resulting residue was subjected to MPLC (50% EtOAc/Hexane) to obtain 335 mg (45%) of a yellow solid.

### Step 2: Synthesis of 1-(5-nitropyridin-2-yl)piperidine-4-carbaldehyde

(1-(5-nitropyridin-2-yl)piperidin-4-yl)methanol (332 mg, 1.4 mmol) was suspended in DCM (10 mL). Then, DMP (712 mg, 1.7 mmol) was added and stirred at room temperature for 50 minutes. NaHCO₃ aqueous solution (30 mL) was added to the reaction solution, and then extracted with DCM (30 mL). The organic layer was dried with anhydrous magnesium sulfate, filtered, and concentrated under reduced pressure. The resulting residue was subjected to MPLC (30% EtOAc/Hexane) to obtain 318 mg (97%) of a yellow solid.

### Step 3: Synthesis of 2-(4-(1,3-dioxolan-2-yl)piperidin-1-yl)-5-nitropyridine

1-(5-nitropyridin-2-yl)piperidine-4-carbaldehyde (318 mg, 1.35 mmol), ethylene glycol (167 mg, 2.7 mmol), and pyridinium p-toluenesulfonate (35 mg, 0.14 mmol) were suspended in toluene (10 mL) and stirred at 115 °C for 1 hour. Distilled water (40 mL) was added to the reaction solution, and then extracted with EtOAc (40 mL). The organic layer was dried over anhydrous sodium sulfate, filtered, and concentrated under reduced pressure. The resulting residue was subjected to MPLC (25% EtOAc/Hexane) to obtain 199 mg (53%) of a yellow solid.

### Step 4: Synthesis of 6-(4-(1,3-dioxolan-2-yl)piperidin-1-yl)pyridin-3-amine

2-(4-(1,3-dioxolan-2-yl)piperidin-1-yl)-5-nitropyridine (192 mg, 0.69 mmol), Fe (192 mg, 3.45 mmol), and NH₄Cl (37 mg, 0.69 mmol) were suspended in EtOH (7 mL) and H₂O (2 mL) and stirred at 70 °C for 1.5 hours. The reaction solution was concentrated under reduced pressure, NaHCO₃ aqueous solution (30 mL) was added, and extracted with EtOAc (30 mL). The organic layer was filtered through Celite and concentrated under reduced pressure. The resulting residue was subjected to MPLC (3% MeOH/DCM) to obtain 126 mg (73%) of a dark red solid.

### Step 5: Synthesis of 3-((6-(4-(1,3-dioxolan-2-yl)piperidin-1-yl)pyridin-3-yl)amino)piperidine-2,6-dione

6-(4-(1,3-dioxolan-2-yl)piperidin-1-yl)pyridin-3-amine (124 mg, 0.5 mmol), 3-bromopiperidine-2,6-dione (115 mg, 0.6 mmol), and NaHCO₃ (84 mg, 1 mmol) were suspended in DMF (2.5 mL) and stirred at 60 °C for 18 hours. Distilled water (40 mL) was added to the reaction solution, and then extracted with EtOAc (40 mL). The organic layer was dried over anhydrous sodium sulfate, filtered, and concentrated under reduced pressure. The resulting residue was subjected to MPLC (5% MeOH/DCM) to obtain 81 mg (45%) of a purple solid.

### Step 6: Synthesis of 1-(5-((2,6-dioxopiperidin-3-yl)amino)pyridin-2-yl)piperidine-4-carbaldehyde

3-((6-(4-(1,3-dioxolan-2-yl)piperidin-1-yl)pyridin-3-yl)amino)piperidine-2,6-dione (79 mg, 0.22 mmol) was suspended in DCM (2 mL). Then, TFA (1 mL) was added and stirred at 50 °C for 15 hours. The reaction solution was concentrated under reduced pressure, and the resulting residue was subjected to MPLC (10% MeOH/DCM) to obtain 56 mg (81%) of a purple solid.

### Intermediates 3-24 to 3-26 were synthesized using the same method as the synthesis of Intermediate 3-23.

| | |
|---|---|
| Intermediate 3-24: 1-(4-((2,6-dioxopiperidin-3-yl)amino)phenyl)piperidine-4-carbaldehyde | 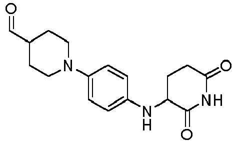 |
| Intermediate 3-25: 1-(3-((2,6-dioxopiperidin-3-yl)amino)phenyl)piperidine-4-carbaldehyde | 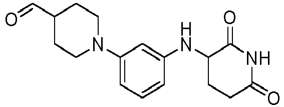 |
| Intermediate 3-26: 1-(4-((2,6-dioxopiperidin-3-yl)amino)-2-fluorophenyl)piperidine-4-carbaldehyde | 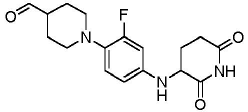 |

### Intermediate 3-27: 3-((6-(piperidin-4-yl)pyridin-3-yl)oxy)piperidine-2,6-dione hydrochloride

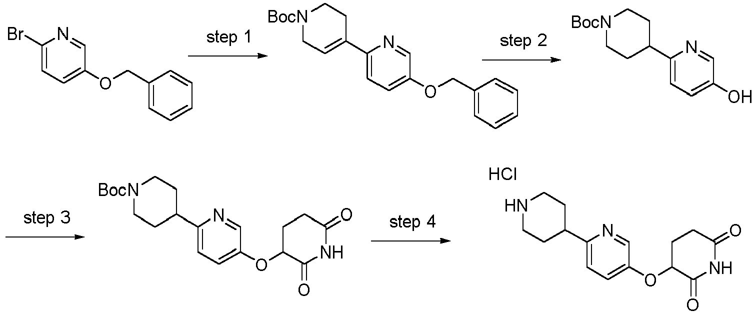

### Step 1: Synthesis of tert-butyl 5-(benzyloxy)-3',6'-dihydro-[2,4'-bipyridine]-1'(2'H)-carboxylate

5-(benzyloxy)-2-bromopyridine (500 mg, 1.89 mmol), tert-butyl 4-(4,4,5,5-tetramethyl-1,3,2-dioxaborolan-2-yl)-3,6-dihydropyridine-1(2H)-carboxylate (702 mg, 2.27 mmol), Pd(dppf)Cl₂ (28 mg, 2 mol%), and K₂CO₃ (758 mg, 5.68 mmol) were suspended in dioxane (20 mL) and H₂O (5 mL), and stirred at 90 °C for 2 hours. Distilled water (80 mL) was added to the reaction solution and extracted with EtOAc (20 mL x 3). The organic layer was dried over anhydrous sodium sulfate, filtered, and concentrated under reduced pressure. The resulting residue was subjected to MPLC (20-50% EtOAc/Hexane) to obtain 728 mg (105%) of a white solid.

### Step 2: Synthesis of tert-butyl 4-(5-hydroxypyridin-2-yl)piperidine-1-carboxylate

Tert-butyl 5-(benzyloxy)-3',6'-dihydro-[2,4'-bipyridine]-1'(2'H)-carboxylate (728 mg, 1.99 mmol) was suspended in MeOH (30 mL). Then, 10% Pd/C (106 mg) was added and stirred for 16 hours at room temperature under a hydrogen stream. The reaction solution was filtered through Celite and concentrated under reduced pressure to obtain 550 mg (99%) of a gray solid.

### Step 3: Synthesis of tert-butyl 4-(5-((2,6-dioxopiperidin-3-yl)oxy)pyridin-2-yl)piperidine-1-carboxylate

Tert-butyl 4-(5-hydroxypyridin-2-yl)piperidine-1-carboxylate (450 mg, 1.62 mmol) was suspended in THF (30 mL). Then, NaH (129 mg, 3.23 mmol) was added and stirred for 10 minutes. Next, 3-bromopiperidine-2,6-dione (372 mg, 1.94 mmol) was added and stirred at 60 °C for 6 hours. 3-bromopiperidine-2,6-dione (94 mg, 0.49 mmol) was additionally added and stirred at 45 °C for 16 hours. Distilled water (80 mL) was added to the reaction solution and extracted with EtOAc (20 mL x 3). The organic layer was dried over anhydrous sodium sulfate, filtered, and concentrated under reduced pressure. The resulting residue was subjected to MPLC (1-4% MeOH/DCM) to obtain 233 mg (37%) of a white solid.

### Step 4: Synthesis of 3-((6-(piperidin-4-yl)pyridin-3-yl)oxy)piperidine-2,6-dione hydrochloride

Tert-butyl 4-(5-((2,6-dioxopiperidin-3-yl)oxy)pyridin-2-yl)piperidine-1-carboxylate (233 mg, 0.60 mmol) was suspended in DCM (4 mL). Then, 4M HCl in dioxane (0.5 mL) was added and stirred at room temperature for 4 hours. The reaction solution was concentrated under reduced pressure to obtain 227 mg (116%) of a light yellow solid.

### Intermediate 3-28: 3-((6-(piperazin-1-yl)pyridin-3-yl)oxy)piperidine-2,6-dione hydrochloride

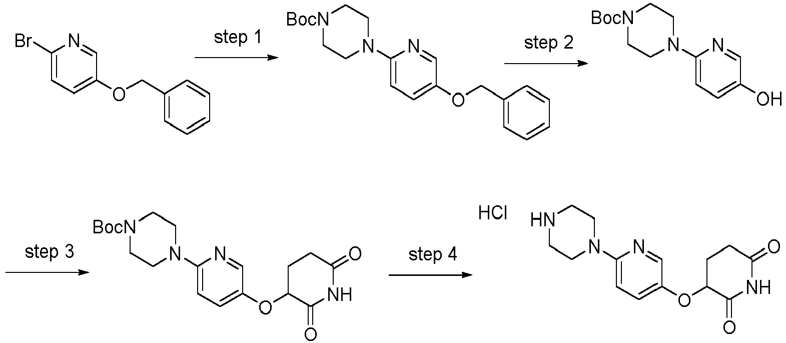

### Step 1: Synthesis of tert-butyl 4-(5-(benzyloxy)pyridin-2-yl)piperazine-1-carboxylate

5-(benzyloxy)-2-bromopyridine (880 mg, 3.33 mmol), tert-butyl piperazine-1-carboxylate acetic acid (984 mg, 4.00 mmol), Pd₂(dba)₃ (610 mg, 0.67 mmol), BINAP (415 mg, 0.67 mmol), and sodium tert-butoxide (640 mg, 6.66 mmol) were suspended in toluene (40 mL) and stirred at 100 °C for 16 hours. Distilled water (100 mL) was added to the reaction solution and extracted with EtOAc (20 mL x 3). The organic layer was dried over anhydrous sodium sulfate, filtered, and concentrated under reduced pressure. The resulting residue was subjected to MPLC (10-30% EtOAc/Hexane) to obtain 1.07 g (87%) of a yellow solid.

### Step 2: Synthesis of tert-butyl 4-(5-hydroxypyridin-2-yl)piperazine-1-carboxylate

Tert-butyl 4-(5-(benzyloxy)pyridin-2-yl)piperazine-1-carboxylate (1.07 g, 2.90 mmol) was suspended in MeOH (40 mL). Then, 10% Pd/C (0.15 g) was added and stirred for 16 hours at room temperature under a hydrogen stream. The reaction solution was filtered through Celite and concentrated under reduced pressure to obtain 0.83 g (102%) of a gray solid.

### Step 3: Synthesis of tert-butyl 4-(5-((2,6-dioxopiperidin-3-yl)oxy)pyridin-2-yl)piperazine-1-carboxylate

Tert-butyl 4-(5-hydroxypyridin-2-yl)piperazine-1-carboxylate (400 mg, 1.43 mmol) was suspended in THF (25 mL). Then, NaH (115 mg, 2.86 mmol) was added and stirred for 10 minutes. 3-bromopiperidine-2,6-dione (330 mg, 1.72 mmol) was added and stirred at 60 °C for 2 hours. Distilled water (80 mL) was added to the reaction solution and extracted with EtOAc (20 mL x 3). The organic layer was dried over anhydrous sodium sulfate, filtered, and concentrated under reduced pressure. The resulting residue was subjected to MPLC (1-4% MeOH/DCM) to obtain 258 mg (46%) of a bright yellow solid.

### Step 4: Synthesis of 3-((6-(piperazin-1-yl)pyridin-3-yl)oxy)piperidine-2,6-dione hydrochloride

Tert-butyl 4-(5-((2,6-dioxopiperidin-3-yl)oxy)pyridin-2-yl)piperazine-1-carboxylate (258 mg, 0.66 mmol) was suspended in DCM (4 mL). 4M HCl in dioxane (0.5 mL) was added and stirred at room temperature for 4 hours. The reaction solution was concentrated under reduced pressure to obtain 281 mg (130%) of a white solid.

### Intermediate 3-29: 1-(4-(2,4-dioxotetrahydropyrimidin-1(2H)-yl)phenyl)piperidine-4-carbaldehyde

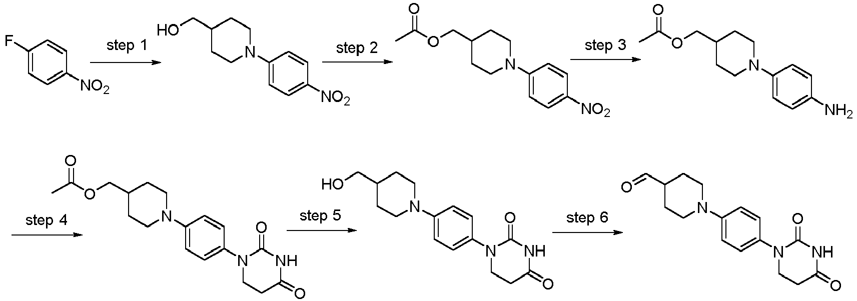

### Step 1: Synthesis of (1-(4-nitrophenyl)piperidin-4-yl)methanol

1-fluoro-4-nitrobenzene (3.0 g, 21.26 mmol), piperidin-4-ylmethanol (2.93 g, 25.50 mmol), and DIPEA (7.40 mL, 42.52 mmol) were suspended in DMF (5 mL) and stirred in a microwave at 120 °C for 2 hours. Distilled water (40 mL) was added to the reaction solution and extracted with EtOAc (30 mL x 3). The organic layer was washed with brine (30 mL), dried over anhydrous sodium sulfate, filtered, and concentrated under reduced pressure. The resulting residue was triturated (EtOAc/Hexane) to obtain 4.90 g (98%) of a yellow solid.

### Step 2: Synthesis of (1-(4-nitrophenyl)piperidin-4-yl)methyl acetate

(1-(4-nitrophenyl)piperidin-4-yl)methanol (2.50 g, 8.98 mmol) and acetyl chloride (0.81 mL, 11.42 mmol) were suspended in EtOH (45 mL). Then, TEA (2.65 mL, 19.0 mmol) was slowly added and stirred at room temperature for 1 hour. Distilled water (50 mL) was added to the reaction solution and extracted with DCM (30 mL x 3). The organic layer was dried over anhydrous sodium sulfate, filtered, and concentrated under reduced pressure. The resulting residue was subjected to MPLC (0-40% EtOAc/Hexane) to obtain 2.5 g (85%) of a yellow solid.

### Step 3: Synthesis of (1-(4-aminophenyl)piperidin-4-yl)methyl acetate

(1-(4-nitrophenyl)piperidin-4-yl)methyl acetate (2.50 g, 8.98 mmol) was dissolved in EtOH (45 mL). Then, 10% Pd/C (250 mg) was added and stirred for 16 hours at room temperature under a hydrogen stream. The reaction solution was filtered through Celite and concentrated under reduced pressure. The resulting residue was triturated (Ether/Hexane) to obtain 1.78 g (80%) of a brown solid.

### Step 4: Synthesis of (1-(4-(2,4-dioxotetrahydropyrimidin-1(2H)-yl)phenyl)piperidin-4-yl)methyl acetate

(1-(4-aminophenyl)piperidin-4-yl)methyl acetate (1.78 g, 7.16 mmol) was dissolved in toluene (12 mL). Then, acrylic acid (0.60 mL, 8.60 mmol) was added and stirred at 50 °C for 16 hours. Urea (2.15 g, 35.8 mmol) and AcOH (10 mL) were added to the reaction solution and stirred at 105 °C for 16 hours. The reaction solution was concentrated under reduced pressure, and the resulting residue was subjected to MPLC (0-100% EtOAc/Hexane) to obtain 860 mg (35%) of a bright pink solid.

### Step 5: Synthesis of 1-(4-(4-(hydroxymethyl)piperidin-1-yl)phenyl)dihydropyrimidine-2,4(1H,3H)-dione

(1-(4-(2,4-dioxotetrahydropyrimidin-1(2H)-yl)phenyl)piperidin-4-yl)methyl acetate (850 mg, 2.46 mmol) was suspended in 2N HCl (12 mL) and stirred at 100 °C for 2 hours. The reaction solution was neutralized with NaHCO₃ aqueous solution, and the resulting solid was filtered to obtain 630 mg (84%) of a pink solid.

### Step 6: Synthesis of 1-(4-(2,4-dioxotetrahydropyrimidin-1(2H)-yl)phenyl)piperidine-4-carbaldehyde

1-(4-(4-(hydroxymethyl)piperidin-1-yl)phenyl)dihydropyrimidine-2,4(1H,3H)-dione (90 mg, 0.29 mmol) was suspended in DCM (2 mL). Then, DMP (150 mg, 4.23 mmol) was added and stirred at room temperature for 15 minutes. NaHCO₃ aqueous solution (80 mL) was added to the reaction solution and extracted with DCM (20 mL x 3). The organic layer was dried over anhydrous sodium sulfate, filtered, and concentrated under reduced pressure. The resulting residue was subjected to MPLC (0-90% EtOAc/Hexane) to obtain 55 mg (62%) of a pink solid.

### Intermediates 3-30 to 3-31 were synthesized using the same method as the synthesis of Intermediate 3-29.

| | |
|---|---|
| Intermediate 3-30: 1-(3-(2,4-dioxotetrahydropyrimidin-1(2H)-yl)phenyl)piperidine-4-carbaldehyde | 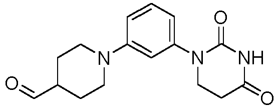 |
| Intermediate 3-31: 1-(4-(2,4-dioxotetrahydropyrimidin-1(2H)-yl)-2-fluorophenyl)piperidine-4-carbaldehyde | 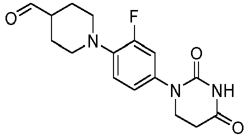 |

### Intermediate 3-32: 1-(3-(piperidin-4-yl)phenyl)dihydropyrimidine-2,4(1H,3H)-dione

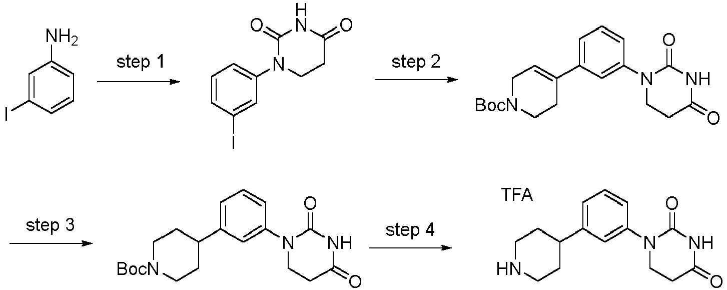

### Step 1: Synthesis of 1-(3-iodophenyl)dihydropyrimidine-2,4(1H,3H)-dione

3-iodoaniline (3.0 g, 13.69 mmol) was suspended in toluene (23 mL). Then, acrylic acid (1.12 mL, 16.43 mmol) was added and stirred at 70 °C for 48 hours. AcOH (34 mL) and urea (2.46 g, 13.69 mmol) were added to the reaction solution and stirred at 105 °C for 16 hours. The reaction solution was concentrated under reduced pressure, distilled water (60 mL) was added, and extracted with EtOAc (30 mL x 4). The organic layer was dried over anhydrous sodium sulfate, filtered, and concentrated under reduced pressure. The resulting residue was subjected to MPLC (0-100% EtOAc/Hexane) to obtain 2.20 g (51%) of a white solid.

### Step 2: Synthesis of tert-butyl 4-(3-(2,4-dioxotetrahydropyrimidin-1(2H)-yl)phenyl)-3,6-dihydropyridine-1(2H)-carboxylate

1-(3-iodophenyl)dihydropyrimidine-2,4(1H,3H)-dione (200 mg, 0.63 mmol), tert-butyl 4-(4,4,5,5-tetramethyl-1,3,2-dioxaborolan-2-yl)-3,6-dihydropyridine-1(2H)-carboxylate (216 mg, 0.70 mmol), Pd(dppf)Cl₂ (22 mg, 0.03 mmol), and K₂CO₃ (174 mg, 1.26 mmol) were suspended in 1,4-dioxane : H₂O=5 : 1 (2 mL) and stirred at 100 °C for 1.5 hours. The reaction solution was filtered through Celite and concentrated under reduced pressure. The resulting residue was subjected to MPLC (0-70% EtOAc/Hexane) to obtain 190 mg (81%) of a white solid.

### Step 3: Synthesis of tert-butyl 4-(3-(2,4-dioxotetrahydropyrimidin-1(2H)-yl)phenyl)pi peridine-1-carboxylate

Tert-butyl 4-(3-(2,4-dioxotetrahydropyrimidin-1(2H)-yl)phenyl)-3,6-dihydropyridine-1(2H)-carboxylate (188 mg, 0.50 mmol) was dissolved in EtOH (15 mL). Then, 10% Pd/C (18 mg) was added and stirred for 16 hours at room temperature under a hydrogen stream. The reaction solution was filtered through Celite and concentrated under reduced pressure to obtain 147 mg (78.7%) of a bright yellow solid.

### Step 4: Synthesis of 1-(3-(piperidin-4-yl)phenyl)dihydropyrimidine-2,4(1H,3H)-dione

Tert-butyl 4-(3-(2,4-dioxotetrahydropyrimidin-1(2H)-yl)phenyl)piperidine-1-carboxylate (147 mg, 0.39 mmol) and TFA (0.9 mL) were suspended in DCM (6 mL) and stirred at room temperature for 4 hours. The reaction solution was concentrated under reduced pressure to obtain 104 mg (72%) of a light yellow solid.

### Intermediate 3-33 was synthesized using the same method as the synthesis of Intermediate 3-32.

| | |
|---|---|
| Intermediate 3-33: 1-(4-(piperidin-4-yl)phenyl)dihydropyrimidine-2,4(1H,3H)-dione | 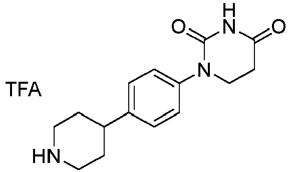 |

### Example 1: N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-3-isopropoxyazetidine-1-carboxamide

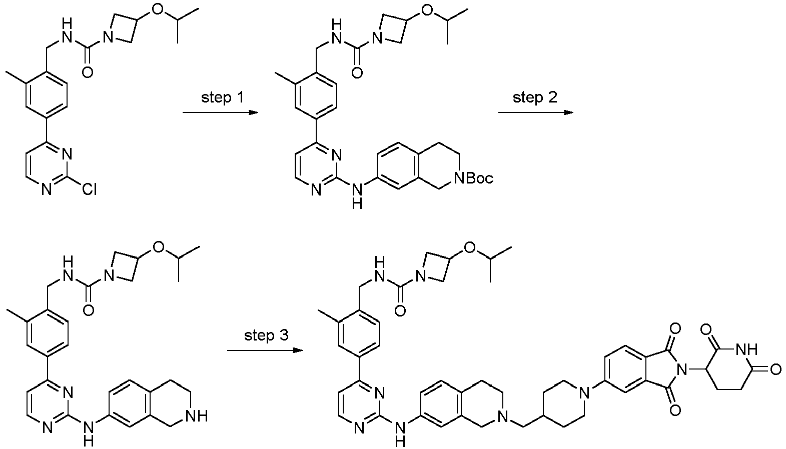

### Step 1: Synthesis of tert-butyl 7-((4-(4-((3-isopropoxyazetidine-1-carboxamido)methyl)-3-methylphenyl)pyrimidin-2-yl)amino)-3,4-dihydroisoquinoline-2(1H)-carboxylate

N-(4-(2-chloropyrimidin-4-yl)-2-methylbenzyl)-3-isopropoxyazetidine-1-carboxamide (Intermediate 1-1, 0.15 g, 0.40 mmol), tert-butyl 7-amino-3,4-dihydroisoquinoline-2(1H)-carboxylate (0.12 g, 0.48 mmol), Pd(OAc)₂ (0.02 g, 0.08 mmol), BINAP (0.05 g, 0.08 mmol), and Cs₂CO₃ (0.33 g, 0.80 mmol) were suspended in dioxane (3 mL) and stirred at 100 °C for 16 hours. Distilled water (30 mL) was added to the reaction solution, and then extracted with EtOAc (10 mL x 3). The organic layer was dried over anhydrous sodium sulfate, filtered, and concentrated under reduced pressure. The resulting residue was subjected to MPLC (80-100% EtOAc/Hexane) to obtain 0.11 g (46%) of a yellow solid.

### Step 2: Synthesis of 3-isopropoxy-N-(2-methyl-4-(2-((1,2,3,4-tetrahydroisoquinolin-7-yl)amino)pyrimidin-4-yl)benzyl)azetidine-1-carboxamide

Tert-butyl 7-((4-(4-((3-isopropoxyazetidine-1-carboxamido)methyl)-3-methylphenyl)pyrimidin-2-yl)amino)-3,4-dihydroisoquinoline-2(1H)-carboxylate (107 mg, 0.18 mmol) was suspended in DCM (2 mL). Then, 4M HCl in dioxane (2 mL, 8.10 mmol) was added and stirred at room temperature for 4 hours. The reaction solution was filtered and concentrated under reduced pressure to obtain 79 mg (89%) of a yellow solid.

### Step 3: Synthesis of N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-3-isopropoxyazetidine-1-carboxamide

3-isopropoxy-N-(2-methyl-4-(2-((1,2,3,4-tetrahydroisoquinolin-7-yl)amino)pyrimidin-4-yl)benzyl)azetidine-1-carboxamide (79 mg, 0.16 mmol) and 1-(2-(2,6-dioxopiperidin-3-yl)-1,3-dioxoisoindolin-5-yl)piperidine-4-carbaldehyde (Intermediate 3-1, 72 mg, 0.19 mmol) were suspended in DCM (4 mL). Then, NaBH(OAc)₃ (103 mg, 0.49 mmol) was added and stirred at room temperature for 2 hours. NaHCO₃ aqueous solution (30 mL) was added to the reaction solution and extracted with DCM (10 mL x 3). The organic layer was dried over anhydrous sodium sulfate, filtered, and concentrated under reduced pressure. The resulting residue was subjected to MPLC (2-6% MeOH/DCM) to obtain 63 mg (46%) of a yellow solid.

### Example 2: 1-(tert-butyl)-N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-1H-pyrazole-4-carboxamide

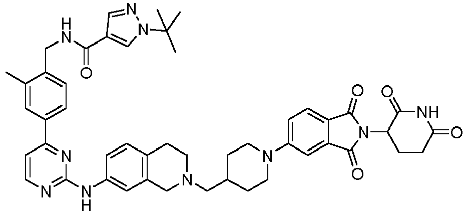

Example 2 was synthesized using the same method as Example 1, using 1-(tert-butyl)-N-(4-(2-chloropyrimidin-4-yl)-2-methylbenzyl)-1H-pyrazole-4-carboxamide (Intermediate 1-4) instead of N-(4-(2-chloropyrimidin-4-yl)-2-methylbenzyl)-3-isopropoxyazetidine-1-carboxamide (Intermediate 1-1).

### Example 3: 1-(tert-butyl)-N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-1H-1,2,3-triazole-4-carboxamide

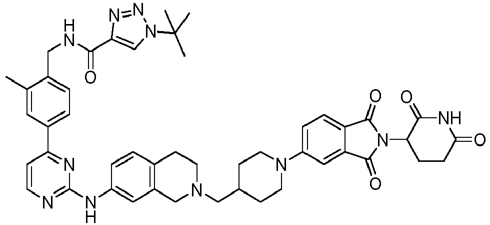

Example 3 was synthesized using the same method as Example 1, using 1-(tert-butyl)-N-(4-(2-chloropyrimidin-4-yl)-2-methylbenzyl)-1H-1,2,3-triazole-4-carboxamide (Intermediate 1-5) instead of N-(4-(2-chloropyrimidin-4-yl)-2-methylbenzyl)-3-isopropoxyazetidine-1-carboxamide (Intermediate 1-1).

### Example 4: N-(3-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-5-fluoro-2-methylphenyl)-3-isopropoxyazetidine-1-carboxamide

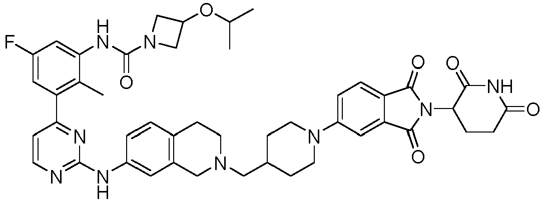

Example 4 was synthesized using the same method as Example 1, using N-(3-(2-chloropyrimidin-4-yl)-5-fluoro-2-methylphenyl)-3-isopropoxyazetidine-1-carboxamide (Intermediate 1-8) instead of N-(4-(2-chloropyrimidin-4-yl)-2-methylbenzyl)-3-isopropoxyazetidine-1-carboxamide (Intermediate 1-1).

### Example 5: 1-(tert-butyl)-N-(3-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-5-fluoro-2-methylphenyl)-1H-pyrazole-4-carboxamide

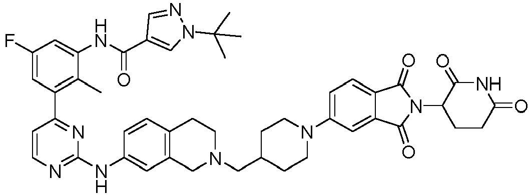

Example 5 was synthesized using the same method as Example 1, using 1-(tert-butyl)-N-(3-(2-chloropyrimidin-4-yl)-5-fluoro-2-methylphenyl)-1H-pyrazole-4-carboxamide (Intermediate 1-9) instead of N-(4-(2-chloropyrimidin-4-yl)-2-methylbenzyl)-3-isopropoxyazetidine-1-carboxamide (Intermediate 1-1).

### Example 6: 1-(tert-butyl)-N-(3-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-5-fluoro-2-methylphenyl)-1H-1,2,3-triazole-4-carboxamide

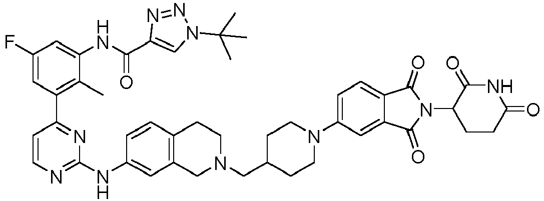

Example 6 was synthesized using the same method as Example 1, using 1-(tert-butyl)-N-(3-(2-chloropyrimidin-4-yl)-5-fluoro-2-methylphenyl)-1H-1,2,3-triazole-4-carboxamide (Intermediate 1-10) instead of N-(4-(2-chloropyrimidin-4-yl)-2-methylbenzyl)-3-isopropoxyazetidine-1-carboxamide (Intermediate 1-1).

### Example 7: N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-3-isopropoxyazetidine-1-carboxamide

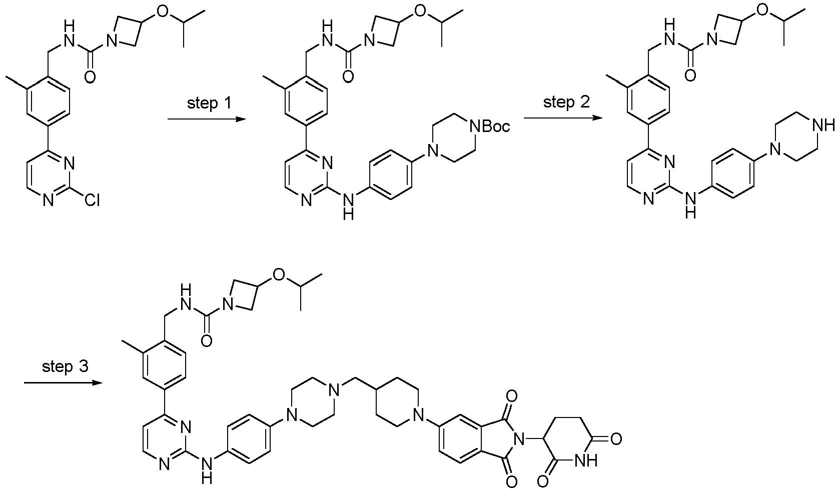

### Step 1: Synthesis of tert-butyl 4-(4-((4-(4-((3-isopropoxyazetidine-1-carboxamido)methyl)-3-methylphenyl)pyrimidin-2-yl)amino)phenyl)piperazine-1-carboxylate

N-(4-(2-chloropyrimidin-4-yl)-2-methylbenzyl)-3-isopropoxyazetidine-1-carboxamide (Intermediate 1-1, 40 mg, 0.11 mmol), tert-butyl 4-(4-aminophenyl)piperazine-1-carboxylate (Intermediate 2-1, 33 mg, 0.12 mmol), Pd₂(dba)₃ (10 mg, 0.01 mmol), S-phos (9 mg, 0.02 mmol), and Cs₂CO₃ (70 mg, 0.21 mmol) were suspended in dioxane (2 mL) and stirred in a microwave at 100 °C for 1 hour. Distilled water (30 mL) was added to the reaction solution, and then extracted with EtOAc (10 mL x 3). The organic layer was dried over anhydrous sodium sulfate, filtered, and concentrated under reduced pressure. The resulting residue was subjected to MPLC (80-100% EtOAc/Hexane) to obtain 29 mg (44%) of a yellow solid.

### Step 2: Synthesis of 3-isopropoxy-N-(2-methyl-4-(2-((4-(piperazin-1-yl)phenyl)amino)pyrimidin-4-yl)benzyl)azetidine-1-carboxamide

Tert-butyl 4-(4-((4-(4-((3-isopropoxyazetidine-1-carboxamido)methyl)-3-methylphenyl)pyrimidin-2-yl)amino)phenyl)piperazine-1-carboxylate (29 mg, 0.05 mmol) was suspended in DCM (2 mL). Then, TFA (0.5 mL) was added and stirred at room temperature for 16 hours. The reaction solution was filtered and concentrated under reduced pressure to obtain 36 mg of a yellow solid.

### Step 3: Synthesis of N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-3-isopropoxyazetidine-1-carboxamide

3-isopropoxy-N-(2-methyl-4-(2-((4-(piperazin-1-yl)phenyl)amino)pyrimidin-4-yl)benzyl)azetidine-1-carboxamide (36 mg, 0.07 mmol) and 1-(2-(2,6-dioxopiperidin-3-yl)-1,3-dioxoisoindolin-5-yl)piperidine-4-carbaldehyde (Intermediate 3-1, 31 mg, 0.08 mmol) were suspended in DCM (4 mL). Then, NaBH(OAc)₃ (44 mg, 0.21 mmol) was added and stirred at room temperature for 16 hours. NaHCO₃ aqueous solution (30 mL) was added to the reaction solution and extracted with DCM (10 mL x 3). The organic layer was dried over anhydrous sodium sulfate, filtered, and concentrated under reduced pressure. The resulting residue was subjected to MPLC (2-5% MeOH/DCM) to obtain 8.8 mg (22%, 2 steps yield) of a yellow solid.

### Example 8: 1-(tert-butyl)-N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1H-pyrazole-4-carboxamide

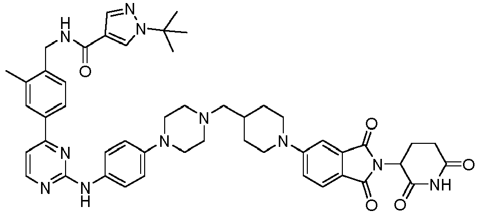

Example 8 was synthesized using the same method as Example 7, using 1-(tert-butyl)-N-(4-(2-chloropyrimidin-4-yl)-2-methylbenzyl)-1H-pyrazole-4-carboxamide (Intermediate 1-4) instead of N-(4-(2-chloropyrimidin-4-yl)-2-methylbenzyl)-3-isopropoxyazetidine-1-carboxamide (Intermediate 1-1).

### Example 9: 1-(tert-butyl)-N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1H-1,2,3-triazole-4-carboxamide

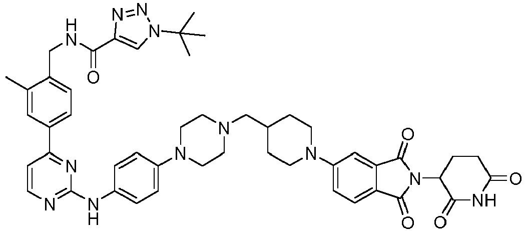

Example 9 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5) instead of N-(4-(2-chloropyrimidin-4-yl)-2-methylbenzyl)-3-isopropoxyazetidine-1-carboxamide (Intermediate 1-1).

### Example 10: N-(4-(2-((4-(4-((1-(6-(2,6-dioxopiperidin-3-yl)-5-oxo-6,7-dihydro-5H-pyrrolo[3,4-b]pyridin-2-yl)piperidin-4-yl)methyl)piperazin-1-yl)phenyl)amino)pyrimidin-4-yl)-2-methylbenzyl)-3-isopropoxyazetidine-1-carboxamide

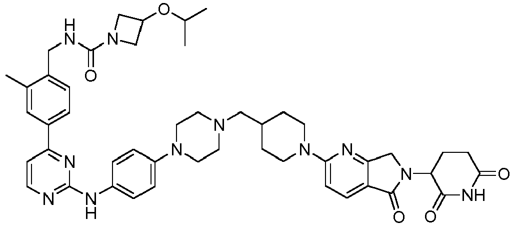

Example 10 was synthesized using the same method as Example 7, using 1-(6-(2,6-dioxopiperidin-3-yl)-5-oxo-6,7-dihydro-5H-pyrrolo[3,4-b]pyridin-2-yl)piperidine-4-carbaldehyde (Intermediate 3-6) instead of 1-(2-(2,6-dioxopiperidin-3-yl)-1,3-dioxoisoindolin-5-yl)piperidine-4-carbaldehyde (Intermediate 3-1).

### Example 11: N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-3-isopropoxyazetidine-1-carboxamide

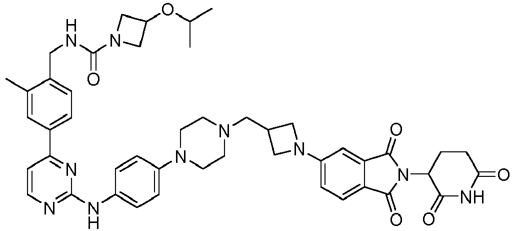

Example 11 was synthesized using the same method as Example 7, using 1-(2-(2,6-dioxopiperidin-3-yl)-1,3-dioxoisoindolin-5-yl)azetidine-3-carbaldehyde (Intermediate 3-2) instead of 1-(2-(2,6-dioxopiperidin-3-yl)-1,3-dioxoisoindolin-5-yl)piperidine-4-carbaldehyde (Intermediate 3-1).

### Example 12: N-(4-(2-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)pyrimidin-4-yl)-2-methylbenzyl)-3-isopropoxyazetidine-1-carboxamide

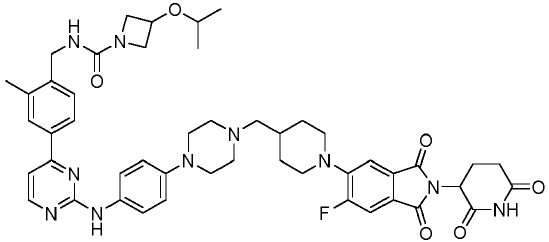

Example 12 was synthesized using the same method as Example 7, using 1-(2-(2,6-dioxopiperidin-3-yl)-6-fluoro-1,3-dioxoisoindolin-5-yl)piperidine-4-carbaldehyde (Intermediate 3-4) instead of 1-(2-(2,6-dioxopiperidin-3-yl)-1,3-dioxoisoindolin-5-yl)piperidine-4-carbaldehyde (Intermediate 3-1).

### Example 13: N-(4-(2-((4-(4-((1-(2-(2,6-dioxopiperidin-3-yl)-6-fluoro-1,3-dioxoisoindolin-5-yl)azetidin-3-yl)methyl)piperazin-1-yl)phenyl)amino)pyrimidin-4-yl)-2-methylbenzyl)-3-isopropoxyazetidine-1-carboxamide

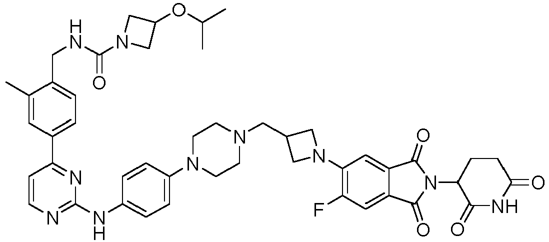

Example 13 was synthesized using the same method as Example 7, using 1-(2-(2,6-dioxopiperidin-3-yl)-6-fluoro-1,3-dioxoisoindolin-5-yl)azetidine-3-carbaldehyde (Intermediate 3-5) instead of 1-(2-(2,6-dioxopiperidin-3-yl)-1,3-dioxoisoindolin-5-yl)piperidine-4-carbaldehyde (Intermediate 3-1).

### Example 14: N-(4-(2-((4-(4-((1-(2-(2,6-dioxopiperidin-3-yl)-1,3-dioxoisoindolin-5-yl)-3-methylazetidin-3-yl)methyl)piperazin-1-yl)phenyl)amino)pyrimidin-4-yl)-2-methylbenzyl)-3-isopropoxyazetidine-1-carboxamide

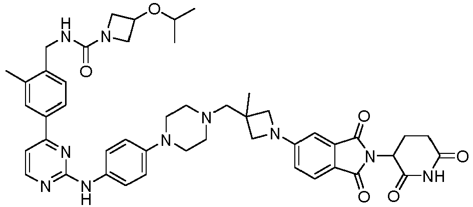

Example 14 was synthesized using the same method as Example 7, using 1-(2-(2,6-dioxopiperidin-3-yl)-1,3-dioxoisoindolin-5-yl)-3-methylazetidine-3-carbaldehyde (Intermediate 3-3) instead of 1-(2-(2,6-dioxopiperidin-3-yl)-1,3-dioxoisoindolin-5-yl)piperidine-4-carbaldehyde (Intermediate 3-1).

### Example 15: N-(4-(2-((4-(1-((1-(2-(2,6-dioxopiperidin-3-yl)-1,3-dioxoisoindolin-5-yl)piperidin-4-yl)methyl)piperidin-4-yl)phenyl)amino)pyrimidin-4-yl)-2-methylbenzyl)-3-isopropoxyazetidine-1-carboxamide

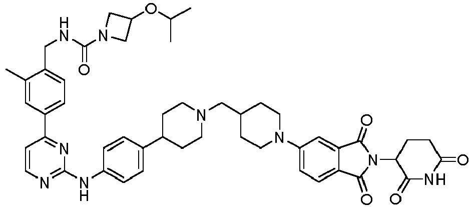

Example 15 was synthesized using the same method as Example 7, using tert-butyl 4-(4-aminophenyl)piperidine-1-carboxylate (Intermediate 2-5) instead of tert-butyl 4-(4-aminophenyl)piperazine-1-carboxylate (Intermediate 2-1).

### Example 16: N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-3-isopropoxyazetidine-1-carboxamide

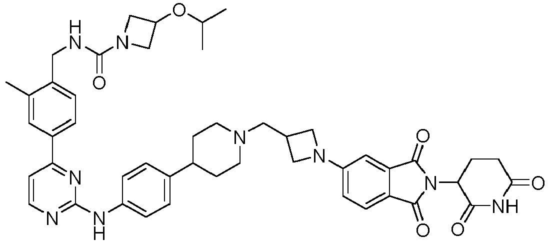

Example 16 was synthesized using the same method as Example 7, using tert-butyl 4-(4-aminophenyl)piperidine-1-carboxylate (Intermediate 2-5) and 1-(2-(2,6-dioxopiperidin-3-yl)-1,3-dioxoisoindolin-5-yl)azetidine-3-carbaldehyde (Intermediate 3-2) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 17: N-(4-(2-((6-(4-((1-(2-(2,6-dioxopiperidin-3-yl)-1,3-dioxoisoindolin-5-yl)piperidin-4-yl)methyl)piperazin-1-yl)pyridin-3-yl)amino)pyrimidin-4-yl)-2-methylbenzyl)-3-isopropoxyazetidine-1-carboxamide

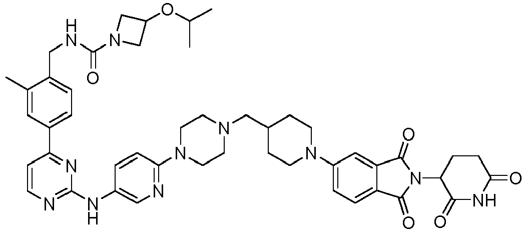

Example 17 was synthesized using the same method as Example 7, using tert-butyl 4-(5-aminopyridin-2-yl)piperazine-1-carboxylate (Intermediate 2-2) instead of tert-butyl 4-(4-aminophenyl)piperazine-1-carboxylate (Intermediate 2-1).

### Example 18: N-(4-(2-((6-(4-((1-(2-(2,6-dioxopiperidin-3-yl)-1,3-dioxoisoindolin-5-yl)azetidin-3-yl)methyl)piperazin-1-yl)pyridin-3-yl)amino)pyrimidin-4-yl)-2-methylbenzyl)-3-isopropoxyazetidine-1-carboxamide

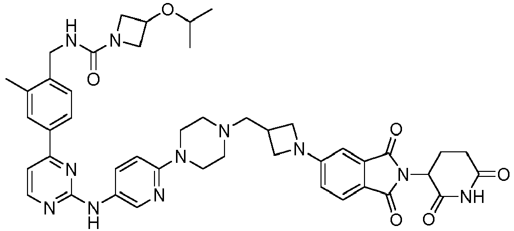

Example 18 was synthesized using the same method as Example 7, using tert-butyl 4-(5-aminopyridin-2-yl)piperazine-1-carboxylate (Intermediate 2-2) and 1-(2-(2,6-dioxopiperidin-3-yl)-1,3-dioxoisoindolin-5-yl)azetidine-3-carbaldehyde (Intermediate 3-2) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 19: N-(4-(2-((6-(1-((1-(2-(2,6-dioxopiperidin-3-yl)-1,3-dioxoisoindolin-5-yl)piperidin-4-yl)methyl)piperidin-4-yl)pyridin-3-yl)amino)pyrimidin-4-yl)-2-methylbenzyl)-3-isopropoxyazetidine-1-carboxamide

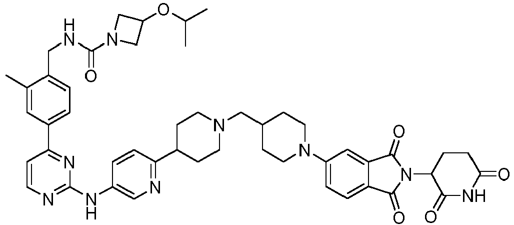

Example 19 was synthesized using the same method as Example 7, using tert-butyl 4-(5-aminopyridin-2-yl)piperidine-1-carboxylate (Intermediate 2-6) instead of tert-butyl 4-(4-aminophenyl)piperazine-1-carboxylate (Intermediate 2-1).

### Example 20: N-(4-(2-((6-(1-((1-(2-(2,6-dioxopiperidin-3-yl)-1,3-dioxoisoindolin-5-yl)azetidin-3-yl)methyl)piperidin-4-yl)pyridin-3-yl)amino)pyrimidin-4-yl)-2-methylbenzyl)-3-isopropoxyazetidine-1-carboxamide

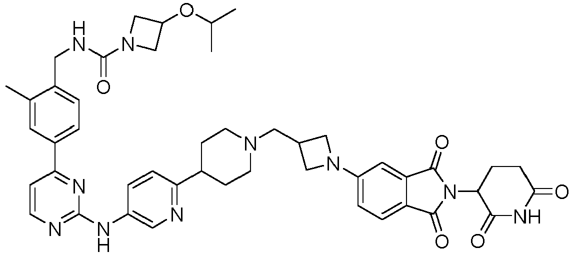

Example 20 was synthesized using the same method as Example 7, using tert-butyl 4-(5-aminopyridin-2-yl)piperidine-1-carboxylate (Intermediate 2-6) and 1-(2-(2,6-dioxopiperidin-3-yl)-1,3-dioxoisoindolin-5-yl)azetidine-3-carbaldehyde (Intermediate 3-2) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 21: N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-3-isopropoxyazetidine-1-carboxamide

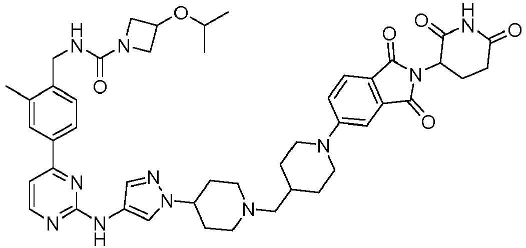

Example 21 was synthesized using the same method as Example 7, using tert-butyl 4-(4-amino-1H-pyrazol-1-yl)piperidine-1-carboxylate (Intermediate 2-7) instead of tert-butyl 4-(4-aminophenyl)piperazine-1-carboxylate (Intermediate 2-1).

### Example 22: N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-3-isopropoxyazetidine-1-carboxamide

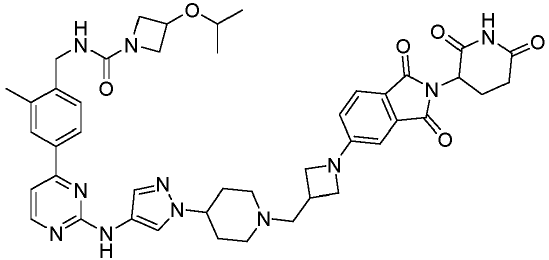

Example 22 was synthesized using the same method as Example 7, using tert-butyl 4-(4-amino-1H-pyrazol-1-yl)piperidine-1-carboxylate (Intermediate 2-7) and 1-(2-(2,6-dioxopiperidin-3-yl)-1,3-dioxoisoindolin-5-yl)azetidine-3-carbaldehyde (Intermediate 3-2) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 23: N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-fluoro-2-methylphenyl)-3-isopropoxyazetidine-1-carboxamide

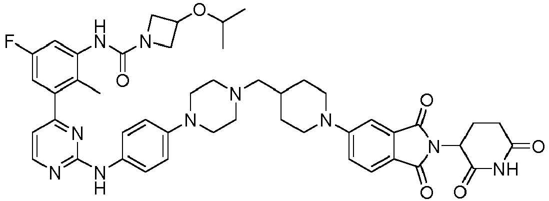

Example 23 was synthesized using the same method as Example 7, using N-(3-(2-chloropyrimidin-4-yl)-5-fluoro-2-methylphenyl)-3-isopropoxyazetidine-1-carboxamide (Intermediate 1-8) instead of N-(4-(2-chloropyrimidin-4-yl)-2-methylbenzyl)-3-isopropoxyazetidine-1-carboxamide (Intermediate 1-1).

### Example 24: 1-(tert-butyl)-N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-fluoro-2-methylphenyl)-1H-pyrazole-4-carboxamide

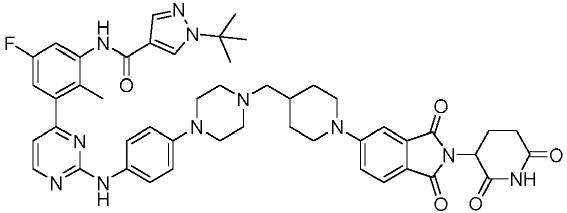

Example 24 was synthesized using the same method as Example 7, using 1-(tert-butyl)-N-(3-(2-chloropyrimidin-4-yl)-5-fluoro-2-methylphenyl)-1H-pyrazole-4-carboxamide (Intermediate 1-9) instead of N-(4-(2-chloropyrimidin-4-yl)-2-methylbenzyl)-3-isopropoxyazetidine-1-carboxamide (Intermediate 1-1).

### Example 25: 1-(tert-butyl)-N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-fluoro-2-methylphenyl)-1H-1,2,3-triazole-4-carboxamide

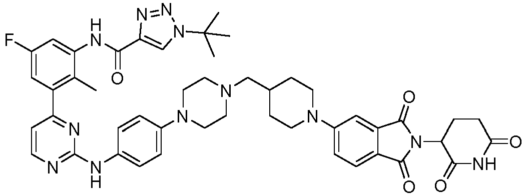

Example 25 was synthesized using the same method as Example 7, using 1-(tert-butyl)-N-(3-(2-chloropyrimidin-4-yl)-5-fluoro-2-methylphenyl)-1H-1,2,3-triazole-4-carboxamide (Intermediate 1-10) instead of N-(4-(2-chloropyrimidin-4-yl)-2-methylbenzyl)-3-isopropoxyazetidine-1-carboxamide (Intermediate 1-1).

### Example 26: N-(3-(2-((4-(4-((1-(6-(2,6-dioxopiperidin-3-yl)-5-oxo-6,7-dihydro-5H-pyrrolo[3,4-b]pyridin-2-yl)piperidin-4-yl)methyl)piperazin-1-yl)phenyl)amino)pyrimidin-4-yl)-5-fluoro-2-methylphenyl)-3-isopropoxyazetidine-1-carboxamide

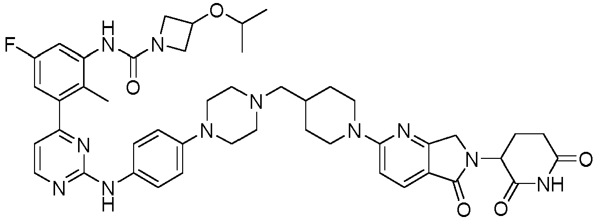

Example 26 was synthesized using the same method as Example 7, using N-(3-(2-chloropyrimidin-4-yl)-5-fluoro-2-methylphenyl)-3-isopropoxyazetidine-1-carboxamide (Intermediate 1-8) and 1-(6-(2,6-dioxopiperidin-3-yl)-5-oxo-6,7-dihydro-5H-pyrrolo[3,4-b]pyridin-2-yl)piperidine-4-carbaldehyde (Intermediate 3-6) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 27: N-(3-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-5-fluoro-2-methylphenyl)-3 -isopropoxy azetidine-1 -carboxamide

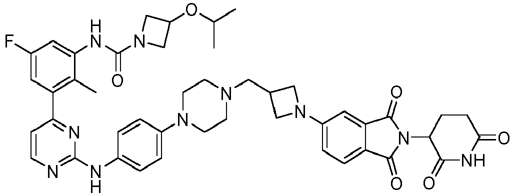

Example 27 was synthesized using the same method as Example 7, using N-(3-(2-chloropyrimidin-4-yl)-5-fluoro-2-methylphenyl)-3-isopropoxyazetidine-1-carboxamide (Intermediate 1-8) and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 28: N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-methylbenzyl)-3-isopropoxyazetidine-1 -carboxamide

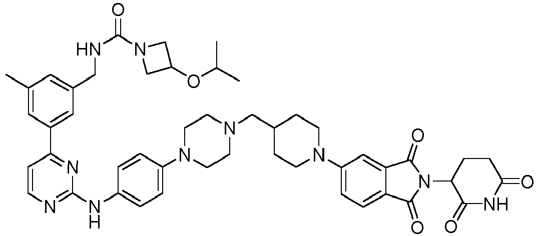

Example 28 was synthesized using the same method as Example 7, using N-(3-(2-chloropyrimidin-4-yl)-5-methylbenzyl)-3-isopropoxyazetidine-1-carboxamide (Intermediate 1-2) instead of N-(4-(2-chloropyrimidin-4-yl)-2-methylbenzyl)-3-isopropoxyazetidine-1-carboxamide (Intermediate 1-1).

### Example 29: N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-methylphenyl)-3-isopropoxyazetidine-1-carboxamide

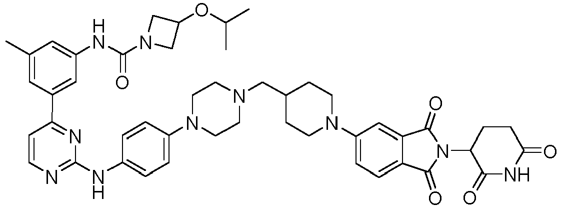

Example 29 was synthesized using the same method as Example 7, using N-(3-(2-chloropyrimidin-4-yl)-5-methylphenyl)-3-isopropoxyazetidine-1-carboxamide (Intermediate 1-3) instead of N-(4-(2-chloropyrimidin-4-yl)-2-methylbenzyl)-3-isopropoxyazetidine-1-carboxamide (Intermediate 1-1).

### Example 30: 4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl 3-isopropoxyazetidine-1-carboxylate

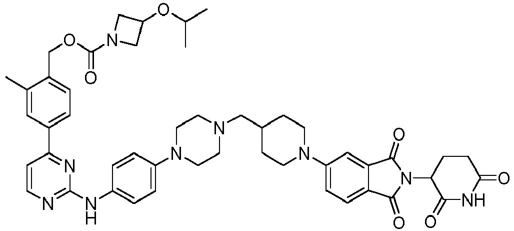

Example 30 was synthesized using the same method as Example 7, using 4-(2-chloropyrimidin-4-yl)-2-methylbenzyl 3-isopropoxyazetidine-1-carboxylate (Intermediate 1-11) instead of N-(4-(2-chloropyrimidin-4-yl)-2-methylbenzyl)-3-isopropoxyazetidine-1-carboxamide (Intermediate 1-1).

### Example 31: 1-(tert-butyl)-N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1H-pyrazole-4-carboxamide

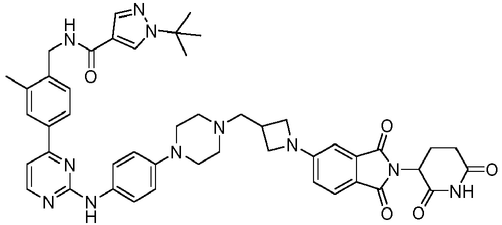

Example 31 was synthesized using the same method as Example 7, using 1-(tert-butyl)-N-(4-(2-chloropyrimidin-4-yl)-2-methylbenzyl)-1H-pyrazole-4-carboxamide (Intermediate 1-4) and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 32: 1-(tert-butyl)-N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1H-1,2,3-triazole-4-carboxamide

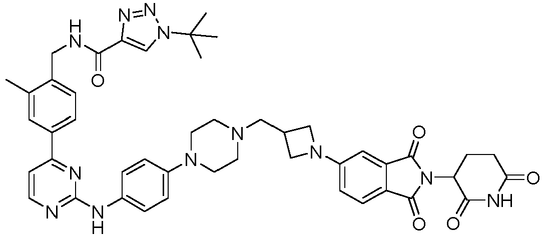

Example 32 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5) and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 33: N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1-isopropyl-1H-pyrazole-4-carboxamide

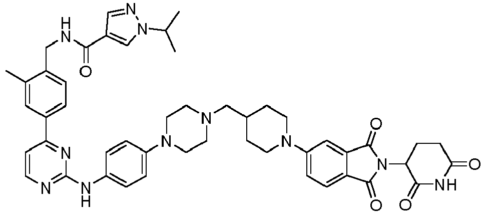

Example 33 was synthesized using the same method as Example 7, using N-(4-(2-chloropyrimidin-4-yl)-2-methylbenzyl)-1-isopropyl-1H-pyrazole-4-carboxamide (Intermediate 1-6) instead of N-(4-(2-chloropyrimidin-4-yl)-2-methylbenzyl)-3-isopropoxyazetidine-1-carboxamide (Intermediate 1-1).

### Example 34: N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1-isopropyl-1H-pyrazole-4-carboxamide

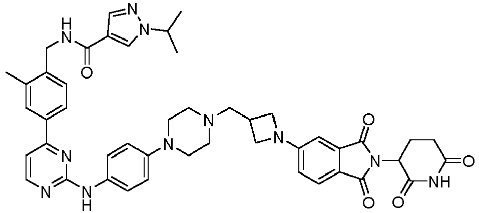

Example 34 was synthesized using the same method as Example 7, using N-(4-(2-chloropyrimidin-4-yl)-2-methylbenzyl)-1-isopropyl-1H-pyrazole-4-carboxamide (Intermediate 1-6) and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 35: N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1-isopropyl-1H-pyrazole-4-carboxamide

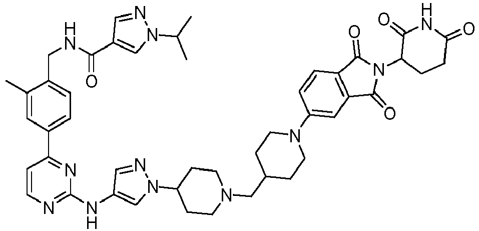

Example 35 was synthesized using the same method as Example 7, using N-(4-(2-chloropyrimidin-4-yl)-2-methylbenzyl)-1-isopropyl-1H-pyrazole-4-carboxamide (Intermediate 1-6) and tert-butyl 4-(4-amino-1H-pyrazol-1-yl)piperidine-1-carboxylate (Intermediate 2-7) instead of N-(4-(2-chloropyrimidin-4-yl)-2-methylbenzyl)-3-isopropoxyazetidine-1-carboxamide (Intermediate 1-1) and tert-butyl 4-(4-aminophenyl)piperazine-1-carboxylate (Intermediate 2-1), respectively.

### Example 36: N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1-isopropyl-1H-pyrazole-4-carboxamide

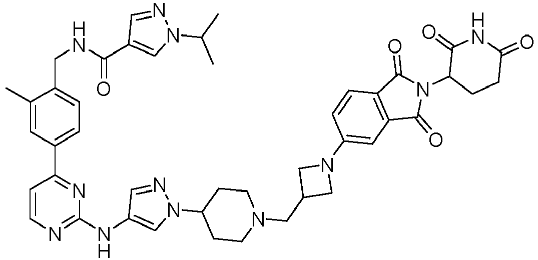

Example 36 was synthesized using the same method as Example 7, using N-(4-(2-chloropyrimidin-4-yl)-2-methylbenzyl)-1-isopropyl-1H-pyrazole-4-carboxamide (Intermediate 1-6), tert-butyl 4-(4-amino-1H-pyrazol-1-yl)piperidine-1-carboxylate (Intermediate 2-7), and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 37: 1-(tert-butyl)-N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1H-pyrazole-4-carboxamide

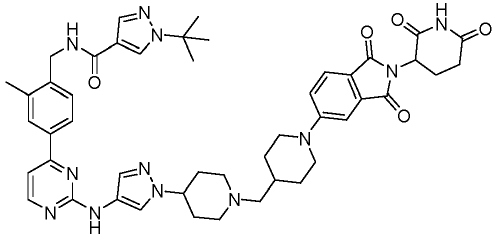

Example 37 was synthesized using the same method as Example 7, using 1-(tert-butyl)-N-(4-(2-chloropyrimidin-4-yl)-2-methylbenzyl)-1H-pyrazole-4-carboxamide (Intermediate 1-4) and tert-butyl 4-(4-amino-1H-pyrazol-1-yl)piperidine-1-carboxylate (Intermediate 2-7) instead of N-(4-(2-chloropyrimidin-4-yl)-2-methylbenzyl)-3-isopropoxyazetidine-1-carboxamide (Intermediate 1-1) and tert-butyl 4-(4-aminophenyl)piperazine-1-carboxylate (Intermediate 2-1), respectively.

### Example 38: 1-(tert-butyl)-N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1H-pyrazole-4-carboxamide

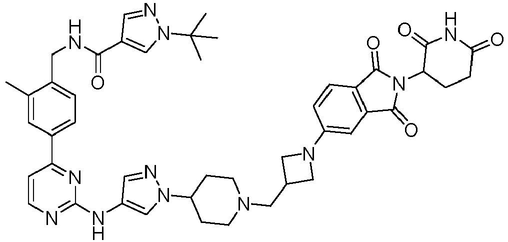

Example 38 was synthesized using the same method as Example 7, using 1-(tert-butyl)-N-(4-(2-chloropyrimidin-4-yl)-2-methylbenzyl)-1H-pyrazole-4-carboxamide (Intermediate 1-4), tert-butyl 4-(4-amino-1H-pyrazol-1-yl)piperidine-1-carboxylate (Intermediate 2-7), and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 39: 1-(tert-butyl)-N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1H-1,2,3-triazole-4-carboxamide

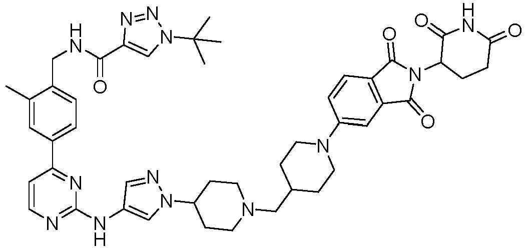

Example 39 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5) and tert-butyl 4-(4-amino-1H-pyrazol-1-yl)piperidine-1-carboxylate (Intermediate 2-7) instead of N-(4-(2-chloropyrimidin-4-yl)-2-methylbenzyl)-3-isopropoxyazetidine-1-carboxamide (Intermediate 1-1) and tert-butyl 4-(4-aminophenyl)piperazine-1-carboxylate (Intermediate 2-1), respectively.

### Example 40: 1-(tert-butyl)-N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1H-1,2,3-triazole-4-carboxamide

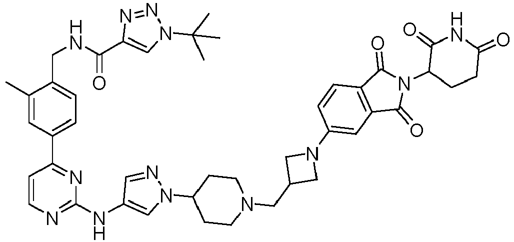

Example 40 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5), tert-butyl 4-(4-amino-1H-pyrazol-1-yl)piperidine-1-carboxylate (Intermediate 2-7), and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 41: N-(4-(2-((4-(1-((1-(2-(2,6-dioxopiperidin-3-yl)-1,3-dioxoisoindolin-5-yl)piperidin-4-yl)methyl)azetidin-3-yl)phenyl)amino)pyrimidin-4-yl)-2-methylbenzyl)-3-isopropoxyazetidine-1 -carboxamide

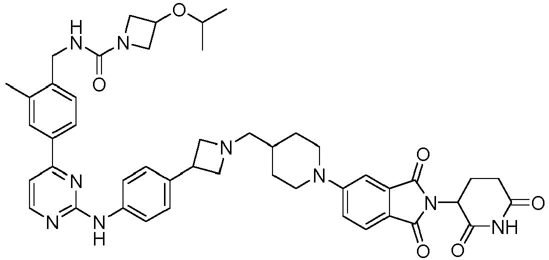

Example 41 was synthesized using the same method as Example 7, using tert-butyl 3-(4-aminophenyl)azetidine-1-carboxylate instead of tert-butyl 4-(4-aminophenyl)piperazine-1-carboxylate (Intermediate 2-1).

### Example 42: N-(4-(2-((4-(4-((1-(3-((2,6-dioxopiperidin-3-yl)oxy)phenyl)piperidin-4-yl)methyl)piperazin-1-yl)phenyl)amino)pyrimidin-4-yl)-2-methylbenzyl)-3-isopropoxyazetidine-1-carboxamide

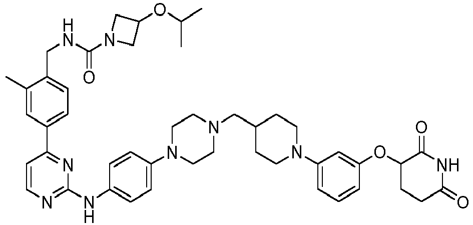

Example 42 was synthesized using the same method as Example 7, using 1-(3-((2,6-dioxopiperidin-3-yl)oxy)phenyl)piperidine-4-carbaldehyde (Intermediate 3-16) instead of 1-(2-(2,6-dioxopiperidin-3-yl)-1,3-dioxoisoindolin-5-yl)piperidine-4-carbaldehyde (Intermediate 3-1).

### Example 43: N-(4-(2-((4-(4-((1-(4-((2,6-dioxopiperidin-3-yl)oxy)phenyl)piperidin-4-yl)methyl)piperazin-1-yl)phenyl)amino)pyrimidin-4-yl)-2-methylbenzyl)-3-isopropoxyazetidine-1-carboxamide

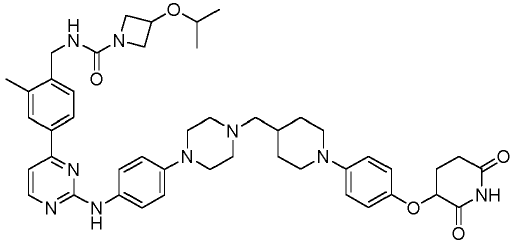

Example 43 was synthesized using the same method as Example 7, using 1-(4-((2,6-dioxopiperidin-3-yl)oxy)phenyl)piperidine-4-carbaldehyde (Intermediate 3-15) instead of 1-(2-(2,6-dioxopiperidin-3-yl)-1,3-dioxoisoindolin-5-yl)piperidine-4-carbaldehyde (Intermediate 3-1).

### Example 44: N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1-isopropyl-1H-pyrazole-4-carboxamide

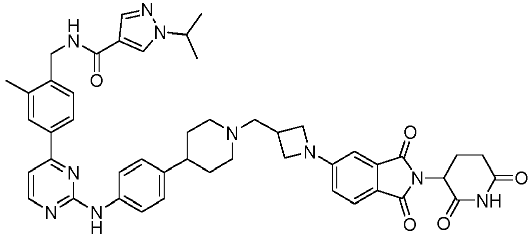

Example 44 was synthesized using the same method as Example 7, using N-(4-(2-chloropyrimidin-4-yl)-2-methylbenzyl)-1-isopropyl-1H-pyrazole-4-carboxamide (Intermediate 1-6), tert-butyl 4-(4-aminophenyl)piperidine-1-carboxylate (Intermediate 2-5), and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 45: 1-(tert-butyl)-N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1H-pyrazole-4-carboxamide

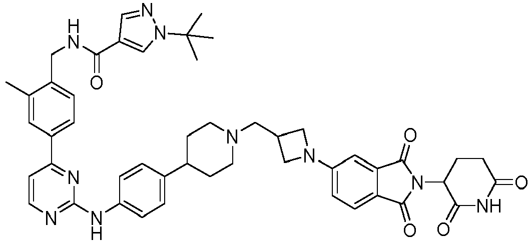

Example 45 was synthesized using the same method as Example 7, using 1-(tert-butyl)-N-(4-(2-chloropyrimidin-4-yl)-2-methylbenzyl)-1H-pyrazole-4-carboxamide (Intermediate 1-4), tert-butyl 4-(4-aminophenyl)piperidine-1-carboxylate (Intermediate 2-5), and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 46: 1-(tert-butyl)-N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1H-1,2,3-triazole-4-carboxamide

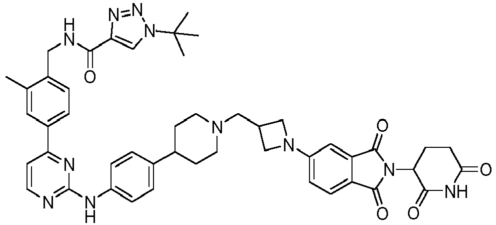

Example 46 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5), tert-butyl 4-(4-aminophenyl)piperidine-1-carboxylate (Intermediate 2-5), and 1-(2-(2,6-dioxopiperidin-3-yl)-1,3-dioxoisoindolin-5-yl)azetidine-3-carbaldehyde (Intermediate 3-2) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 47: N-(4-(2-((4-(1-((1-(4-((2,6-dioxopiperidin-3-yl)oxy)phenyl)piperidin-4-yl)methyl)piperidin-4-yl)phenyl)amino)pyrimidin-4-yl)-2-methylbenzyl)-3-isopropoxyazetidine-1-carboxamide

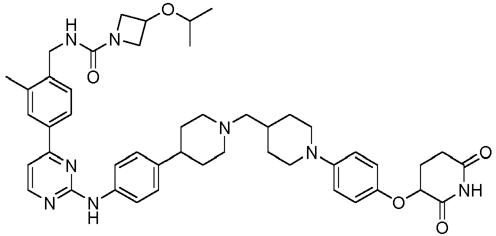

Example 47 was synthesized using the same method as Example 7, using tert-butyl 4-(4-aminophenyl)piperidine-1-carboxylate (Intermediate 2-5) and 1-(4-((2,6-dioxopiperidin-3-yl)oxy)phenyl)piperidine-4-carbaldehyde (Intermediate 3-15) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 48: N-(4-(2-((4-(4-((1-(4-((2,6-dioxopiperidin-3-yl)oxy)phenyl)piperidin-4-yl)methyl)piperazin-1-yl)phenyl)amino)pyrimidin-4-yl)-2-methylbenzyl)-1-isopropyl-1H-pyrazole-4-carboxamide

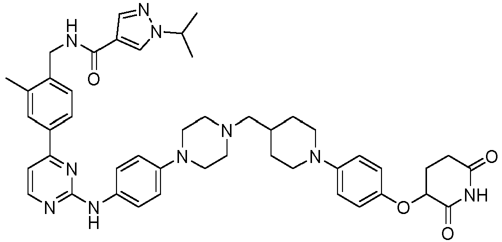

Example 48 was synthesized using the same method as Example 7, using N-(4-(2-chloropyrimidin-4-yl)-2-methylbenzyl)-1-isopropyl-1H-pyrazole-4-carboxamide (Intermediate 1-6) and 1-(4-((2,6-dioxopiperidin-3-yl)oxy)phenyl)piperidine-4-carbaldehyde (Intermediate 3-15) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 49: N-(4-(2-((4-(1-((1-(4-((2,6-dioxopiperidin-3-yl)oxy)phenyl)piperidin-4-yl)methyl)piperidin-4-yl)phenyl)amino)pyrimidin-4-yl)-2-methylbenzyl)-1-isopropyl-1H-pyrazole-4-carboxamide

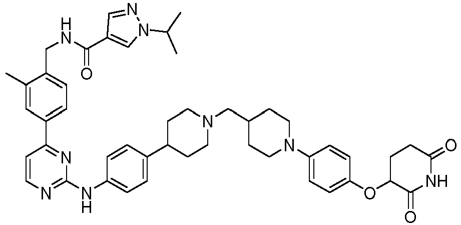

Example 49 was synthesized using the same method as Example 7, using N-(4-(2-chloropyrimidin-4-yl)-2-methylbenzyl)-1-isopropyl-1H-pyrazole-4-carboxamide (Intermediate 1-6), tert-butyl 4-(4-aminophenyl)piperidine-1-carboxylate (Intermediate 2-5), and 1-(4-((2,6-dioxopiperidin-3-yl)oxy)phenyl)piperidine-4-carbaldehyde (Intermediate 3-15) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 50: N-(4-(2-((4-(4-((1-(5-((2,6-dioxopiperidin-3-yl)oxy)pyridin-2-yl)piperidin-4-yl)methyl)piperazin-1-yl)phenyl)amino)pyrimidin-4-yl)-2-methylbenzyl)-3-isopropoxyazetidine-1-carboxamide

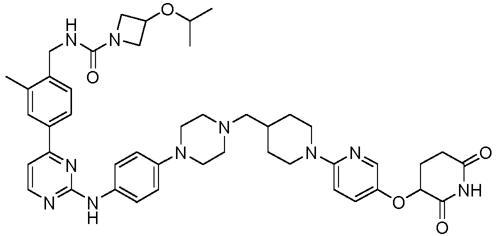

Example 50 was synthesized using the same method as Example 7, using 1-(5-((2,6-dioxopiperidin-3-yl)oxy)pyridin-2-yl)piperidine-4-carbaldehyde (Intermediate 3-18) instead of 1-(2-(2,6-dioxopiperidin-3-yl)-1,3-dioxoisoindolin-5-yl)piperidine-4-carbaldehyde (Intermediate 3-1).

### Example 51: N-(4-(2-((4-(1-((1-(5-((2,6-dioxopiperidin-3-yl)oxy)pyridin-2-yl)piperidin-4-yl)methyl)piperidin-4-yl)phenyl)amino)pyrimidin-4-yl)-2-methylbenzyl)-3-isopropoxyazetidine-1-carboxamide

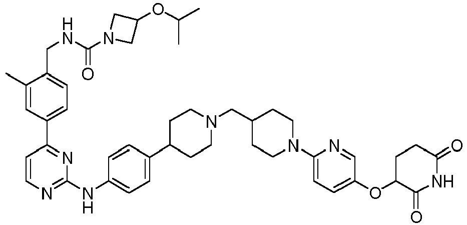

Example 51 was synthesized using the same method as Example 7, using tert-butyl 4-(4-aminophenyl)piperidine-1-carboxylate (Intermediate 2-5) and 1-(5-((2,6-dioxopiperidin-3-yl)oxy)pyridin-2-yl)piperidine-4-carbaldehyde (Intermediate 3-18) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 52: N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-3-isopropoxyazetidine-1-carboxamide

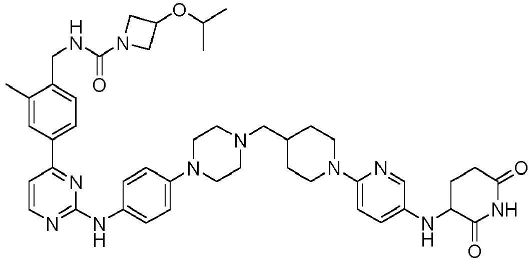

Example 52 was synthesized using the same method as Example 7, using 1-(5-((2,6-dioxopiperidin-3-yl)amino)pyridin-2-yl)piperidine-4-carbaldehyde (Intermediate 3-23) instead of 1-(2-(2,6-dioxopiperidin-3-yl)-1,3-dioxoisoindolin-5-yl)piperidine-4-carbaldehyde (Intermediate 3-1).

### Example 53: N-(4-(2-((4-(1-((1-(5-((2,6-dioxopiperidin-3-yl)amino)pyridin-2-yl)piperidin-4-yl)methyl)piperidin-4-yl)phenyl)amino)pyrimidin-4-yl)-2-methylbenzyl)-3-isopropoxyazetidine-1-carboxamide

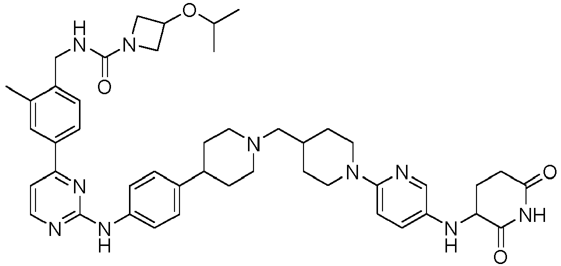

Example 53 was synthesized using the same method as Example 7, using tert-butyl 4-(4-aminophenyl)piperidine-1-carboxylate (Intermediate 2-5) and 1-(5-((2,6-dioxopiperidin-3-yl)amino)pyridin-2-yl)piperidine-4-carbaldehyde (Intermediate 3-23) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 54: N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-3-isopropoxyazetidine-1-carboxamide

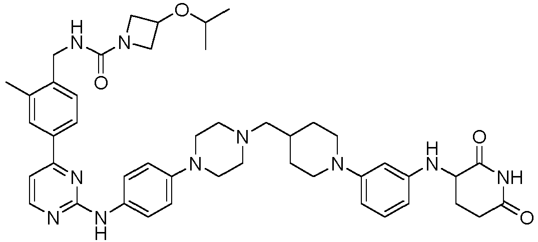

Example 54 was synthesized using the same method as Example 7, using 1-(3-((2,6-dioxopiperidin-3-yl)amino)phenyl)piperidine-4-carbaldehyde (Intermediate 3-25) instead of 1-(2-(2,6-dioxopiperidin-3-yl)-1,3-dioxoisoindolin-5-yl)piperidine-4-carbaldehyde (Intermediate 3-1).

### Example 55: N-(4-(2-((4-(4-((1-(4-((2,6-dioxopiperidin-3-yl)amino)phenyl)piperidin-4-yl)methyl)piperazin-1-yl)phenyl)amino)pyrimidin-4-yl)-2-methylbenzyl)-3-isopropoxyazetidine-1-carboxamide

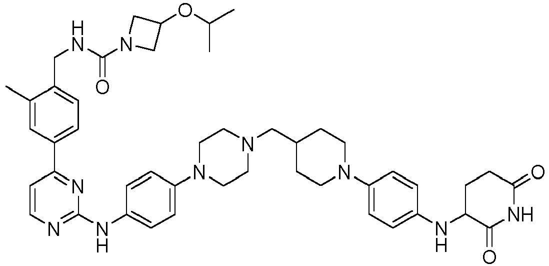

Example 55 was synthesized using the same method as Example 7, using 1-(4-((2,6-dioxopiperidin-3-yl)amino)phenyl)piperidine-4-carbaldehyde (Intermediate 3-24) instead of 1-(2-(2,6-dioxopiperidin-3-yl)-1,3-dioxoisoindolin-5-yl)piperidine-4-carbaldehyde (Intermediate 3-1).

### Example 56: N-(4-(2-((4-(1-((1-(4-((2,6-dioxopiperidin-3-yl)amino)phenyl)piperidin-4-yl)methyl)piperidin-4-yl)phenyl)amino)pyrimidin-4-yl)-2-methylbenzyl)-3-isopropoxyazetidine-1-carboxamide

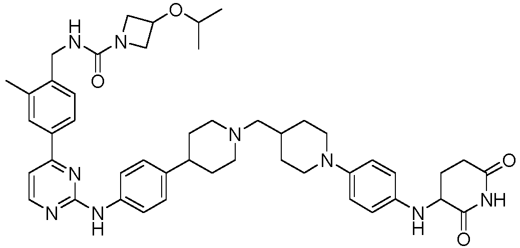

Example was synthesized using the same method as Example 7, using tert-butyl 4-(4-aminophenyl)piperidine-1-carboxylate (Intermediate 2-5) and 1-(4-((2,6-dioxopiperidin-3-yl)amino)phenyl)piperidine-4-carbaldehyde (Intermediate 3-24) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 57: N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1-isopropyl-1H-pyrazole-4-carboxamide

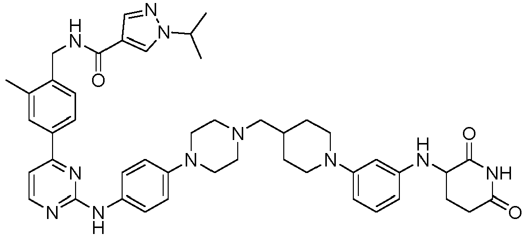

Example 57 was synthesized using the same method as Example 7, using N-(4-(2-chloropyrimidin-4-yl)-2-methylbenzyl)-1-isopropyl-1H-pyrazole-4-carboxamide (Intermediate 1-6) and 1-(3-((2,6-dioxopiperidin-3-yl)amino)phenyl)piperidine-4-carbaldehyde (Intermediate 3-25) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 58: N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1-isopropyl-1H-pyrazole-4-carboxamide

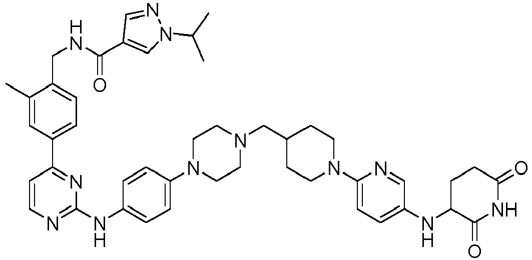

Example 58 was synthesized using the same method as Example 7, using N-(4-(2-chloropyrimidin-4-yl)-2-methylbenzyl)-1-isopropyl-1H-pyrazole-4-carboxamide (Intermediate 1-6) and 1-(5-((2,6-dioxopiperidin-3-yl)amino)pyridin-2-yl)piperidine-4-carbaldehyde (Intermediate 3-23) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 59: 1-(tert-butyl)-N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1H-pyrazole-4-carboxamide

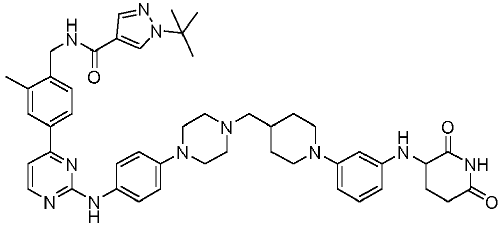

Example 59 was synthesized using the same method as Example 7, using 1-(tert-butyl)-N-(4-(2-chloropyrimidin-4-yl)-2-methylbenzyl)-1H-pyrazole-4-carboxamide (Intermediate 1-4) and 1-(3-((2,6-dioxopiperidin-3-yl)amino)phenyl)piperidine-4-carbaldehyde (Intermediate 3-25) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 60: 1-(tert-butyl)-N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1H-pyrazole-4-carboxamide

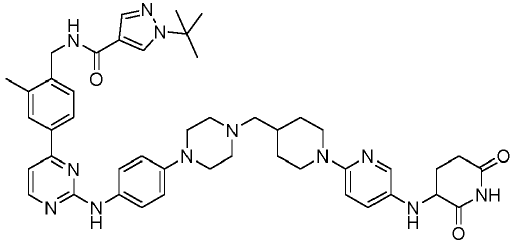

Example 60 was synthesized using the same method as Example 7, using 1-(tert-butyl)-N-(4-(2-chloropyrimidin-4-yl)-2-methylbenzyl)-1H-pyrazole-4-carboxamide (Intermediate 1-4) and 1-(5-((2,6-dioxopiperidin-3-yl)amino)pyridin-2-yl)piperidine-4-carbaldehyde (Intermediate 3-23) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 61: 1-(tert-butyl)-N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1H-1,2,3-triazole-4-carboxamide

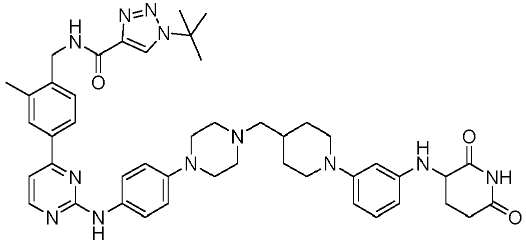

Example 61 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5) and 1-(3-((2,6-dioxopiperidin-3-yl)amino)phenyl)piperidine-4-carbaldehyde (Intermediate 3-25) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 62: 1-(tert-butyl)-N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1H-1,2,3-triazole-4-carboxamide

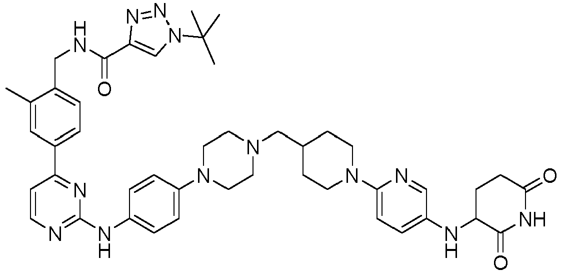

Example 62 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5) and 1-(5-((2,6-dioxopiperidin-3-yl)amino)pyridin-2-yl)piperidine-4-carbaldehyde (Intermediate 3-23) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 63: N-(4-(2-((4-((4-((1-(3-((2,6-dioxopiperidin-3-yl)oxy)phenyl)piperidin-4-yl)methyl)piperazin-1-yl)methyl)phenyl)amino)pyrimidin-4-yl)-2-methylbenzyl)-3-isopropoxyazetidine-1-carboxamide

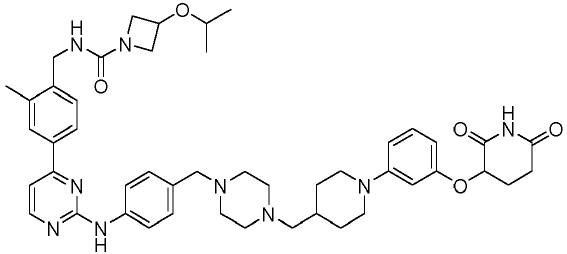

Example 63 was synthesized using the same method as Example 7, using tert-butyl 4-(4-aminobenzyl)piperazine-1-carboxylate (Intermediate 2-13) and 1-(3-((2,6-dioxopiperidin-3-yl)oxy)phenyl)piperidine-4-carbaldehyde (Intermediate 3-16) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 64: N-(4-(2-((4-((4-((1-(3-((2,6-dioxopiperidin-3-yl)amino)phenyl)piperidin-4-yl)methyl)piperazin-1-yl)methyl)phenyl)amino)pyrimidin-4-yl)-2-methylbenzyl)-3-isopropoxyazetidine-1-carboxamide

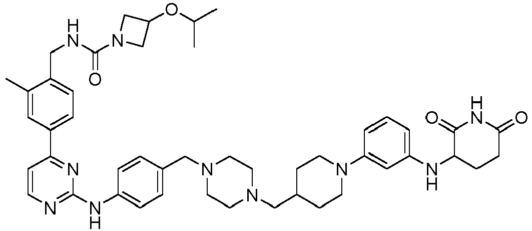

Example 64 was synthesized using the same method as Example 7, using tert-butyl 4-(4-aminobenzyl)piperazine-1-carboxylate (Intermediate 2-13) and 1-(3-((2,6-dioxopiperidin-3-yl)amino)phenyl)piperidine-4-carbaldehyde (Intermediate 3-25) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 65: N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-3 -isopropoxy azetidine-1 -carboxamide

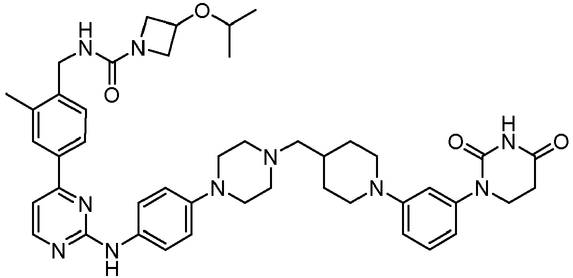

Example 65 was synthesized using the same method as Example 7, using 1-(3-(2,4-dioxotetrahydropyrimidin-1(2H)-yl)phenyl)piperidine-4-carbaldehyde (Intermediate 3-30) instead of 1-(2-(2,6-dioxopiperidin-3-yl)-1,3-dioxoisoindolin-5-yl)piperidine-4-carbaldehyde (Intermediate 3-1).

### Example 66: N-(4-(2-((4-(4-((1-(4-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-3 -isopropoxy azetidine-1 -carboxamide

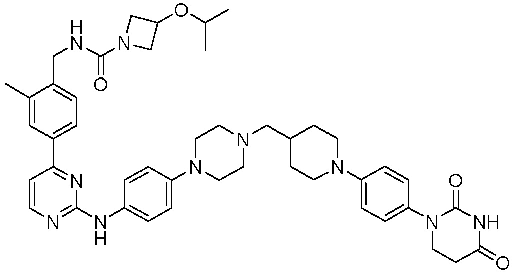

Example 66 was synthesized using the same method as Example 7, using 1-(4-(2,4-dioxotetrahydropyrimidin-1(2H)-yl)phenyl)piperidine-4-carbaldehyde (Intermediate 3-29) instead of 1-(2-(2,6-dioxopiperidin-3-yl)-1,3-dioxoisoindolin-5-yl)piperidine-4-carbaldehyde (Intermediate 3-1).

### Example 67: N-(4-(2-((4-((4-((1-(3-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-3-isopropoxyazetidine-1-carboxamide

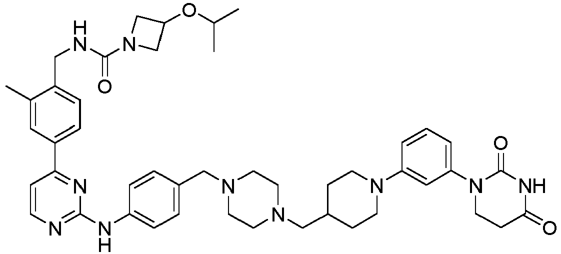

Example 67 was synthesized using the same method as Example 7, using tert-butyl 4-(4-aminobenzyl)piperazine-1-carboxylate (Intermediate 2-13) and 1-(3-(2,4-dioxotetrahydropyrimidin-1(2H)-yl)phenyl)piperidine-4-carbaldehyde (Intermediate 3-30) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 68: N-(4-(2-((4-((4-((1-(4-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-3-isopropoxyazetidine-1-carboxamide

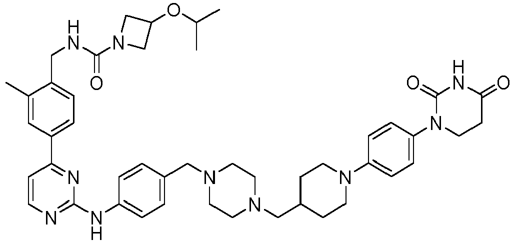

Example 68 was synthesized using the same method as Example 7, using tert-butyl 4-(4-aminobenzyl)piperazine-1-carboxylate (Intermediate 2-13) and 1-(4-(2,4-dioxotetrahydropyrimidin-1(2H)-yl)phenyl)piperidine-4-carbaldehyde (Intermediate 3-29) instead of tert-butyl 4-(4-aminophenyl)piperazine-1-carboxylate (Intermediate 2-1) and 1-(2-(2,6-dioxopiperidin-3-yl)-1,3-dioxoisoindolin-5-yl)piperidine-4-carbaldehyde (Intermediate 3-1), respectively.

### Example 69: 1-(tert-butyl)-N-(4-(2-((4-((4-((1-(3-((2,6-dioxopiperidin-3-yl)amino)phenyl)piperidin-4-yl)methyl)piperazin-1-yl)methyl)phenyl)amino)pyrimidin-4-yl)-2-methylbenzyl)-1H-1,2,3-triazole-4-carboxamide

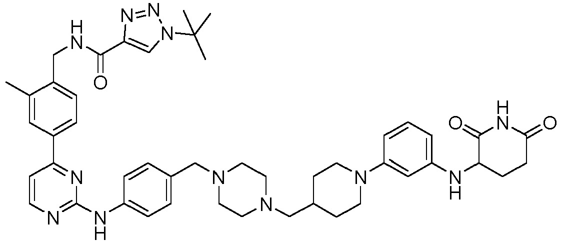

Example 69 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5), tert-butyl 4-(4-aminobenzyl)piperazine-1-carboxylate (Intermediate 2-13), and 1-(3-((2,6-dioxopiperidin-3-yl)amino)phenyl)piperidine-4-carbaldehyde (Intermediate 3-25) instead ofN-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 70: 1-(tert-butyl)-N-(4-(2-((4-(4-((1-(3 -(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1H-1,2,3-triazole-4-carboxamide

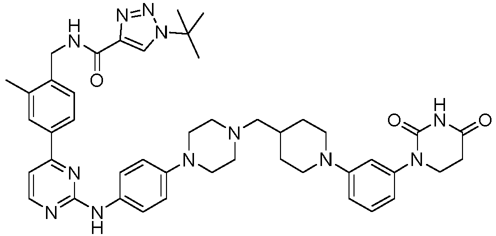

Example 70 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5) and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 71: 1-(tert-butyl)-N-(4-(2-((4-(4-((1-(4-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1H-1,2,3-triazole-4-carboxamide

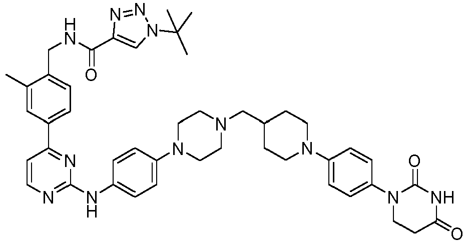

Example 71 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5) and 1-(4-(2,4-dioxotetrahydropyrimidin-1(2H)-yl)phenyl)piperidine-4-carbaldehyde (Intermediate 3-29) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 72: 1-(tert-butyl)-N-(4-(2-((4-(4-((1-(4-((2,6-dioxopiperidin-3-yl)amino)-2-fluorophenyl)piperidin-4-yl)methyl)piperazin-1-yl)phenyl)amino)pyrimidin-4-yl)-2-methylbenzyl)-1H-1,2,3-triazole-4-carboxamide

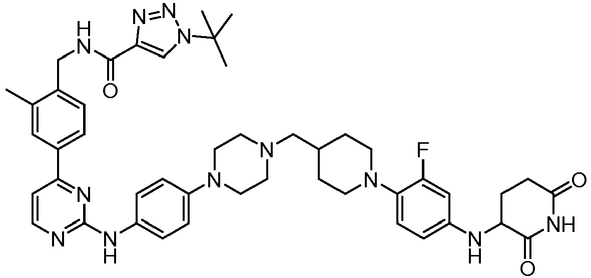

Example 72 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5) and 1-(4-((2,6-dioxopiperidin-3-yl)amino)-2-fluorophenyl)piperidine-4-carbaldehyde (Intermediate 3-26) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 73: 1-(tert-butyl)-N-(4-(2-((4-(4-((1-(4-(2,4-dioxotetrahydropyrimidin-1(2H)-yl)-2-fluorophenyl)piperidin-4-yl)methyl)piperazin-1-yl)phenyl)amino)pyrimidin-4-yl)-2-methylbenzyl)-1H-1,2,3-triazole-4-carboxamide

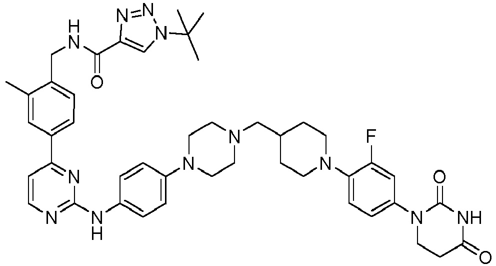

Example 73 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5) and 1 -(4-(2,4-dioxotetrahydropyrimidin-1 (2H)-yl)-2-fluorophenyl)piperidine-4-carbaldehyde (Intermediate 3-31) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 74: 1-(tert-butyl)-N-(4-(2-((4-((4-((1-(3-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-1H-1,2,3-triazole-4-carboxamide

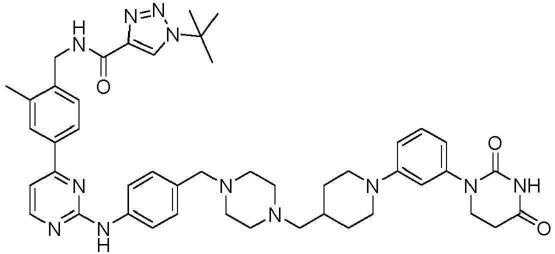

Example 74 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5), tert-butyl 4-(4-aminobenzyl)piperazine-1-carboxylate (Intermediate 2-13), and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 75: 1-(tert-butyl)-N-(4-(2-((4-((4-((1-(4-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-1H-1,2,3-triazole-4-carboxamide

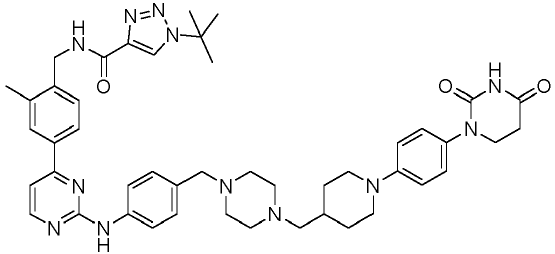

Example 75 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5), tert-butyl 4-(4-aminobenzyl)piperazine-1-carboxylate (Intermediate 2-13), and 1-(4-(2,4-dioxotetrahydropyrimidin-1(2H)-yl)phenyl)piperidine-4-carbaldehyde (Intermediate 3-29) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 76: 1-(tert-butyl)-N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1H-1,2,3-triazole-4-carboxamide

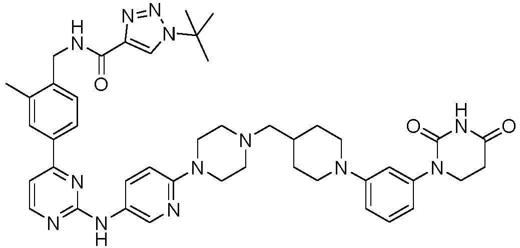

Example 76 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5), tert-butyl 4-(5-aminopyridin-2-yl)piperazine-1-carboxylate (Intermediate 2-2), and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 77: 1-(tert-butyl)-N-(4-(2-((6-(4-((1-(4-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1H-1,2,3-triazole-4-carboxamide

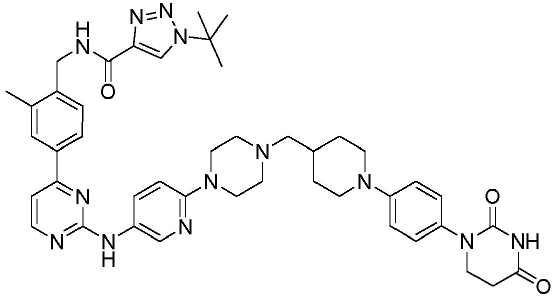

Example 77 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5), tert-butyl 4-(5-aminopyridin-2-yl)piperazine-1-carboxylate (Intermediate 2-2), and 1-(4-(2,4-dioxotetrahydropyrimidin-1(2H)-yl)phenyl)piperidine-4-carbaldehyde (Intermediate 3-29) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 78: 1-(tert-butyl)-N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1H-1,2,3-triazole-4-carboxamide

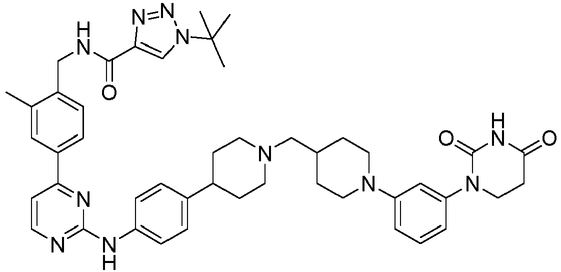

Example 78 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5), tert-butyl 4-(4-aminophenyl)piperidine-1-carboxylate (Intermediate 2-5), and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 79: 1-(tert-butyl)-N-(4-(2-((4-(1-((1-(4-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1H-1,2,3-triazole-4-carboxamide

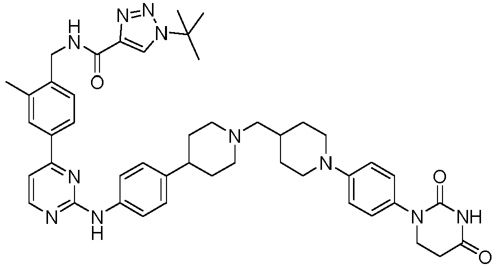

Example 79 was synthesized using the same method as Example 7, using 1-(tert-butyl)-N-(4-(2-chloropyrimidin-4-yl)-2-methylbenzyl)-1H-1,2,3-triazole-4-carboxamide (Intermediate 1-5), tert-butyl 4-(4-aminophenyl)piperidine-1-carboxylate (Intermediate 2-5), and 1-(4-(2,4-dioxotetrahydropyrimidin-1(2H)-yl)phenyl)piperidine-4-carbaldehyde (Intermediate 3-29) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 80: N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1-isopropyl-1H-pyrazole-4-carboxamide

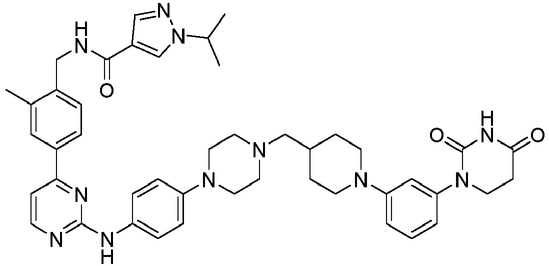

Example 80 was synthesized using the same method as Example 7, using N-(4-(2-chloropyrimidin-4-yl)-2-methylbenzyl)-1-isopropyl-1H-pyrazole-4-carboxamide (Intermediate 1-6) and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 81: 1-(tert-butyl)-N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1H-pyrazole-4-carboxamide

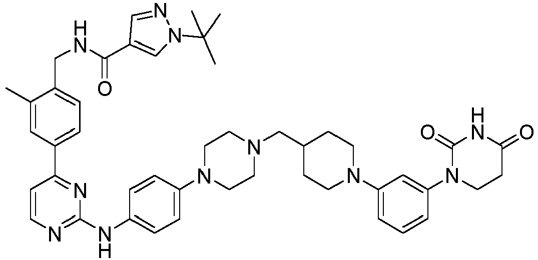

Example 81 was synthesized using the same method as Example 7, using 1-(tert-butyl)-N-(4-(2-chloropyrimidin-4-yl)-2-methylbenzyl)-1H-pyrazole-4-carboxamide (Intermediate 1-4) and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1) and 1-(2-(2,6-dioxopiperidin-3-yl)-1,3-dioxoisoindolin-5-yl)piperidine-4-carbaldehyde (Intermediate 3-1), respectively.

### Example 82: N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1-isopropyl-1H-pyrazole-4-carboxamide

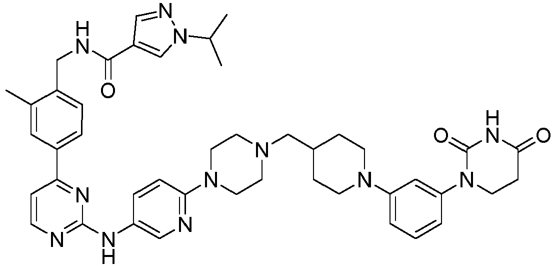

Example 82 was synthesized using the same method as Example 7, using N-(4-(2-chloropyrimidin-4-yl)-2-methylbenzyl)-1-isopropyl-1H-pyrazole-4-carboxamide (Intermediate 1-6), tert-butyl 4-(5-aminopyridin-2-yl)piperazine-1-carboxylate (Intermediate 2-2), and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 83: 1-(tert-butyl)-N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1H-pyrazole-4-carboxamide

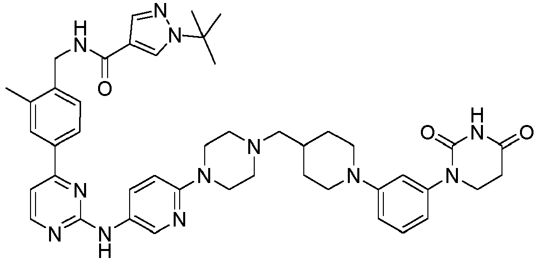

Example 83 was synthesized using the same method as Example 7, using 1-(tert-butyl)-N-(4-(2-chloropyrimidin-4-yl)-2-methylbenzyl)-1H-pyrazole-4-carboxamide (Intermediate 1-4), tert-butyl 4-(5-aminopyridin-2-yl)piperazine-1-carboxylate (Intermediate 2-2), and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 84: N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1-isopropyl-1H-pyrazole-4-carboxamide

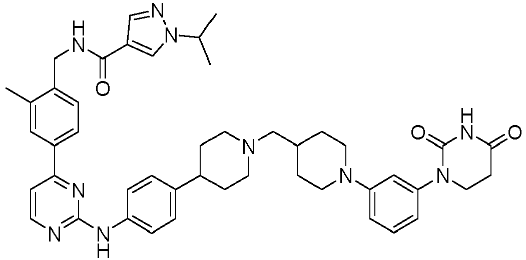

Example 84 was synthesized using the same method as Example 7, using N-(4-(2-chloropyrimidin-4-yl)-2-methylbenzyl)-1-isopropyl-1H-pyrazole-4-carboxamide (Intermediate 1-6), tert-butyl 4-(4-aminophenyl)piperidine-1-carboxylate (Intermediate 2-5), and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 85: 1-(tert-butyl)-N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1H-pyrazole-4-carboxamide

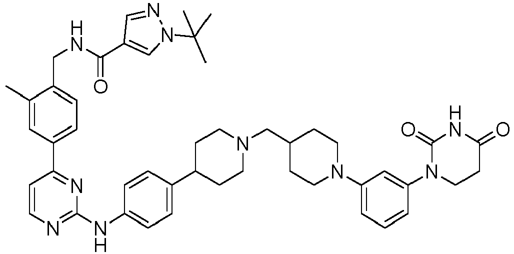

Example 85 was synthesized using the same method as Example 7, using 1-(tert-butyl)-N-(4-(2-chloropyrimidin-4-yl)-2-methylbenzyl)-1H-pyrazole-4-carboxamide (Intermediate 1-4), tert-butyl 4-(4-aminophenyl)piperidine-1-carboxylate (Intermediate 2-5), and 1-(3-(2,4-dioxotetrahydropyrimidin-1(2H)-yl)phenyl)piperidine-4-carbaldehyde (Intermediate 3-30) instead of N-(4-(2-chloropyrimidin-4-yl)-2-methylbenzyl)-3-isopropoxyazetidine-1-carboxamide (Intermediate 1-1), tert-butyl 4-(4-aminophenyl)piperazine-1-carboxylate (Intermediate 2-1), and 1-(2-(2,6-dioxopiperidin-3-yl)-1,3-dioxoisoindolin-5-yl)piperidine-4-carbaldehyde (Intermediate 3-1), respectively.

### Example 86: N-(4-(2-((4-(4-((1-(3-(2,4-dioxotetrahydropyrimidin-1(2H)-yl)-4-methoxybenzoyl)piperidin-4-yl)methyl)piperazin-1-yl)phenyl)amino)pyrimidin-4-yl)-2-methylbenzyl)-3-isopropoxyazetidine-1-carboxamide

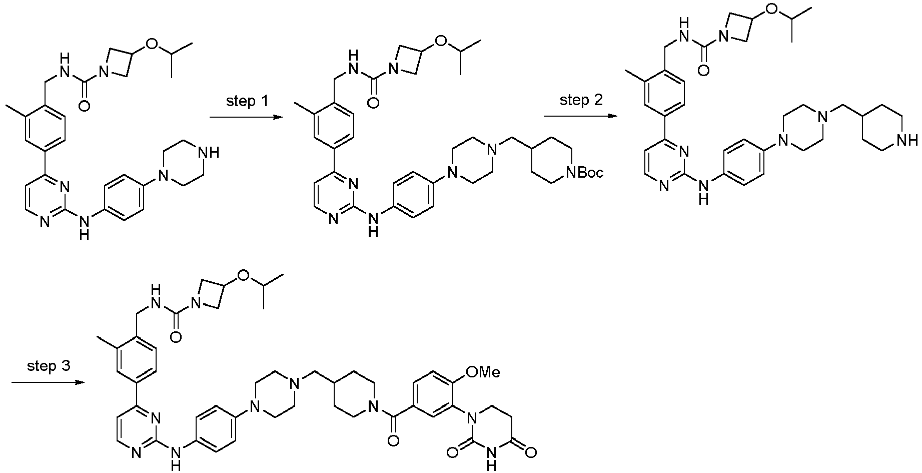

### Step 1: Synthesis of tert-butyl 4-((4-(4-((4-(4-((3-isopropoxyazetidine-1-carboxamido)methyl)-3-methylphenyl)pyrimidin-2-yl)amino)phenyl)piperazin-1-yl)methyl)piperidine-1-carboxylate

3-isopropoxy-N-(2-methyl-4-(2-((4-(piperazin-1-yl)phenyl)amino)pyrimidin-4-yl)benzyl)azetidine-1-carboxamide (100 mg, 0.19 mmol) and tert-butyl 4-formylpiperidine-1-carboxylate (50 mg, 0.23 mmol) were suspended in DCM (4 mL). Then, NaBH(OAc)₃ (123 mg, 0.58 mmol) was added and stirred at room temperature for 2 hours. NaHCO₃ aqueous solution (30 mL) was added to the reaction solution and extracted with DCM (10 mL x 3). The organic layer was dried over anhydrous sodium sulfate, filtered, and concentrated under reduced pressure. The resulting residue was subjected to MPLC (5-8% MeOH/DCM) to obtain 129 mg (93%) of a yellow solid.

### Step 2: Synthesis of 3-isopropoxy-N-(2-methyl-4-(2-((4-(4-(piperidin-4-ylmethyl)piperazin-1-yl)phenyl)amino)pyrimidin-4-yl)benzyl)azetidine-1-carboxamide

Tert-butyl 4-((4-(4-((4-(4-((3-isopropoxyazetidine-1-carboxamido)methyl)-3-methylphenyl)pyrimidin-2-yl)amino)phenyl)piperazin-1-yl)methyl)piperidine-1-carboxylate (128 mg, 0.18 mmol) was suspended in DCM (8 mL). Then, TFA (2 mL) was added and stirred at room temperature for 2 hours. NaHCO₃ aqueous solution (30 mL) was added to the reaction solution and extracted with DCM (10 mL x 3). The organic layer was dried over anhydrous sodium sulfate, filtered, and concentrated under reduced pressure to obtain 125 mg of an orange solid.

### Step 3: Synthesis of N-(4-(2-((4-(4-((1-(3-(2,4-dioxotetrahydropyrimidin-1(2H)-yl)-4-methoxybenzoyl)piperidin-4-yl)methyl)piperazin-1-yl)phenyl)amino)pyrimidin-4-yl)-2-methylbenzyl)-3-isopropoxyazetidine-1-carboxamide

3-(2,4-dioxotetrahydropyrimidin-1(2H)-yl)-4-methoxybenzoic acid (Intermediate 3-12, 16 mg, 0.06 mmol) was suspended in DMF (0.5 mL). Then, HOBT (10 mg, 0.07 mmol), TBTU (24 mg, 0.07 mmol), and DIPEA (0.03 mL, 0.15 mmol) were added. 3-isopropoxy-N-(2-methyl-4-(2-((4-(4-(piperidin-4-ylmethyl)piperazin-1-yl)phenyl)amino)pyrimidin-4-yl)benzyl)azetidine-1-carboxamide (30 mg, 0.05 mmol) was dissolved in DMF (0.5 mL), added, and stirred at room temperature for 16 hours. NHCl₄ aqueous solution (30 mL) was added to the reaction solution, and then extracted with EtOAc (10 mL x 3). The organic layer was dried over anhydrous sodium sulfate, filtered, and concentrated under reduced pressure. The resulting residue was subjected to MPLC (3-8% MeOH/DCM) to obtain 22 mg (52%) of a yellow solid.

### Example 87: N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-3-isopropoxyazetidine-1-carboxamide

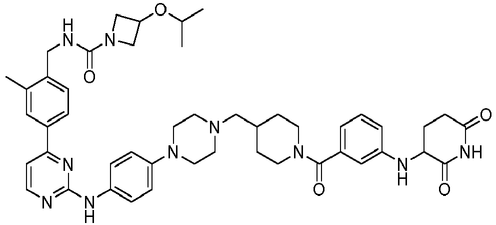

Example 87 was synthesized using the same method as Example 86, using 3-((2,6-dioxopiperidin-3-yl)amino)benzoic acid (Intermediate 3-8) instead of 3-(2,4-dioxotetrahydropyrimidin-1(2H)-yl)-4-methoxybenzoic acid (Intermediate 3-12).

### Example 88: N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-3-isopropoxyazetidine-1-carboxamide

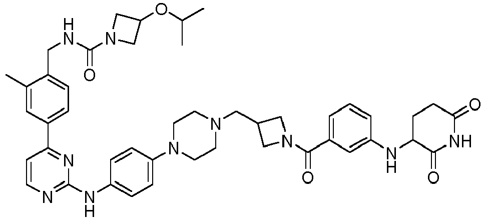

Example 88 was synthesized using the same method as Example 86, using tert-butyl 3-formylazetidine-1-carboxylate and 3-((2,6-dioxopiperidin-3-yl)amino)benzoic acid (Intermediate 3-8) instead of tert-butyl 4-formylpiperidine-1-carboxylate and 3-(2,4-dioxotetrahydropyrimidin-1(2H)-yl)-4-methoxybenzoic acid (Intermediate 3-12), respectively.

### Example 89: N-(4-(2-((4-(4-((1-(3-(2,4-dioxotetrahydropyrimidin-1(2H)-yl)benzoyl)piperidin-4-yl)methyl)piperazin-1-yl)phenyl)amino)pyrimidin-4-yl)-2-methylbenzyl)-3 -isopropoxy azetidine-1 -carboxamide

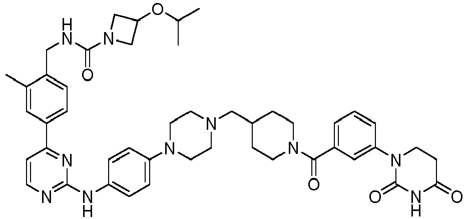

Example 89 was synthesized using the same method as Example 86, using 3-(2,4-dioxotetrahydropyrimidin-1(2H)-yl)benzoic acid (Intermediate 3-11) instead of 3-(2,4-dioxotetrahydropyrimidin-1(2H)-yl)-4-methoxybenzoic acid (Intermediate 3-12).

### Example 90: N-(3-(2-((4-(4-((1-(3-(2,4-dioxotetrahydropyrimidin-1(2H)-yl)-4-methoxybenzoyl)piperidin-4-yl)methyl)piperazin-1-yl)phenyl)amino)pyrimidin-4-yl)-5-fluoro-2-methylphenyl)-3-isopropoxyazetidine-1-carboxamide

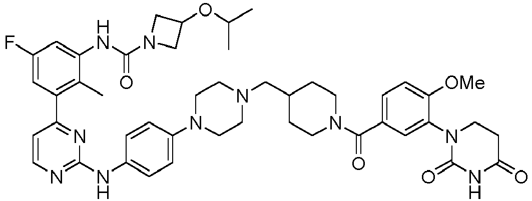

Example 90 was synthesized using the same method as Step 1 and Step 2 of Example 7 and Example 86, using N-(3-(2-chloropyrimidin-4-yl)-5-fluoro-2-methylphenyl)-3-isopropoxyazetidine-1-carboxamide (Intermediate 1-8) instead of N-(4-(2-chloropyrimidin-4-yl)-2-methylbenzyl)-3-isopropoxyazetidine-1-carboxamide (Intermediate 1-1).

### Example 91: N-(4-(2-((1-(1-((1-(3-((2,6-dioxopiperidin-3-yl)amino)benzoyl)piperidin-4-yl)methyl)piperidin-4-yl)-1H-pyrazol-4-yl)amino)pyrimidin-4-yl)-2-methylbenzyl)-3-isopropoxyazetidine-1-carboxamide

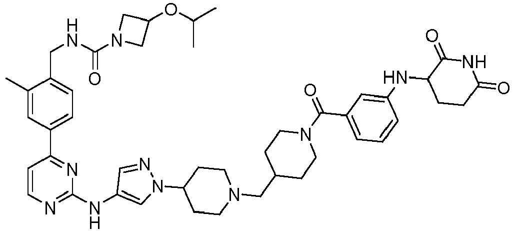

Example 91 was synthesized using the same method as Step 1 and Step 2 of Example 7 and Example 86, using tert-butyl 4-(4-amino-1H-pyrazol-1-yl)piperidine-1-carboxylate (Intermediate 2-7) and 3-((2,6-dioxopiperidin-3-yl)amino)benzoic acid (Intermediate 3-8) instead of tert-butyl 4-(4-aminophenyl)piperazine-1-carboxylate (Intermediate 2-1) and 3-(2,4-dioxotetrahydropyrimidin-1(2H)-yl)-4-methoxybenzoic acid (Intermediate 3-12), respectively.

### Example 92: N-(4-(2-((1-(1-((1-(3-((2,6-dioxopiperidin-3-yl)amino)benzoyl)azetidin-3-yl)methyl)piperidin-4-yl)-1H-pyrazol-4-yl)amino)pyrimidin-4-yl)-2-methylbenzyl)-3-isopropoxyazetidine-1-carboxamide

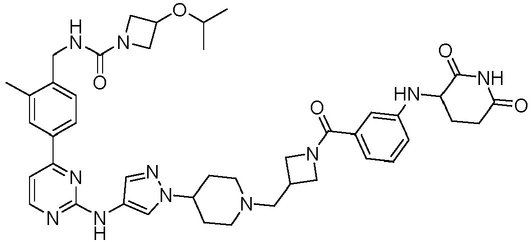

Example 92 was synthesized using the same method as Step 1 and Step 2 of Example 7 and Example 86, using tert-butyl 4-(4-amino-1H-pyrazol-1-yl)piperidine-1-carboxylate (Intermediate 2-7), tert-butyl 3-formylazetidine-1-carboxylate, and 3-((2,6-dioxopiperidin-3-yl)amino)benzoic acid (Intermediate 3-8) instead of tert-butyl 4-(4-aminophenyl)piperazine-1-carboxylate (Intermediate 2-1), tert-butyl 4-formylpiperidine-1-carboxylate, and 3-(2,4-dioxotetrahydropyrimidin-1(2H)-yl)-4-methoxybenzoic acid (Intermediate 3-12), respectively.

### Example 93: N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1-isopropyl-1H-pyrazole-4-carboxamide

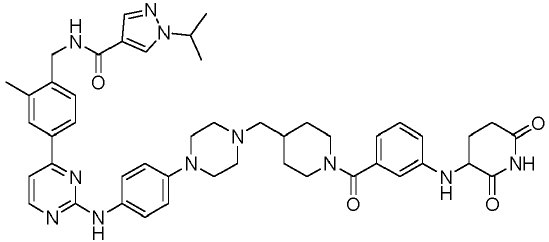

Example 93 was synthesized using the same method as Step 1 and Step 2 of Example 7 and Example 86, using N-(4-(2-chloropyrimidin-4-yl)-2-methylbenzyl)-1-isopropyl-1H-pyrazole-4-carboxamide (Intermediate 1-6) and 3-((2,6-dioxopiperidin-3-yl)amino)benzoic acid (Intermediate 3-8) instead of N-(4-(2-chloropyrimidin-4-yl)-2-methylbenzyl)-3-isopropoxyazetidine-1-carboxamide (Intermediate 1-1) and 3-(2,4-dioxotetrahydropyrimidin-1(2H)-yl)-4-methoxybenzoic acid (Intermediate 3-12), respectively.

### Example 94: N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1-isopropyl-1H-pyrazole-4-carboxamide

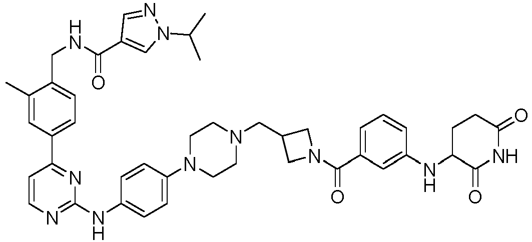

Example 94 was synthesized using the same method as Step 1 and Step 2 of Example 7 and Example 86, using N-(4-(2-chloropyrimidin-4-yl)-2-methylbenzyl)-1-isopropyl-1H-pyrazole-4-carboxamide (Intermediate 1-6), tert-butyl 3-formylazetidine-1-carboxylate, and 3-((2,6-dioxopiperidin-3-yl)amino)benzoic acid (Intermediate 3-8) instead of N-(4-(2-chloropyrimidin-4-yl)-2-methylbenzyl)-3-isopropoxyazetidine-1-carboxamide (Intermediate 1-1), tert-butyl 4-formylpiperidine-1-carboxylate, and 3-(2,4-dioxotetrahydropyrimidin-1(2H)-yl)-4-methoxybenzoic acid (Intermediate 3-12), respectively.

### Example 95: 1-(tert-butyl)-N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1H-pyrazole-4-carboxamide

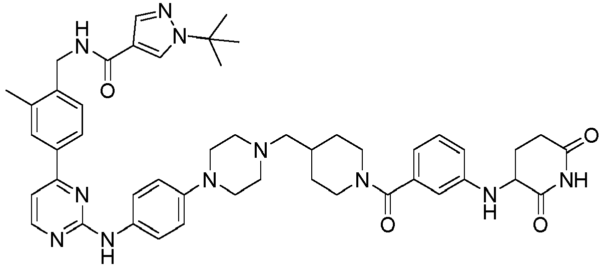

Example 95 was synthesized using the same method as Step 1 and Step 2 of Example 7 and Example 86, using 1-(tert-butyl)-N-(4-(2-chloropyrimidin-4-yl)-2-methylbenzyl)-1H-pyrazole-4-carboxamide (Intermediate 1-4) and 3-((2,6-dioxopiperidin-3-yl)amino)benzoic acid (Intermediate 3-8) instead of N-(4-(2-chloropyrimidin-4-yl)-2-methylbenzyl)-3-isopropoxyazetidine-1-carboxamide (Intermediate 1-1) and 3-(2,4-dioxotetrahydropyrimidin-1(2H)-yl)-4-methoxybenzoic acid (Intermediate 3-12), respectively.

### Example 96: 1-(tert-butyl)-N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1H-pyrazole-4-carboxamide

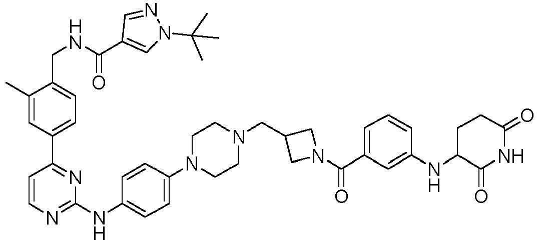

Example 96 was synthesized using the same method as Step 1 and Step 2 of Example 7 and Example 86, using 1-(tert-butyl)-N-(4-(2-chloropyrimidin-4-yl)-2-methylbenzyl)-1H-pyrazole-4-carboxamide (Intermediate 1-4), tert-butyl 3-formylazetidine-1-carboxylate, and 3-((2,6-dioxopiperidin-3-yl)amino)benzoic acid (Intermediate 3-8) instead of N-(4-(2-chloropyrimidin-4-yl)-2-methylbenzyl)-3-isopropoxyazetidine-1-carboxamide (Intermediate 1-1), tert-butyl 4-formylpiperidine-1-carboxylate, and 3-(2,4-dioxotetrahydropyrimidin-1(2H)-yl)-4-methoxybenzoic acid (Intermediate 3-12), respectively.

### Example 97: 1-(tert-butyl)-N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1H-1,2,3-triazole-4-carboxamide

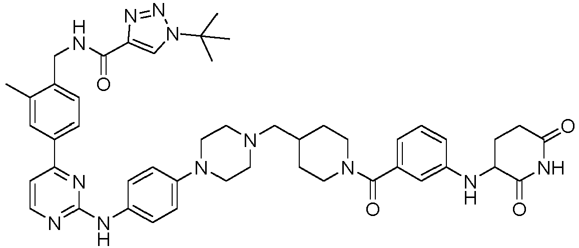

Example 97 was synthesized using the same method as Step 1 and Step 2 of Example 7 and Example 86, using 1-(tert-butyl)-N-(4-(2-chloropyrimidin-4-yl)-2-methylbenzyl)-1H-1,2,3-triazole-4-carboxamide (Intermediate 1-5) and 3-((2,6-dioxopiperidin-3-yl)amino)benzoic acid (Intermediate 3-8) instead of N-(4-(2-chloropyrimidin-4-yl)-2-methylbenzyl)-3-isopropoxyazetidine-1-carboxamide (Intermediate 1-1) and 3-(2,4-dioxotetrahydropyrimidin-1(2H)-yl)-4-methoxybenzoic acid (Intermediate 3-12), respectively.

### Example 98: 1-(tert-butyl)-N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1H-1,2,3-triazole-4-carboxamide

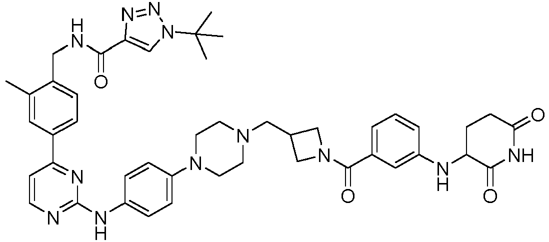

Example 98 was synthesized using the same method as Step 1 and Step 2 of Example 7 and Example 86, using 1-(tert-butyl)-N-(4-(2-chloropyrimidin-4-yl)-2-methylbenzyl)-1H-1,2,3-triazole-4-carboxamide (Intermediate 1-5), tert-butyl 3-formylazetidine-1-carboxylate, and 3-((2,6-dioxopiperidin-3-yl)amino)benzoic acid (Intermediate 3-8) instead of N-(4-(2-chloropyrimidin-4-yl)-2-methylbenzyl)-3-isopropoxyazetidine-1-carboxamide (Intermediate 1-1), tert-butyl 4-formylpiperidine-1-carboxylate, and 3-(2,4-dioxotetrahydropyrimidin-1(2H)-yl)-4-methoxybenzoic acid (Intermediate 3-12), respectively.

### Example 99: N-(4-(2-((4-(1-((1-(3-((2,6-dioxopiperidin-3-yl)amino)benzoyl)piperidin-4-yl)methyl)piperidin-4-yl)phenyl)amino)pyrimidin-4-yl)-2-methylbenzyl)-3-isopropoxyazetidine-1-carboxamide

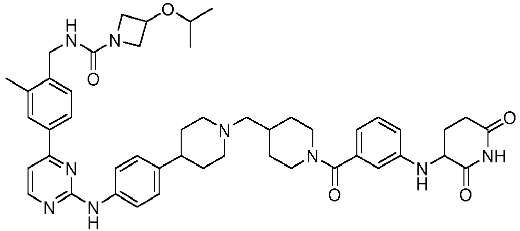

Example 99 was synthesized using the same method as Step 1 and Step 2 of Example 7 and Example 86, using tert-butyl 4-(4-aminophenyl)piperidine-1-carboxylate (Intermediate 2-5) and 3-((2,6-dioxopiperidin-3-yl)amino)benzoic acid (Intermediate 3-8) instead of tert-butyl 4-(4-aminophenyl)piperazine-1-carboxylate (Intermediate 2-1) and 3-(2,4-dioxotetrahydropyrimidin-1(2H)-yl)-4-methoxybenzoic acid (Intermediate 3-12), respectively.

### Example 100: N-(4-(2-((4-(1-((1-(3-((2,6-dioxopiperidin-3-yl)amino)benzoyl)azetidin-3-yl)methyl)piperidin-4-yl)phenyl)amino)pyrimidin-4-yl)-2-methylbenzyl)-3-isopropoxyazetidine-1-carboxamide

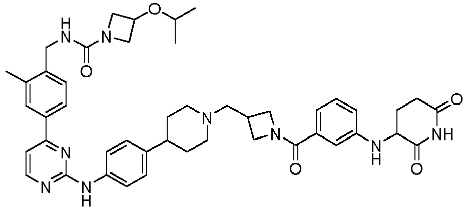

Example 100 was synthesized using the same method as Step 1 and Step 2 of Example 7 and Example 86, using tert-butyl 4-(4-aminophenyl)piperidine-1-carboxylate (Intermediate 2-5), tert-butyl 3-formylazetidine-1-carboxylate, and 3-((2,6-dioxopiperidin-3-yl)amino)benzoic acid (Intermediate 3-8) instead of tert-butyl 4-(4-aminophenyl)piperazine-1-carboxylate (Intermediate 2-1), tert-butyl 4-formylpiperidine-1-carboxylate, and 3-(2,4-dioxotetrahydropyrimidin-1(2H)-yl)-4-methoxybenzoic acid (Intermediate 3-12), respectively.

### Example 101: N-(4-(2-((4-(1-((1-(3-((2,6-dioxopiperidin-3-yl)amino)benzoyl)piperidin-4-yl)methyl)azetidin-3-yl)phenyl)amino)pyrimidin-4-yl)-2-methylbenzyl)-3-isopropoxyazetidine-1 -carboxamide

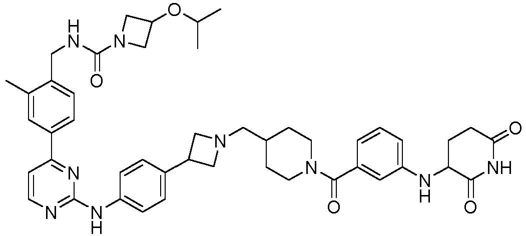

Example 101 was synthesized using the same method as Step 1 and Step 2 of Example 7 and Example 86, using tert-butyl 3-(4-aminophenyl)azetidine-1-carboxylate and 3-((2,6-dioxopiperidin-3-yl)amino)benzoic acid (Intermediate 3-8) instead of tert-butyl 4-(4-aminophenyl)piperazine-1-carboxylate (Intermediate 2-1) and 3-(2,4-dioxotetrahydropyrimidin-1(2H)-yl)-4-methoxybenzoic acid (Intermediate 3-12), respectively.

### Example 102: N-(4-(2-((4-(4-((1-(3-((2,6-dioxopiperidin-3-yl)oxy)benzoyl)piperidin-4-yl)methyl)piperazin-1-yl)phenyl)amino)pyrimidin-4-yl)-2-methylbenzyl)-3-isopropoxyazetidine-1-carboxamide

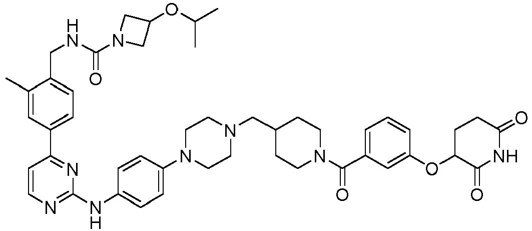

Example 102 was synthesized using the same method as Example 86, using 3-((2,6-dioxopiperidin-3-yl)oxy)benzoic acid (Intermediate 3-14) instead of 3-(2,4-dioxotetrahydropyrimidin-1(2H)-yl)-4-methoxybenzoic acid (Intermediate 3-12).

### Example 103: N-(4-(2-((4-(4-(1-(3-((2,6-dioxopiperidin-3-yl)amino)benzoyl)piperidin-4-yl)piperazin-1-yl)phenyl)amino)pyrimidin-4-yl)-2-methylbenzyl)-3-isopropoxyazetidine-1-carboxamide

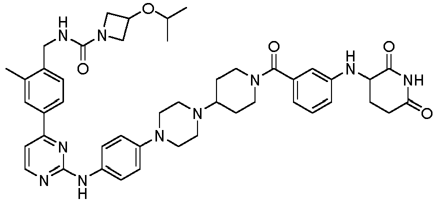

Example 103 was synthesized using the same method as Example 86, using tert-butyl 4-oxopiperidine-1-carboxylate and 3-((2,6-dioxopiperidin-3-yl)amino)benzoic acid (Intermediate 3-8) instead of tert-butyl 4-formylpiperidine-1-carboxylate and 3-(2,4-dioxotetrahydropyrimidin-1(2H)-yl)-4-methoxybenzoic acid (Intermediate 3-12), respectively.

### Example 104: N-(4-(2-((4-(1'-(3-((2,6-dioxopiperidin-3-yl)amino)benzoyl)-[1,4'-bipiperidin]-4-yl)phenyl)amino)pyrimidin-4-yl)-2-methylbenzyl)-3-isopropoxy azetidine-1-carboxamide

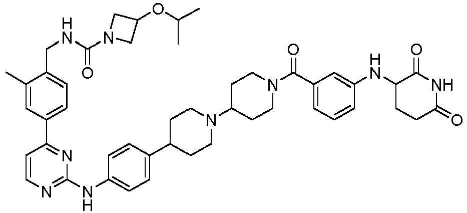

Example 104 was synthesized using the same method as Step 1 and Step 2 of Example 7 and Example 86, using tert-butyl 4-(4-aminophenyl)piperidine-1-carboxylate (Intermediate 2-5), tert-butyl 4-oxopiperidine-1-carboxylate, and 3-((2,6-dioxopiperidin-3-yl)amino)benzoic acid (Intermediate 3-8) instead of tert-butyl 4-(4-aminophenyl)piperazine-1-carboxylate (Intermediate 2-1), tert-butyl 4-formylpiperidine-1-carboxylate, and 3-(2,4-dioxotetrahydropyrimidin-1(2H)-yl)-4-methoxybenzoic acid (Intermediate 3-12), respectively.

### Example 105: N-(4-(2-((4-(4-((1-(2-((3-((2,6-dioxopiperidin-3-yl)amino)phenyl)amino)-2-oxoethyl)piperidin-4-yl)methyl)piperazin-1-yl)phenyl)amino)pyrimidin-4-yl)-2-methylbenzyl)-3-isopropoxyazetidine-1-carboxamide

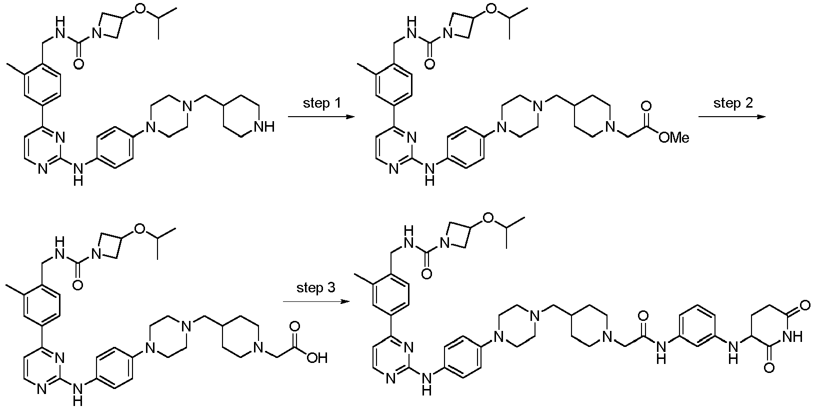

### Step 1: Synthesis of methyl 2-(4-((4-(4-((4-(4-((3-isopropoxyazetidine-1-carboxamido)methyl)-3-methylphenyl)pyrimidin-2-yl)amino)phenyl)piperazin-1-yl)methyl)piperidin-1 -yl)acetate

3-isopropoxy-N-(2-methyl-4-(2-((4-(4-(piperidin-4-ylmethyl)piperazin-1-yl)phenyl)amino)pyrimidin-4-yl)benzyl)azetidine-1-carboxamide (50 mg, 0.08 mmol) and methyl 2-bromoacetate (15 mg, 0.10 mmol) were suspended in DCM (10 mL). Then, triethylamine (0.03 mL, 0.25 mmol) was added at 0 °C and stirred at room temperature for 2 hours. The reaction solution was concentrated under reduced pressure, and the resulting residue was subjected to MPLC (5-8% MeOH/DCM) to obtain 41 mg (73%) of a yellow solid.

### Step 2: Synthesis of 2-(4-((4-(4-((4-(4-((3-isopropoxyazetidine-1-carboxamido)methyl)-3-methylphenyl)pyrimidin-2-yl)amino)phenyl)piperazin-1-yl)methyl)piperidin-1-yl)acetic acid

Methyl 2-(4-((4-(4-((4-(4-((3-isopropoxyazetidine-1-carboxamido)methyl)-3-methylphenyl)pyrimidin-2-yl)amino)phenyl)piperazin-1-yl)methyl)piperidin-1-yl)acetate (41 mg, 0.06 mmol) was suspended in EtOH (2 mL). Then, lithium hydroxide (5 mg, 0.12 mmol) was added and stirred at room temperature for 16 hours. The reaction solution was concentrated under reduced pressure to obtain 34 mg of a yellow solid.

### Step 3: Synthesis of N-(4-(2-((4-(4-((1-(2-((3-((2,6-dioxopiperidin-3-yl)amino)phenyl)amino)-2-oxoethyl)piperidin-4-yl)methyl)piperazin-1-yl)phenyl)amino)pyrimidin-4-yl)-2-methylbenzyl)-3-isopropoxyazetidine-l-carboxamide

3-((3-aminophenyl)amino)piperidine-2,6-dione (Intermediate 3-7, 33 mg, 0.05 mmol) was suspended in DMF (0.5 mL). Then, HOBT (8 mg, 0.06 mmol), TBTU (19 mg, 0.06 mmol), and DIPEA (0.03 mL, 0.15 mmol) were added. 2-(4-((4-(4-((4-(4-((3-isopropoxyazetidine-1-carboxamido)methyl)-3-methylphenyl)pyrimidin-2-yl)amino)phenyl)piperazin-1-yl)methyl)piperidin-1-yl)acetic acid (33 mg, 0.05 mmol) dissolved in DMF(0.5 mL) was added thereto and stirred at room temperature for 16 hours. NHCl₄ aqueous solution (30 mL) was added to the reaction solution, and then extracted with EtOAc (10 mL x 3). The organic layer was dried over anhydrous sodium sulfate, filtered, and concentrated under reduced pressure. The resulting residue was subjected to MPLC (5-8% MeOH/DCM) to obtain 8 mg (15%, 2 steps yield) of a yellow solid.

### Example 106: N-(3-(2-((4-(4-((1-(2-((3-((2,6-dioxopiperidin-3-yl)amino)phenyl)amino)-2-oxoethyl)piperidin-4-yl)methyl)piperazin-1-yl)phenyl)amino)pyrimidin-4-yl)-5-fluoro-2-methylphenyl)-3-isopropoxyazetidine-1-carboxamide

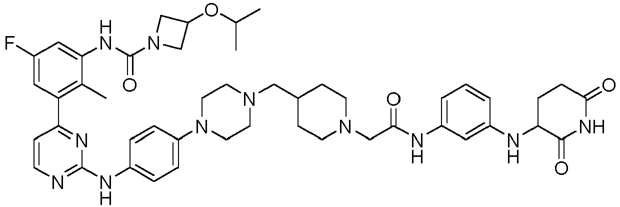

Example 106 was synthesized using the same method as Step 1 and Step 2 of Example 7, Step 1 and Step 2 of Example 86, and Example 105, using N-(3-(2-chloropyrimidin-4-yl)-5-fluoro-2-methylphenyl)-3-isopropoxyazetidine-1-carboxamide (Intermediate 1-8) instead of N-(4-(2-chloropyrimidin-4-yl)-2-methylbenzyl)-3-isopropoxyazetidine-1-carboxamide (Intermediate 1-1).

### Example 107: N-(4-(2-((4-(4-((1-(6-(2,6-dioxopiperidin-3-yl)-5-oxo-6,7-dihydro-5H-pyrrolo[3,4-b]pyridin-2-yl)azetidin-3-yl)methyl)piperazin-1-yl)phenyl)amino)pyrimidin-4-yl)-2-methylbenzyl)-3-isopropoxyazetidine-1-carboxamide

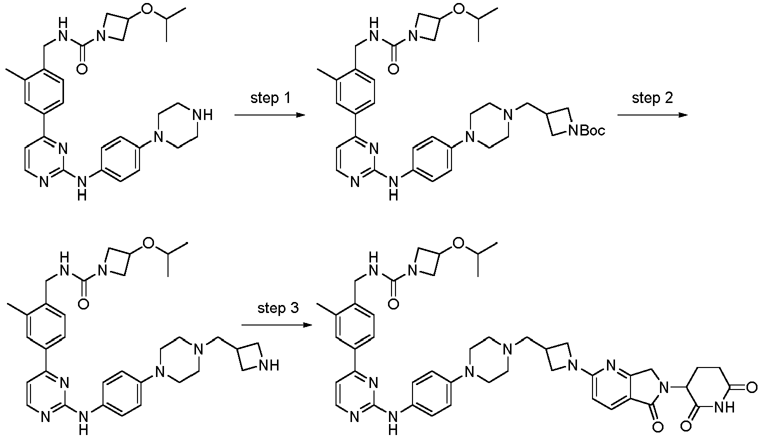

### Step 1: Synthesis of tert-butyl 3-((4-(4-((4-(4-((3-isopropoxyazetidine-1-carboxamido)methyl)-3-methylphenyl)pyrimidin-2-yl)amino)phenyl)piperazin-1-yl)methyl)azetidine-1-carboxylate

3-isopropoxy-N-(2-methyl-4-(2-((4-(piperazin-1-yl)phenyl)amino)pyrimidin-4-yl)benzyl)azetidine-1-carboxamide (50 mg, 0.10 mmol) and tert-butyl 3-formylazetidine-1-carboxylate (22 mg, 0.12 mmol) were suspended in DCM (2 mL). Then, NaBH(OAc)₃ (64 mg, 0.30 mmol) was added and stirred at room temperature for 2 hours. NaHCO₃ aqueous solution (30 mL) was added to the reaction solution and extracted with DCM (10 mL x 3). The organic layer was dried over anhydrous sodium sulfate, filtered, and concentrated under reduced pressure. The resulting residue was subjected to MPLC (5-8% MeOH/DCM) to obtain 65 mg (98%) of a yellow solid.

### Step 2: Synthesis of N-(4-(2-((4-(4-(azetidin-3-ylmethyl)piperazin-1-yl)phenyl)amino)pyrimidin-4-yl)-2-methylbenzyl)-3-isopropoxyazetidine-l-carboxamide

Tert-butyl 3-((4-(4-((4-(4-((3-isopropoxyazetidine-1-carboxamido)methyl)-3-methylphenyl)pyrimidin-2-yl)amino)phenyl)piperazin-1-yl)methyl)azetidine-1-carboxylate (65 mg, 0.10 mmol) was suspended in DCM (4 mL). Then, TFA (1 mL) was added and stirred at room temperature for 2 hours. NaHCO₃ aqueous solution (30 mL) was added to the reaction solution and extracted with DCM (10 mL x 3). The organic layer was dried over anhydrous sodium sulfate, filtered, and concentrated under reduced pressure to obtain 53 mg (95%) of a yellow solid.

### Step 3: Synthesis of N-(4-(2-((4-(4-((1-(6-(2,6-dioxopiperidin-3-yl)-5-oxo-6,7-dihydro-SH-pyrrolo[3,4-b]pyridin-2-yl)azetidin-3-yl)methyl)piperazin-1-yl)phenyl)amino)pyrimidin-4-yl)-2-methylbenzyl)-3-isopropoxyazetidine-1-carboxamide

N-(4-(2-((4-(4-(azetidin-3-ylmethyl)piperazin-1-yl)phenyl)amino)pyrimidin-4-yl)-2-methylbenzyl)-3-isopropoxyazetidine-1-carboxamide (33 mg, 0.06 mmol), 3-(2-chloro-5-oxo-5,7-dihydro-6H-pyrrolo[3,4-b]pyridin-6-yl)piperidine-2,6-dione (25 mg, 0.09 mmol), and DIPEA (0.03 mL, 0.18 mmol) were suspended in DMSO (2 mL) and stirred at 120 °C for 16 hours. NH₄Cl aqueous solution (30 mL) was added to the reaction solution, and then extracted with EtOAc (10 mL x 3). The organic layer was dried over anhydrous sodium sulfate, filtered, and concentrated under reduced pressure. The resulting residue was subjected to MPLC (5-8% MeOH/DCM) to obtain 19 mg (41%) of a yellow solid.

### Example 108: N-(3-(2-((4-(4-((1-(6-(2,6-dioxopiperidin-3-yl)-5-oxo-6,7-dihydro-5H-pyrrolo[3,4-b]pyridin-2-yl)azetidin-3-yl)methyl)piperazin-1-yl)phenyl)amino)pyrimidin-4-yl)-5-fluoro-2-methylphenyl)-3-isopropoxyazetidine-1-carboxamide

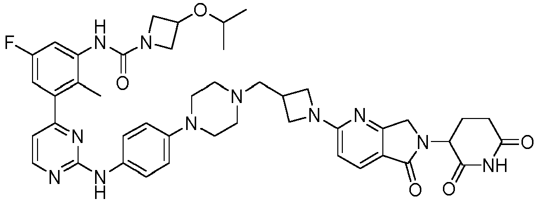

Example 108 was synthesized using the same method as Step 1 and Step 2 of Example 7 and Example 107, using N-(3-(2-chloropyrimidin-4-yl)-5-fluoro-2-methylphenyl)-3-isopropoxyazetidine-1-carboxamide (Intermediate 1-8) instead of N-(4-(2-chloropyrimidin-4-yl)-2-methylbenzyl)-3-isopropoxyazetidine-1-carboxamide (Intermediate 1-1).

### Example 109: N-(4-(2-((4-(4-(1-(2-(2,6-dioxopiperidin-3-yl)-1,3-dioxoisoindolin-5-yl)piperidin-4-yl)piperazin-1-yl)phenyl)amino)pyrimidin-4-yl)-2-methylbenzyl)-3-isopropoxyazetidine-1 -carboxamide

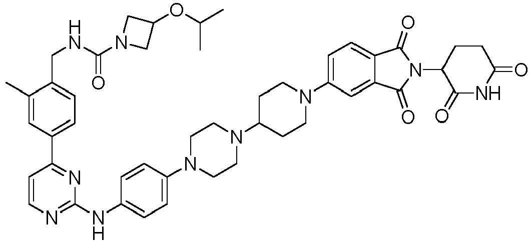

Example 109 was synthesized using the same method as Example 107, using tert-butyl 4-oxopiperidine-1-carboxylate and 2-(2,6-dioxopiperidin-3-yl)-5-fluoroisoindoline-1,3-dione instead of tert-butyl 3-formylazetidine-1-carboxylate and 3-(2-chloro-5-oxo-5,7-dihydro-6H-pyrrolo[3,4-b]pyridin-6-yl)piperidine-2,6-dione, respectively.

### Example 110: N-(4-(2-((4-(1'-(2-(2,6-dioxopiperidin-3-yl)-1,3-dioxoisoindolin-5-yl)-[1,4'-bipiperidin]-4-yl)phenyl)amino)pyrimidin-4-yl)-2-methylbenzyl)-3-isopropoxyazetidine-1-carboxamide

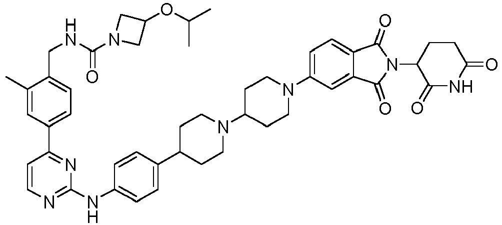

Example 110 was synthesized using the same method as Step 1 and Step 2 of Example 7 and Example 107, using tert-butyl 4-(4-aminophenyl)piperidine-1-carboxylate (Intermediate 2-5), tert-butyl 4-oxopiperidine-1-carboxylate, and 2-(2,6-dioxopiperidin-3-yl)-5-fluoroisoindoline-1,3-dione instead of tert-butyl 4-(4-aminophenyl)piperazine-1-carboxylate (Intermediate 2-1), tert-butyl 3-formylazetidine-1-carboxylate, and 3-(2-chloro-5-oxo-5,7-dihydro-6H-pyrrolo[3,4-b]pyridin-6-yl)piperidine-2,6-dione, respectively.

### Example 111: N-(4-(2-((4-(4-((4-(2-(2,6-dioxopiperidin-3-yl)-1,3-dioxoisoindolin-5-yl)piperazin-1-yl)methyl)piperidin-1-yl)phenyl)amino)pyrimidin-4-yl)-2-methylbenzyl)-3-isopropoxyazetidine-1 -carboxamide

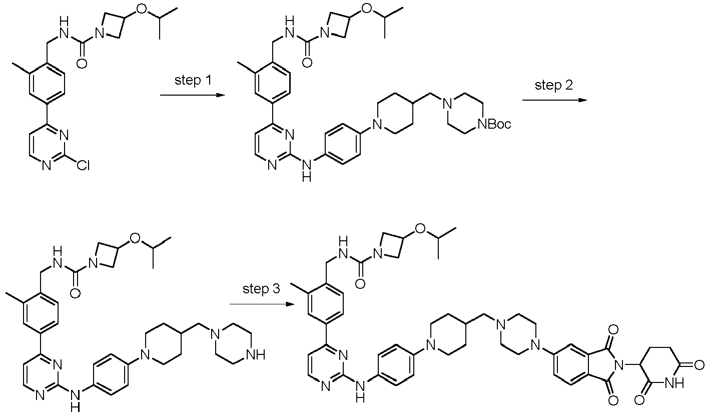

### Step 1: Synthesis of tert-butyl 4-((1-(4-((4-(4-((3-isopropoxyazetidine-1-carboxamido)methyl)-3-methylphenyl)pyrimidin-2-yl)amino)phenyl)piperidin-4-yl)methyl)piperazine-1-carboxylate

N-(4-(2-chloropyrimidin-4-yl)-2-methylbenzyl)-3-isopropoxyazetidine-1-carboxamide (Intermediate 1-1, 53 mg, 0.14 mmol), tert-butyl 4-((1-(4-aminophenyl)piperidin-4-yl)methyl)piperazine-1-carboxylate (Intermediate 2-8, 58 mg, 0.15 mmol), Pd₂(dba)₃ (13 mg, 0.01 mmol), S-phos (12 mg, 0.03 mmol), and Cs₂CO₃ (91.2 mg, 0.28 mmol) were suspended in dioxane (2 mL) and stirred at 100 °C for 3 hours. The reaction solution was filtered and concentrated under reduced pressure, and the resulting residue was subjected to MPLC (80-100% EtOAc/Hexane) to obtain 47 mg (47%) of a yellow solid.

### Step 2: Synthesis of 3-isopropoxy-N-(2-methyl-4-(2-((4-(4-(piperazin-1-ylmethyl)piperidin-1-yl)phenyl)amino)pyrimidin-4-yl)benzyl)azetidine-1-carboxamide

Tert-butyl 4-((1-(4-((4-(4-((3-isopropoxyazetidine-1-carboxamido)methyl)-3-methylphenyl)pyrimidin-2-yl)amino)phenyl)piperidin-4-yl)methyl)piperazine-1-carboxylate (47 mg, 0.06 mmol) was suspended in DCM (2 mL). Then, TFA (0.2 mL) was added and stirred at room temperature for 3 hours. The reaction solution was diluted with DCM (2 mL), then an aqueous sodium bicarbonate solution was added (pH=8∼9) and extracted with DCM (20 mL x 2). The organic layer was dried over anhydrous magnesium sulfate, filtered, and concentrated under reduced pressure to obtain 28 mg (70%) of a yellow solid.

### Step 3: Synthesis of N-(4-(2-((4-(4-((4-(2-(2,6-dioxopiperidin-3-yl)-1,3-dioxoisoindolin-5-yl)piperazin-1-yl)methyl)piperidin-1-yl)phenyl)amino)pyrimidin-4-yl)-2-methylbenzyl)-3-isopropoxyazetidine-1 -carboxamide

3-isopropoxy-N-(2-methyl-4-(2-((4-(4-(piperazin-1-ylmethyl)piperidin-1-yl)phenyl)amino)pyrimidin-4-yl)benzyl)azetidine-1-carboxamide (20 mg, 0.03 mmol), 2-(2,6-dioxopiperidin-3-yl)-5-fluoroisoindoline-1,3-dione (11 mg, 0.04 mmol), and DIPEA (0.015 mL, 0.09 mmol) were suspended in DMSO (1.15 mL) and stirred at 100 °C for 16 hours. Distilled water (30 mL) was added to the reaction solution and extracted with EtOAc (20 mL x 2). The organic layer was washed with brine (20 mL), dried over anhydrous sodium sulfate, filtered, and concentrated under reduced pressure. The resulting residue was subjected to MPLC (50-100% EA/Hex) to obtain 10 mg (38%) of a yellow solid.

### Example 112: N-(4-(2-((4-(3-((4-(2-(2,6-dioxopiperidin-3-yl)-1,3-dioxoisoindolin-5-yl)piperazin-1-yl)methyl)azetidin-1-yl)phenyl)amino)pyrimidin-4-yl)-2-methylbenzyl)-3-isopropoxyazetidine-1 -carboxamide

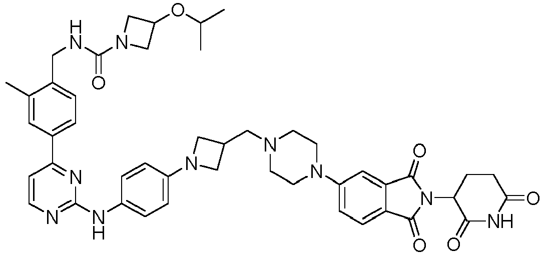

Example 112 was synthesized using the same method as Example 111, using tert-butyl 4-((1-(4-aminophenyl)azetidin-3-yl)methyl)piperazine-1-carboxylate (Intermediate 2-9) instead of tert-butyl 4-((1-(4-aminophenyl)piperidin-4-yl)methyl)piperazine-1 -carboxylate (Intermediate 2-8).

### Example 113: N-(4-(2-((4-(3-((4-(3-((2,6-dioxopiperidin-3-yl)amino)benzoyl)piperazin-1-yl)methyl)azetidin-1-yl)phenyl)amino)pyrimidin-4-yl)-2-methylbenzyl)-3-isopropoxyazetidine-1-carboxamide

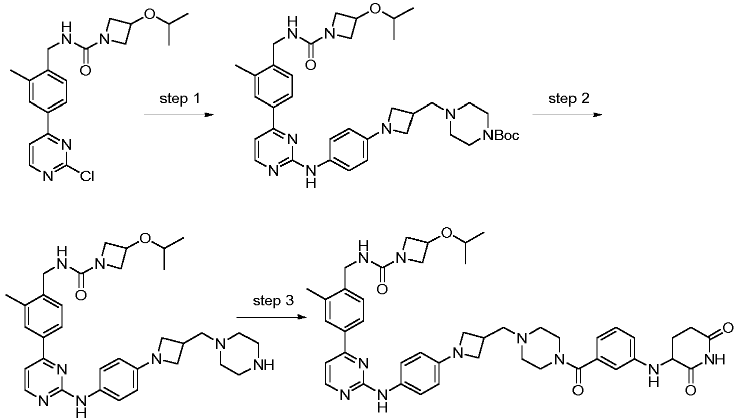

### Step 1: Synthesis of tert-butyl 4-((1-(4-((4-(4-((3-isopropoxyazetidine-1-carboxamido)methyl)-3-methylphenyl)pyrimidin-2-yl)amino)phenyl)azetidin-3-yl)methyl)piperazine-1-carboxylate

N-(4-(2-chloropyrimidin-4-yl)-2-methylbenzyl)-3-isopropoxyazetidine-1-carboxamide (Intermediate 1-1, 150 mg, 0.40 mmol), tert-butyl 4-((1-(4-aminophenyl)azetidin-3-yl)methyl)piperazine-1-carboxylate (Intermediate 2-9, 166 mg, 0.48 mmol), Pd₂(dba)₃ (37 mg, 0.04 mmol), S-phos (33 mg, 0.08 mmol), and Cs₂CO₃ (326 mg, 1.00 mmol) were suspended in dioxane (8 mL) and stirred at 100 °C for 16 hours. Distilled water (50 mL) was added to the reaction solution and extracted with EtOAc (20 mL x 3). The organic layer was dried over anhydrous sodium sulfate, filtered, and concentrated under reduced pressure. The resulting residue was subjected to MPLC (50-100% EtOAc/Hexane, 2-4% MeOH/DCM) to obtain 167 mg (61%) of a brown solid.

### Step 2: Synthesis of 3-isopropoxy-N-(2-methyl-4-(2-((4-(3-(piperazin-1-ylmethyl)azetidin-1-yl)phenyl)amino)pyrimidin-4-yl)benzyl)azetidine-1-carboxamide

Tert-butyl 4-((1-(4-((4-(4-((3-isopropoxyazetidine-1-carboxamido)methyl)-3-methylphenyl)pyrimidin-2-yl)amino)phenyl)azetidin-3-yl)methyl)piperazine-1-carboxylate (167 mg, 0.24 mmol) and TFA (0.25 mL) were suspended in DCM (0.75 mL) and stirred at room temperature for 2 hours. The reaction solution was concentrated under reduced pressure to obtain 124 mg (87%) of a brown solid.

### Step 3: Synthesis of N-(4-(2-((4-(3-((4-(3-((2,6-dioxopiperidin-3-yl)amino)benzoyl)piperazin-1-yl)methyl)azetidin-1-yl)phenyl)amino)pyrimidin-4-yl)-2-methylbenzyl)-3-isopropoxyazetidine-1-carboxamide

3-((2,6-dioxopiperidin-3-yl)amino)benzoic acid (Intermediate 3-8, 20 mg, 0.08 mmol) was suspended in DMF (2 mL). Then, HATU (10 mg, 0.07 mmol) and DIPEA (0.04 mL, 0.21 mmol) were added and 3-isopropoxy-N-(2-methyl-4-(2-((4-(3-(piperazin-1-ylmethyl)azetidin -1-yl)phenyl)amino)pyrimidin-4-yl)benzyl)azetidine-1-carboxamide (40 mg, 0.07 mmol) was slowly added to the reaction solution. The mixture was stirred at room temperature for 16 hours, distilled water (30 mL) was added to the reaction solution, and the mixture was extracted with EtOAc (15 mL x 3). The organic layer was dried over anhydrous sodium sulfate, filtered, and concentrated under reduced pressure. The resulting residue was subjected to MPLC (amine silica, 1-3% MeOH/DCM) to obtain 26 mg (47%) of a yellow solid.

### Example 114: N-(4-(2-((4-(4-((4-(3-((2,6-dioxopiperidin-3-yl)amino)benzoyl)piperazin-1-yl)methyl)piperidin-1-yl)phenyl)amino)pyrimidin-4-yl)-2-methylbenzyl)-3-isopropoxyazetidine-1 -carboxamide

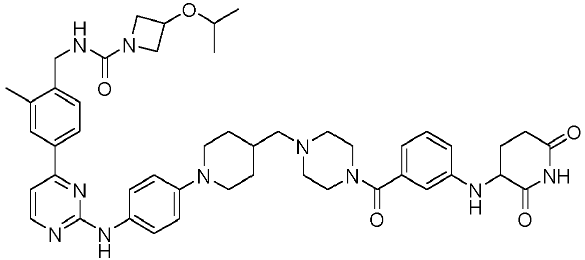

Example 114 was synthesized using the same method as Example 113, using tert-butyl 4-((1-(4-aminophenyl)piperidin-4-yl)methyl)piperazine-1-carboxylate (Intermediate 2-8) instead of tert-butyl 4-((1-(4-aminophenyl)azetidin-3-yl)methyl)piperazine-1-carboxylate (Intermediate 2-9).

### Example 115: N-(4-(2-((4-(4-((1-(3-((2,6-dioxopiperidin-3-yl)amino)benzyl)piperidin-4-yl)methyl)piperazin-1-yl)phenyl)amino)pyrimidin-4-yl)-2-methylbenzyl)-3-isopropoxyazetidine-1-carboxamide

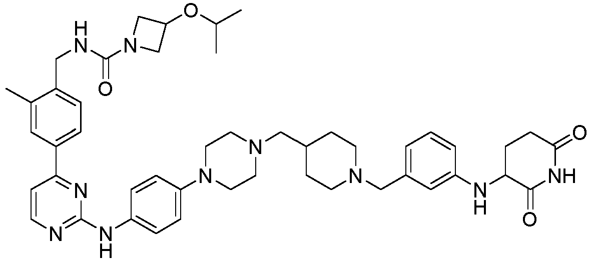

3-isopropoxy-N-(2-methyl-4-(2-((4-(4-(piperidin-4-ylmethyl)piperazin-1-yl)phenyl)amino)pyrimidin-4-yl)benzyl)azetidine-1-carboxamide (20 mg, 0.03 mmol), 3-((3-(bromomethyl)phenyl)amino)piperidine-2,6-dione (Intermediate 3-13, 14 mg, 0.05 mmol), and K₂CO₃ (9 mg, 0.07 mmol) were suspended in DMF (1 mL) and stirred at 80 °C for 16 hours. Distilled water (30 mL) was added to the reaction solution, and then extracted with EtOAc (10 mL x 3). The organic layer was dried over anhydrous sodium sulfate, filtered, and concentrated under reduced pressure. The resulting residue was subjected to MPLC (NH silica, 1-2% MeOH/DCM) to obtain 6.2 mg (23%) of a yellow solid.

### Example 116: N-(4-(2-((4-(4-(4-(3-((2,6-dioxopiperidin-3-yl)amino)phenyl)piperidine-1-carbonyl)piperidin-1-yl)phenyl)amino)pyrimidin-4-yl)-2-methylbenzyl)-3-isopropoxyazetidine-1-carboxamide

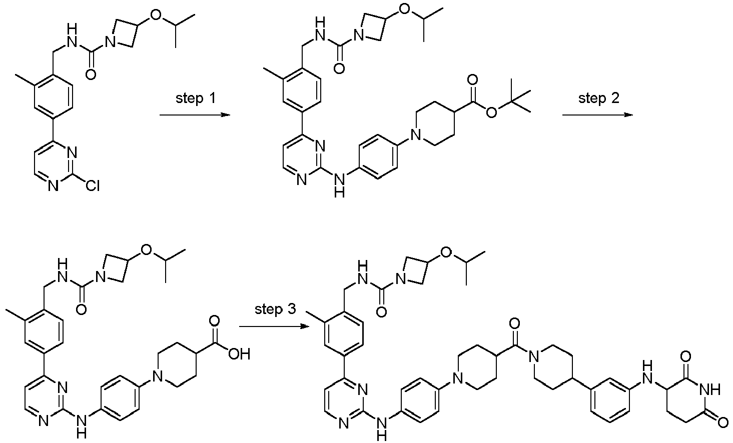

### Step 1: Synthesis of tert-butyl 1-(4-((4-(4-((3-isopropoxyazetidine-1-carboxamido)methyl)-3-methylphenyl)pyrimidin-2-yl)amino)phenyl)piperidine-4-carboxylate

N-(4-(2-chloropyrimidin-4-yl)-2-methylbenzyl)-3-isopropoxyazetidine-1-carboxamide (Intermediate 1-1, 300 mg, 0.80 mmol), tert-butyl 1-(4-aminophenyl)piperidine-4-carboxylate (Intermediate 2-10, 442 mg, 1.60 mmol), Pd₂(dba)₃ (147 mg, 0.16 mmol), S-Phos (99 mg, 0.24 mmol), and Cs₂CO₃ (782 mg, 2.40 mmol) were suspended in 1,4-dioxane (5 mL) and stirred at 100 °C for 16 hours. The reaction solution was filtered through Celite and concentrated under reduced pressure. The resulting residue was subjected to MPLC (0-100% EtOAc/Hexane) to obtain 266 mg (54%) of a brown solid.

### Step 2: Synthesis of 1-(4-((4-(4-((3-isopropoxyazetidine-1-carboxamido)methyl)-3-methylphenyl)pyrimidin-2-yl)amino)phenyl)piperidine-4-carboxylic acid

Tert-butyl 1-(4-((4-(4-((3-isopropoxyazetidine-1-carboxamido)methyl)-3-methylphenyl)pyrimidin-2-yl)amino)phenyl)piperidine-4-carboxylate (260 mg, 0.42 mmol) and TFA (2 mL) were suspended in DCM (6 mL) and stirred at room temperature for 3 hours. The reaction solution was concentrated under reduced pressure to obtain 230 mg (97%) of a yellow solid.

### Step 3: Synthesis of N-(4-(2-((4-(4-(4-(3-((2,6-dioxopiperidin-3-yl)amino)phenyl)piperidine-1-carbonyl)piperidin-1-yl)phenyl)amino)pyrimidin-4-yl)-2-methylbenzyl)-3-isopropoxyazetidine-1-carboxamide

1-(4-((4-(4-((3-isopropoxyazetidine-1-carboxamido)methyl)-3-methylphenyl)pyrimidin-2-yl)amino)phenyl)piperidine-4-carboxylic acid (60 mg, 0.10 mmol), 3-((3-(piperidin-4-yl)phenyl)amino)piperidine-2,6-dione hydrochloride (Intermediate 3-10, 40 mg, 0.14 mmol), HATU (61 mg, 0.16 mmol), and DIPEA (0.1 mL, 0.50 mmol) were suspended in DMF (2 mL) and stirred at room temperature for 16 hours. Distilled water (30 mL) was added to the reaction solution and extracted with EtOAc (20 mL x 2). The organic layer was washed with brine (20 mL), dried over anhydrous sodium sulfate, filtered, and concentrated under reduced pressure. The resulting residue was subjected to PTLC (7% MeOH/DCM) to obtain 41 mg (46%) of a yellow solid.

### Example 117: N-(4-(2-((4-(4-(4-(4-((2,6-dioxopiperidin-3-yl)amino)phenyl)piperidine-1-carbonyl)piperidin-1-yl)phenyl)amino)pyrimidin-4-yl)-2-methylbenzyl)-3-isopropoxyazetidine-1-carboxamide

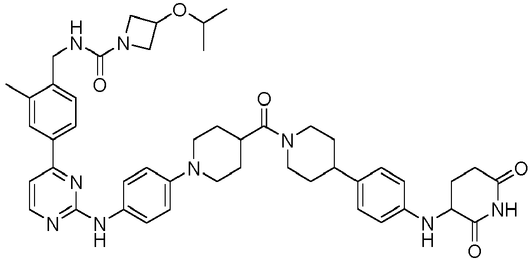

Example 117 was synthesized using the same method as Example 116, using 3-((4-(piperidin-4-yl)phenyl)amino)piperidine-2,6-dione hydrochloride (Intermediate 3-9) instead of 3-((3-(piperidin-4-yl)phenyl)amino)piperidine-2,6-dione hydrochloride (Intermediate 3-10).

### Example 118: N-(4-(2-((4-(4-(4-(4-((2,6-dioxopiperidin-3-yl)oxy)phenyl)piperidine-1-carbonyl)piperidin-1-yl)phenyl)amino)pyrimidin-4-yl)-2-methylbenzyl)-3-isopropoxyazetidine-1-carboxamide

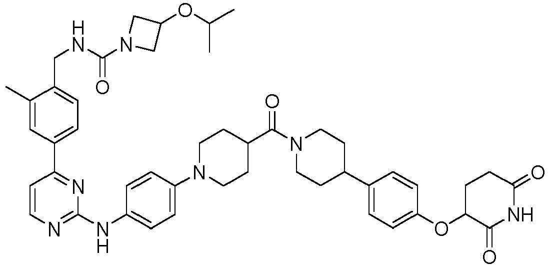

Example 118 was synthesized using the same method as Example 116, using 3-(4-(piperidin-4-yl)phenoxy)piperidine-2,6-dione hydrochloride (Intermediate 3-17) instead of 3-((3-(piperidin-4-yl)phenyl)amino)piperidine-2,6-dione hydrochloride (Intermediate 3-10).

### Example 119: N-(4-(2-((4-(4-((4-(4-((2,6-dioxopiperidin-3-yl)oxy)phenyl)piperidin-1-yl)methyl)piperidin-1-yl)phenyl)amino)pyrimidin-4-yl)-2-methylbenzyl)-3-isopropoxyazetidine-1-carboxamide

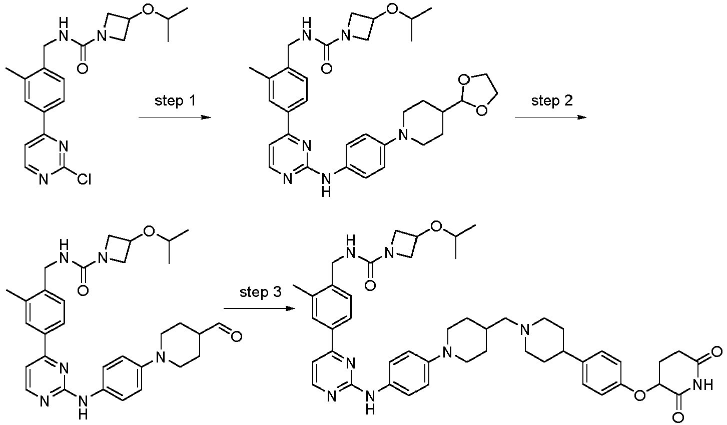

### Step 1: Synthesis of N-(4-(2-((4-(4-(1,3-dioxolan-2-yl)piperidin-1-yl)phenyl)amino)pyrimidin-4-yl)-2-methylbenzyl)-3-isopropoxyazetidine-l-carboxamide

N-(4-(2-chloropyrimidin-4-yl)-2-methylbenzyl)-3-isopropoxyazetidine-1-carboxamide (Intermediate 1-1, 100 mg, 0.27 mmol), 4-(4-(1,3-dioxolan-2-yl)piperidin-1-yl)aniline (Intermediate 2-11, 133 mg, 0.53 mmol), Pd₂(dba)₃ (49 mg, 0.053 mmol), S-phos (33 mg, 0.08 mmol), and Cs₂CO₃ (174 mg, 0.53 mmol) were suspended in dioxane (4 mL) and stirred at 100 °C for 16 hours. Distilled water (30 mL) was added to the reaction solution, and then extracted with EtOAc (10 mL x 3). The organic layer was dried over anhydrous sodium sulfate, filtered, and concentrated under reduced pressure. The resulting residue was subjected to MPLC (50-100% EtOAc/Hexane) to obtain 87 mg (56%) of a yellow solid.

### Step 2: Synthesis of N-(4-(2-((4-(4-formylpiperidin-1-yl)phenyl)amino)pyrimidin-4-yl)-2-methylbenzyl)-3-isopropoxyazetidine-1-carboxamide

N-(4-(2-((4-(4-(1,3-dioxolan-2-yl)piperidin-1-yl)phenyl)amino)pyrimidin-4-yl)-2-methylbenzyl)-3-isopropoxyazetidine-1-carboxamide (87 mg, 0.15 mmol) was suspended in DCM (2 mL). Then, TFA (2 mL) was added and stirred at 50 °C for 16 hours. The reaction solution was concentrated under reduced pressure, and the resulting residue was subjected to MPLC (5-8% MeOH/DCM) to obtain 44 mg (55%) of a yellow solid.

### Step 3: Synthesis of N-(4-(2-((4-(4-((4-(4-((2,6-dioxopiperidin-3-yl)oxy)phenyl)piperidin-1-yl)methyl)piperidin-1-yl)phenyl)amino)pyrimidin-4-yl)-2-methylbenzyl)-3-isopropoxyazetidine-1-carboxamide

N-(4-(2-((4-(4-formylpiperidin-1-yl)phenyl)amino)pyrimidin-4-yl)-2-methylbenzyl)-3-isopropoxyazetidine-1-carboxamide (22 mg, 0.04 mmol) and 3-(4-(piperidin-4-yl)phenoxy)piperidine-2,6-dione hydrochloride (Intermediate 3-17, 16 mg, 0.05 mmol) were suspended in DCM (2 mL) and stirred for 10 minutes. NaBH(OAc)₃ (17 mg, 0.08 mmol) was added and stirred at room temperature for 16 hours. NaHCO₃ aqueous solution (30 mL) was added to the reaction solution and extracted with DCM (10 mL x 3). The organic layer was dried over anhydrous sodium sulfate, filtered, and concentrated under reduced pressure. The resulting residue was subjected to MPLC (2-8% MeOH/DCM) to obtain 11 mg (32%) of a yellow solid.

### Example 120: N-(4-(2-((4-(4-((4-(3-((2,6-dioxopiperidin-3-yl)amino)phenyl)piperazin-1-yl)methyl)piperidin-1-yl)phenyl)amino)pyrimidin-4-yl)-2-methylbenzyl)-3-isopropoxyazetidine-1-carboxamide

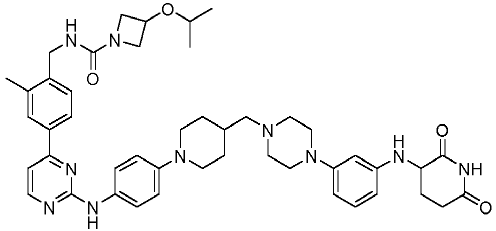

Example 120 was synthesized using the same method as Example 119, using 3-((3-(piperazin-1-yl)phenyl)amino)piperidine-2,6-dione hydrochloride (Intermediate 3-21) instead of 3-(4-(piperidin-4-yl)phenoxy)piperidine-2,6-dione hydrochloride (Intermediate 3-17).

### Example 121: N-(4-(2-((4-(4-((4-(4-((2,6-dioxopiperidin-3-yl)amino)phenyl)piperazin-1-yl)methyl)piperidin-1-yl)phenyl)amino)pyrimidin-4-yl)-2-methylbenzyl)-3-isopropoxyazetidine-1-carboxamide

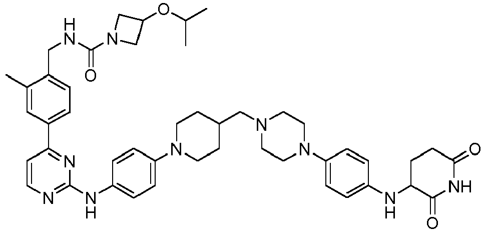

Example 121 was synthesized using the same method as Example 119, using 3-((4-(piperazin-1-yl)phenyl)amino)piperidine-2,6-dione hydrochloride (Intermediate 3-22) instead of 3-(4-(piperidin-4-yl)phenoxy)piperidine-2,6-dione hydrochloride (Intermediate 3-17).

### Example 122: N-(4-(2-((4-(4-((4-(3-((2,6-dioxopiperidin-3-yl)amino)phenyl)piperidin-1-yl)methyl)piperidin-1-yl)phenyl)amino)pyrimidin-4-yl)-2-methylbenzyl)-3-isopropoxyazetidine-1-carboxamide

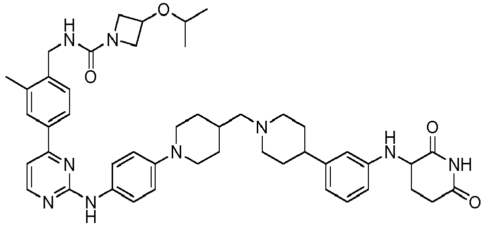

Example 122 was synthesized using the same method as Example 119, using 3-((3-(piperidin-4-yl)phenyl)amino)piperidine-2,6-dione hydrochloride (Intermediate 3-10) instead of 3-(4-(piperidin-4-yl)phenoxy)piperidine-2,6-dione hydrochloride (Intermediate 3-17).

### Example 123: N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-3-isopropoxyazetidine-1-carboxamide

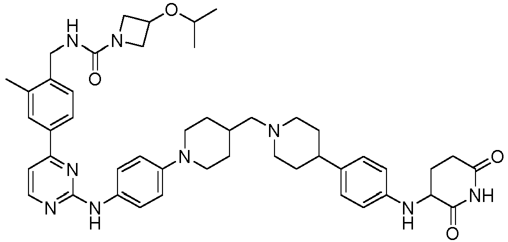

Example 123 was synthesized using the same method as Example 119, using 3-((4-(piperidin-4-yl)phenyl)amino)piperidine-2,6-dione hydrochloride (Intermediate 3-9) instead of 3-(4-(piperidin-4-yl)phenoxy)piperidine-2,6-dione hydrochloride (Intermediate 3-17).

### Example 124: N-(4-(2-((4-(4-((4-(5-((2,6-dioxopiperidin-3-yl)oxy)pyridin-2-yl)piperidin-1 -yl)methyl)piperidin-1 -yl)phenyl)amino)pyrimidin-4-yl)-2-methylbenzyl)-3 - isopropoxyazetidine-1 -carboxamide

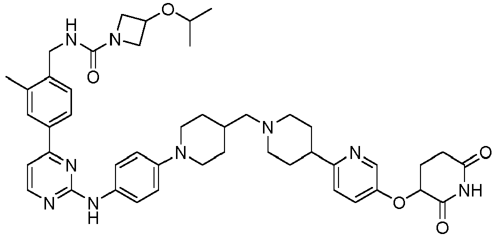

Example 124 was synthesized using the same method as Example 119, using 3-((6-(piperidin-4-yl)pyridin-3-yl)oxy)piperidine-2,6-dione hydrochloride (Intermediate 3-27) instead of 3-(4-(piperidin-4-yl)phenoxy)piperidine-2,6-dione hydrochloride (Intermediate 3-17).

### Example 125: N-(4-(2-((4-(4-((4-(5-((2,6-dioxopiperidin-3-yl)oxy)pyridin-2-yl)piperazin-1-yl)methyl)piperidin-1-yl)phenyl)amino)pyrimidin-4-yl)-2-methylbenzyl)-3-isopropoxyazetidine-1 -carboxamide

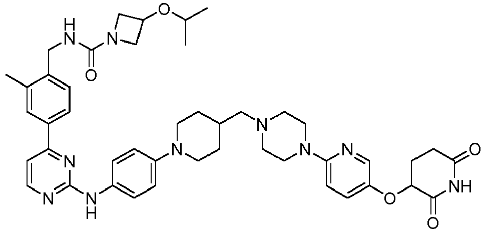

Example 125 was synthesized using the same method as Example 119, using 3-((6-(piperazin-1-yl)pyridin-3-yl)oxy)piperidine-2,6-dione hydrochloride (Intermediate 3-28) instead of 3-(4-(piperidin-4-yl)phenoxy)piperidine-2,6-dione hydrochloride (Intermediate 3-17).

### Example 126: N-(4-(2-((4-(4-((4-(5-((2,6-dioxopiperidin-3-yl)amino)pyridin-2-yl)piperidin-1 -yl)methyl)piperidin-1 -yl)phenyl)amino)pyrimidin-4-yl)-2-methylbenzyl)-3 - isopropoxyazetidine-1 -carboxamide

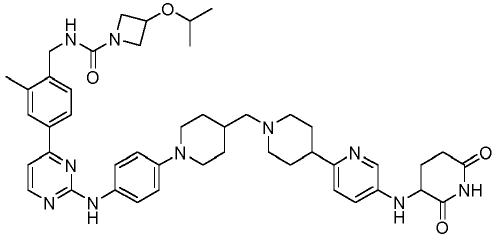

Example 126 was synthesized using the same method as Example 119, using 3-((6-(piperidin-4-yl)pyridin-3-yl)amino)piperidine-2,6-dione hydrochloride (Intermediate 3-19) instead of 3-(4-(piperidin-4-yl)phenoxy)piperidine-2,6-dione hydrochloride (Intermediate 3-17).

### Example 127: N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-3-isopropoxyazetidine-1 -carboxamide

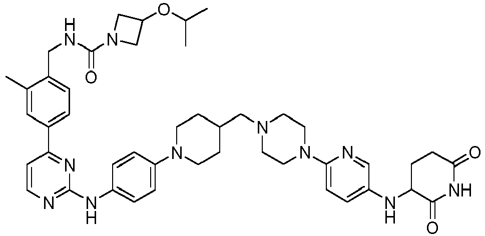

Example 127 was synthesized using the same method as Example 119, using 3-((6-(piperazin-1-yl)pyridin-3-yl)amino)piperidine-2,6-dione hydrochloride (Intermediate 3-20) instead of 3-(4-(piperidin-4-yl)phenoxy)piperidine-2,6-dione hydrochloride (Intermediate 3-17).

### Example 128: N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1-isopropyl-1H-pyrazole-4-carboxamide

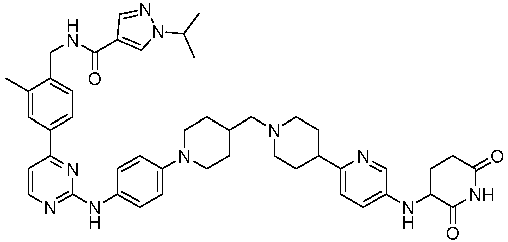

Example 128 was synthesized using the same method as Example 119, using N-(4-(2-chloropyrimidin-4-yl)-2-methylbenzyl)-1-isopropyl-1H-pyrazole-4-carboxamide (Intermediate 1-6) and 3-((6-(piperidin-4-yl)pyridin-3-yl)amino)piperidine-2,6-dione hydrochloride (Intermediate 3-19) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 129: N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1-isopropyl-1H-pyrazole-4-carboxamide

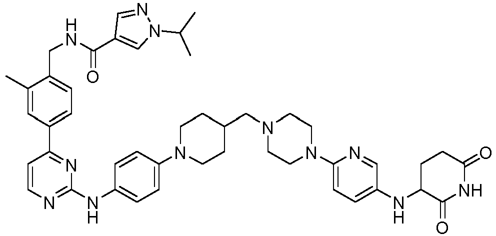

Example 129 was synthesized using the same method as Example 119, using N-(4-(2-chloropyrimidin-4-yl)-2-methylbenzyl)-1-isopropyl-1H-pyrazole-4-carboxamide (Intermediate 1-6) and 3-((6-(piperazin-1-yl)pyridin-3-yl)amino)piperidine-2,6-dione hydrochloride (Intermediate 3-20) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 130: 1-(tert-butyl)-N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1H-pyrazole-4-carboxamide

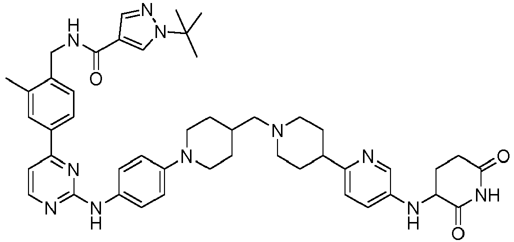

Example 130 was synthesized using the same method as Example 119, using 1-(tert-butyl)-N-(4-(2-chloropyrimidin-4-yl)-2-methylbenzyl)-1H-pyrazole-4-carboxamide (Intermediate 1-4) and 3-((6-(piperidin-4-yl)pyridin-3-yl)amino)piperidine-2,6-dione hydrochloride (Intermediate 3-19) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 131: 1-(tert-butyl)-N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1H-pyrazole-4-carboxamide

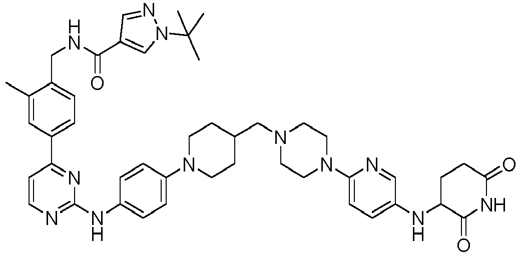

Example 131 was synthesized using the same method as Example 119, using 1-(tert-butyl)-N-(4-(2-chloropyrimidin-4-yl)-2-methylbenzyl)-1H-pyrazole-4-carboxamide (Intermediate 1-4) and 3-((6-(piperazin-1-yl)pyridin-3-yl)amino)piperidine-2,6-dione hydrochloride (Intermediate 3-20) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 132: 1-(tert-butyl)-N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1H-1,2,3-triazole-4-carboxamide

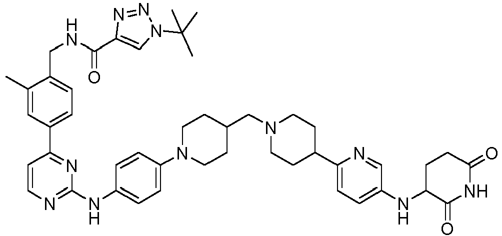

Example 132 was synthesized using the same method as Example 119, using 1-(tert-butyl)-N-(4-(2-chloropyrimidin-4-yl)-2-methylbenzyl)-1H-1,2,3-triazole-4-carboxamide (Intermediate 1-5) and 3-((6-(piperidin-4-yl)pyridin-3-yl)amino)piperidine-2,6-dione hydrochloride (Intermediate 3-19) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 133: 1-(tert-butyl)-N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1H-1,2,3-triazole-4-carboxamide

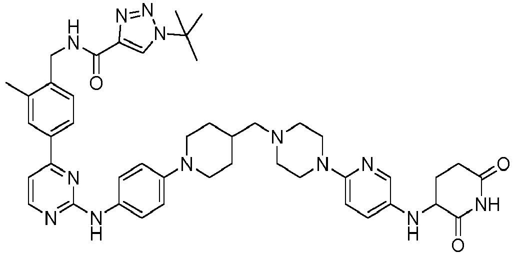

Example 133 was synthesized using the same method as Example 119, using 1-(tert-butyl)-N-(4-(2-chloropyrimidin-4-yl)-2-methylbenzyl)-1H-1,2,3-triazole-4-carboxamide (Intermediate 1-5) and 3-((6-(piperazin-1-yl)pyridin-3-yl)amino)piperidine-2,6-dione hydrochloride (Intermediate 3-20) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 134: N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1-isopropyl-1H-pyrazole-4-carboxamide

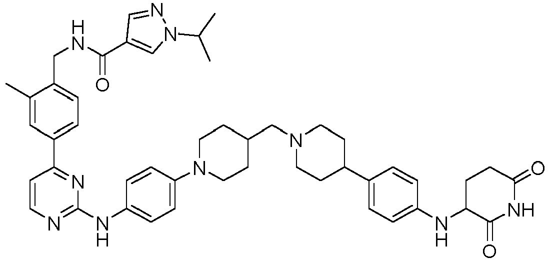

Example 134 was synthesized using the same method as Example 119, using N-(4-(2-chloropyrimidin-4-yl)-2-methylbenzyl)-1-isopropyl-1H-pyrazole-4-carboxamide (Intermediate 1-6) and 3-((4-(piperidin-4-yl)phenyl)amino)piperidine-2,6-dione hydrochloride (Intermediate 3-9) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 135: 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1H-pyrazole-4-carboxamide

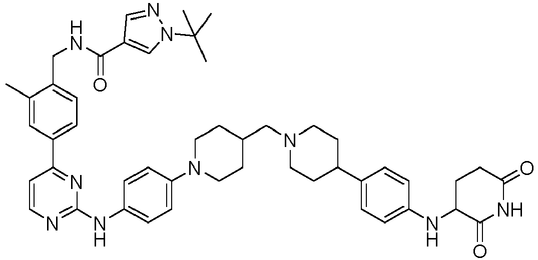

Example 135 was synthesized using the same method as Example 119, using 1-(tert-butyl)-N-(4-(2-chloropyrimidin-4-yl)-2-methylbenzyl)-1H-pyrazole-4-carboxamide (Intermediate 1-4) and 3-((4-(piperidin-4-yl)phenyl)amino)piperidine-2,6-dione hydrochloride (Intermediate 3-9) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 136: 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1H-1,2,3-triazole-4-carboxamide

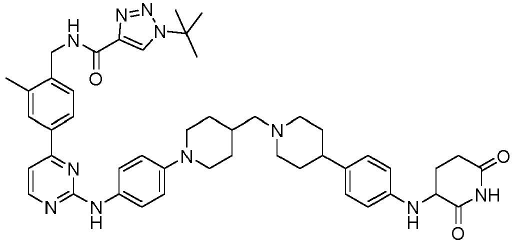

Example 136 was synthesized using the same method as Example 119, using 1-(tert-butyl)-N-(4-(2-chloropyrimidin-4-yl)-2-methylbenzyl)-1H-1,2,3-triazole-4-carboxamide (Intermediate 1-5) and 3-((4-(piperidin-4-yl)phenyl)amino)piperidine-2,6-dione hydrochloride (Intermediate 3-9) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 137: 1-(tert-butyl)-N-((5-(2-((4-(4-((4-(4-((2,6-dioxopiperidin-3-yl)amino)phenyl)piperidin-1-yl)methyl)piperidin-1-yl)phenyl)amino)pyrimidin-4-yl)-3-methylpyridin-2-yl)methyl)-1H-1,2,3-triazole-4-carboxamide

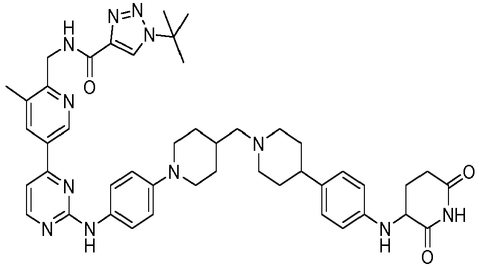

Example 137 was synthesized using the same method as Example 119, using 1-(tert-butyl)-N-((5-(2-chloropyrimidin-4-yl)-3-methylpyridin-2-yl)methyl)-1H-1,2,3-triazole-4-carboxamide (Intermediate 1-7) and 3-((4-(piperidin-4-yl)phenyl)amino)piperidine-2,6-dione hydrochloride (Intermediate 3-9) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 138: 1 -(tert-butyl)-N-(4-(2-((4-(4-((4-(3 -(2,4-dioxotetrahydropyrimidin-1 (2H)-yl)phenyl)piperidin-1-yl)methyl)piperidin-1-yl)phenyl)amino)pyrimidin-4-yl)-2-methylbenzyl)-1H-1,2,3-triazole-4-carboxamide

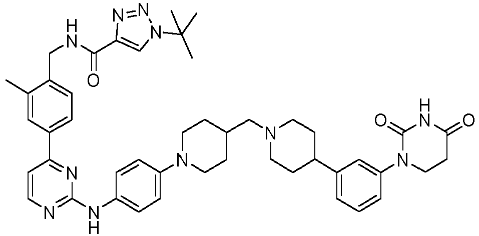

Example 138 was synthesized using the same method as Example 119, using 1-(tert-butyl)-N-(4-(2-chloropyrimidin-4-yl)-2-methylbenzyl)-1H-1,2,3-triazole-4-carboxamide (Intermediate 1-5) and 1-(3-(piperidin-4-yl)phenyl)dihydropyrimidine-2,4(1H,3H)-dione (Intermediate 3-32) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 139: 1-(tert-butyl)-N-(4-(2-((4-(4-((4-(4-(2,4-dioxotetrahydropyrimidin-1(2H)-yl)phenyl)piperidin-1-yl)methyl)piperidin-1-yl)phenyl)amino)pyrimidin-4-yl)-2-methylbenzyl)-1H-1,2,3-triazole-4-carboxamide

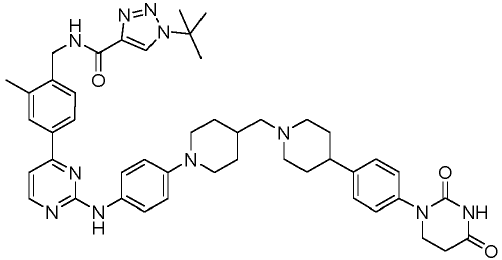

Example 139 was synthesized using the same method as Example 119, using 1-(tert-butyl)-N-(4-(2-chloropyrimidin-4-yl)-2-methylbenzyl)-1H-1,2,3-triazole-4-carboxamide (Intermediate 1-5) and 1-(4-(piperidin-4-yl)phenyl)dihydropyrimidine-2,4(1H,3H)-dione (Intermediate 3-33) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 140: 1-(tert-butyl)-N-(4-(2-((4-((4-((4-(3-(2,4-dioxotetrahydropyrimidin-1(2H)-yl)phenyl)piperidin-1-yl)methyl)piperidin-1-yl)methyl)phenyl)amino)pyrimidin-4-yl)-2-methylbenzyl)-1H-1,2,3-triazole-4-carboxamide

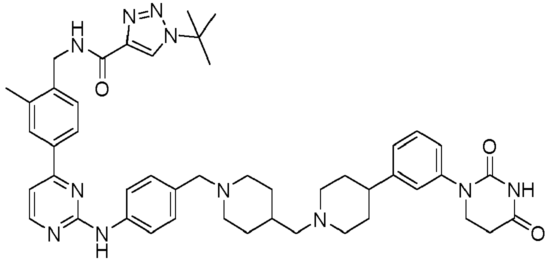

Example 140 was synthesized using the same method as Example 119, using 1-(tert-butyl)-N-(4-(2-chloropyrimidin-4-yl)-2-methylbenzyl)-1H-1,2,3-triazole-4-carboxamide (Intermediate 1-5), 4-((4-(1,3-dioxolan-2-yl)piperidin-1-yl)methyl)aniline (Intermediate 2-14), and 1-(3-(piperidin-4-yl)phenyl)dihydropyrimidine-2,4(1H,3H)-dione (Intermediate 3-32) instead of N-(4-(2-chloropyrimidin-4-yl)-2-methylbenzyl)-3-isopropoxyazetidine-1-carboxamide (Intermediate 1-1), 4-(4-(1,3-dioxolan-2-yl)piperidin-1-yl)aniline (Intermediate 2-11), and 3-(4-(piperidin-4-yl)phenoxy)piperidine-2,6-dione hydrochloride (Intermediate 3-17), respectively.

### Example 141: 1-(tert-butyl)-N-(4-(2-((4-((4-((4-(4-(2,4-dioxotetrahydropyrimidin-1(2H)-yl)phenyl)piperidin-1-yl)methyl)piperidin-1-yl)methyl)phenyl)amino)pyrimidin-4-yl)-2-methylbenzyl)-1H-1,2,3-triazole-4-carboxamide

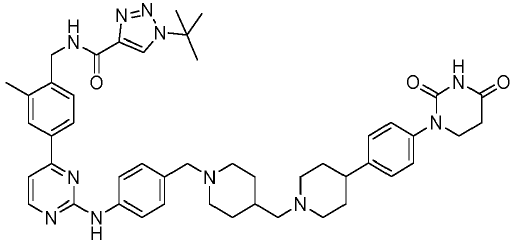

Example 141 was synthesized using the same method as Example 119, using 1-(tert-butyl)-N-(4-(2-chloropyrimidin-4-yl)-2-methylbenzyl)-1H-1,2,3-triazole-4-carboxamide (Intermediate 1-5), 4-((4-(1,3-dioxolan-2-yl)piperidin-1-yl)methyl)aniline (Intermediate 2-14), and 1-(4-(piperidin-4-yl)phenyl)dihydropyrimidine-2,4(1H,3H)-dione (Intermediate 3-33) instead of N-(4-(2-chloropyrimidin-4-yl)-2-methylbenzyl)-3-isopropoxyazetidine-1-carboxamide (Intermediate 1-1), 4-(4-(1,3-dioxolan-2-yl)piperidin-1-yl)aniline (Intermediate 2-11), and 3-(4-(piperidin-4-yl)phenoxy)piperidine-2,6-dione hydrochloride (Intermediate 3-17), respectively.

### Example 142: 1-(tert-butyl)-N-(4-(2-((4-((4-((4-(4-((2,6-dioxopiperidin-3-yl)amino)phenyl)piperidin-1-yl)methyl)piperidin-1-yl)methyl)phenyl)amino)pyrimidin-4-yl)-2-methylbenzyl)-1H-1,2,3-triazole-4-carboxamide

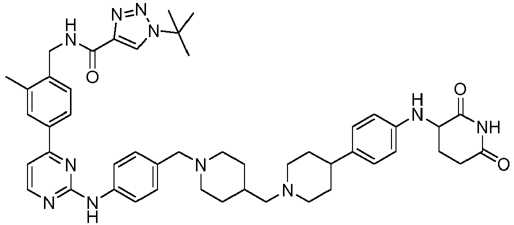

Example 142 was synthesized using the same method as Example 119, using 1-(tert-butyl)-N-(4-(2-chloropyrimidin-4-yl)-2-methylbenzyl)-1H-1,2,3-triazole-4-carboxamide (Intermediate 1-5), 4-((4-(1,3-dioxolan-2-yl)piperidin-1-yl)methyl)aniline (Intermediate 2-14), and 3-((4-(piperidin-4-yl)phenyl)amino)piperidine-2,6-dione hydrochloride (Intermediate 3-9) instead of N-(4-(2-chloropyrimidin-4-yl)-2-methylbenzyl)-3-isopropoxyazetidine-1-carboxamide (Intermediate 1-1), 4-(4-(1,3-dioxolan-2-yl)piperidin-1-yl)aniline (Intermediate 2-11), and 3-(4-(piperidin-4-yl)phenoxy)piperidine-2,6-dione hydrochloride (Intermediate 3-17), respectively.

### Example 143: 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-(hydroxymethyl)benzy1)-1H-1,2,3-triazole-4-carboxamide

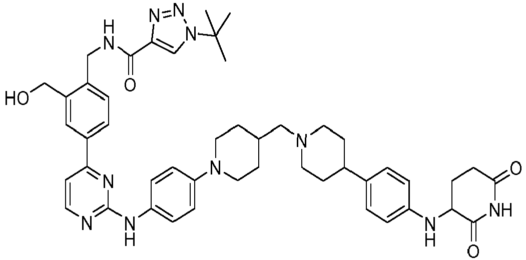

Example 143 was synthesized using the same method as Example 119, using 1-(tert-butyl)-N-(4-(2-chloropyrimidin-4-yl)-2-(hydroxymethyl)benzyl)-1H-1,2,3-triazole-4-carboxamide (Intermediate 1-12) and 3-((4-(piperidin-4-yl)phenyl)amino)piperidine-2,6-dione hydrochloride (Intermediate 3-9) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

### Example 144: N-(4-(2-((6-(4-((4-(4-((2,6-dioxopiperidin-3-yl)oxy)phenyl)piperidin-1-yl)methyl)piperidin-1-yl)pyridin-3-yl)amino)pyrimidin-4-yl)-2-methylbenzyl)-3-isopropoxyazetidine-1 -carboxamide

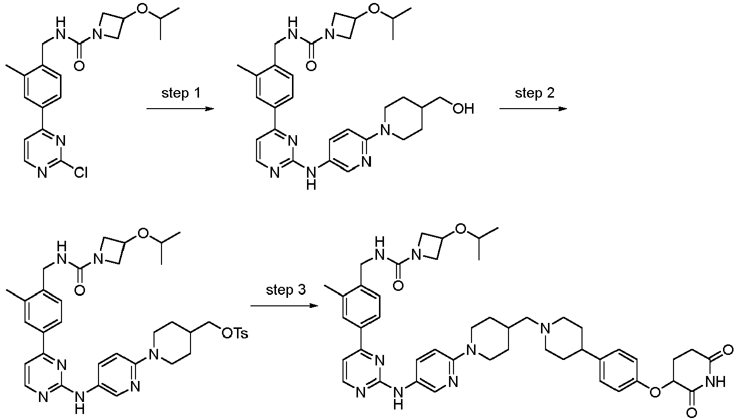

### Step 1: Synthesis of N-(4-(2-((6-(4-(hydroxymethyl)piperidin-1-yl)pyridin-3-yl)amino)pyrimidin-4-yl)-2-methylbenzyl)-3-isopropoxyazetidine-1-carboxamide

N-(4-(2-chloropyrimidin-4-yl)-2-methylbenzyl)-3-isopropoxyazetidine-1-carboxamide (Intermediate 1-1, 300 mg, 0.80 mmol), (1-(5-aminopyridin-2-yl)piperidin-4-yl)methanol (Intermediate 2-4, 245 mg, 1.18 mmol), Pd₂(dba)₃ (147 mg, 0.16 mmol), S-phos (99 mg, 0.24 mmol), and Cs₂CO₃ (521 mg, 1.60 mmol) were suspended in dioxane (8 mL) and stirred at 90 °C for 24 hours. Distilled water (70 mL) was added to the reaction solution and extracted with EtOAc (20 mL x 3). The organic layer was dried over anhydrous sodium sulfate, filtered, and concentrated under reduced pressure. The resulting residue was subjected to MPLC (50-100% EtOAc/Hexane, 5-8% MeOH/DCM) to obtain 300 mg (69%) of a yellow solid.

### Step 2: Synthesis of (1-(5-((4-(4-((3-isopropoxyazetidine-1-carboxamido)methyl)-3-methylphenyl)pyrimidin-2-yl)amino)pyridin-2-yl)piperidin-4-yl)methyl 4-methylbenzenesulfonate

N-(4-(2-((6-(4-(hydroxymethyl)piperidin-1-yl)pyridin-3-yl)amino)pyrimidin-4-yl)-2-methylbenzyl)-3-isopropoxyazetidine-1-carboxamide (146 mg, 0.27 mmol) was suspended in DCM (10 mL) and TsCl (103 mg, 0.54 mmol) was added thereto. Triethylamine (0.11 mL, 0.81 mmol) was slowly added to the reaction solution and stirred at room temperature for 16 hours. Distilled water (30 mL) was added to the reaction solution and extracted with DCM (10 mL x 3). The organic layer was dried over anhydrous sodium sulfate, filtered, and concentrated under reduced pressure. The resulting residue was subjected to MPLC (2-3% MeOH/DCM) to obtain 88 mg (47%) of a yellow solid.

### Step 3: Synthesis of N-(4-(2-((6-(4-((4-(4-((2,6-dioxopiperidin-3-yl)oxy)phenyl)piperidin-1-yl)methyl)piperidin-1-yl)pyridin-3-yl)amino)pyrimidin-4-yl)-2-methylbenzyl)-3-isopropoxyazetidine-1-carboxamide

(1-(5-((4-(4-((3-isopropoxyazetidine-1-carboxamido)methyl)-3-methylphenyl)pyrimidin-2-yl)amino)pyridin-2-yl)piperidin-4-yl)methyl 4-methylbenzenesulfonate (88 mg, 0.13 mmol), 3-(4-(piperidin-4-yl)phenoxy)piperidine-2,6-dione hydrochloride (Intermediate 3-17, 52 mg, 0.16 mmol), DIPEA (0.07 mL, 0.378 mmol), and KI (10 mg, 0.06 mmol) were suspended in MeCN (2 mL) and stirred at 85 °C for 2 hours. The reaction solution was concentrated under reduced pressure, distilled water (30 mL) was added, and extracted with DCM (10 mL x 3). The organic layer was dried over anhydrous sodium sulfate, filtered, and concentrated under reduced pressure. The resulting residue was subjected to MPLC (2-8% MeOH/DCM) to obtain 28 mg (28%) of a yellow solid.

### Example 145: N-(4-(2-((6-(4-((4-(4-((2,6-dioxopiperidin-3-yl)amino)phenyl)piperidin-1-yl)methyl)piperidin-1-yl)pyridin-3-yl)amino)pyrimidin-4-yl)-2-methylbenzyl)-3-isopropoxyazetidine-1 -carboxamide

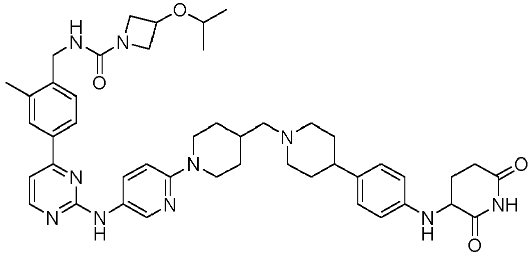

Example 145 was synthesized using the same method as Example 144, using 3-((4-(piperidin-4-yl)phenyl)amino)piperidine-2,6-dione hydrochloride (Intermediate 3-9) instead of 3-(4-(piperidin-4-yl)phenoxy)piperidine-2,6-dione hydrochloride (Intermediate 3-17).

### Example 146: 1-(tert-butyl)-N-(4-(2-((6-(4-((4-(4-((2,6-dioxopiperidin-3-yl)amino)phenyl)piperidin-1-yl)methyl)piperidin-1-yl)pyridin-3-yl)amino)pyrimidin-4-yl)-2-methylbenzyl)-1H-1,2,3-triazole-4-carboxamide

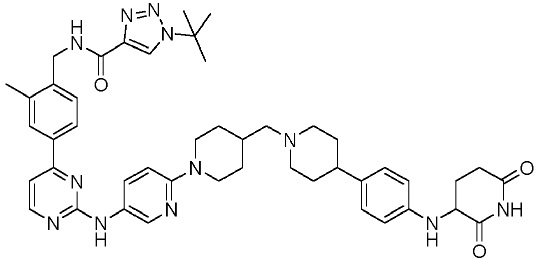

Example 146 was synthesized using the same method as Example 144, using 1-(tert-butyl)-N-(4-(2-chloropyrimidin-4-yl)-2-methylbenzyl)-1H-1,2,3-triazole-4-carboxamide (Intermediate 1-5) and 3-((4-(piperidin-4-yl)phenyl)amino)piperidine-2,6-dione hydrochloride (Intermediate 3-9) instead of N-(4-(2-chloropyrimidin-4-yl)-2-methylbenzyl)-3-isopropoxyazetidine-1-carboxamide (Intermediate 1-1) and 3-(4-(piperidin-4-yl)phenoxy)piperidine-2,6-dione hydrochloride (Intermediate 3-17), respectively.

The NMR and mass results of the compounds obtained in Example 1-146 are summarized in Table 2 below.

**[Table 2]**

| Exam ple (Com pound ) | NMR and m/z |
|---|---|
| 1 | ¹H NMR (400 MHz, CDCl₃) δ 8.42 (d, *J*=5.3 Hz, 1H), 8.30 (s, 1H), 7.90 (s, 1H), 7.84 (d, *J*=7.9 Hz 1H), 7.67 (d, *J*=8.6 Hz, 1H), 7.48 (s, 2H), 7.42 (dd, *J*=8.4, 2.0 Hz, 1H), 7.37 (d, *J*=8.0 Hz, 1H), 7.29 (d, *J*=2.1 Hz, 1H), 7.12-7.09 (m, 2H), 7.05 (dd, *J*=8.6, 2.2 Hz, 1H), 4.94 (dd, *J*=12.3, 5.3 Hz, 1H), 4.45 (d, *J*=5.6 Hz, 2H), 4.38-4.32 (m, 1H), 4.26 (m, 1H), 4.13 (t, *J*=8.0 Hz, 2H), 3.97 (d, *J*=13.0 Hz, 2H), 3.86 (dd, *J*=8.5, 4.9 Hz, 2H), 3.67 (s, 2H), 3.64-3.57 (m, 1H), 3.00 (t, *J*=11.8 Hz, 2H), 2.92-2.84 (m, 4H), 2.79-2.72 (m, 3H), 2.43 (s, 3H), 2.42 (d, *J*=8.0 Hz, 1H), 1.96 (d, *J*=12.0 Hz, 2H), 1.37-1.29 (m, 2H), 1.15 (d, *J*=6.1 Hz, 6H). m/z 840.49 [M+H]⁺. |
| 2 | ¹H NMR (400 MHz, CDCl₃) δ 8.44 (d, *J*=5.2 Hz, 1H), 8.17 (s, 1H), 8.04 (s, 1H), 7.93 (s, 1H), 7.85 (dd, *J*=8.0, 1.6 Hz 1H), 7.75 (s, 1H), 7.67 (d, *J*=8.6 Hz, 1H), 7.47 (s, 1H), 7.42-7.39 (m, 2H), 7.28 (d, *J*=2.3 Hz, 1H), 7.18 (s, 1H), 7.12 (d, *J*=8.0 Hz, 1H), 7.10 (d, *J*=8.0 Hz, 1H), 7.05 (dd, *J*=8.6, 2.3 Hz, 1H), 5.91 (t, *J*=5.4 Hz, 1H), 4.94 (dd, *J*=12.3, 5.3 Hz, 1H), 4.66 (d, *J*=5.6 Hz, 2H), 3.97 (d, *J*=13.1 Hz, 2H), 3.66 (s, 2H), 3.00 (t, *J*=11.8 Hz, 2H), 2.92-2.81 (m, 4H), 2.78-2.72 (m, 3H), 2.46 (s, 3H), 2.41 (d, *J*=6.5 Hz, 2H), 2.15-2.11 (m, 2H), 1.96 (d, *J*=12.0 Hz, 2H), 1.60 (s, 9H), 1.38-1.29 (m, 2H). m/z 849.46 [M+H]⁺. |
| 3 | ¹H NMR (400 MHz, CDCl₃) δ 8.43 (d, *J*=5.3 Hz, 1H), 8.23 (s, 1H), 8.19 (s, 1H), 7.93 (s, 1H), 7.85 (dd, *J*=8.0, 1.6 Hz 1H), 7.67 (d, *J*=8.6 Hz, 1H), 7.50 (d, *J*=1.8 Hz, 1H), 7.45-7.43 (m, 2H), 7.40 (dd, J=8.3, 2.2 Hz, 1H), 7.34 (s, 1H), 7.29 (d, *J*=2.2 Hz, 1H), 7.12 (d, *J*=5.2 Hz, 1H), 7.09 (d, *J*=8.4 Hz, 1H), 7.05 (dd, *J*=8.6, 2.3 Hz, 1H), 4.94 (dd, *J*=12.3, 5.3 Hz, 1H), 4.71 (d, *J*=5.9 Hz, 2H), 3.97 (d, *J*=13.0 Hz, 2H), 3.67 (s, 2H), 3.00 (t, *J*=11.7 Hz, 2H), 2.92-2.81 (m, 4H), 2.79-2.72 (m, 3H), 2.47 (s, 3H), 2.42-2.40 (m, 2H), 2.15-2.11 (m, 2H), 1.96 (d, *J*=11.9 Hz, 2H), 1.70 (s, 9H), 1.38-1.29 (m, 2H). m/z 850.46 [M+H]⁺. |
| 4 | ¹H NMR (400 MHz, CDCl₃) δ 8.46 (d, *J*=5.0 Hz, 1H), 8.19 (s, 1H), 7.79 (dd, *J*=10.6, 2.5 Hz, 1H), 7.67 (d, *J*=8.6 Hz, 1H), 7.40-7.34 (m, 2H), 7.28 (s, 1H), 7.21 (s, 1H), 7.06 (t, *J*=9.0 Hz, 2H), 6.89 (dd, *J*=8.6, 2.5 Hz, 1H), 6.75 (d, *J*=5.0 Hz, 1H), 5.95 (s, 1H), 4.94 (m, 1H), 4.45-4.36 (m, 1H), 4.24 (t, *J*=7.5 Hz, 2H), 3.97 (m, 4H), 3.65 (m, 3H), 3.49 (s, 1H), 3.00 (t, *J*=12.4 Hz, 2H), 2.83 (m, 4H), 2.78-2.66 (m, 3H), 2.40 (d, *J*=6.3 Hz, 2H), 2.19 (s, 3H), 2.13 (m, 1H), 1.95 (d, *J*=12.1 Hz, 2H), 1.32 (m, 2H), 1.18 (d, *J*=6.1 Hz, 6H). m/z 844.48 [M+H]⁺. |
| 5 | ¹H NMR (400 MHz, CDCl₃) δ 8.48 (d, *J*=5.0 Hz, 1H), 8.30 (s, 1H), 8.13 (s, 1H), 7.89 (dd, *J*=10.2, 2.6 Hz, 1H), 7.85 (s, 1H), 7.67 (d, *J*=8.6 Hz, 1H), 7.45 (s, 1H), 7.40-7.34 (m, 2H), 7.28 (d, *J*=2.3 Hz, 1H), 7.25 (s, 1H), 7.09-6.99 (m, 3H), 6.78 (d, *J*=5.0 Hz, 1H), 4.94 (m, 1H), 3.96 (d, *J*=13.0 Hz, 2H), 3.63 (s, 2H), 2.99 (t, *J*=11.7 Hz, 2H), 2.91-2.81 (m, 4H), 2.78-2.70 (m, 3H), 2.40 (d, *J*=6.7 Hz, 2H), 2.31 (s, 3H), 2.17-2.09 (m, 1H), 1.95 (d, *J*=12.2 Hz, 3H), 1.63 (s, 9H), 1.37-1.26 (m, 2H). m/z 853.45 [M+H]⁺. |
| 6 | ¹H NMR (400 MHz, CDCl₃) δ 9.14 (s, 1H), 8.49 (d, *J*=5.0 Hz, 1H), 8.27 (s, 1H), 8.15 (dd, *J*=10.3, 2.6 Hz, 2H), 7.67 (d, J=8.6 Hz, 1H), 7.38 (m, 2H), 7.28 (d, *J*=2.3 Hz, 1H), 7.22 (s, 1H), 7.07 (dd, *J*=9.2, 5.4 Hz, 1H), 7.04-6.98 (m, 2H), 6.79 (d, *J*=5.0 Hz, 1H), 4.94 (m, 1H), 3.96 (d, *J*=12.9 Hz, 2H), 3.63 (s, 2H), 3.00 (t, *J*=11.7 Hz, 2H), 2.93-2.82 (m, 4H), 2.82-2.75 (m, 1H), 2.74 (m, 2H), 2.40 (m, 5H), 2.13 (m, 1H), 1.94 (m, 3H), 1.74 (s, 9H), 1.32 (d, *J*=10.6 Hz, 2H). m/z 854.43 [M+H]⁺. |
| 7 | ¹H NMR (400 MHz, CDCl₃) δ 8.42 (d, *J*=5.2 Hz, 1H), 7.99 (s, 1H), 7.85-7.84 (m, 2H), 7.68 (d, *J*=8.6 Hz, 1H), 7.57 (d, *J*=9.0 Hz, 2H), 7.37 (d, *J*=7.8 Hz, 1H), 7.29 (d, *J*=5.3 Hz, 1H), 7.08 (d, *J*=8.0 Hz, 1H) 7.07-7.04 (m, 2H), 6.97 (d, *J*=9.0 Hz, 2H), 4.94 (dd, *J*=12.4, 5.3 Hz, 1H), 4.45 (d, *J*=5.6 Hz, 2H), 4.37-4.34 (m, 1H), 4.22 (t, *J*=5.6 Hz, 1H), 4.13 (t, *J*=8.0 Hz, 2H), 3.97 (d, *J*=12.8 Hz, 2H), 3.87 (dd, *J*=8.9, 4.7 Hz, 2H), 3.64-3.58 (m, 1H), 3.18 (t, *J*=4.0 Hz, 4H), 2.99 (t, *J*=11.5 Hz, 2H), 2.92-2.88 (m, 1H), 2.85-2.72 (m, 2H), 2.61 (t, *J*=4.8 Hz, 4H), 2.42 (s, 3H), 2.28 (*J*=6.8 Hz, 2H), 2.16-2.11 (m, 1H), 1.93 (d, *J*=13.1 Hz, 2H), 1.35-1.25 (m, 2H), 1.16 (d, *J*=6.4 Hz, 6H). m/z 435.45 [M+2H]²⁺. |
| 8 | ¹H NMR (400 MHz, CDCl₃) δ 8.39 (d, *J*=5.4 Hz, 1H), 8.05 (s, 1H), 8.03-8.00 (m, 2H), 7.88 (t, *J*=11.0 Hz, 2H), 7.75 (s, 1H), 7.68 (d, *J*=8.5 Hz, 1H), 7.60 (d, *J*=8.4 Hz, 1H), 7.44-7.40 (m, 2H), 7.17 (t, *J*=7.6 Hz, 1H), 7.09 (d, *J*=5.1 Hz, 1H) 7.05 (d, *J*=7.5 Hz, 1H), 6.98 (t, *J*=8.0 Hz, 2H), 5.91-5.87 (m, 1H), 4.94 (dd, *J*=11.6, 4.8 Hz, 1H), 4.67 (d, *J*=5.2 Hz, 2H), 3.97 (d, *J*=12.6 Hz, 2H), 3.23-3.14 (m, 4H), 3.00 (t, *J*=11.9 Hz, 2H), 2.93-2.79 (m, 3H), 2.73-2.68 (m, 4H), 2.47 (s, 3H), 2.38-2.35 (m, 2H), 2.15-2.14 (m, 1H), 1.97-1.92 (m, 3H), 1.61 (s, 9H), 1.38-1.31 (m, 2H). m/z 878.50 [M+H]⁺. |
| 9 | ¹H NMR (400 MHz, CDCl₃) δ 8.41 (d, *J*=5.2 Hz, 1H), 8.19 (s, 1H), 8.00 (s, 1H), 7.88 (s, 1H), 7.86 (d, *J*=8.6 Hz, 1H), 7.68 (d, *J*=8.0 Hz, 1H), 7.57 (d, *J*=8.9 Hz, 2H), 7.45-7.40 (m, 2H), 7.29 (d, *J*=2.2 Hz, 1H), 7.17 (s, 1H), 7.08 (d, *J*=5.2 Hz, 1H), 7.05 (dd, *J*=8.6, 2.2 Hz, 1H), 6.97 (d, *J*=9.0 Hz, 2H), 4.94 (dd, *J*=12.3, 5.3 Hz, 1H), 4.71 (d, *J*=5.9 Hz, 2H), 3.97 (d, *J*=13.0 Hz, 2H), 3.18 (t, *J*=4.8 Hz, 4H), 2.99 (t, *J*=11.6 Hz, 2H), 2.92-2.88 (m, 1H), 2.85-2.72 (m, 2H), 2.61 (t, *J*=4.6 Hz, 4H), 2.46 (s, 3H), 2.28 (d, *J*=7.2 Hz, 2H), 2.11 (s, 1H), 1.93 (d, *J*=13.1 Hz, 2H), 1.87-1.82 (m, 1H), 1.71 (s, 9H), 1.36-1.26 (m, 2H). m/z 879.50 [M+H]⁺. |
| 10 | ¹H NMR (400 MHz, DMSO-*d₆*) δ 10.95 (s, 1H), 9.38 (s, 1H), 8.45 (d, *J*=5.2 Hz, 1H), 7.93-7.92 (m, 2H), 7.73 (d, *J*=8.9 Hz, 1H), 7.65 (d, *J*=9.0 Hz, 2H), 7.34 (d, *J*=8.5 Hz, 1H), 7.28 (d, *J*=5.3 Hz, 1H), 6.93-6.84 (m, 4H), 5.06 (dd, *J*=13.3, 5.0 Hz, 1H), 4.44 (d, *J*=12.4 Hz, 2H), 4.35-4.27 (m, 1H), 4.22 (d, *J*=7.8 Hz, 2H), 4.10-4.02 (m, 3H), 3.62 (dd, *J*=8.9, 4.5 Hz, 2H), 3.60-3.54 (m, 1H), 3.11-3.05 (m, 4H), 2.99-2.85 (m, 3H), 2.67-2.64 (m, 2H), 2.62-2.59 (m, 1H), 2.55-2.53 (m, 4H), 2.36 (s, 3H), 2.33-2.31 (m, 2H), 2.20 (d, *J*=12.6 Hz, 2H), 1.98-1.93 (m, 2H), 1.81 (d, *J*=12.6 Hz, 2H), 1.08 (d, *J*=6.1 Hz, 6H). m/z 856.53 [M+H]⁺. |
| 11 | ¹H NMR (400 MHz, CDCl₃) δ 8.41 (d, J=5.2 Hz, 1H), 8.06 (brs, 1H), 7.86-7.82 (m, 2H), 7.63 (d, *J*=8.3 Hz, 1H), 7.57 (d, *J*=9.0 Hz, 2H), 7.37 (d, *J*=7.8 Hz, 1H), 7.08 (d, *J*=5.3 Hz, 1H), 7.07 (brs, 1H), 6.97 (d, *J*=9.0 Hz, 2H), 6.79 (d, *J*=2.0 Hz, 1H), 6.52 (dd, *J*=8.3, 2.1 Hz, 1H), 4.93 (dd, *J*=12.3, 5.3 Hz, 1H), 4.45 (d, *J*=5.6 Hz, 2H), 4.39-4.33 (m, 1 H), 4.23 (t, *J*=4.4 Hz, 1H), 4.20-4.12 (m, 4H), 3.87 (dd, *J*=8.9, 4.7 Hz, 2H), 3.75 (dd, *J*=8.0, 5.4 Hz, 2H), 3.65-3.56 (m, 1H), 3.19 (t, *J*=4.6 Hz, 4H), 3.12-3.05 (m, 1H), 2.92-2.87 (m, 1H), 2.85-2.80 (m, 1H), 2.78-2.73 (m, 2H), 2.65 (t, *J*=4.8 Hz, 4H), 2.42 (s, 3H), 2.16-2.12 (m, 1H), 1.16 (d, *J*=6.2 Hz, 6H). m/z 841.52 [M+H]⁺. |
| 12 | ¹H NMR (400 MHz, CDCl₃) δ 8.42 (d, *J*=5.2 Hz, 1H), 8.33 (s, 1H), 7.87-7.83 (m, 2H), 7.58 (d, *J*=8.9 Hz, 2H), 7.46 (d, *J*=11.0 Hz, 1H), 7.38 (dd, *J*=9.8, 8.0 Hz, 2H), 7.18 (s, 1H), 7.08 (d, *J*=5.3 Hz, 1H), 6.99-6.94 (m, 2H), 4.93 (dd, *J*=12.4, 5.3 Hz, 1H), 4.45 (d, *J*=5.6 Hz, 2H), 4.35 (tt, *J*=6.6, 4.7 Hz, 1H), 4.24 (t, *J*=5.6 Hz, 1H), 4.16-4.10 (m, 2H), 3.86 (dd, *J*=8.8, 4.7 Hz, 2H), 3.70-3.57 (m, 5H), 3.23 (s, 4H), 2.94-2.60 (m, 9H), 2.42 (s, 3H), 2.17-2.09 (m, 1H), 1.96 (d, *J*=11.9 Hz, 2H), 1.44 (d, *J*=11.5 Hz, 2H), 1.32-1.27 (m, 1H), 1.16 (d, *J*=6.1 Hz, 6H). m/z 887.54 [M+H]⁺. |
| 13 | ¹H NMR (400 MHz, CDCl₃) δ 8.41 (d, *J*=5.2 Hz, 1H), 8.05 (s, 1H), 7.88-7.83 (m, 2H), 7.59-7.55 (m, 2H), 7.39-7.34 (m, 2H), 7.08 (d, *J*=5.3 Hz, 1H), 7.04 (s, 1H), 6.98-6.94 (m, 2H), 6.82 (d, *J*=7.5 Hz, 1H), 4.91 (dd, *J*=12.4, 5.5 Hz, 1H), 4.45 (d, *J*=5.6 Hz, 2H), 4.38-4.29 (m, 3H), 4.22 (t, *J*=5.7 Hz, 1H), 4.15-4.11 (m, 2H), 3.92-3.84 (m, 4H), 3.64-3.57 (m, 1H), 3.21-3.15 (m, 4H), 3.10-3.01 (m, 1H), 2.93-2.85 (m, 1H), 2.85-2.78 (m, 1H), 2.78-2.70 (m, 3H), 2.67-2.62 (m, 4H), 2.42 (s, 3H), 2.16-2.10 (m, 1H), 1.16 (d, *J*=6.1 Hz, 6H). m/z 859.46 [M+H] +. |
| 14 | ¹H NMR (400 MHz, CDCl₃) δ 8.41 (d, *J*=5.2 Hz, 1H), 8.09 (s, 1H), 7.85 (d, *J*=7.3 Hz, 2H), 7.65 (d, *J*=8.3 Hz, 1H), 7.57 (d, *J*=9.0 Hz, 2H), 7.37 (d, *J*=7.9 Hz, 1H), 7.12 (s, 1H), 7.08 (d, *J*=5.3 Hz, 1H), 6.95 (d, *J*=9.0 Hz, 2H), 6.78 (d, *J*=2.1 Hz, 1H), 6.51 (dd, *J*=8.3, 2.1 Hz, 1H), 4.93 (m, 1H), 4.45(d, *J*=5.6 Hz, 2H), 4.38-4.31 (m, 1H), 4.23 (m, 1H), 4.13 (dd, *J*=8.1, 7.2 Hz, 2H), 3.87 (dd, *J*=8.9, 4.7 Hz, 2H), 3.81 (d, *J*=7.7 Hz, 2H), 3.74 (d, *J*=7.7 Hz, 2H), 3.64-3.58 (m, 1H), 3.20-3.14 (m, 4H), 2.92-2.69 (m, 3H), 2.62 (m, 6H), 2.42 (s, 3H), 2.11 (m, 1H), 1.47 (s, 3H), 1.16 (d, *J*=6.1 Hz, 6H). m/z 855.22 [M+H]⁺. |
| 15 | ¹H NMR (400 MHz, CDCl₃) δ 8.43 (d, *J*=5.3 Hz, 1H), 8.10 (brs, 1H), 7.87-7.85 (m, 2H), 7.68 (d, *J*=8.6 Hz, 1H), 7.65 (d, *J*=8.5 Hz, 2H), 7.44 (brs, 1H), 7.38 (d, *J*=7.8 Hz, 1H), 7.29 (d, *J*=2.2 Hz, 1H), 7.24 (d, *J*=8.4 Hz, 2H), 7.12 (d, *J*=5.3 Hz, 1H), 7.05 (dd, *J*=8.6, 2.3 Hz, 1H), 4.94 (dd, *J*=12.3, 5.4 Hz, 1H), 4.46 (d, *J*=5.6 Hz, 2H), 4.39-4.33 (m, 1H), 4.26 (t, *J*=5.6 Hz, 1H), 4.14 (t, *J*=7.4 Hz, 2H), 3.97 (d, *J*=13.4 Hz, 2H), 3.87 (dd, *J*=8.4, 4.7 Hz, 2H), 3.64-3.58 (m, 1H), 3.13 (d, *J*=11.0 Hz, 2H), 2.99 (t, *J*=12.0 Hz, 2H), 2.92-2.88 (m, 1H), 2.85-2.72 (m, 2H), 2.58-2.49 (m, 1H), 2.43 (s, 3H), 2.36 (d, *J*=6.3 Hz, 2H), 2.21-2.13 (m, 3H), 1.98-1.89 (m, 6H), 1.39-1.30 (m, 2H), 1.16 (d, *J*=6.1 Hz, 6H). m/z 868.56 [M+H]⁺. |
| 16 | ¹H NMR (400 MHz, CDCl₃) δ 8.44 (d, *J*=5.2 Hz, 1H), 8.05 (brs, 1H), 7.87-7.85 (m, 2H), 7.65 (d, *J*=5.2 Hz, 1H), 7.63 (d, *J*=6.0 Hz, 2H), 7.38 (d, *J*=7.6 Hz, 1H), 7.24-7.22 (m, 3H), 7.12 (d, *J*=5.3 Hz, 1H), 6.78 (d, *J*=2.1 Hz, 1H), 6.51 (dd, *J*=8.3, 2.1 Hz, 1H), 4.93 (dd, *J*=12.3, 5.4 Hz, 1H), 4.46 (d, *J*=5.6 Hz, 2H), 4.39-4.33 (m, 1H), 4.24 (t, *J*=6.0 Hz, 1H), 4.19 (t, *J*=8.0 Hz, 2H), 4.16-4.11 (m, 2H), 3.87 (dd, *J*=8.7, 4.7 Hz, 2H), 3.74 (dd, *J*=7.6, 5.2 Hz, 2H), 3.64-3.56 (m, 1H), 3.14-3.08 (m, 1H), 3.02 (d, *J*=9.4 Hz, 2H), 2.92-2.87 (m, 1H), 2.85-2.80 (m, 1H), 2.78-2.72 (m, 3H), 2.55-2.48 (m, 1H), 2.43 (s, 3H), 2.19-2.13 (m, 3H), 1.88-1.80 (m, 3H), 1.16 (d, *J*=6.2 Hz, 6H). m/z 840.52 [M+H]⁺. |
| 17 | ¹H NMR (400 MHz, CDCl₃) δ 8.40-8.39 (m, 2H), 8.11 (brs, 1H), 7.97 (dd, *J*=8.8, 2.4 Hz, 1H), 7.85-7.83 (m, 2H), 7.68 (d, *J*=8.6 Hz, 1H), 7.37 (d, *J*=8.2 Hz, 1H), 7.29 (s, 1H), 7.20 (brs, 1H), 7.09 (d, J=5.3 Hz, 1H), 7.06 (d, *J*=8.6 Hz, 1H), 6.72 (d, *J*=9.2 Hz, 1H), 4.94 (dd, *J*=12.4, 5.3 Hz, 1H), 4.45 (d, *J*=5.5 Hz, 2H), 4.38-4.33 (m, 1H), 4.24 (t, *J*=5.8 Hz, 1H), 4.13 (t, *J*=7.7 Hz, 2H), 3.97 (d, *J*=13.2 Hz, 2H), 3.87 (dd, *J*=8.7, 4.8 Hz, 2H), 3.64-3.58 (m, 1H), 3.56-3.50 (m, 4H), 3.00 (d, *J*=12.4 Hz, 2H), 2.92-2.88 (m, 1H), 2.84-2.73 (m, 2H), 2.62-2.56 (m, 4H), 2.42 (s, 3H), 2.30 (d, *J*=6.5 Hz, 2H), 2.17-2.14 (m, 1H), 1.96 (d, *J*=13.2 Hz, 2H), 1.35-1.27 (m, 2H), 1.16 (d, *J*=6.1 Hz, 6H). m/z 870.52 [M+H]⁺. |
| 18 | ¹H NMR (400 MHz, CDCl₃) δ 8.49 (s, 1H), 8.40 (d, *J*=2.7 Hz, 1H), 8.39 (d, *J*=5.3 Hz, 1H), 7.98 (dd, *J*=9.0, 2.7 Hz, 1H), 7.85-7.83 (m, 2H), 7.65 (d, *J*=8.3 Hz, 1H), 7.37 (d, *J*=7.7 Hz, 2H), 7.10 (d, *J*=4.0 Hz, 1H), 6.81 (d, *J*=2.0 Hz, 1H), 6.72 (d, *J*=9.1 Hz, 1H), 6.51 (dd, *J*=8.3, 2.1 Hz, 1H), 4.93 (dd, *J*=12.3, 5.3 Hz, 1H), 4.45 (d, *J*=5.6 Hz, 2H), 4.38-4.33 (m, 1H), 4.26 (t, *J*=5.5 Hz, 1H), 4.20-4.12 (m, 4H), 3.87 (dd, *J*=8.8, 4.7 Hz, 2H), 3.77-3.73 (m, 2H), 3.64-3.58 (m, 1H), 3.54 (t, *J*=4.8 Hz, 4H), 3.13-3.07 (m, 1H), 2.91-2.87 (m, 1H), 2.85-2.80 (m, 1H), 2.78-2.72 (m, 2H), 2.62 (t, *J*=4.8 Hz, 4H), 2.42 (s, 3H), 2.15-2.12 (m, 1H), 1.16 (d, *J*=6.1 Hz, 6H). m/z 842.46 [M+H]⁺. |
| 19 | ¹H NMR (400 MHz, CDCl₃) δ 8.74 (d, *J*=2.6 Hz, 1H), 8.46 (d, *J*=5.3 Hz, 2H), 8.20 (dd, *J*=8.5, 2.7 Hz, 1H), 7.86 (m, 2H), 7.67 (d, *J*=8.6 Hz, 1H), 7.44-7.36 (m, 2H), 7.28 (d, *J*=2.3 Hz, 1H), 7.19 (d, *J*=8.6 Hz, 1H), 7.17 (d, *J*=5.3 Hz, 1H), 7.05 (dd, *J*=8.7, 2.3 Hz, 1H), 4.94 (dd, *J*=12.3, 5.4 Hz, 1H), 4.45 (d, *J*=5.6 Hz, 2H), 4.38-4.34 (m, 1H), 4.29-4.26 (m, 1H), 4.17-4.11 (m, 2H), 3.96 (d, *J*=13.0 Hz, 2H), 3.87 (dd, *J*=8.9, 4.7 Hz, 2H), 3.64-3.58 (m, 1H), 3.06-2.68 (m, 8H), 2.42 (s, 3H), 2.27 (d, *J*=6.9 Hz, 2H), 2.15-2.08 (m, 3H), 1.94-1.82 (m, 7H), 1.32-1.24 (m, 2H), 1.16 (d, *J*=6.1 Hz, 6H). m/z 869.51 [M+H]⁺. |
| 20 | ¹H NMR (400 MHz, CDCl₃) δ 8.73 (d, *J*=2.6 Hz, 1H), 8.58 (s, 1H), 8.46 (d, *J*=5.3 Hz, 1H), 8.22 (dd, *J*=8.6, 2.7 Hz, 1H), 7.89-7.83 (m, 2H), 7.64 (d, *J*=8.3 Hz, 1H), 7.42-7.35 (m, 2H), 7.17 (d, *J*=5.3 Hz, 1H), 6.80 (d, *J*=2.1 Hz, 1H), 6.80 (d, *J*=2.1 Hz, 1H), 6.50 (dd, *J*=8.3, 2.1 Hz, 1H), 4.93 (dd, *J*=12.3, 5.4 Hz, 1H), 4.45 (d, *J*=5.6 Hz, 2H), 4.38-4.34 (m, 1H), 4.27 (t, *J*=5.6 Hz, 1H), 4.22-4.11 (m, 4H), 3.87 (dd, *J*=8.9, 4.7 Hz, 2H), 3.75-3.72 (m, 2H), 3.67-3.55 (m, 1H), 3.13-2.98 (m, 3H), 2.92-2.68 (m, 6H), 2.43 (s, 3H), 2.24-2.10 (m, 3H), 1.98 (d, *J*=11.8 Hz, 2H), 1.90-1.82 (m, 2H), 1.16 (d, *J*=6.1 Hz, 6H). m/z 841.51 [M+H]⁺. |
| 21 | ¹H NMR (400 MHz, CDCl₃) δ 8.42 (d, *J*=5.2 Hz, 2H), 7.96 (s, 1H), 7.89 (s, 1H), 7.82 (d, *J*=7.9 Hz, 1H), 7.68 (d, *J*=8.6 Hz, 1H), 7.63 (s, 1H), 7.37 (d, *J*=8.0 Hz, 1H), 7.29 (d, *J*=2.3 Hz, 1H), 7.13 (s, 1H), 7.08-7.05 (m, 2H), 4.94 (dd, *J*=12.3, 5.4 Hz, 1H), 4.45 (d, *J*=5.6 Hz, 2H), 4.37-4.32 (m, 1H), 4.26 (t, *J*=5.5 Hz, 1H), 4.15-4.11 (m, 3H), 3.97 (d, J=13.1 Hz, 2H), 3.86 (dd, *J*=8.8, 4.6 Hz, 2H), 3.63-3.57 (m, 1H), 3.02-2.96 (m, 4H), 2.94-2.67 (m, 3H), 2.42 (s, 3H), 2.26 (d, *J*=6.8 Hz, 2H), 2.21-2.02 (m, 7H), 1.88 (d, *J*=12.8 Hz, 2H), 1.83-1.78 (m, 1H), 1.33-1.25 (m, 2H), 1.15 (d, *J*=6.1 Hz, 6H). m/z 858.52 [M+H]⁺. |
| 22 | ¹H NMR (400 MHz, CDCl₃) δ 8.63 (s, 1H), 8.42 (d, *J*=5.2 Hz, 1H), 7.96 (s, 1H), 7.88 (s, 1H), 7.83 (d, *J*=7.9 Hz, 1H), 7.65 (d, *J*=8.3 Hz, 1H), 7.63 (s, 1H), 7.38 (d, *J*=8.0 Hz, 1H), 7.22 (s, 1H), 7.07 (d, *J*=5.3 Hz, 1H), 6.80 (d, *J*=2.0 Hz, 1H), 6.52 (dd, *J*=8.3, 2.1 Hz, 1H), 4.93 (dd, *J*=12.2, 5.4 Hz, 1H), 4.45 (d, *J*=5.6 Hz, 2H), 4.39-4.28 (m, 2H), 4.19-4.09 (m, 4H), 3.86 (dd, *J*=8.6, 4.7 Hz, 2H), 3.77-3.69 (m, 2H), 3.63-3.57 (m, 1H), 3.07-2.99 (m, 3H), 2.94-2.69 (m, 5H), 2.42 (s, 3H), 2.31-2.05 (m, 7H), 1.15 (d, *J*=6.1 Hz, 6H). m/z 830.47 [M+H]⁺. |
| 23 | ¹H NMR (400 MHz, CDCl₃) δ 8.44 (d, *J*=5.0 Hz, 1H), 8.17 (s, 1H), 7.80 (dd, *J*=10.7, 2.7 Hz, 1H), 7.68 (d, *J*=8.6 Hz, 1H), 7.51 (d, *J*=5.7 Hz, 2H), 7.29 (d, *J*=2.2 Hz, 1H), 7.14 (s, 1H), 7.05 (dd, *J*=8.6, 2.3 Hz, 1H), 6.94 (d, *J*=9.0 Hz, 2H), 6.88 (dd, *J*=8.6, 2.7 Hz, 1H), 6.71 (d, *J*=5.0 Hz, 1H), 5.95 (s, 1H), 4.96-4.89 (m, 1H), 4.41 (m, 1H), 4.28-4.19 (m, 2H), 3.98 (m, 4H), 3.69-3.59 (m, 1H), 3.21-3.11 (m, 4H), 3.05-2.90 (m, 2H), 2.90-2.79 (m, 2H), 2.78-2.70 (m, 1H), 2.66-2.55 (m, 4H), 2.28 (d, *J*=7.1 Hz, 2H), 2.18 (d, *J*=5.5 Hz, 3H), 2.14-2.09 (m, 1H), 1.93 (d, *J*=13.5 Hz, 2H), 1.87-1.79 (m, 1H), 1.33-1.24 (m, 2H), 1.18 (d, *J*=6.1 Hz, 6H). m/z 873.48 [M+H]⁺. |
| 24 | ¹H NMR (400 MHz, CDCl₃) δ 8.46 (d, *J*=5.0 Hz, 1H), 8.22 (s, 1H), 8.13 (d, *J*=0.6 Hz, 1H), 7.91 (dd, *J*=10.2, 2.6 Hz, 1H), 7.85 (s, 1H), 7.67 (d, *J*=8.6 Hz, 1H), 7.50 (d, *J*=6.1 Hz, 2H), 7.43 (s, 1H), 7.28 (d, *J*=2.3 Hz, 1H), 7.16 (s, 1H), 7.05 (dd, *J*=8.6, 2.3 Hz, 1H), 7.02-6.98 (m, 1H), 6.94 (d, *J*=9.0 Hz, 2H), 6.75 (d, *J*=5.0 Hz, 1H), 4.94 (m, 1H), 3.96 (d, *J*=13.0 Hz, 2H), 3.20-3.12 (m, 4H), 2.98 (dd, *J*=12.4, 10.6 Hz, 2H), 2.93-2.84 (m, 1H), 2.84-2.70 (m, 2H), 2.62-2.56 (m, 4H), 2.33-2.26 (m, 5H), 2.16-2.10 (m, 1H), 1.92 (d, *J*=13.3 Hz, 2H), 1.88-1.79 (m, 1H), 1.64 (s, 9H), 1.35-1.24 (m, 2H). m/z 882.49 [M+H]⁺. |
| 25 | ¹H NMR (400 MHz, CDCl₃) δ 9.13 (s, 1H), 8.46 (d, *J*=5.0 Hz, 1H), 8.27 (s, 1H), 8.15 (dd, *J*=10.4, 2.7 Hz, 1H), 8.09 (s, 1H), 7.67 (d, *J*=8.6 Hz, 1H), 7.51 (d, *J*=9.0 Hz, 2H), 7.28 (d, *J*=2.3 Hz, 1H), 7.12 (s, 1H), 7.05 (dd, *J*=8.6, 2.3 Hz, 1H), 6.99 (dd, *J*=8.6, 2.7 Hz, 1H), 6.94 (d, *J*=9.0 Hz, 2H), 6.76 (d, *J*=5.0 Hz, 1H), 4.94 (m, 1H), 3.96 (d, *J*=13.1 Hz, 2H), 3.17 (m, 4H), 2.97 (m, 2H), 2.90-2.78 (m, 2H), 2.77-2.69 (m, 1H), 2.59 (m, 4H), 2.39 (s, 3H), 2.28 (d, *J*=6.9 Hz, 2H), 2.18-2.10 (m, 1H), 1.93 (d, *J*=12.8 Hz, 2H), 1.84 (m, 1H), 1.75 (s, 9H), 1.37-1.23 (m, 2H). m/z 883.40 [M+H]⁺. |
| 26 | ¹H NMR (400 MHz, DMSO-*d₆*) δ 10.95 (s, 1H), 9.48 (s, 1H), 8.48 (d, *J*=5.0 Hz, 1H), 8.02 (s, 1H), 7.73 (d, *J*=8.9 Hz, 1H), 7.57 (d, *J*=9.0 Hz, 2H), 7.37 (dd, *J*=10.6, 2.8 Hz, 1H), 7.01 (dd, *J*=9.0, 2.8 Hz, 1H), 6.88(m, 3H), 6.81 (d, *J*=5.0 Hz, 1H), 5.06 (dd, *J*=13.3, 5.1 Hz, 1H), 4.44 (d, J=12.6 Hz, 2H), 4.39-4.35 (m, 1H), 4.26 (d, *J*=17.4 Hz, 1H), 4.21-4.15 (m, 2H), 4.08 (d, *J*=17.3 Hz, 1H), 3.76 (dd, *J*=8.9, 4.4 Hz, 2H), 3.64-3.58 (m, 1H), 3.05 (m, 4H), 2.99-2.84 (m, 2H), 2.69-2.55 (m, 2H), 2.52 (m, 4H), 2.40-2.29 (m, 1H), 2.19 (d, *J*=6.9 Hz, 2H), 2.15 (s, 3H), 1.99-1.94 (m, 1H), 1.90-1.86 (m, 1H), 1.81 (d, *J*=12.6 Hz, 2H), 1.10 (d, *J*=6.1 Hz, 8H). m/z 860.48 [M+H]⁺. |
| 27 | ¹H NMR (400 MHz, CDCl₃) δ 8.44 (d, *J*=5.0 Hz, 1H), 8.21 (s, 1H), 7.80 (dd, *J*=10.6, 2.7 Hz, 1H), 7.65 (d, *J*=8.3 Hz, 1H), 7.51 (d, *J*=8.9 Hz, 2H), 7.10 (s, 1H), 6.94 (d, J=9.0 Hz, 2H), 6.88 (dd, *J*=8.6, 2.7 Hz, 1H), 6.80 (d, *J*=2.0 Hz, 1H), 6.72 (d, *J*=5.0 Hz, 1H), 6.51 (dd, *J*=8.3, 2.1 Hz, 1H), 5.94 (s, 1H), 4.93 (m, 1H), 4.41 (m, 1H), 4.29-4.22 (m, 2H), 4.17 (t, J=8.0 Hz, 2H), 3.98 (dd, *J*=8.9, 4.6 Hz, 2H), 3.78-3.73 (m, 2H), 3.70-3.61 (m, 1H), 3.17 (m, 4H), 3.08 (m, 1H), 2.85 (m, 2H), 2.75 (m, 3H), 2.65 (m, 4H), 2.19 (s, 3H), 2.16-2.09 (m, 1H), 1.18 (d, *J*=6.1 Hz, 6H). m/z 845.49 [M+H]⁺. |
| 28 | ¹H NMR (400 MHz, CDCl₃) δ 8.42 (d, *J*=5.2 Hz, 1H), 8.06 (s, 1H), 7.76 (d, *J*=6.7 Hz, 2H), 7.68 (d, *J*=8.6 Hz, 1H), 7.56 (d, *J*=8.9 Hz, 2H), 7.29 (d, *J*=2.9 Hz, 1H), 7.11-7.04 (m, 3H), 6.97 (d, *J*=9.0 Hz, 2H), 4.94 (m, 1H), 4.45 (d, *J*=5.7 Hz, 2H), 4.38-4.30 (m, 2H), 4.17-4.10 (m, 2H), 3.97 (d, *J*=13.0 Hz, 2H), 3.86 (dd, *J*=8.6, 4.7 Hz, 2H), 3.60 (m, 1H), 3.18 (m, 4H), 2.99 (t, *J*=11.5 Hz, 2H), 2.86 (m, 1H), 2.80-2.68 (m, 1H), 2.61 (m, 4H), 2.43 (s, 3H), 2.29 (d, *J*=7.1 Hz, 2H), 2.16-2.09 (m, 1H), 1.93 (d, *J*=13.3 Hz, 2H), 1.85 (s, 1H), 1.31 (m, 2H), 1.15 (d, *J*=6.1 Hz, 6H). m/z 869.56 [M+H]⁺. |
| 29 | ¹H NMR (400 MHz, DMSO-*d₆*) δ 11.08 (s, 1H), 9.38 (s, 1H), 8.58 (s, 1H), 8.46 (d, *J*=5.2 Hz, 1H), 8.14 (s, 1H), 7.69-7.64 (m, 3H), 7.52 (s, 1H), 7.44 (s, 1H), 7.31 (d, *J*=1.8 Hz, 1H), 7.23 (dd, *J*=8.7, 2.0 Hz, 1H), 7.16 (d, *J*=5.2 Hz, 1H), 6.93 (d, *J*=9.1 Hz, 2H), 5.06 (dd, *J*=12.9, 5.4 Hz, 1H), 4.41-4.34 (m, 1H), 4.21-4.16 (m, 2H), 4.09-4.02 (m, 2H), 3.75 (dd, *J*=9.0, 4.4 Hz, 2H), 3.65-3.58 (m, 1H), 3.38 (m, 4H), 3.08 (m, 4H), 2.97 (t, *J*=12.2 Hz, 2H), 2.64-2.53 (m, 3H), 2.35 (s, 3H), 2.20 (d, *J*=6.8 Hz, 2H), 2.05-1.97 (m, 1H), 1.82 (d, *J*=12.9 Hz, 2H), 1.23 (m, 1H), 1.20-1.14 (m, 2H), 1.10 (d, *J*=6.1 Hz, 6H). m/z 855.55 [M+H]⁺. |
| 30 | ¹H NMR (400 MHz, CDCl₃) δ 8.42 (d, *J*=5.2 Hz, 1H), 7.97 (s, 1H), 7.86 (m, 2H), 7.68 (d, *J*=8.6 Hz, 1H), 7.56 (d, *J*=8.9 Hz, 2H), 7.43 (d, *J*=7.7 Hz, 1H), 7.29 (d, *J*=2.2 Hz, 1H), 7.09 (d, *J*=5.2 Hz, 1H), 7.06 (m, 2H), 6.97 (d, *J*=8.9 Hz, 2H), 5.15 (s, 2H), 4.94 (m, 1H), 4.33 (m, 1H), 4.23-4.14 (m, 2H), 4.02-3.88 (m, 4H), 3.59 (m, 1H), 3.17 (m, 4H), 2.99 (m, 2H), 2.91-2.68 (m, 3H), 2.61 (m, 4H), 2.43 (s, 3H), 2.29 (d, *J*=7.2 Hz, 2H), 2.16-2.09 (m, 1H), 1.93 (d, *J*=14.4 Hz, 2H), 1.84 (s, 1H), 1.32 (m, 2H), 1.15 (d, *J*=6.1 Hz, 6H). m/z 870.47 [M+H]⁺. |
| 31 | ¹H NMR (400 MHz, CDCl₃) δ 8.42 (d, *J*=5.2 Hz, 1H), 8.09 (s, 1H), 8.04 (s, 1H), 7.90-7.84 (m, 2H), 7.74 (s, 1H), 7.65 (d, *J*=8.3 Hz, 1H), 7.57 (d, *J*=8.9 Hz, 2H), 7.44-7.38 (m, 1H), 7.09 (m, 2H), 6.96 (d, *J*=9.0 Hz, 2H), 6.79 (d, *J*=2.0 Hz, 1H), 6.51 (dd, *J*=8.3, 2.1 Hz, 1H), 5.89 (m, 1H), 4.93 (m, 1H), 4.66 (d, *J*=5.5 Hz, 2H), 4.18 (t, *J*=7.9 Hz, 2H), 3.80-3.71 (m, 2H), 3.19 (m, 4H), 3.09 (m, 1H), 2.94-2.80 (m, 2H), 2.74 (m, 3H), 2.66 (m, 4H), 2.46 (s, 3H), 2.12 (m, 1H), 1.60 (s, 9H). m/z 850.25 [M+H]⁺. |
| 32 | ¹H NMR (400 MHz, CDCl₃) δ 8.42 (d, *J*=5.2 Hz, 1H), 8.18 (s, 1H), 8.01 (s, 1H), 7.89 (s, 1H), 7.87 (d, *J*=8.0 Hz, 1H), 7.65 (d, *J*=8.3 Hz, 1H), 7.57 (d, *J*=8.9 Hz, 2H), 7.44 (d, *J*=8.0 Hz, 1H), 7.42-7.39 (m, 1H), 7.09 (d, *J*=5.3 Hz, 1H), 7.06 (s, 1H), 6.97( d, *J*=8.7 Hz, 2H), 6.79 (d, *J*=2.0 Hz, 1H), 6.51 (dd, *J*=8.0, 2.0 Hz, 1H), 4.93 (dd, *J*=12.1, 5.2 Hz, 1H), 4.71 (d, *J*=5.9 Hz, 2H), 4.18 (t, *J*=7.9 Hz, 2H), 3.77-3.73 (m, 2H), 3.19 (t, *J*=4.6 Hz, 4H), 3.12-3.5 (m, 1H), 2.92-2.87 (m, 1H), 2.85-2.80 (m, 1H), 2.78-2.72 (m, 3H), 2.65 (t, *J*=4.8 Hz, 4H), 2.47 (s, 3H), 2.15-2.12 (m, 1H), 1.71 (s, 9H). m/z 851.54 [M+H]⁺. |
| 33 | ¹H NMR (400 MHz, DMSO-*d₆*) δ 11.06 (s, 1H), 9.36 (s, 1H), 8.49 (t, *J*=5.8 Hz, 1H), 8.45 (d, *J*=5.2 Hz, 1H), 8.26 (s, 1H), 7.97-7.91 (m, 3H), 7.65 (d, *J*=8.5 Hz, 3H), 7.37 (d, *J*=8.1 Hz, 1H), 7.31 (d, *J*=1.6 Hz, 1H), 7.28 (d, *J*=5.3 Hz, 1H), 7.23 (dd, *J*=8.7, 2.0 Hz, 1H), 6.91( d, *J*=9.1 Hz, 2H), 5.06 (dd, *J*=12.9, 5.4 Hz, 1H), 4.55-4.48 (m, 1H), 4.47 (d, *J*=5.3 Hz, 2H), 4.05 (d, *J*=13.3 Hz, 2H), 3.12-3.04 (m, 4H), 2.98 (t, *J*=12.0 Hz, 2H), 2.93-2.84 (m, 1H), 2.67-2.65 (m, 4H), 2.62-2.60 (m, 1H), 2.41 (s, 3H), 2.35-2.32 (m, 2H), 2.21 (d, *J*=6.7 Hz, 2H), 2.05-1.99 (m, 1H), 1.83 (d, *J*=13.0 Hz, 2H), 1.42 (d, *J*=6.7 Hz, 6H), 1.23-1.13 (m, 2H). m/z 864.52 [M+H]⁺. |
| 34 | ¹H NMR (400 MHz, DMSO-*d₆*) δ 11.05 (s, 1H), 9.36 (s, 1H), 8.48 (t, *J*=5.7 Hz, 1H), 8.45 (d, *J*=5.2 Hz, 1H), 8.26 (s, 1H), 7.97-7.92 (m, 3H), 7.41 (s, 1H), 7.65 (t, *J*=8.6 Hz, 3H), 7.37 (d, *J*=8.0 Hz, 1H), 7.29 (d, *J*=5.2 Hz, 1H), 6.92 (d, *J*=9.1 Hz, 2H), 6.78 (d, *J*=1.9 Hz, 1H), 6.66 (dd, *J*=8.4, 2.0 Hz, 1H), 5.05 (dd, *J*=12.9, 5.4 Hz, 1H), 4.55-4.48 (m, 1H), 4.47 (d, *J*=5.6 Hz, 2H), 4.16 (m, 2H), 3.74-3.70 (m, 2H), 3.11-3.05 (m, 4H), 2.93-2.83 (m, 1H), 2.67-2.65 (m, 4H), 2.62-2.59 (m, 1H), 2.57-2.55 (m, 2H), 2.41 (s, 3H), 2.34-2.32 (m, 2H), 2.04-1.97 (m, 1H), 1.42 (d, *J*=6.6 Hz, 6H). m/z 836.46 [M+H]⁺. |
| 35 | ¹H NMR (400 MHz, CDCl₃) δ 8.42 (d, *J*=5.2 Hz, 1H), 8.29 (brs, 1H), 7.97-7.95 (m, 2H), 7.92 (s, 1H), 7.85 (d, *J*=8.4 Hz, 1H), 7.74 (s, 1H), 7.68 (d, *J*=8.5 Hz, 1H), 7.64 (s, 1H), 7.42 (d, *J*=7.6 Hz, 1H), 7.30-7.29 (m, 2H), 7.09-7.06 (m, 2H), 5.93 (s, 1H), 4.94 (dd, *J*=12.3, 5.3 Hz, 1H), 4.67 (d, *J*=5.6 Hz, 2H), 4.55-4.48 (m, 1H), 4.18-4.09 (m, 1H), 3.98 (d, *J*=13.4 Hz, 2H), 3.00 (t, *J*=11.9 Hz, 4H), 2.93-2.88 (m, 1H), 2.85-2.79 (m, 1H), 2.77-2.72 (m, 1H), 2.46 (s, 3H), 2.30-2.25 (m, 2H), 2.18-2.14 (m, 6H), 1.92-1.86 (m 3H), 1.52 (d, *J*=6.7 Hz, 6H), 1.37-1.26 (m, 3H). m/z 853.48 [M+H]⁺. |
| 36 | ¹H NMR (400 MHz, CDCl₃) δ 8.41 (d, *J*=5.2 Hz, 1H), 8.22 (brs, 1H), 7.94 (d, *J*=5.2 Hz, 2H), 7.90 (s, 1H), 7.83 (d, *J*=7.9 Hz, 1H), 7.72 (s, 1H), 7.63 (d, *J*=8.4 Hz, 1H), 7.61 (s, 1H), 7.40 (d, *J*=8.3 Hz, 1H), 7.06 (d, *J*=5.3 Hz, 2H), 6.77 (s, 1H), 6.51 (dd, *J*=8.3, 2.1 Hz, 1H), 5.94 (brs, 1H), 4.92 (dd, *J*=12.3, 5.2 Hz, 1H), 4.65 (d, *J*=5.6 Hz, 2H), 4.53-4.47 (m, 1H), 4.15 (t, *J*=8.3 Hz, 3H), 3.74-3.70 (m, 2H), 3.04-2.97 (m, 3H), 2.90-2.85 (m, 1H), 2.82-2.79 (m, 2H), 2.70 (d, *J*=7.4 Hz, 2H), 2.45 (s, 3H), 2.26-2.14 (m, 5H), 2.09-2.02 (m, 2H), 1.51 (d, *J*=6.7 Hz, 6H). m/z 825.46 [M+H]⁺. |
| 37 | ¹H NMR (400 MHz, CDCl₃) δ 8.42 (d, *J*=5.2 Hz, 1H), 8.32 (s, 1H), 8.04 (s, 1H), 7.96 (s, 1H), 7.92 (s, 1H), 7.84 (d, *J*=8.1 Hz, 1H), 7.75 (s, 1H), 7.68 (d, *J*=8.5 Hz, 1H), 7.62 (s, 1H), 7.42 (d, *J*=8.0 Hz, 1H), 7.29 (d, *J*=2.2 Hz, 1H), 7.16-7.04 (m, 3H), 5.94 (s, 1H), 4.94 (dd, *J*=12.3, 5.4 Hz, 1H), 4.66 (d, *J*=5.6 Hz, 2H), 4.12 (q, *J*=7.1 Hz, 2H), 3.97 (d, *J*=12.9 Hz, 2H), 2.99 (t, *J*=11.4 Hz, 4H), 2.91-2.64 (m, 3H), 2.46 (s, 3H), 2.26 (d, *J*=6.5 Hz, 2H), 2.16 (d, *J*=10.5 Hz, 4H), 2.08 (m, 2H), 1.88 (d, *J*=14.3 Hz, 2H), 1.78 (m, 1H), 1.60 (s, 9H), 1.31 (m, 2H). m/z 867.51 [M+H]⁺. |
| 38 | ¹H NMR (400 MHz, CDCl₃) δ 8.43 (d, *J*=5.2 Hz, 1H), 8.05 (s, 1H), 7.96 (s, 1H), 7.91 (s, 1H), 7.84 (d, *J*=7.9 Hz, 1H), 7.76 (s, 1H), 7.65 (d, *J*=8.3 Hz, 1H), 7.62 (s, 1H), 7.42 (d, *J*=8.0 Hz, 1H), 7.08 (t, *J*=5.7 Hz, 2H), 6.80 (d, *J*=2.1 Hz, 1H), 6.52 (dd, *J*=8.3, 2.1 Hz, 1H), 5.99 (s, 1H), 4.93 (dd, *J*=12.3, 5.4 Hz, 1H), 4.66 (d, *J*=5.6 Hz, 2H), 4.14 (m, 3H), 3.77-3.69 (m, 2H), 3.08-2.95 (m, 3H), 2.83 (m, 2H), 2.71 (d, *J*=7.3 Hz, 2H), 2.46 (s, 3H), 2.28-2.00 (m, 9H), 1.60 (s, 9H). m/z 839.45 [M+H]⁺. |
| 39 | ¹H NMR (400 MHz, CDCl₃) δ 8.41 (d, J=5.2 Hz, 1H), 8.25 (brs, 1H), 8.19 (s, 1H), 7.99 (s, 1H), 7.92 (s, 1H), 7.83 (d, *J*=7.8 Hz, 1H), 7.68 (d, *J*=8.5 Hz, 1H), 7.61 (s, 1H), 7.47-7.41 (m, 2H), 7.30 (d, *J*=2.2 Hz, 1H), 7.15 (brs, 1H), 7.08-7.05 (m, 2H), 4.95 (dd, *J*=12.2, 5.4 Hz, 1H), 4.71 (d, *J*=5.9 Hz, 2H), 4.18-4.10 (m, 1H), 3.97 (d, *J*=13.1 Hz, 2H), 2.99 (t, *J*=11.4 Hz, 4H), 2.93-2.88 (m, 1H), 2.84-2.80 (m, 1H), 2.77-2.72 (m, 1H), 2.47 (s, 3H), 2.26 (d, *J*=6.9 Hz, 2H), 2.19-2.14 (m, 5H), 2.08-2.03 (m, 2H), 1.89 (d, *J*=12.9 Hz, 3H), 1.70 (s, 9H), 1.35-1.29 (m, 2H). m/z 868.53 [M+H]⁺. |
| 40 | ¹H NMR (400 MHz, CDCl₃) δ 8.40 (d, *J*=5.2 Hz, 1H), 8.27 (brs, 1H), 8.18 (s, 1H), 7.98 (s, 1H), 7.90 (s, 1H), 7.83 (d, *J*=8.0 Hz, 1H), 7.64 (d, *J*=8.3 Hz, 1H), 7.61 (s, 1H), 7.48-7.42 (m, 2H), 7.07 (d, *J*=5.3 Hz, 1H), 6.78 (d, *J*=2.0 Hz, 1H), 6.51 (dd, *J*=8.3, 2.0 Hz, 1H), 4.94 (dd, *J*=12.1, 5.5 Hz, 1H), 4.70 (d, *J*=5.9 Hz, 2H), 4.17-4.10 (m, 3H), 3.73 (t, *J*=5.8 Hz, 2H), 3.07-2.98 (m, 3H), 2.91-2.86 (m, 1H), 2.82-2.76 (m, 2H), 2.71 (d, *J*=7.3 Hz, 2H), 2.47 (s, 3H), 2.27-2.17 (m, 5H), 2.06-2.02 (m, 2H), 1.69 (s, 9H). m/z 840.48 [M+H]⁺. |
| 41 | ¹H NMR (400 MHz, CDCl₃) δ 8.45 (d, *J*=5.2 Hz, 1H), 8.24 (s, 1H), 7.87 (dd, *J*=3.8, 2.5 Hz, 2H), 7.70-7.61 (m, 3H), 7.41-7.36 (m, 1H), 7.29 (dd, *J*=6.9, 4.9 Hz, 4H), 7.13 (d, *J*=5.3 Hz, 1H), 7.05 (dd, *J*=8.6, 2.3 Hz, 1H), 4.94 (m, 1H), 4.46 (d, *J*=5.6 Hz, 2H), 4.36 (m, 1H), 4.25 (m, 1H), 4.16-4.09 (m, 2H), 3.96 (d, *J*=13.3 Hz, 2H), 3.87 (dd, *J*=8.9, 4.7 Hz, 2H), 3.79 (m, 2H), 3.61 (m, 1H), 3.16 (s, 2H), 3.03-2.65 (m, 5H), 2.44 (d, *J*=9.7 Hz, 5H), 2.18-2.11 (m, 1H), 1.88 (d, *J*=11.7 Hz, 2H), 1.64 (s, 2H), 1.35 (m, 2H), 1.16 (d, *J*=6.1 Hz, 6H). m/z 840.66 [M+H]⁺. |
| 42 | ¹H NMR (400 MHz, CDCl₃) δ 8.41 (d, *J*=5.2 Hz, 1H), 8.04 (s, 1H), 7.85 (m, 2H), 7.57 (d, *J*=9.0 Hz, 2H), 7.36 (d, *J*=7.8 Hz, 1H), 7.20-7.12 (m, 2H), 7.07 (d, *J*=5.3 Hz, 1H), 6.99-6.93 (m, 2H), 6.66-6.60 (m, 2H), 6.51-6.46 (m, 1H), 4.87 (dd, *J*=7.5, 4.3 Hz, 1H), 4.44 (d, *J*=5.6 Hz, 2H), 4.37-4.33 (m, 1H), 4.24-4.22 (m, 1H), 4.13 (dd, *J*=8.2, 7.3 Hz, 2H), 3.86 (dd, *J*=8.9, 4.7 Hz, 2H), 3.69 (d, *J*=12.3 Hz, 2H), 3.65-3.56 (m, 1H), 3.23-3.13 (m, 4H), 2.95 (ddd, *J*=17.9, 7.7, 5.3 Hz, 1H), 2.76-2.58 (m, 7H), 2.42 (s, 3H), 2.38-2.25 (m, 4H), 1.88 (d, *J*=11.8 Hz, 2H), 1.72-1.68 (m, 1H), 1.34 (dd, *J*=12.3, 3.0 Hz, 2H), 1.16 (d, J=6.1 Hz, 6H). m/z 816.32 [M+H]⁺. |
| 43 | ¹H NMR (400 MHz, CDCl₃) δ 8.41 (d, *J*=5.2 Hz, 1H), 7.92 (s, 1H), 7.85 (m, 2H), 7.56 (d, J=8.9 Hz, 2H), 7.36 (d, *J*=7.7 Hz, 1H), 7.11-7.04 (m, 2H), 7.00-6.95 (m, 4H), 6.93-6.88 (m, 2H), 4.74 (dd, *J*=7.6, 4.2 Hz, 1H), 4.45 (d, *J*=5.6 Hz, 2H), 4.37-4.34 (m, 1H), 4.22 (t, *J*=5.6 Hz, 1H), 4.17-4.10 (m, 2H), 3.86 (dd, *J*=8.7, 4.7 Hz, 2H), 3.64-3.55 (m, 3H), 3.22-3.14 (m, 4H), 3.00-2.90 (m, 1H), 2.68-2.60 (m, 7H), 2.42 (s, 3H), 2.34-2.23 (m, 4H), 1.90 (d, *J*=11.6 Hz, 2H), 1.67 (s, 1H), 1.39 (dd, *J*=12.0, 8.5 Hz, 2H), 1.16 (d, *J*=6.1 Hz, 6H). m/z 816.54 [M+H]⁺. |
| 44 | ¹H NMR (400 MHz, DMSO-*d₆*) δ 11.06 (s, 1H), 9.55 (s, 1H), 3.51-8.48 (m, 2H), 8.25 (s, 1H), 7.97 (s, 1H), 7.94 (d, *J*=8.0 Hz, 1H), 7.91 (s, 1H), 7.72 (d, *J*=8.5 Hz, 2H), 7.62 (d, *J*=8.3 Hz, 1H), 7.36 (d, *J*=8.0 Hz, 1H), 7.33 (d, *J*=5.2 Hz, 1H), 7.17 (d, *J*=8.6 Hz, 2H), 6.77 (d, *J*=1.9 Hz, 1H), 6.64 (dd, *J*=8.4, 2.0 Hz, 1H), 5.04 (dd, *J*=12.9, 5.4 Hz, 1H), 4.53-4.47 (m, 1H), 4.45 (d, *J*=5.6 Hz, 2H), 4.15-4.09 (m, 2H), 3.70-3.67 (m, 2H), 3.04-2.98 (m, 1H), 2.94 (d, *J*=11.0 Hz, 2H), 2.90-2.82 (m, 1H), 2.66-2.58 (m, 4H), 2.40 (s, 3H), 2.32-2.31 (m, 1H), 2.07-1.97 (m, 3H), 1.74-1.57 (m, 4H), 1.41 (d, *J*=6.7 Hz, 6H). m/z 835.51 [M+H]⁺. |
| 45 | ¹H NMR (400 MHz, CDCl₃) δ 8.43 (d, *J*=5.2 Hz, 1H), 8.24 (brs, 1H), 8.05 (d, *J*=0.6 Hz, 1H), 7.91 (s, 1H), 7.88 (d, *J*=8.0 Hz, 1H), 7.75 (s, 1H), 7.64 (m, 3H), 7.44-7.41 (m, 2H), 7.22 (d, *J*=8.5 Hz, 2H), 7.12 (d, *J*=5.3 Hz, 1H), 6.79 (d, *J*=1.8 Hz, 1H), 6.51 (dd, *J*=8.3, 2.0 Hz, 1H), 5.92 (t, *J*=5.4 Hz, 1H), 4.93 (dd, *J*=12.2, 5.2 Hz, 1H), 4.67 (d, *J*=5.6 Hz, 2H), 4.18 (t, *J*=8.0 Hz, 2H), 3.74 (t, *J*=6.6 Hz, 2H), 3.14-3.07 (m, 1H), 3.03 (d, *J*=10.8 Hz, 2H), 2.91-2.87 (m, 1H), 2.85-2.80 (m, 1H), 2.76-2.71 (m, 3H), 2.56-2.49 (m, 1H), 2.46 (s, 3H), 2.19-2.13 (m, 3H), 1.82-1.76 (m, 4H), 1.60 (d, *J*=5.0 Hz, 9H). m/z 849.50 [M+H]⁺. |
| 46 | ¹H NMR (400 MHz, CDCl₃) δ 8.43 (d, *J*=5.2 Hz, 1H), 8.17 (s, 1H), 8.06 (brs, 1H), 7.89 (s, 1H), 7.85 (d, *J*=8.0 Hz, 1H), 7.62 (m, 3H), 7.44-7.35 (m, 2H), 7.21 (d, *J*=8.5 Hz, 2H), 7.15 (s, 1H), 7.11 (d, *J*=5.3 Hz, 1H), 6.77 (s, 1H), 6.50 (dd, *J*=8.3, 2.0 Hz, 1H), 4.92 (dd, *J*=12.3, 5.3 Hz, 1H), 4.70 (d, *J*=3.8 Hz, 2H), 4.16 (t, *J*=8.0 Hz, 2H), 3.74-3.70 (m, 2H), 3.09-3.03 (m, 1H), 2.98 (d, *J*=10.4 Hz, 2H), 2.90-2.74 (m, 3H), 2.71-2.69 (m, 2H), 2.53-2.48 (m, 1H), 2.46 (s, 3H), 2.15-2.09 (m, 3H), 1.87-1.75 (m, 4H), 1.70 (s, 9H). m/z 850.46 [M+H]⁺. |
| 47 | ¹H NMR (400 MHz, CDCl₃) δ 8.44 (d, *J*=5.2 Hz, 1H), 7.91-7.80 (m, 3H), 7.62 (d, *J*=8.4 Hz, 2H), 7.38 (d, *J*=7.9 Hz, 1H), 7.24 (d, *J*=8.5 Hz, 2H), 7.16 (s, 1H), 7.12 (d, *J*=5.2 Hz, 1H), 6.97 (d, *J*=9.2 Hz, 2H), 6.91 (d, *J*=9.2 Hz, 2H), 4.75 (m, 1H), 4.46 (d, *J*=5.6 Hz, 2H), 4.35 (m, 1H), 4.23 (m, 1H), 4.17-4.09 (m, 2H), 3.87 (dd, *J*=8.4, 4.7 Hz, 2H), 3.59 (m, 3H), 3.02 (m, 3H), 2.71-2.60 (m, 3H), 2.49 (m, 1H), 2.43 (s, 3H), 2.36-2.23 (m, 4H), 2.04 (d, *J*=7.3 Hz, 2H), 1.92-1.79 (m, 5H), 1.65 (m, 2H), 1.38 (m, 2H), 1.16 (d, *J*=6.1 Hz, 6H). m/z 815.30 [M+H]⁺. |
| 48 | ¹H NMR (400 MHz, CDCl₃) 8.42 (d, *J*=5.2 Hz, 1H), 8.07 (s, 1H), 7.95 (s, 1H), 7.92-7.83 (m, 2H), 7.73 (s, 1H), 7.57 (d, *J*=8.9 Hz, 2H), 7.40 (d, *J*=7.9 Hz, 1H), 7.15 (s, 1H), 7.08 (d, *J*=5.2 Hz, 1H), 6.99-6.94 (m, 4H), 6.93-6.88 (m, 2H), 5.89 (s, 1H), 4.75 (dd, *J*=7.6, 4.3 Hz, 1H), 4.66 (d, *J*=5.5 Hz, 2H), 4.59-4.46 (m, 1H), 3.57 (d, *J*=12.2 Hz, 2H), 3.21-3.13 (m, 4H), 2.94 (m, 1H), 2.68-2.56 (m, 8H), 2.45 (s, 3H), 2.32-2.26 (m, 3H), 1.90 (d, *J*=11.7 Hz, 2H), 1.67 (s, 1H), 1.52 (d, *J*=6.7 Hz, 6H), 1.43-1.32 (m, 2H). m/z 811.47 [M+H]⁺. |
| 49 | ¹H NMR (400 MHz, DMSO-*d₆*) δ 10.89 (s, 1H), 9.57 (s, 1H), 8.55-8.48 (m, 2H), 8.27 (s, 1H), 7.99 (s, 1H), 7.95 (d, *J*=7.9 Hz, 1H), 7.92 (s, 1H), 7.74 (d, *J*=8.6 Hz, 2H), 7.36 (d, *J*=6.6 Hz, 2H), 7.19 (d, *J*=8.7 Hz, 2H), 6.87 (t, *J*=5.8 Hz, 4H), 5.01 (m, 1H), 4.52 (m, 1H), 4.46 (d, *J*=5.5 Hz, 2H), 3.54 (m, 2H), 2.95 (d, *J*=10.2 Hz, 2H), 2.63 (m, 6H), 2.42 (m, 5H), 2.22-2.11 (m, 3H), 2.01-1.92 (m, 2H), 1.82-1.56 (m, 7H), 1.42 (d, *J*=6.7 Hz, 6H). m/z 810.48 [M+H]⁺. |
| 50 | ¹H NMR (400 MHz, CDCl₃) δ 8.40 (d, *J*=5.2 Hz, 1H), 8.01 (d, J=5.2 Hz, 1H), 7.85-7.83 (m, 2H), 7.79 (brs, 1H), 7.55 (d, *J*=8.9 Hz, 2H), 7.35 (d, *J*=7.8 Hz, 1H), 7.28 (dd, *J*=9.1, 3.2 Hz, 2H), 7.06 (d, *J*=5.2 Hz, 1H), 7.04 (s, 1H), 6.95 (d, *J*=9.0 Hz, 2H), 6.63 (d, *J*=9.2 Hz, 1H), 4.63 (dd, *J*=7.6, 4.5 Hz, 1H), 4.43 (d, *J*=5.5 Hz, 2H), 4.38-4.31 (m, 1H), 4.22-4.01 (m, 5H), 3.85 (dd, *J*=8.6, 4.7 Hz, 2H), 3.63-3.57 (m, 1H), 3.17 (t, *J*=4.8 Hz, 4H), 2.97-2.89 (m, 1H), 2.81-2.75 (m, 2H), 2.69-2.62 (m, 1H), 2.60-2.56 (m, 4H), 2.41 (s, 3H), 2.35-2.24 (m, 4H), 1.87 (d, *J*=11.6 Hz, 2H), 1.78-1.72 (m, 1H), 1.34-1.20 (m, 3H), 1.14 (d, *J*=6.1 Hz, 6H). m/z 817.49 [M+H]⁺. |
| 51 | ¹H NMR (400 MHz, CDCl₃) δ 8.44 (d, *J*=5.2 Hz, 1H), 8.03 (d, *J*=3.0 Hz, 1H), 7.88-7.82 (m, 3H), 7.63 (d, *J*=8.5 Hz, 2H), 7.38 (d, *J*=7.8 Hz, 1H), 7.30 (d, *J*=3.1 Hz, 1H), 7.28 (d, *J*=3.1 Hz, 1H), 7.24 (d, *J*=8.5 Hz, 2H), 7.20 (s, 1H), 7.12 (d, *J*=5.2 Hz, 1H), 6.65 (d, *J*=9.2 Hz, 1H), 4.64 (dd, *J*=7.6, 4.6 Hz, 1H), 4.46 (d, *J*=5.6 Hz, 2H), 4.39-4.33 (m, 1H), 4.25-4.12 (m, 5H), 3.87 (dd, *J*=8.7, 4.7 Hz, 2H), 3.64-3.58 (m, 1H), 3.02 (d, *J*=10.4 Hz, 2H), 2.99-2.91 (m, 1H), 2.83-2.76 (m, 2H), 2.69-2.62 (m, 1H), 2.54-2.47 (m, 1H), 2.43 (s, 3H), 2.36-2.27 (m, 2H), 2.25 (d, *J*=6.4 Hz, 2H), 2.07-2.02 (m, 2H), 1.90-1.82 (m, 5H), 1.33-1.25 (m, 3H), 1.16 (d, *J*=6.1 Hz, 6H). m/z 816.49 [M+H]⁺. |
| 52 | ¹H NMR (400 MHz, CDCl₃) δ 8.41 (d, *J*=5.2 Hz,1H), 8.06 (s, 1H), 7.85 (m, 2H), 7.80 (d, *J*=2.9 Hz, 1H), 7.56 (d, *J*=8.9 Hz. 2H), 7.36 (d, *J*=7.7 Hz, 1H), 7.11-7.02 (m, 3H), 6.97 (d, *J*=9.0 Hz, 2H), 6.66 (d, *J*=9.0 Hz, 1H), 4.45 (d, *J*=5.6 Hz, 2H), 4.35 (ddd, *J*=11.3, 6.6, 4.7 Hz, 1H), 4.29 (s, 1H), 4.23 (t, *J*=5.4 Hz, 1H), 4.15-4.09 (m, 4H), 3.95 (d, *J*=10.7 Hz, 1H), 3.86 (dd, *J*=8.7, 4.7 Hz, 2H), 3.61 (dt, *J*=12.3, 6.1 Hz, 1H), 3.21-3.13 (m, 4H), 2.97-2.64 (m, 5H), 2.61 (d, *J*=4.7 Hz, 4H), 2.54-2.46 (m, 1H), 2.42 (s, 3H), 2.28 (d, *J*=7.1 Hz, 2H), 1.90 (dd, *J*=15.0, 10.8 Hz, 2H), 1.79-1.75 (m, 1H), 1.27 (dd, *J*=13.4, 6.1 Hz, 2H), 1.16 (d, *J*=6.1 Hz, 6H). m/z 816.54 [M+H]⁺. |
| 53 | ¹H NMR (400 MHz, CDCl₃) δ 8.44 (d, *J*=5.2 Hz, 1H), 8.03 (s, 1H), 7.90-7.83 (m, 2H), 7.80 (d, *J*=2.9 Hz, 1H), 7.62 (d, *J*=8.5 Hz, 2H), 7.38 (d, *J*=7.9 Hz, 1H), 7.25-7.18 (m, 3H), 7.11 (d, *J*=5.3 Hz, 1H), 7.05 (dd, *J*=9.0, 3.0 Hz, 1H), 6.67 (d, *J*=9.0 Hz, 1H), 4.45 (d, J=5.6 Hz, 2H), 4.40-4.33 (m, 1H), 4.29 (s, 1H), 4.24 (t, *J*=5.5 Hz, 1H), 4.12 (dd, *J*=15.5, 8.9 Hz, 4H), 3.95 (dd, *J*=12.4, 4.3 Hz, 1H), 3.87 (dd, *J*=8.7, 4.7 Hz, 2H), 3.61 (dt, *J*=12.3, 6.1 Hz, 1H), 3.03-3.01 (m, 2H), 2.91-2.60 (m, 5H), 2.54-2.45 (m, 2H), 2.43 (s, 3H), 2.26-2.24 (m, 2H), 2.10-1.97 (m, 2H), 1.94-1.81 (m, 5H), 1.29 (dd, *J*=19.6, 12.2 Hz, 4H), 1.16 (d, *J*=6.1 Hz, 6H). m/z 815.34 [M+H]⁺. |
| 54 | ¹H NMR (400 MHz, CDCl₃) δ 8.42 (d, *J*=5.2 Hz, 1H), 8.09 (s, 1H), 7.86-7.84 (m, 2H), 7.57 (d, *J*=8.9 Hz, 2H), 7.37 (d, *J*=7.8 Hz, 1H), 7.13-7.07 (m, 3H), 6.97 (d, *J*=9.0 Hz, 2H), 6.46 (dd, *J*=8.2, 2.0 Hz, 1H), 6.29 (t, *J*=2.0 Hz, 1H), 6.16 (dd, *J*=7.9, 1.8 Hz, 1H), 4.68 (d, *J*=3.5 Hz, 1H), 4.45 (d, *J*=5.6 Hz, 2H), 4.39-4.33 (m, 1H), 4.23 (t, *J*=5.4 Hz, 1H), 4.10-4.05 (m, 2H), 3.87 (dd, *J*=8.7, 4.7 Hz, 2H), 3.67 (d, *J*=12.4 Hz, 2H), 3.64-3.58 (m, 1H), 3.19-3.17 (m, 4H), 2.90-2.84 (m, 1H), 2.80-2.75 (m, 1H), 2.73-2.67 (m, 2H), 2.62-2.56 (m, 5H), 2.42 (s, 3H), 2.29 (d, *J*=7.1 Hz, 2H), 1.95-1.86 (m, 3H), 1.76-1.65 (m, 1H), 1.41-1.33 (m, 2H), 1.16 (d, *J*=6.1 Hz, 6H). m/z 815.47 [M+H]⁺. |
| 55 | ¹H NMR (400 MHz, CDCl₃) δ 8.42 (d, *J*=5.2 Hz, 1H), 7.99 (s, 1H), 7.85 (m, 2H), 7.56 (d, *J*=9.0 Hz, 2H), 7.37 (d, *J*=7.7 Hz, 1H), 7.08 (d, *J*=5.3 Hz, 2H), 6.97 (d, *J*=9.0 Hz, 2H), 6.91 (d, *J*=8.9 Hz, 2H), 6.67 (d, *J*=8.9 Hz, 2H), 4.49 (d, *J*=2.9 Hz, 1H), 4.45 (d, *J*=5.5 Hz, 2H), 4.38-4.32 (m, 1H), 4.23 (t, *J*=5.5 Hz, 1H), 4.16-4.08 (m, 2H), 4.01 (d, *J*=11.1 Hz, 1H), 3.87 (dd, *J*=8.8, 4*.*7 Hz, 2H), 3.65-3.58 (m, 1H), 3.50 (d, *J*=9.6 Hz, 2H), 3.24-3.14 (m, 4H), 2.93-2.82 (m, 1H), 2.76 (dd, *J*=13.2, 5.1 Hz, 1H), 2.66-2.50 (m, 7H), 2.42 (s, 3H), 2.30 (d, *J*=7.1 Hz, 2H), 1.91 (dd, *J*=12.5, 4.8 Hz, 3H), 1.66 (m, 1H), 1.40 (d, *J*=11.9 Hz, 2H), 1.16 (d, *J*=6.1 Hz, 6H). m/z 815.51 [M+H]⁺. |
| 56 | ¹H NMR (400 MHz, CDCl₃) δ 8.44 (d, *J*=5.2 Hz, 1H), 7.88 (m, 3H), 7.62 (d, *J*=8.5 Hz, 2H), 7.38 (d, *J*=7.9 Hz, 1H), 7.24 (d, *J*=8.6 Hz, 2H), 7.15 (s, 1H), 7.12 (d, *J*=5.2 Hz, 1H), 6.91 (d, *J*=8.9 Hz, 2H), 6.67 (d, *J*=8.9 Hz, 2H), 4.49 (d, *J*=3.3 Hz, 1H), 4.46 (d, *J*=5.6 Hz, 2H), 4.35 (m, 1H), 4.23 (m, 1H), 4.17-4.10 (m, 2H), 4.06-3.98 (m, 1H), 3.87 (dd, *J*=8.6, 4.7 Hz, 2H), 3.66-3.57 (m, 1H), 3.51 (m, 2H), 3.02 (d, *J*=11.5 Hz, 2H), 2.85 (m, 1H), 2.74 (m, 1H), 2.61 (m, 4H), 2.43 (s, 3H), 2.26 (d, *J*=7.0 Hz, 2H), 2.07-1.98 (m, 2H), 1.94-1.75 (m, 7H), 1.65 (s, 1H), 1.40 (d, *J*=9.0 Hz, 2H), 1.16 (d, *J*=6.1 Hz, 6H). m/z 814.52 [M+H]⁺. |
| 57 | ¹H NMR (400 MHz, CDCl₃) δ 8.42 (d, *J*=5.2 Hz, 1H), 8.06 (s, 1H), 7.95 (s, 1H), 7.89 (s, 1H), 7.87 (d, *J*=7.9 Hz, 1H), 7.73 (s, 1H), 7.56 (d, *J*=8.9 Hz, 2H), 7.41 (d, *J*=7.9 Hz, 1H), 7.12-7.08 (m, 3H), 6.97 (d, *J*=9.0 Hz, 2H), 6.46 (dd, *J*=8.2, 2.0 Hz, 1H), 6.29 (t, *J*=2.0 Hz, 1H), 6.16 (dd, *J*=7.9, 1.8 Hz, 1H), 5.88 (t, *J*=5.4 Hz, 1H), 4.68-4.65 (m, 3H), 4.56-4.67 (m, 1H), 4.09 (dt, *J*=12.5, 4.2 Hz, 1H), 3.67 (d, *J*=12.3 Hz, 2H), 3.18 (t, *J*=4.2 Hz, 4H), 2.91-2.84 (m, 1H), 2.80-2.75 (m, 1H), 2.73-2.67 (m, 2H), 2.60-2.55 (m, 5H), 2.45 (s, 3H), 2.29 (d, *J*=6.9 Hz, 2H), 1.93-1.87 (m, 2H), 1.72-1.65 (m, 1H), 1.53 (d, *J*=6.7 Hz, 6H), 1.41-1.30 (m, 2H). m/z 810.47 [M+H]⁺. |
| 58 | ¹H NMR (400 MHz, CDCl₃) δ 8.42 (d, *J*=5.2 Hz, 1H), 7.95 (s, 1H), 7.89-7.86 (m, 3H), 7.80 (d, *J*=2.9 Hz, 1H), 7.73 (s, 1H), 7.56 (d, *J*=8.9 Hz, 2H), 7.41 (d, *J*=7.9 Hz, 1H), 7.09-7.08 (m, 2H), 7.05 (dd, *J*=9.0, 3.0 Hz, 1H), 6.97 (d, *J*=9.1 Hz, 2H), 6.67 (d, *J*=9.1 Hz, 1H), 5.87 (t, *J*=5.2 Hz, 1H), 4.66 (d, *J*=5.6 Hz, 2H), 4.55-4.49 (m, 1H), 4.30 (brs 1H), 4.15-4.10 (m, 2H), 3.95 (dd, *J*=12.3, 4.8 Hz, 1H), 3.20-3.17 (m, 4H), 2.90-3.83 (m, 1H), 2.79-2.66 (m, 3H), 2.64-2.56 (m, 4H), 2.55-2.47 (m, 1H), 2.45 (s, 3H), 2.28 (d, *J*=7.0 Hz, 2H), 1.98-1.90 (m, 2H), 1.78-1.72 (m, 1H), 1.53 (d, *J*=6.7 Hz, 6H), 1.33-1.27 (m, 2H). m/z 811.49 [M+H]⁺. |
| 59 | ¹H NMR (400 MHz, CDCl₃) δ 8.42 (d, *J*=5.2 Hz, 1H), 8.04 (s, 1H), 7.93 (s, 1H), 7.93 (s, 1H), 7.87 (d, *J*=7.9 Hz, 1H), 7.74 (s, 1H), 7.56 (d, *J*=8.9 Hz, 2H), 7.41 (d, *J*=7.9 Hz, 1H), 7.13-7.05 (m, 3H), 6.97 (d, *J*=9.0 Hz, 2H), 6.46 (dd, *J*=8.3, 2.0 Hz, 1H), 6.29 (t, *J*=1.9 Hz, 1H), 6.16 (dd, *J*=7.9, 1.8 Hz, 1H), 5.88 (t, *J*=5.2 Hz, 1H), 4.67 (d, *J*=5.5 Hz, 3H), 4.09 (dt, *J*=12.3, 4.2 Hz, 1H), 3.67 (d, *J*=12.0 Hz, 2H), 3.18 (t, *J*=4.4 Hz, 4H), 2.91-2.85 (m, 1H), 2.79-2.76 (m, 1H), 2.70 (t, *J*=11.2 Hz, 2H), 2.61-2.56 (m, 5H), 2.46 (s, 3H), 2.29 (d, *J*=7.1 Hz, 2H), 1.96-1.87 (m, 2H), 1.74-1.66 (m, 1H), 1.61 (s, 9H), 1.41-1.31 (m, 2H). m/z 824.50 [M+H]⁺. |
| 60 | ¹H NMR (400 MHz, CDCl₃) δ 8.42 (d, *J*=5.2 Hz, 1H), 8.04 (s, 1H), 7.95 (brs, 1H), 7.89 (s, 1H), 7.87 (d, *J*=7.9 Hz, 1H), 7.80 (d, *J*=2.9 Hz, 1H), 7.74 (s, 1H), 7.56 (d, *J*=8.9 Hz, 2H), 7.41 (d, *J*=7.9 Hz, 1H), 7.09-7.03 (m, 3H), 6.96 (d, *J*=9.0 Hz, 2H), 6.67 (d, *J*=9.0 Hz, 1H), 5.89 (t, *J*=5.3 Hz, 1H), 4.66 (d, *J*=5.5 Hz, 2H), 4.30 (d, *J*=2.5 Hz, 1H), 4.13-4.10 (m, 2H), 3.98-3.92 (m, 1H), 3.18 (t, *J*=4.6 Hz, 4H), 2.89-2.83 (m, 1H), 2.79-2.68 (m, 3H), 2.60 (t, *J*=4.4 Hz, 4H), 2.54-2.49 (m, 1H), 2.45 (s, 3H), 2.28 (d, *J*=7.1 Hz, 2H), 1.98-1.90 (m, 2H), 1.79-1.72 (m, 1H), 1.60 (s, 9H), 1.34-1.28 (m, 2H). m/z 825.52 [M+H]⁺. |
| 61 | ¹H NMR (400 MHz, CDCl₃) δ 8.41 (d, *J*=5.3 Hz, 1H), 8.18 (s, 1H), 7.93 (s, 1H), 7.90-7.82 (m, 2H), 7.56 (d, *J*=8.6 Hz, 2H), 7.46-7.38 (m, 2H), 7.14-7.04 (m, 3H), 6.97 (d, *J*=8.8 Hz, 2H), 6.46 (d, *J*=8.5 Hz, 1H), 6.29 (s, 1H), 6.16 (d, *J*=7.5 Hz, 1H), 4.69 (dd, *J*=13.4, 4.5 Hz, 3H), 4.09 (d, *J*=12.3 Hz, 1H), 3.67 (d, *J*=12.3 Hz, 2H), 3.18 (s, 4H), 2.85 (s, 1H), 2.80-2.66 (m, 3H), 2.60 (s, 5H), 2.46 (s, 3H), 2.29 (d, *J*=7.0 Hz, 2H), 1.88 (d, *J*=11.5 Hz, 3H), 1.71 (s, 9H), 1.36 (d, *J*=16.2 Hz, 2H). m/z 825.50 [M+H]⁺. |
| 62 | ¹H NMR (400 MHz, CDCl₃) δ 8.41 (d, *J*=5.2 Hz, 1H), 8.19 (s, 1H), 8.06 (s, 1H), 7.90-7.83 (m, 2H), 7.80 (d, *J*=2.9 Hz, 1H), 7.57 (d, *J*=8.9 Hz, 2H), 7.43 (d, *J*=8.0 Hz, 2H), 7.10-7.03 (m, 3H), 6.97 (d, *J*=9.0 Hz, 2H), 6.67 (d, *J*=9.0 Hz, 1H), 4.71 (d, *J*=5.9 Hz, 2H), 4.30 (s, 1H), 4.12 (dd, *J*=9.5, 5.0 Hz, 2H), 3.95 (dd, *J*=12.4, 3.3 Hz, 1H), 3.21-3.12 (m, 4H), 2.84 (m, 1H), 2.77-2.65 (m, 3H), 2.61 (m, 4H), 2.46 (m, 4H), 2.28 (d, *J*=7.1 Hz, 2H), 1.97-1.84 (m, 3H), 1.71 (s, 9H), 1.35-1.28 (m, 2H). m/z 826.51 [M+H]⁺. |
| 63 | ¹H NMR (400 MHz, CDCl₃) δ 8.46 (d, *J*=5.2 Hz, 1H), 8.01 (s, 1H), 7.87 (m, 2H), 7.67 (d, *J*=8.5 Hz, 2H), 7.38 (d, *J*=8.5 Hz, 1H), 7.31 (d, *J*=8.4 Hz, 2H), 7.25 (s, 1H), 7.15 (m, 2H), 6.62 (m, 2H), 6.51-6.45 (m, 1H), 4.87 (dd, *J*=7.6, 4.3 Hz, 1H), 4.46 (d, *J*=5.6 Hz, 2H), 4.36 (m, 1H), 4.24 (t, *J*=5.5 Hz, 1H), 4.17-4.09 (m, 2H), 3.87 (dd, *J*=8.7, 4.7 Hz, 2H), 3.71-3.59 (m, 3H), 3.50 (d, *J*=5.1 Hz, 2H), 2.95 (m, 1H), 2.67 (m, 3H), 2.58-2.36 (m, 11H), 2.30 (m, 2 H), 2.22 (d, *J*=7.1 Hz, 2H), 1.84 (d, *J*=11.4 Hz, 2H), 1.30 (dd, *J*=22.5, 13.5, 2H), 1.16 (d, *J*=6.1 Hz, 6H). m/z 830.47 [M+H]⁺. |
| 64 | ¹H NMR (400 MHz, CDCl₃) δ 8.46 (d, *J*=5.2 Hz, 1H), 8.14 (s, 1H), 7.87-7.86 (m, 2H), 7.67 (d, *J*=8.4 Hz, 2H), 7.38 (d, *J*=8.5 Hz, 1H), 7.31 (d, *J*=8.4 Hz, 2H), 7.27 (s, 1H), 7.13 (d, *J*=5.2 Hz, 1H), 7.10 (t, *J*=8.1 Hz, 1H), 6.44 (dd, *J*=8.2, 2.0 Hz, 1H), 6.27 (t, *J*=2.0, 1H), 6.15 (dd, *J*=7.9, 1.8 Hz, 1H), 4.67 (d, *J*=3.6 Hz, 1H), 4.46 (d, J=5.6 Hz, 2H), 4.39-4.32 (m, 1H), 4.25 (t, *J*=5.5 Hz, 1H), 4.18-4.12 (m, 2H), 4.08 (dt, *J*=12.4, 4.3 Hz, 1H), 3.87 (dd, *J*=8.5, 4.7 Hz, 2H), 3.68-3.57 (m, 3H), 3.51 (s, 2H), 2.91-2.83 (m, 1H), 2.79-2.72 (m, 1H), 2.70-2.63 (m, 2H), 2.61-2.41 (m, 11H), 2.22 (d, *J*=7.1 Hz, 2H), 1.95-1.83 (m, 3H), 1.40-1.24 (m, 3H), 1.16 (d, *J*=6.1 Hz, 6H). m/z 829.50 [M+H]⁺. |
| 65 | ¹H NMR (400 MHz, CDCl₃) δ 8.41 (d, *J*=5.2 Hz, 1H), 7.86-7.84 (m, 2H), 7.57 (d, *J*=8.9 Hz, 3H), 7.37 (d, *J*=7.9 Hz, 1H), 7.29-7.27 (m, 1H), 7.18 (s, 1H), 7.08 (d, *J*=5.2 Hz, 1H), 6.97 (d, *J*=9.0 Hz, 2H), 6.90-6.84 (m, 2H), 6.72-6.70 (m, 1H), 4.45 (d, *J*=5.6 Hz, 2H), 4.40-4.31 (m, 1H), 4.23 (t, *J*=5.5 Hz, 1H), 4.17-4.09 (m, 2H), 3.90-3.83 (m, 4H), 3.70 (d, *J*=12.3 Hz, 2H), 3.64-3.60 (m, 1H), 3.22-3.13 (m, 4H), 2.82 (t, J=6.7 Hz, 2H), 2.75 (td, *J*=12.0, 2.0 Hz, 2H), 2.65-2.58 (m, 4H), 2.42 (s, 3H), 2.30 (d, *J*=7.1 Hz, 2H), 1.90 (d, *J*=12.1 Hz, 2H), 1.35 (ddd, *J*=15.5, 12.5, 3.4 Hz, 3H), 1.16 (d, *J*=6.1 Hz, 6H). m/z 801.54 [M+H]⁺. |
| 66 | ¹H NMR (400 MHz, CDCl₃) δ 8.42 (d, J=5.2 Hz, 1H), 7.85 (m, 2H), 7.57 (m, 3H), 7.37 (d, J=7.8 Hz, 1H), 7.15 (m, 3H), 7.08 (d, J=5.2 Hz, 1H), 7.01-6.92 (m, 4H), 4.45 (d, J=5.6 Hz, 2H), 4.35 (m, 1H), 4.23 (t, J=5.4 Hz, 1H), 4.16-4.09 (m, 2H), 3.90-3.79 (m, 4H), 3.70 (d, J=12.3 Hz, 2H), 3.61 (m, 1H), 3.18 (m, 4H), 2.82 (t, J=6.7 Hz, 2H), 2.73 (dd, J=12.1, 10.1 Hz, 2H), 2.61 (m, 4H), 2.42 (s, 3H), 2.29 (d, J=7.1 Hz, 2H), 1.90 (d, J=13.3 Hz, 2H), 1.71 (s, 1H), 1.35 (m, 2H), 1.16 (d, J=6.1 Hz, 6H). m/z 801.56 [M+H]⁺. |
| 67 | ¹H NMR (400 MHz, CDCl₃) δ 8.48 (d, *J*=5.2 Hz, 1H), 7.88-7.86 (m, 2H), 7.76 (d, *J*=7.2 Hz, 2H), 7.60 (s, 1H), 7.50-7.38 (m, 4H), 7.17 (d, *J*=5.2 Hz, 1H), 6.84-6.82 (m, 2H), 6.70 (d, *J*=8.1 Hz, 1H), 4.46 (d, *J*=5.6 Hz, 2H), 4.41-4.32 (m, 1H), 4.29 (t, *J*=5.5 Hz, 1H), 4.14 (t, *J*=7.6 Hz, 2H), 3.93-3.82 (m, 5H), 3.67 (d, *J*=13.1 Hz, 2H), 3.64-3.58 (m, 1H), 3.07-2.80 (m, 9H), 2.72 (t, *J*=12.0 Hz, 3H), 2.43 (s, 3H), 1.94-1.80 (m, 3H), 1.42-1.21 (m, 5H), 1.16 (d, *J*=6.1 Hz, 6H). m/z 815.55 [M+H]⁺. |
| 68 | ¹H NMR (400 MHz, CDCl₃) δ 8.45 (d, *J*=5.2 Hz, 1H), 7.87 (m, 2H), 7.66 (d, *J*=8.4 Hz, 2H), 7.38 (d, *J*=8.0 Hz, 1H), 7.31 (d, *J*=8.6 Hz, 2H), 7.21 (s, 1H), 7.14 (m, 3H), 6.93 (d, *J*=9.0 Hz, 2H), 4.46 (d, J=5.6 Hz, 2H), 4.39-4.32 (m, 1H), 4.24 (m, 1H), 4.17-4.09 (m, 2H), 3.87 (dd, *J*=8.7, 4.7 Hz, 2H), 3.81 (t, *J*=6.7 Hz, 2H), 3.72-3.64 (m, 2H), 3.60 (m, 1H), 3.51 (s, 2H), 2.81 (t, *J*=6.7 Hz, 2H), 2.70 (dd, *J*=12.3, 10.1 Hz, 2H), 2.64-2.30 (m, 11H), 2.22 (d, *J*=7.0 Hz, 2H), 1.85 (d, *J*=11.4 Hz, 2H), 1.35-1.28 (m, 4H), 1.16 (d, *J*=6.1 Hz, 6H). m/z 815.54 [M+H]⁺. |
| 69 | ¹H NMR (400 MHz, CDCl₃) δ 8.45 (d, *J*=5.2 Hz, 1H), 8.19 (s, 1H), 7.97 (brs, 1H), 7.90 (s, 1H), 7.88 (d, *J*=8.0 Hz, 1H), 7.68 (d, *J*=8.2 Hz, 2H), 7.45 (d, *J*=7.8 Hz, 2H), 7.37 (s, 1H), 7.31 (d, *J*=8.3 Hz, 2H), 7.14 (d, *J*=5.2 Hz, 1H), 7.09 (t, *J*=8.1 Hz, 1H), 6.43 (d, *J*=8.2 Hz, 1H), 6.26 (s, 1H), 6.15 (d, *J*=7.9 Hz, 1H), 4.71 (d, *J*=5.9 Hz, 2H), 4.66 (brs, 1H), 4.08 (dd, *J*=12.4, 4.7 Hz, 1H), 3.63 (d, *J*=12.0 Hz, 2H), 3.59-2.51 (m, 2H), 2.91-2.82 (m, 1H), 2.80-2.72 (m, 1H), 2.70-2.50 (m, 9H), 2.47 (s, 3H), 2.27-2.20 (m, 2H), 1.96-1.86 (m, 2H), 1.84 (d, *J*=13.7 Hz, 2H), 1.71 (s, 9H), 1.37-1.27 (m, 3H). m/z 839.58 [M+H]⁺. |
| 70 | ¹H NMR (400 MHz, CDCl₃) δ 8.42 (d, *J*=5.2 Hz, 1H), 8.19 (s, 1H), 7.88 (s, 1 H), 7.86 (d, *J*=7.9 Hz, 1H), 7.68 (s, 1H), 7.57 (d, *J*=8.9 Hz, 2H), 7.43 (d, *J*=7.9 Hz, 2H), 7.17 (s, 1H), 7.08 (d, *J*=5.2 Hz, 1H), 6.96 (d, *J*=9.0 Hz, 2H), 6.88-6.85 (m, 2H), 6.71 (d, *J*=8.5 Hz, 1H), 4.70 (d, *J*=5.9 Hz, 2H), 3.86 (t, *J*=6.7 Hz, 2H), 3.70 (d, *J*=12.3 Hz, 2H), 3.25-3.15 (m, 4H), 2.82 (t, *J*=6.7 Hz, 2H), 2.75 (td, *J*=12.1, 2.1 Hz, 2H), 2.66-2.59 (m, 4H), 2.46 (s, 3H), 2.31 (d, *J*=6.8 Hz, 2H), 1.91 (d, *J*=12.6 Hz, 2H), 1.71 (s, 9H), 1.68-1.67 (m, 1H), 1.40-1.30 (m, 2H). m/z 811.52 [M+H]⁺. |
| 71 | ¹H NMR (400 MHz, DMSO-*d*₆) δ 10.26 (s, 1H), 9.38 (s, 1H), 9.01 (t, *J*=6.1 Hz, 1H), 8.71 (s, 1H), 8.45 (d, *J*=5.2 Hz, 1H), 7.99-7.88 (m, 2H), 7.66 (d, *J*=9.0 Hz, 2H), 7.37 (d, *J*=8.1 Hz, 1H), 7.28 (d, *J*=5.3 Hz, 1H), 7.13 (d, *J*=8.9 Hz, 2H), 6.94-6.90 (m, 4H), 4.51 (d, *J*=6.0 Hz, 2H), 3.69 (t, *J*=6.7 Hz, 4H), 3.08 (s, 4H), 2.65 (m, 8H), 2.42 (s, 3H), 2.22 (d, *J*=7.0 Hz, 2H), 1.81 (d, *J*=12.5 Hz, 2H), 1.70 (m, 1H), 1.64 (s, 9H), 1.22 (d, *J*=9.5 Hz, 2H). m/z 811.54 [M+H]⁺. |
| 72 | ¹H NMR (400 MHz, DMSO-*d₆*) δ 10.77 (s, 1H), 9.37 (s, 1H), 9.00 (t, *J*=6.1 Hz, 1H), 8.71 (s, 1H), 8.45 (d, *J*=5.2 Hz, 1H), 7.96 (s, 1H), 7.92 (d, *J*=8.0 Hz, 1H), 7.65 (d, *J*=9.0 Hz, 2H), 7.37 (d, *J*=8.0 Hz, 1H), 7.28 (d, *J*=5.2 Hz, 1H), 6.91 (d, *J*=9.2 Hz, 2H), 6.83 (t, *J*=9.4 Hz, 1H), 6.50 (d, *J*=15.0 Hz, 1H), 6.42 (d, *J*=8.6 Hz, 1H), 5.78 (d, *J*=7.7 Hz, 1H), 4.51 (d, *J*=5.7 Hz, 2H), 4.29-4.21 (m, 1H), 3.12 (d, *J*=10.8 Hz, 2H), 3.07 (s, 4H), 2.70-2.73 (m, 2H), 2.58 (m, 4H), 2.56 (m, 2H), 2.42 (s, 3H), 2.23 (d, *J*=7.1 Hz, 2H), 2.13-2.05 (m, 1H), 2.03-1.82 (m, 3H), 1.82-1.75 (m, 2H), 1.64 (s, 9H). m/z 843.60 [M+H]⁺. |
| 73 | ¹H NMR (400 MHz, DMSO-*d₆*) δ 10.36 (s, 1H), 9.38 (s, 1H), 9.02 (t, *J*=5.9 Hz, 1H), 8.71 (s, 1H), 8.45 (d, *J*=5.2 Hz, 1H), 7.96 (s, 1H), 7.93 (d, *J*=8.2 Hz, 1H), 7.65 (d, *J*=9.0 Hz, 2H), 7.36 (d, *J*=8.0 Hz, 1H), 7.28 (d, *J*=5.3 Hz, 1H), 7.19-7.13 (m, 1H), 7.04 (m, 2H), 6.92 (d, *J*=9.1 Hz, 2H), 4.51 (d, *J*=5.9 Hz, 2H), 3.74 (t, *J*=6.7 Hz, 2H), 3.10-3.15 (m, 2H), 3.08 (m, 4H), 2.73-2.61 (m, 6H), 2.50 (m, 2H), 2.42 (s, 3H), 2.24 (d, *J*=6.9 Hz, 2H), 1.83 (d, *J*=13.1 Hz, 2H), 1.74-1.67 (m, 1H), 1.64 (s, 9H), 1.36-1.27 (m, 2H). m/z 829.57 [M+H]⁺. |
| 74 | ¹H NMR (400 MHz, CDCl₃) δ 8.45 (d, *J*=5.2 Hz, 1H), 8.18 (s, 1H), 7.90 (brs, 1H), 7.88 (d, J*=*7.9 Hz, 1H), 7.66 (d, J=8.5 Hz, 2H), 7.47 (s, 1H), 7.46-7.41 (m, 2H), 7.31 (d, *J*=8.3 Hz, 2H), 7.24 (s, 1H), 7.13 (d, *J*=5.3 Hz, 1H), 6.85-6.83 (m, 2H), 6.70-6.68 (m, 1H), 4.71 (d, J=5.9 Hz, 2H), 3.84 (t, *J*=6.7 Hz, 2H), 3.66 (d, *J*=12.3 Hz, 2H), 3.51 (s, 2H), 2.81 (t, *J*=6.7 Hz, 2H), 2.72 (td, *J*=12.1, 2.1 Hz, 2H), 2.57-2.41 (m, 11H), 2.22 (d, *J*=7.0 Hz, 2H), 1.85 (d, *J*=12.7 Hz, 2H), 1.71 (s, 9H), 1.36-1.26 (m, 3H). m/z 825.58 [M+H]⁺. |
| 75 | ¹H NMR (400 MHz, CDCl₃) δ 8.45 (d, *J*=5.2 Hz, 1H), 8.18 (s, 1H), 7.90 (s, 1H), 7.88 (d, *J*=7.9 Hz, 1H), 7.66 (d, *J*=8.4 Hz, 2H), 7.46-7.04 (m, 3H), 7.31 (d, *J*=7.9 Hz, 2H), 7.21 (s, 1H), 7.16-7.11 (m, 3H), 6.93 (d, *J*=9.0 Hz, 2H), 4.71 (d, *J*=5.9 Hz, 2H), 3.80 (t, *J*=6.7 Hz, 2H), 3.67 (d, *J*=12.4 Hz, 2H), 3.51 (s, 2H), 2.81 (t, *J*=6.7 Hz, 2H), 2.70 (td, *J*=12.1, 2.0 Hz, 2H), 2.55-2.39 (m, 1 1H), 2.22 (d, *J*=6.7 Hz, 2H), 1.85 (d, *J*=12.1 Hz, 2H), 1.71 (s, 9H), 1.37-1.26 (m, 3H). m/z 825.60 [M+H]⁺. |
| 76 | ¹H NMR (400 MHz, CDCl₃) δ 8.40 (d, *J*=5.2 Hz, 1H), 8.34 (d, *J*=2.7 Hz, 1H), 8.18 (s, 1H), 7.98 (dd, *J*=9.1, 2.7 Hz, 1H), 7.86-7.83 (m, 2H), 7.47-7.38 (m, 3H), 7.30 (d, *J*=2.8 Hz, 1H), 7.09 (d, *J*=5.3 Hz, 1H), 6.93 (s, 1H), 6.89-6.83 (m, 2H), 6.73-6.70 (m, 2H), 4.70 (d, *J*=5.9 Hz, 2H), 3.86 (t, *J*=6.7 Hz, 2H), 3.70 (d, *J*=12.3 Hz, 2H), 3.54-3.49 (m, 4H), 2.82 (t, *J*=6.7 Hz, 2H), 2.75 (td, *J*=11.8, 1.8 Hz, 2H), 2.60-2.52 (m, 4H), 2.46 (s, 3H), 2.28 (d, *J*=7.1 Hz, 2H), 1.90 (d, *J*=11.3 Hz, 2H), 1.77-1.73 (m, 1H), 1.71 (s, 9H), 1.35 (qd, *J*=12.4, 3.9 Hz, 2H). m/z 812.58 [M+H]⁺. |
| 77 | ¹H NMR (400 MHz, DMSO-*d*₆) δ 10.23 (s, 1H), 9.37 (s, 1H), 8.98 (t, *J*=6.1 Hz, 1H), 8.70 (s, 1H), 8.47 (d, J=2.6 Hz, 1H), 8.45 (d, *J*=5.2 Hz, 1H), 7.97 (dd, *J*=9.1, 2.5 Hz, 1H), 7.94 (s, 1H), 7.90 (d, *J*=8.0 Hz, 1H), 7.37 (d, *J*=8.1 Hz, 1H), 7.29 (d, *J*=5.3 Hz, 1H), 7.13 (d, *J*=8.9 Hz, 2H), 6.93 (d, *J*=9.0 Hz, 2H), 6.84 (d, *J*=9.1 Hz, 1H), 4.51 (d, *J*=5.9 Hz, 2H), 3.71-3.68 (m, 4H), 3.44-3.38 (m, 4H), 2.70-2.64 (m, 4H), 2.48-2.44 (d, *J*=4.2 Hz, 4H), 2.42 (s, 3H), 2.22 (d, *J*=7.1 Hz, 2H), 1.82 (d, *J*=12.5 Hz, 2H), 1.77-1.70 (m, 1H), 1.64 (s, 9H), 1.28-1.19 (m, 2H). m/z 812.58 [M+H]⁺. |
| 78 | ¹H NMR (400 MHz, CDCl₃) δ 8.45 (d, *J*=5.2 Hz, 1H), 8.19 (s, 1H), 7.90 (s, 1H), 7.87 (dd, *J*=8.0, 1.4 Hz, 1H), 7.73 (s, 1H), 7.63 (d, *J*=8.5 Hz, 2H), 7.44 (d, *J*=7.8 Hz, 2H), 7.31 (s, 1H), 7.23 (d, *J*=8.5 Hz, 2H), 7.12 (d, *J*=5.3 Hz, 1H), 6.87 (m, 2H), 6.73-6.68 (m, 1H), 4.71 (d, *J*=5.9 Hz, 2H), 3.86 (t, *J*=6.7 Hz, 2H), 3.70 (d, *J*=12.4 Hz, 2H), 3.02 (d, *J*=11.2 Hz, 2H), 2.82 (t, *J*=6.7 Hz, 2H), 2.74 (dd, *J*=12.2, 10.1 Hz, 2H), 2.51 (m, 1H), 2.47 (s, 3H), 2.26 (d, *J*=7.0 Hz, 2H), 2.04 (td, *J*=10.6, 2.9 Hz, 2H), 1.93-1.77 (m, 7H), 1.71 (s, 9H), 1.30-1.35 (m, 2H). m/z 810.57 [M+H]⁺. |
| 79 | ¹H NMR (400 MHz, DMSO-*d₆*) δ 10.22 (s, 1H), 9.52 (s, 1H), 8.98 (t, *J*=6.1 Hz, 1H), 8.69 (s, 1H), 8.49 (d, *J*=5.2 Hz, 1H), 7.98 (s, 1H), 7.93 (d, *J*=8.0 Hz, 1H), 7.73 (d, *J*=8.6 Hz, 2H), 7.38 (d, *J*=8.0 Hz, 1H), 7.33 (d, *J*=5.2 Hz, 1H), 7.18 (d, *J*=8.6 Hz, 2H), 7.13 (d, *J*=8.9 Hz, 2H), 6.92 (d, *J*=9.0 Hz, 2H), 4.51 (d, *J*=6.0 Hz, 2H), 3.73-3.65 (m, 4H), 2.95 (d, *J*=11.0 Hz, 2H), 2.72-2.65 (m, 4H), 2.45-2.39 (m, 4H), 2.19 (d, *J*=7.1 Hz, 2H), 1.98 (t, *J*=10.7 Hz, 2H), 1.86-1.68 (m, 6H), 1.65 (s, 9H), 1.28-1.14 (m, 3H). m/z 810.58 [M+H]⁺. |
| 80 | ¹H NMR (400 MHz, CDCI3) δ 8.41 (d, *J*=5.2 Hz, 1H), 7.93 (s, 1H), 7.88 (s, 1H), 7.85 (d, *J*=7.9 Hz, 1H), 7.71 (s, 1H), 7.55 (d, *J*=8.9 Hz, 2H), 7.46 (brs, 1H), 7.39 (d, *J*=7.9 Hz, 1H), 7.07-7.06 (m, 2H), 6.95 (d, *J*=9.0 Hz, 2H), 6.86-6.84 (m, 2H), 6.69 (d, *J*=8.3 Hz, 1H), 5.86 (t, *J*=5.7 Hz, 1H), 4.65 (d, *J*=5.5 Hz, 2H), 4.55-4.45 (m, 1H), 3.84 (t, *J*=6.7 Hz, 2H), 3.68 (d, *J*=12.4 Hz, 2H), 3.20-3.12 (m, 4H), 2.81 (t, *J*=6.7 Hz, 2H), 2.73 (td, *J*=12.1, 2.2 Hz, 2H), 2.63-2.56 (m, 4H), 2.44 (s, 3H), 2.27 (d, *J*=7.2 Hz, 2H), 1.88 (d, *J*=12.6 Hz, 2H),1.74-1.66 (m, 1H), 1.51 (d, *J*=6.7 Hz, 6H), 1.38-1.29 (m, 2H). m/z 796.66 [M+H]⁺. |
| 81 | ¹H NMR (400 MHz, CDCl₃) δ 8.42 (d, *J*=5.2 Hz, 1H), 8.04 (s, 1H), 7.89 (s, 1H), 7.87 (d, *J*=7.9 Hz, 1H), 7.74 (s, 1H), 7.62 (s, 1H), 7.57 (d, *J*=9.0 Hz, 2H), 7.41 (d, *J*=7.9 Hz, 1H), 7.16 (s, 1H), 7.08 (d, *J*=5.3 Hz, 1H), 6.96 (d, *J*=9.0 Hz, 2H), 6.87-6.85 (m, 2H), 6.71 (d, *J*=8.8 Hz, 1H), 5.89 (t, *J*=5.4 Hz, 1H), 4.66 (d, *J*=5.6 Hz, 2H), 3.86 (t, *J*=6.7 Hz, 2H), 3.70 (d, *J*=12.4 Hz, 2H), 3.24-3.13 (m, 4H), 2.82 (t, *J*=6.7 Hz, 2H), 2.75 (td, *J*=12.0, 1.9 Hz, 2H), 2.63-2.56 (m, 4H), 2.45 (s, 3H), 2.29 (d, *J*=7.1 Hz, 2H), 1.90 (d, *J*=11.7 Hz, 2H), 1.75-1.71 (m, 1H), 1.60 (s, 9H), 1.40-1.31 (m, 2H). m/z 810.57 [M+H]⁺. |
| 82 | ¹H NMR (400 MHz, CDCl₃) δ 8.41 (d, J=5.2 Hz, 1H), 8.37 (d, J=2.7 Hz, 1H), 7.98-7.92 (m, 2H), 7.85 (m, 2H), 7.73 (s, 1H), 7.65 (s, 1H), 7.40 (d, *J*=8.1 Hz, 1H), 7.09 (d, *J*=5.2 Hz, 1H), 7.06 (s, 1H), 6.90-6.83 (m, 2H), 6.75-6.67 (m, 2H), 5.92 (t, *J*=5.3 Hz, 1H), 4.65 (d, *J*=5.6 Hz, 2H), 4.52 (hept, *J*=6.6 Hz, 1H), 3.86 (t, *J*=6.7 Hz, 2H), 3.70 (d, *J*=12.3 Hz, 2H), 3.58-3.45 (m, 4H), 2.82 (t, *J*=6.7 Hz, 2H), 2.75 (td, *J*=12.2, 2.2 Hz, 2H), 2.60-2.52 (m, 4H), 2.45 (s, 3H), 2.28 (d, *J*=7.1 Hz, 2H), 1.90 (d, *J*=11.9 Hz, 2H), 1.76-1.67 (m, 1H), 1.52 (d, *J*=6.7 Hz, 6H), 1.41-1.29 (m, 2H). m/z 797.50 [M+H]⁺. |
| 83 | ¹H NMR (400 MHz, CDCl₃) δ 8.41 (d, *J*=5.2 Hz, 1H), 8.37 (d, *J*=2.7 Hz, 1H), 8.04 (s, 1H), 7.95 (dd, *J*=9.1, 2.7 Hz, 1H), 7.85 (d, *J*=7.5 Hz, 2H), 7.75 (s, 1H), 7.61 (s, 1H), 7.40 (d, *J*=7.9 Hz, 1H), 7.09 (d, *J*=5.2 Hz, 1H), 7.04 (s, 1H), 6.89-6.84 (m, 2H), 6.75-6.67 (m, 2H), 5.92 (t, *J*=5.6 Hz, 1H), 4.66 (d, *J*=5.6 Hz, 2H), 3.86 (t, *J*=6.7 Hz, 2H), 3.70 (d, *J*=12.4 Hz, 2H), 3.57-3.46 (m, 4H), 2.82 (t, *J*=6.7 Hz, 2H), 2.75 (td, *J*=12.2, 2.2 Hz, 2H), 2.60-2.51 (m, 4H), 2.45 (s, 3H), 2.28 (d, *J*=7.1 Hz, 2H), 1.90 (d, *J*=11.9 Hz, 2H), 1.76-1.69 (m, 1H), 1.60 (s, 9H), 1.41-1.29 (m, 2H). m/z 811.53 [M+H]⁺. |
| 84 | ¹H NMR (400 MHz, CDCl₃) δ 8.45 (d, *J*=5.2 Hz, 1H), 7.95 (s, 1H), 7.91 (s, 1H), 7.87 (d, *J*=7.9 Hz, 1H), 7.74 (s, 1H), 7.63 (m, 3H), 7.41 (d, *J*=8.0 Hz, 1H), 7.23 (d, *J*=8.5 Hz, 2H), 7.12 (d, *J*=5.2 Hz, 1H), 6.86 (m, 2H), 6.70 (d, *J*=8.4 Hz, 1H), 5.90 (m, 1H), 4.66 (d, J=5.6 Hz, 2H), 4.60-4.46 (m, 1H), 3.86 (t, *J*=6.7 Hz, 2H), 3.70 (d, *J*=12.4 Hz, 2H), 3.02 (d, *J*=11.3 Hz, 2H), 2.82 (t, *J*=6.7 Hz, 2H), 2.74 (dd, *J*=12.1, 10.1 Hz, 2H), 2.48 (m, 4H), 2.26 (d, *J*=6.9 Hz, 2H), 2.04 (t, *J*=9.4 Hz, 2H), 1.93-1.76 (m, 6H), 1.66 (m, 2H), 1.53 (d, *J*=6.7 Hz, 6H), 1.43-1.29 (m, 3H). m/z 795.51 [M+H]⁺. |
| 85 | ¹H NMR (400 MHz, CDCl₃) δ 8.45 (d, *J*=5.2 Hz, 1H), 8.04 (s, 1H), 7.93-7.84 (m, 2H), 7.75 (s, 1H), 7.63 (d, *J*=8.5 Hz, 2H), 7.56 (s, 1H), 7.42 (d, *J*=7.9 Hz, 2H), 7.23 (m, 3H), 7.12 (d, *J*=5.2 Hz, 1H), 6.86 (m, 2H), 6.70 (d, *J*=8.4 Hz, 1H), 5.89 (t, *J*=5.3 Hz, 1H), 4.67 (d, *J*=5.6 Hz, 2H), 3.86 (t, *J*=6.7 Hz, 2H), 3.70 (d, *J*=12.3 Hz, 2H), 3.01 (d, *J*=11.2 Hz, 2H), 2.87-2.68 (m, 4H), 2.54-2.42 (m, 4H), 2.26 (d, *J*=7.0 Hz, 2H), 2.04 (dd, *J*=10.9, 8.0 Hz, 2H), 1.86 (m, 6H), 1.60 (s, 9H), 1.34 (dd, *J*=16.7, 7.3 Hz, 3H). m/z 809.58 [M+H]⁺. |
| 86 | ¹H NMR (400 MHz, CDCl₃) δ 8.41 (d, *J*=5.2 Hz, 1H), 7.86-7.84 (m, 2H), 7.56 (m, 3H), 7.43 (dd, *J*=8.5, 2.1 Hz, 1H), 7.38-7.36 (m, 2H), 7.10 (brs, 1H), 7.07 (m, 2H), 7.00 (d, *J*=8.4 Hz, 1H), 6.96 (d, *J*=9.0 Hz, 2H), 4.72-4.58 (m, 1H), 4.45 (d, *J*=5.6 Hz, 2H), 4.39-4.31 (m, 1H), 4.24-4.22 (m, 1H), 4.13 (t, *J*=7.6 Hz, 2H), 3.90 (s, 3H), 3.86 (dd, *J*=8.7, 4.7 Hz, 2H), 3.80-3.69 (m, 2H), 3.67-3.56 (m, 1H), 3.19-3.12 (m, 4H), 3.08-3.0 (m, 1H), 2.82 (t, *J*=6.6 Hz, 2H), 2.62-2.55 (m, 4H), 2.42 (s, 3H), 2.28-2.24 (m, 2H), 1.91-1.79 (m, 3H), 1.34-1.19 (m, 2H), 1.16 (d, *J*=6.0 Hz, 6H), 1.12-1.07 (m, 1H). m/z 859.54 [M+H]⁺. |
| 87 | ¹H NMR (400 MHz, CDCl₃) δ 8.41 (d, *J*=5.2 Hz, 1H), 8.18 (s, 1H), 7.85 (m, 2H), 7.56 (d, *J*=9.0 Hz, 2H), 7.36 (d, *J*=8.1 Hz, 1H), 7.22 (m, 1H), 7.13 (s, 1H), 7.07 (d, *J*=5.3 Hz, 1H), 6.96 (d, *J*=9.0 Hz, 2H), 6.78 (d, *J*=7.5 Hz, 1H), 6.73-6.68 (m, 2H), 4.81 (d, *J*=3.9 Hz, 1H), 4.70 (s, 1H), 4.45 (d, *J*=5.6 Hz, 2H), 4.35 (m, 1H), 4.25 (t, *J*=5.5 Hz, 1H), 4.17-4.07 (m, 3H), 3.86 (m, 3H), 3.61 (m, 1H), 3.20-3.11 (m, 4H), 2.97 (m, 1H), 2.90-2.67 (m, 3H), 2.58 (m, 5H), 2.42 (s, 3H), 2.28 (s, 2H), 1.93-1.79 (m, 3H), 1.16 (d, *J*=6.2 Hz, 6H), 1.03 (m, 2H). m/z 843.49 [M+H]⁺. |
| 88 | ¹H NMR (400 MHz, CDCl₃) δ 8.41 (d, *J*=5.2 Hz, 1H), 8.01 (s, 1H), 7.85 (m, 2H), 7.56 (d, *J*=9.0 Hz, 2H), 7.37 (d, *J*=7.8 Hz, 1H), 7.23 (m, 1H), 7.09-7.05 (m, 2H), 7.01-6.92 (m, 4H), 6.78 (dd, *J*=8.1, 1.8 Hz, 1H), 4.82 (d, *J*=4.0 Hz, 1H), 4.45 (d, *J*=5.6 Hz, 2H), 4.41-4.26 (m, 3H), 4.24 (d, *J*=5.3 Hz, 1H), 4.17-4.08 (m, 3H), 4.03-3.97 (m, 1H), 3.86 (m, 3H), 3.61 (m, 1H), 3.14 (m, 4H), 2.91 (m, 1H), 2.84 (m, 1H), 2.79 (m, 1H), 2.69 (d, *J*=7.4 Hz, 2H), 2.64-2.55 (m, 5H), 2.42 (s, 3H), 1.96-1.85 (m, 1H), 1.16 (d, *J*=6.1 Hz, 6H). m/z 815.42 [M+H]⁺. |
| 89 | ¹H NMR (400 MHz, CDCl₃) δ 8.42 (d, *J*=5.2 Hz, 1H), 7.93 (s, 1H), 7.86-7.83 (m, 2H), 7.59-7.54 (m, 2H), 7.47-7.41 (m, 1H), 7.39-7.34 (m, 3H), 7.30 (dt, *J*=7.5, 1.3 Hz, 1H), 7.22 (s, 1H), 7.07 (d, *J*=5.3 Hz, 1H), 6.97-6.92 (m, 2H), 4.75-4.64 (m, 1H), 4.44 (d, *J*=5.6 Hz, 2H), 4.35 (tt, *J*=6.5, 4.7 Hz, 1H), 4.25 (t, *J*=5.6 Hz, 1H), 4.16-4.09 (m, 2H), 3.93-3.78 (m, 5H), 3.65-3.55 (m, 1H), 3.18-3.12 (m, 4H), 3.08-2.98 (m, 1H), 2.86-2.70 (m, 3H), 2.62-2.55 (m, 4H), 2.41 (s, 3H), 2.27 (d, *J*=6.2 Hz, 2H), 1.98-1.89 (m, 1H), 1.87-1.76 (m, 2H), 1.33-1.18 (m, 2H), 1.15 (d, *J*=6.1 Hz, 6H). m/z 829.55 [M+H]⁺. |
| 90 | ¹H NMR (400 MHz, CDCl₃) δ 8.44 (d, *J*=5.0 Hz, 1H), 7.83-7.75 (m, 2H), 7.52-7.47 (m, 2H), 7.42 (dd, *J*=8.5, 2.1 Hz, 1H), 7.37 (d, *J*=2.1 Hz, 1H), 7.24 (s, 1H), 6.99 (d, *J*=8.6 Hz, 1H), 6.94-6.89 (m, 2H), 6.87 (dd, *J*=8.6, 2.7 Hz, 1H), 6.71 (d, *J*=5.0 Hz, 1H), 5.97 (s, 1H), 4.40 (tt, *J*=6.6, 4.6 Hz, 1H), 4.27-4.21 (m, 2H), 3.97 (dd, *J*=8.8, 4.6 Hz, 2H), 3.89 (s, 3H), 3.74-3.60 (m, 4H), 3.17-3.10 (m, 4H), 2.82 (t, *J*=6.7 Hz, 2H), 2.61-2.53 (m, 4H), 2.26 (d, *J*=6.7 Hz, 2H), 2.18 (s, 3H), 1.91-1.75 (m, 2H), 1.62 (s, 3H), 1.18 (d, *J*=6.1 Hz, 6H). m/z 863.51 [M+H]⁺. |
| 91 | ¹H NMR (400 MHz, CDCl₃) δ 8.41 (d, *J*=5.2 Hz, 1H), 8.07 (brs, 1H), 8.00 (s, 1H), 7.88 (s, 1H), 7.82 (d, *J*=6.5 Hz, 1H), 7.58 (s, 1H), 7.37 (d, *J*=8.2 Hz, 1H), 7.23 (t, *J*=7.8 Hz, 1H), 7.07 (d, *J*=5.2 Hz, 1H), 6.90 (brs, 1H), 6.78 (d, *J*=7.6 Hz, 1H), 6.73-6.69 (m, 2H), 4.80 (d, *J*=4.4 Hz, 1H), 4.73-4.68 (m, 1H), 4.44 (d, *J*=5.5 Hz, 2H), 4.37-4.34 (m, 1H), 4.31-4.27 (m, 1H), 4.15-4.01 (m, 4H), 3.87 (dd, *J*=8.7, 4.7 Hz, 2H), 3.64-3.58 (m, 1H), 2.99 (d, *J*=9.9 Hz, 3H), 2.90-2.85 (m, 1H), 2.81-2.72 (m, 2H), 2.60-2.54 (m, 1H), 2.42 (s, 3H), 2.26 (d, *J*=6.4 Hz, 2H), 2.18-2.12 (m, 4H), 2.01-2.02 (m, 3H), 1.93-1.89 (m, 2H), 1.82-1.75 (m, 2H), 1.16 (d, *J*=6.1 Hz, 6H). m/z 832.54 [M+H]⁺. |
| 92 | ¹H NMR (400 MHz, CDCl₃) δ 8.41 (d, *J*=5.2 Hz, 1H), 8.17 (brs, 1H), 8.01 (s, 1H), 7.87 (s, 1H), 7.82 (d, *J*=7.7 Hz 1H), 7.58 (s, 1H), 7.38 (d, *J*=8.0 Hz, 1H), 7.22 (d, J=1.9 Hz, 1H), 7.07 (d, *J*=5.3 Hz, 1H), 7.00-6.97 (m, 3H), 6.96 (s, 1H), 6.79 (dd, *J*=8.1, 1.7 Hz, 1H), 4.82 (d, *J*=3.9 Hz, 1H), 4.44 (d, *J*=8.9 Hz, 2H), 4.42-4.38 (m, 1H), 4.37-4.33 (m, 2H), 4.31-4.26 (m, 1H), 4.17-4.10 (m, 4H), 4.01-3.96 (m, 1H), 3.88-3.85 (m, 3H), 3.64-3.58 (m, 1H), 2.98-2.92 (m, 2H), 2.90-2.85 (m, 2H), 2.83-2.76 (m, 1H), 2.66 (d, *J*=7.6 Hz, 2H), 2.62-2.55 (m, 1H), 2.41 (s, 3H), 2.21-2.15 (m, 3H), 2.08-2.02 (m, 2H), 1.97-1.87 (m, 1H), 1.16 (d, *J*=6.1 Hz, 6H). m/z 804.49 [M+H]⁺. |
| 93 | ¹H NMR (400 MHz, CDCl₃) δ 8.41 (d, *J*=5.2 Hz, 1H), 8.08 (s, 1H), 7.95 (s, 1H), 7.88-7.85 (m, 2H), 7.73 (s, 1H), 7.56 (d, *J*=9.0 Hz, 2H), 7.40 (d, *J*=7.9 Hz, 1H), 7.22 (t, *J*=7.8 Hz, 1H), 7.08 (d, *J*=5.3 Hz, 2H), 6.95 (d, *J*=9.0 Hz, 2H), 6.78 (d, *J*=7.5 Hz, 1H), 6.72-6.69 (m, 2H), 5.91 (t, *J*=5.6 Hz, 1H), 4.80 (d, J=3.9 Hz, 1H), 4.77-4.70 (m, 1H), 4.65 (d, *J*=5.6 Hz, 2H), 4.57-4.46 (m, 1H), 4.16-4.07 (m, 2H), 3.86-3.79 (m, 1H), 3.15 (t, *J*=4.8 Hz, 4H), 3.04-2.95 (m, 1H), 2.09-2.84 (m, 1H), 2.81-2.72 (m, 2H), 2.63-2.54 (m, 5H), 2.45 (s, 3H), 2.28 (d, *J*=5.7 Hz, 2H), 1.91 (dt, *J*=12.9, 8.6 Hz, 2H), 1.84-1.76 (m, 2H), 1.52 (d, *J*=6.7 Hz, 6H). m/z 838.51 [M+H]⁺. |
| 94 | ¹H NMR (400 MHz, CDCl₃) δ 8.42 (d, *J*=5.2 Hz, 1H), 8.28 (s, 1H), 7.95 (s, 1H), 7.88-7.85 (m, 2H), 7.74 (s, 1H), 7.56 (d, *J*=8.9 Hz, 2H), 7.40 (d, *J*=7.9 Hz, 1H), 7.23 (t, *J*=7.9 Hz, 1H), 7.11 (s, 1H), 7.08 (d, *J*=5.3 Hz, 1H), 6.99-6.97 (m, 2H), 6.94 (d, *J*=9.0 Hz, 2H), 6.78 (dd, *J*=8.2, 1.5 Hz, 1H), 5.96 (t, *J*=5.4 Hz, 1H), 4.82 (d, *J*=3.9 Hz, 1H), 4.65 (d, *J*=5.5 Hz, 2H), 4.55-4.48 (m, 1H), 4.40 (t, *J*=8.3 Hz, 1H), 4.30 (t, *J*=9.4 Hz, 1H), 4.16-4.12 (m, 1H), 4.02-3.98 (m, 1H), 3.87 (dd, *J*=10.3, 5.5 Hz, 1H), 3.14 (t, *J*=5.4 Hz, 4H), 2.93-2.88 (m, 1H), 2.86-2.77 (m, 2H), 2.69 (d, *J*=7.4 Hz, 2H), 2.62-2.56 (m, 5H), 2.44 (s, 3H), 1.96-1.85 (m, 1H), 1.52 (d, *J*=6.7 Hz, 6H). m/z 810.50 [M+H]⁺. |
| 95 | ¹H NMR (400 MHz, CDCl₃) δ 8.41 (d, *J*=5.2 Hz, 1H), 8.04 (d, *J*=0.5 Hz, 1H), 7.91-7.84 (m, 2H), 7.75 (s, 1H), 7.55 (d, *J*=9.0 Hz, 2H), 7.40 (d, *J*=7.9 Hz, 1H), 7.22 (t, *J*=7.8 Hz, 1H), 7.08 (d, *J*=5.2 Hz, 2H), 6.95 (d, *J*=9.0 Hz, 2H), 6.78 (d, *J*=7.5 Hz, 1H), 6.73-6.65 (m, 2H), 5.91 (s, 1H), 4.80 (d, *J*=3.8 Hz, 1H), 4.66 (d, *J*=5.6 Hz, 3H), 4.11 (m, 1H), 3.80 (m, 1H), 3.19-3.12 (m, 4H), 2.96 (m, 1H), 2.90-2.68 (m, 3H), 2.59 (m, 5H), 2.45 (s, 3H), 2.28 (d, *J*=5.9 Hz, 2H), 1.85 (m, 6H), 1.60 (s, 9H). m/z 852.55 [M+H]⁺. |
| 96 | ¹H NMR (400 MHz, CDCl₃) δ 8.41 (d, *J*=5.2 Hz, 1H), 8.06 (m, 2H), 7.92-7.83 (m, 2H), 7.75 (s, 1H), 7.55 (d, *J*=9.4 Hz, 2H), 7.41 (d, *J*=7.9 Hz, 1H), 7.23 (t, *J*=7.8 Hz, 1H), 7.08 (m, 2H), 7.02-6.91 (m, 4H), 6.78 (dd, *J*=8.1, 2.3 Hz, 1H), 5.92 (s, 1H), 4.82 (d, *J*=3.8 Hz, 1H), 4.66 (d, *J*=5.6 Hz, 2H), 4.41 (t, *J*=8.7 Hz, 1H), 4.30 (t, *J*=9.1 Hz, 1H), 4.14 (m, 1H), 4.00 (m, 1H), 3.92-3.83 (m, 1H), 3.15 (m, 4H), 2.99-2.74 (m, 3H), 2.70 (d, *J*=7.4 Hz, 2H), 2.66-2.55 (m, 5H), 2.45 (s, 3H), 1.91 (m, 1H), 1.60 (s, 9H). m/z 824.46 [M+H]⁺. |
| 97 | ¹H NMR (400 MHz, CDCl₃) δ 8.41 (d, *J*=5.2 Hz, 1H), 8.27 (s, 1H), 8.19 (s, 1H), 7.86 (m, 2H), 7.56 (d, *J*=9.0 Hz, 2H), 7.43 (d, *J*=7.9 Hz, 2H), 7.22 (t, *J*=7.8 Hz, 1H), 7.15 (s, 1H), 7.08 (d, *J*=5.3 Hz, 1H), 6.98-6.92 (m, 2H), 6.78 (d, *J*=7.6 Hz, 1H), 6.73-6.67 (m, 2H), 4.81 (d, *J*=3.9 Hz, 1H), 4.70 (d, *J*=5.9 Hz, 3H), 4.11 (m, 1H), 3.80 (m, 1H), 3.22-3.09 (m, 4H), 2.97 (d, *J*=9.2 Hz, 1H), 2.89-2.67 (m, 3H), 2.65-2.52 (m, 5H), 2.46 (s, 3H), 2.28 (d, *J*=4.9 Hz, 2H), 1.98-1.75 (m, 6H), 1.71 (s, 9H). m/z 853.57 [M+H]⁺. |
| 98 | ¹H NMR(400 MHz, CDCl₃) δ 8.41 (d, *J*=5.2 Hz, 1H), 8.19 (s, 1H), 8.09 (s, 1H), 7.89-7.82 (m, 2H), 7.55 (d, *J*=10.1 Hz, 2H), 7.43 (d, *J*=8.0 Hz, 2H), 7.23 (t, *J*=7.8 Hz, 1H), 7.08 (m, 2H), 6.96-6.93 (m, 4H), 6.78 (dd, *J*=8.1, 2.4 Hz, 1H), 4.82 (d, *J*=3.9 Hz, 1H), 4.70 (d, *J*=5.9 Hz, 2H), 4.41 (t, *J*=8.4 Hz, 1H), 4.30 (t, *J*=9.2 Hz, 1H), 4.14 (m, 1H), 4.00 (m, 1H), 3.93-3.83 (m, 1H), 3.15 (m, 4H), 2.95-2.74 (m, 3H), 2.69 (d, *J*=7.4 Hz, 2H), 2.64-2.53 (m, 5H), 2.46 (s, 3H), 1.98-1.84 (m, 1H), 1.71 (s, 9H). m/z 825.53 [M+H]⁺. |
| 99 | ¹H NMR(400 MHz, CDCl₃) δ 8.52 (s, 1H), 8.44 (d, *J*=5.2 Hz, 1H), 7.87-7.85 (m, 2H), 7.63 (d, *J*=8.6 Hz, 2H), 7.37 (d, *J*=7.8 Hz, 1H), 7.30 (s, 1H), 7.24-7.20 (m, 3H), 7.11 (d, *J*=5.3 Hz, 1H), 6.78 (d, *J*=7.5 Hz, 1H), 6.72-6.70 (m, 2H), 4.82 (d, *J*=4.0 Hz, 1H), 4.75-4.68 (m, 1H), 4.45 (d, *J*=5.6 Hz, 2H), 4.38-4.32 (m, 1H), 4.27 (t, *J*=5.5 Hz, 1H), 4.15-4.08 (m, 3H), 3.88-3.78 (m, 3H), 3.64-3.58 (m, 1H), 2.98 (d, *J*=10.3 Hz, 2H), 2.86-2.71 (m, 4H), 2.60-2.54 (m, 1H), 2.51-2.45 (m, 1H), 2.42 (s, 3H), 2.24 (d, *J*=5.2 Hz, 2H), 2.08-2.01 (m, 3H), 1.93-1.75 (m, 8H), 1.16 (d, *J*=6.1 Hz, 6H). m/z 842.60 [M+H]_{*}. |
| 100 | ¹H NMR (400 MHz, CDCl₃) δ 8.42 (d, *J*=5.2 Hz, 1H), 8.01 (s, 1H), 7.87-7.85 (m, 2H), 7.63 (d, *J*=8.4 Hz, 2H), 7.38 (d, *J*=7.8 Hz, 1H), 7.34 (s, 1H), 7.23-7.20 (m, 3H), 7.11 (d, *J*=5.3 Hz, 1H), 7.00-6.98 (m, 2H), 6.79-6.77 (m, 1H), 4.82 (d, *J*=3.2 Hz, 1H), 4.45 (d, *J*=5.6 Hz, 2H), 4.42-4.39 (m, 1H), 4.37-4.33 (m, 1H), 4.31-4.29 (m, 1H), 4.23 (t, *J*=5.6 Hz, 1H), 4.18-4.12 (m, 3H), 4.04-3.99 (m, 1H), 3.87 (dd, *J*=8.5, 4.7 Hz, 3H), 3.65-3.56 (m, 1H), 3.02-2.90 (m, 3H), 2.87-2.79 (m, 2H), 2.75-2.65 (m, 2H), 2.63-2.56 (m, 1H), 2.54-2.46 (m, 1H), 2.43 (s, 3H), 1.97-1.87 (m, 6H), 1.16 (d, *J*=6.2 Hz, 6H). m/z 814.52 [M+H]⁺. |
| 101 | ¹H NMR (400 MHz, CDCl₃) δ 8.44 (d, *J*=5.2 Hz, 1H), 8.29 (s, 1H), 7.87 (m, 2H), 7.66 (d, J=8.5 Hz, 2H), 7.39 (d, *J*=8.5 Hz, 1H), 7.29 (d, *J*=8.5 Hz, 2H), 7.24-7.19 (m, 2H), 7.13 (d, *J*=5.3 Hz, 1H), 6.78 (d, *J*=7.5 Hz, 1H), 6.74-6.58 (m, 2H), 4.80 (d, *J*=4.0 Hz, 1H), 4.69 (s, 1H), 4.45 (d, *J*=5.7 Hz, 2H), 4.35 (m, 1H), 4.28 (s, 1H), 4.16-4.04 (m, 3H), 3.92-3.86 (m, 2H), 3.74 (m, 4H), 3.61 (m, 1H), 3.15 (s, 2H), 3.05-2.65 (m, 4H), 2.56 (dd, *J*=10.7, 5.5 Hz, 1H), 2.41 (d, *J*=7.7 Hz, 5H), 1.87 (m, 4H), 1.28 (m, 2H), 1.17 (d, *J*=6.2 Hz, 6H). m/z 814.56 [M+H]⁺. |
| 102 | ¹H NMR (400 MHz, CDCl₃) δ 8.41 (d, *J*=5.2 Hz, 1H), 8.10 (s, 1H), 7.84 (m, 2H), 7.56 (d, *J*=8.9 Hz, 2H), 7.38-7.30 (m, 2H), 7.13-7.03 (m, 5H), 6.95 (d, *J*=9.0 Hz, 2H), 4.91 (dd, *J*=7.6, 5.0 Hz, 1H), 4.72-4.69 (m, 1H), 4.44 (d, *J*=5.6 Hz, 2H), 4.39-4.32 (m, 1H), 4.24 (t, *J*=5.5 Hz, 1H), 4.16-4.09 (m, 2H), 3.86 (dd, *J*=8.4, 4.7 Hz, 2H), 3.78-3.76 (m, 1H), 3.60 (dt, *J*=12.3, 6.1 Hz, 1H), 3.15 (m, 4H), 3.04-2.90 (m, 2H), 2.73-2.59 (m, 2H), 2.59 (m, 4H), 2.41 (s, 3H), 2.38-2.25 (m, 4H), 1.95-1.80 (m, 3H), 1.28-1.24 (m, 2H), 1.15 (d, *J*=6.1 Hz, 6H). m/z 844.55 [M+H]⁺. |
| 103 | ¹H NMR (400 MHz, CDCl₃) δ 8.41 (d, *J*=5.2 Hz, 1H), 7.99 (s, 1H), 7.86-7.84 (m, 2H), 7.57 (d, *J*=8.9 Hz, 2H), 7.37 (d, *J*=7.8 Hz, 1H), 7.23 (d, *J*=7.8 Hz, 1H), 7.08 (d, *J*=5.2 Hz, 1H), 7.05 (s, 1H), 6.97 (d, *J*=9.0 Hz, 2H), 6.79 (d, *J*=7.5 Hz, 1H), 6.74-6.70 (m, 2H), 4.80 (d, *J*=3.9 Hz, 1H), 4.75 (brs, 1H), 4.45 (d, *J*=5.6 Hz, 2H), 4.38-4.33(m, 1H), 4.23 (m, 1H), 4.15-4.09 (m, 4H), 3.86 (dd, *J*=8.4, 4.6 Hz, 2H), 3.64-3.58 (m, 1H), 3.19 (t, *J*=4.4 Hz, 4H), 3.03 (brs, 1H), 2.91-2.80 (m, 2H), 2.77-2.73 (m, 5H), 2.60-2.52 (m, 2H), 2.42 (s, 3H), 2.03-1.84 (m, 3H), 1.50-1.42 (m, 1H), 1.16 (d, *J*=6.1 Hz, 6H). m/z 829.51 [M+H]⁺. |
| 104 | ¹H NMR (400 MHz, CDCl₃) δ 8.44 (d, *J*=5.2 Hz, 1H), 8.10 (s, 1H), 7.87 (s, 1H), 7.85 (dd, *J*=7.9, 1.5 Hz, 1H), 7.62 (d, *J*=8.5 Hz, 2H), 7.38 (d, *J*=7.9 Hz, 1H), 7.23 (d, *J*=8.2 Hz, 2H), 7.19 (s, 1H), 7.11 (d, *J*=5.2 Hz, 1H), 6.78 (dd, *J*=4.8, 3.7 Hz, 1H), 6.72 (dd, *J*=8.1, 1.8 Hz, 1H), 6.70 (d, *J*=1.3 Hz, 1H), 4.80 (m, 2H), 4.45 (d, *J*=5.6 Hz, 2H), 4.35 (m, 1H), 4.24 (m, 1H), 4.12 (m, 2H), 3.87 (dd, *J*=9.0, 4.7 Hz, 3H), 3.61 (m, 1H), 3.07 (m, 4H), 2.90-2.84 (m, 1H), 2.81-2.73 (m, 2H), 2.63-2.54 (m, 2H), 2.53-2.47 (m, 1H), 2.42 (s, 3H), 2.32 (m, 2H), 2.01 (m, 1H), 1.95-1.86 (m, 3H), 1.85-1.72 (m, 3H), 1.46 (m, 2H), 1.16 (d, *J*=6.1 Hz, 6H). m/z 828.40 [M+H]⁺. |
| 105 | ¹H NMR (400 MHz, CDCl₃) δ 9.19 (s, 1H), 8.41 (d, *J*=5.2 Hz, 1H), 7.90 (brs, 1H), 7.86-7.83 (m, 2H), 7.56 (d, *J*=9.0 Hz, 2H), 7.45 (s, 1H), 7.37 (d, *J*=7.6 Hz, 1H), 7.15 (t, *J*=8.0 Hz, 1H), 7.08 (d, *J*=5.3 Hz, 1H), 7.03 (s, 1H), 6.96 (d, *J*=9.0 Hz, 2H), 6.64 (dd, *J*=8.0, 1.2 Hz, 1H), 6.44 (dd, *J*=8.0, 2.4 Hz, 1H), 4.80 (d, *J*=4.0 Hz, 1H), 4.45 (d, *J*=5.5 Hz, 2H), 4.39-4.33 (m, 1H), 4.22 (t, *J*=4.4 Hz, 1H), 4.17-4.11 (m, 3H), 4.86 (dd, *J*=8.8, 4.7 Hz, 2H), 3.64-3.58 (m, 1H), 3.49 (d, *J*=5.1 Hz, 1H), 3.17 (t, *J*=5.0 Hz, 4H), 3.09 (s, 2H), 2.92 (d, *J*=11.5 Hz, 2H), 2.87-2.85 (m, 1H), 2.83-2.77 (m, 1H), 2.60 (t, *J*=4.8 Hz, 4H), 2.42 (s, 3H), 2.30-2.24 (m, 3H), 1.95-1.91 (m, 1H), 1.88-1.84 (m, 2H), 1.33-1.28 (m, 2H), 1.16 (d, *J*=6.1 Hz, 6H). m/z 872.59 [M+H]⁺. |
| 106 | ¹H NMR(400 MHz, CDCl₃) δ 9.18 (s, 1H), 8.43(d, *J*=5.0 Hz. 1H), 8.07 (s, 1H), 7.79 (dd, *J*=10.6, 2.7 Hz, 1H), 7.52-7.47 (m, 2H), 7.44 (t, *J*=2.0 Hz, 1H), 7.18-7.08 (m, 2H), 6.95-6.91 (m, 2H), 6.87 (dd, *J*=8.6, 2.7 Hz, 1H), 6.72-6.69 (m, 1H), 6.63 (dd, *J*=7.9, 1.2 Hz, 1H), 6.44 (dd, *J*=7.8, 2.0 Hz, 1H), 5.95 (s, 1H), 4.80 (d, *J*=3.8 Hz, 1H), 4.41 (tt, *J*=6.3, 4.6 Hz, 1H), 4.27-4.21 (m, 2H), 4.17-4.10 (m, 1H), 3.97 (dd, *J*=9.2, 4.6 Hz, 2H), 3.64 (dt, *J*=12.3, 6.1 Hz, 1H), 3.19-3.12 (m, 4H), 3.09 (s, 2H), 2.91 (d, *J*=11.7 Hz, 2H), 2.87-2.74 (m, 2H), 2.67-2.60 (m, 1H), 2.60-2.54 (m, 4H), 2.31-2.21 (m, 4H), 2.18 (s, 3H), 2.07-1.97 (m, 3H), 1.95-1.81 (m, 3H), 1.18 (d, *J*=6.1 Hz, 6H). m/z 876.49 [M+H]⁺. |
| 107 | ¹H NMR (400 MHz, CDCl₃) δ 8.42 (d, *J*=5.2 Hz, 1H), 8.25 (s, 1H), 7.86-7.84 (m, 2H), 7.82 (d, *J*=8.6 Hz, 1H), 7.58 (d, *J*=8.9 Hz, 2H), 7.37 (d, *J*=8.5 Hz, 1H), 7.16 (s, 1H), 7.08 (d, *J*=5.3 Hz, 1H), 6.96 (d, *J*=9.0 Hz, 2H) 6.25 (d, *J*=8.6 Hz, 1H), 5.19 (dd, *J*=13.3, 5.2 Hz, 1H), 4.45 (d, *J*=5.5 Hz, 2H), 4.37-4.33 (m, 1 H), 4.30-4.25 (m, 3H), 4.17-4.11 (m, 2H), 3.88-3.82 (m, 3H), 3.65-3.58 (m, 1H), 3.28-3.17 (m, 4H), 3.12-3.07 (m, 1H), 2.35-2.88 (m, 1H), 2.86-2.81 (m, 1H), 2.79-2.77 (m, 1H), 2.72-2.63 (m, 4H), 2.43 (s, 3H), 2.38-2.27 (m, 1H), 2.24-2.17 (m, 1H), 1.55-1.52 (m, 2H), 1.44 (d, J=6.7 Hz, 1H), 1.16 (d, J=6.2 Hz, 6H). m/z 414.99 [M+H]²⁺. |
| 108 | ¹H NMR (400 MHz, CDCl₃) δ 8.43 (d, *J*=5.0 Hz, 1H), 8.11 (s, 1H), 7.83-7.77 (m, 2H), 7.51 (d, *J*=9.0 Hz, 2H), 7.12 (s, 1H), 6.93 (d, *J*=9.0 Hz, 2H), 6.87 (dd, *J*=8.6, 2.7 Hz, 1H), 6.71 (d, *J*=5.0 Hz, 1H), 6.24 (d, *J*=8.6 Hz, 1H), 5.95 (s, 1H), 5.18 (dd, *J*=13.2, 5.2 Hz, 1H), 4.44-4.37 (m, 1H), 4.35-4.14 (m, 6H), 3.97 (dd, *J*=8.6, 4.5 Hz, 2H), 3.85-3.80 (m, 2H), 3.67-3.61 (m, 1H), 3.15 (s, 4H), 3.06-3.02 (m, 1H), 2.94-2.72 (m, 4H), 2.63 (m, 4H), 2.37-2.26 (m, 1H), 2.20 (m, 2H), 2.18 (s, 3H), 1.18 (d, *J*=6.1 Hz, 6H). m/z 832.48 [M+H]⁺. |
| 109 | ¹H NMR (400 MHz, CDCl₃) δ 8.41 (d, *J*=5.1 Hz, 1H), 8.06 (s, 1H), 7.86-7.84 (m, 2H), 7.69 (d, *J*=8.5 Hz, 1H), 7.57 (d, *J*=8.8 Hz, 2H), 7.37 (d, *J*=7.7 Hz, 1H), 7.30 (s, 1H), 7.08-7.06 (m, 3H), 6.97 (d, *J*=8.8 Hz, 2H), 4.94 (dd, *J*=12.2, 5.3 Hz, 1H), 4.45 (d, *J*=5.5 Hz, 2H), 4.38-4.33 (m, 1H), 4.23 (d, *J*=5.4 Hz, 1H), 4.13 (t, *J*=7.2 Hz, 2H), 4.02 (d, *J*=12.7 Hz, 2H), 3.86 (dd, *J*=8.1, 4.6 Hz, 2H), 3.64-3.58 (m, 1H), 3.19 (t, *J*=4.2 Hz, 4H), 3.03 (t, *J*=11.8 Hz, 2H), 2.93-2.85 (m, 2H), 2.77 (t, *J*=8.4 Hz, 4H), 2.74-2.69 (m, 1H), 2.61-2.54 (m, 1H), 2.42 (s, 3H), 2.18-2.10 (m, 1H), 2.03 (d, *J*=14.7 Hz, 2H), 1.72-1.63 (m, 2H), 1.16 (d, *J*=6.1 Hz, 6H). m/z 855.55 [M+H]⁺. |
| 110 | ¹H NMR (400 MHz, CDCl₃) δ 8.44 (d, *J*=5.2 Hz, 1H), 7.98 (s, 1H), 7.87 (s, 1H), 7.85 (d, *J*=7.9 Hz, 1H), 7.69 (d, *J*=8.5 Hz, 1H), 7.63 (d, *J*=8.2 Hz, 2H), 7.38 (d, *J*=7.9 Hz, 1H), 7.30 (d, *J*=2.3 Hz, 1H), 7.23 (d, *J*=8.5 Hz, 2H), 7.19 (s, 1H), 7.12 (d, *J*=5.2 Hz, 1H), 7.09-7.05 (m, 1H), 4.94 (m, 1H), 4.45 (d, *J*=5.6 Hz, 2H), 4.35 (m, 1H), 4.26-4.18 (m, 1H), 4.16-4.09 (m, 2H), 4.04 (d, *J*=12.0 Hz, 2H), 3.87 (dd, *J*=9.0, 4.7 Hz, 2H), 3.61 (m, 1H), 3.09 (s, 2H), 3.04-2.98 (m, 2H), 2.94-2.87 (m, 2H), 2.85-2.79 (m, 2H), 2.73 (m, 2H), 2.62 (m, 2H), 2.53 (m, 2H), 2.43 (s, 3H), 2.35 (d, *J*=7.6 Hz, 1H), 2.16-2.12 (m, 1H), 2.01 (m, 2H), 1.91 (m, 2H), 1.21-1.11 (m, 6H). m/z 854.75 [M+H]⁺. |
| 111 | ¹H NMR (400 MHz, DMSO-*d₆*) δ 11.09 (s, 1H), 9.36 (s, 1H), 8.45 (d, *J*=5.2 Hz, 1H), 7.93 (d, *J*=4.4 Hz, 2H), 7.66 (m, 3H), 7.34 (d, *J*=8.5 Hz, 2H), 7.27 (m, 2H), 6.92 (d, *J*=9.1 Hz, 2H), 6.86 (t, *J*=5.8 Hz, 1H), 5.07 (m, 1H), 4.33 (t, *J*=5.5 Hz, 1H), 4.23 (d, *J*=5.6 Hz, 2H), 4.08-4.00 (m, 2H), 3.66-3.55 (m, 6H), 3.45 (s, 5H), 2.87 (m, 1H), 2.70-2.64 (m, 5H), 2.61 (d, *J*=1.7 Hz, 3H), 2.36 (s, 3H), 2.24 (d, *J*=6.9 Hz, 2H), 2.02 (m, 1H), 1.83 (d, *J*=11.6 Hz, 2H), 1.68 (s, 1H), 1.08 (d, *J*=6.1 Hz, 6H). m/z 869.58 [M+H]⁺. |
| 112 | ¹H NMR (400 MHz, CDCl₃) δ 8.39 (d, *J*=5.2 Hz, 1H), 8.06 (s, 1H), 7.85-7.83 (m, 2H), 7.49 (d, *J*=8.7 Hz, 2H), 7.36 (d, *J*=7.9 Hz, 1H), 7.30 (d, *J*=2.2 Hz, 1H), 7.08-7.04 (m, 2H), 7.00 (s, 1H), 6.50 (d, *J*=8.8 Hz, 2H), 4.94 (dd, *J*=12.4, 5.3 Hz, 1H), 4.45 (d, *J*=5.5 Hz, 2H), 4.38-4.33 (m, 1H), 4.22 (t, *J*=5.3 Hz, 1H), 4.13 (t, *J*=7.4 Hz, 2H), 4.04 (t, *J*=7.3 Hz, 2H), 3.86 (dd, *J*=8.4, 4.6 Hz, 2H), 3.64-3.58 (m, 1H), 3.57 (t, *J*=6.2 Hz, 2H), 3.44 (t, *J*=5.0 Hz, 4H), 3.02-2.95 (m, 1H), 2.92-2.88 (m, 1H), 2.85-2.81 (m, 1H), 2.79-2.76 (m, 1H), 2.74-2.70 (m, 2H), 2.62 (t, *J*=5.0 Hz, 4H), 2.43 (s, 3H), 2.16-2.10 (m, 1H), 1.16 (d, *J*=6.1 Hz, 6H). m/z 841.46 [M+H]⁺. |
| 113 | ¹H NMR (400 MHz, CDCl₃) δ 8.39 (d, *J*=5.2 Hz, 1H), 8.22 (s, 1H), 7.85-7.83 (m, 2H), 7.48 (d, *J*=8.7 Hz, 2H), 7.36 (d, *J*=7.8 Hz, 1H), 7.23 (t, *J*=7.7 Hz, 1H), 7.05-7.03 (m, 2H), 6.78 (d, *J*=7.5 Hz, 1H), 6.73-6.71 (m, 2H), 6.48 (d, *J*=8.8 Hz, 2H), 4.81 (d, *J*=4.0 Hz, 1H), 4.44 (d, *J*=5.6 Hz, 2H), 4.38-4.32 (m, 1H), 4.23 (t, *J*=5.4 Hz, 1H), 4.15-4.09 (m, 3H), 4.01 (t, *J*=7.3 Hz, 2H), 3.86 (dd, J=8.5, 4.5 Hz, 2H), 3.79 (m, 1H), 3.64-3.58 (m, 1H), 3.54 (t, *J*=6.3 Hz, 2H), 3.47-3.42 (m, 2H), 2.98-2.93 (m, 1H), 2.91-2.85 (m, 1H), 2.81-2.75 (m, 1H), 2.68 (d, *J*=7.3 Hz, 2H), 2.60-2.50 (m, 3H), 2.38 (s, 3H), 2.40-2.36 (m, 2H), 1.91 (qd, *J*=13.3, 4.8 Hz, 1H), 1.16 (d, *J*=4.8 Hz, 6H). m/z 815.49 [M+H]⁺. |
| 114 | ¹H NMR (400 MHz, CDCl₃) δ 8.41 (d, *J*=5.2 Hz, 1H), 8.11 (s, 1H), 7.85 (m, 2H), 7.55 (d, *J*=8.9 Hz, 2H), 7.36 (d, *J*=8.0 Hz, 1H), 7.23 (t, *J*=7.8 Hz, 1H), 7.11-7.05 (m, 2H), 6.98 (d, *J*=8.8 Hz, 2H), 6.79 (d, *J*=7.5 Hz, 1H), 6.72 (m, 2H), 4.80 (d, *J*=4.0 Hz, 1H), 4.45 (d, *J*=5.6 Hz, 2H), 4.35 (m, 1H), 4.23 (t, *J*=5.5 Hz, 1H), 4.17-4.05 (m, 3H), 3.86 (dd, *J*=8.5, 4.5 Hz, 2H), 3.83-3.70 (m, 2H), 3.61 (m, 3H), 3.46 (m, 2H), 2.88 (m, 1H), 2.81-2.74 (m, 1H), 2.69 (m, 2H), 2.59-2.45 (m, 3H), 2.42 (s, 3H), 2.37 (s, 2H), 2.27 (d, *J*=6.9 Hz, 2H), 1.97-1.85 (m, 3H), 1.38 (dd, *J*=25.1, 14.7 Hz, 3H), 1.16 (d, *J*=6.1 Hz, 6H). m/z 843.37 [M+H]⁺. |
| 115 | ¹H NMR (400 MHz, CDCl₃) δ 8.41 (d, *J*=5.2 Hz, 1H), 7.91 (s, 1H), 7.86-7.83 (m, 2H), 7.55 (d, *J*=8.9 Hz, 2H), 7.36 (d, *J*=7.8 Hz, 1H), 7.16 (t, *J*=7.8 Hz, 1H), 7.07 (d, *J*=5.3 Hz, 1H), 7.03 (s, 1H), 6.95 (d, *J*=9.0 Hz, 2H), 6.78 (dd, *J*=15.5, 7.8 Hz, 1H), 6.70 (s, 1H), 6.63-6.55 (m, 1H), 4.70 (d, *J*=3.9 Hz, 1H), 4.65 (s, 1H), 4.45 (d, *J*=5.6 Hz, 2H), 4.38-4.33 (m, 1H), 4.21 (t, *J*=5.2 Hz, 1H), 4.15 (m, 4H), 3.86 (dd, *J*=8.7, 4.7 Hz, 2H), 3.64-3.58 (m, 1H), 3.45 (s, 2H), 3.15 (t, *J*=4.8 Hz, 4H), 2.92-2.73 (m, 5H), 2.62-2.56 (m, 5H), 2.42(s, 3H), 2.24 (d, *J*=7.0 Hz, 2H), 2.02-1.90 (m, 3H), 1.75 (d, *J*=12.2 Hz, 2H), 1.16 (d, *J*=6.1 Hz, 6H). m/z 829.60 [M+H]⁺. |
| 116 | ¹H NMR (400 MHz, CDCl₃) δ 8.41 (m, 1H), 8.12 (s, 1H), 7.85 (m, 2H), 7.56 (d, *J*=8.7 Hz, 2H), 7.37 (d, *J*=8.0 Hz, 1H), 7.19 (t, *J*=7.6 Hz, 1H), 7.12 (s, 1H), 7.08 (d, *J*=5.2 Hz, 1H), 6.99 (d, *J*=8.8 Hz, 2H), 6.67 (d, *J*=7.6 Hz, 1H), 6.53 (m, 2H), 4.84 (d, *J*=13.2 Hz, 1H), 4.73 (d, *J*=3.5 Hz, 1H), 4.45 (d, *J*=5.5 Hz, 2H), 4.35 (m, 1H), 4.23 (t, *J*=5.4 Hz, 1H), 4.15-4.02 (m, 4H), 3.87 (dd, *J*=8.2, 4.7 Hz, 2H), 3.71 (d, *J*=11.3 Hz, 2H), 3.61 (m, 1H), 3.17 (t, *J*=12.9 Hz, 1H), 2.90 (d, *J*=17.5 Hz, 1H), 2.77 (m, 4H), 2.67-2.53 (m, 3H), 2.42 (s, 3H), 2.10-2.01 (m, 2H), 1.91 (m, 5H), 1.70-1.60 (m, 2H), 1.16 (dd, *J*=6.1, 0.7 Hz, 6H). m/z 828.50 [M+H]⁺. |
| 117 | ¹H NMR (400 MHz, CDCl₃) δ 8.42 (d, *J*=5.2 Hz, 1H), 8.19 (s, 1H), 7.85 (m, 2H), 7.56 (d, *J*=8.8 Hz, 2H), 7.37 (d, *J*=8.2 Hz, 1H), 7.14 (s, 1H), 7.08 (dd, *J*=6.2, 0.6 Hz, 3H), 6.98 (d, *J*=8.9 Hz, 2H), 6.66 (d, *J*=8.4 Hz, 2H), 4.82 (d, *J*=12.8 Hz, 1H), 4.69 (d, *J*=3.5 Hz, 1H), 4.45 (d, *J*=5.5 Hz, 2H), 4.39-4.32 (m, 1H), 4.24 (t, *J*=5.5 Hz, 1H), 4.17-4.10 (m, 2H), 4.06 (m, 2H), 3.87 (dd, *J*=8.4, 4.7 Hz, 2H), 3.70 (d, *J*=11.5 Hz, 2H), 3.60 (m, 1H), 3.16 (t, J=12.2 Hz, 1H), 2.92-2.83 (m, 1H), 2.82-2.51 (m, 7H), 2.42 (s, 3H), 2.11-1.98 (m, 2H), 1.91 (m, 5H), 1.57 (d, *J*=3.9 Hz, 1H), 1.47 (d, *J*=17.5 Hz, 1H), 1.18-1.13 (m, 6H). m/z 828.50 [M+H]⁺. |
| 118 | ¹H NMR (400 MHz, CDCl₃) δ 8.41 (d, *J*=5.2 Hz, 1H), 7.89 (s, 1H), 7.86-7.84 (m, 2H), 7.56 (d, *J*=8.9 Hz, 2H), 7.37 (d, *J*=8.2 Hz, 1H), 7.15 (d, *J*=8.7 Hz, 2H), 7.07 (d, *J*=5.7 Hz, 1H), 7.05 (s, 1H), 7.01-6.97 (m, 4H), 4.86 (dd, *J*=7.7, 4.4 Hz, 1H), 4.81 (s, 1H), 4.45 (d, *J*=5.6 Hz, 2H), 4.18-4.33 (m, 1H), 4.22 (t, *J*=5.5 Hz, 1H), 4.15-4.11 (m, 2H), 4.10-4.05 (m, 1H), 3.86 (dd, *J*=8.7, 4.7 Hz, 2H), 3.70 (d, *J*=11.3 Hz, 2H), 3.64-3.58 (m, 1H), 3.17 (t, *J*=12.9 Hz, 1H), 3.00-2.92 (m, 1H), 2.77-2.61 (m, 6H), 2.42 (s, 3H), 2.37-2.26 (m, 2H), 2.09-2.01 (m, 2H), 1.96-1.84 (m, 4H), 1.67-1.61 (m, 2H), 1.16 (d, *J*=6.1 Hz, 6H). m/z 829.47 [M+H]⁺. |
| 119 | ¹H NMR (400 MHz, CDCl₃) δ 8.40 (d, *J*=5.2 Hz, 1H), 7.86-7.82 (m, 3H), 7.55 (d, *J*=8.9 Hz, 2H), 7.37 (d, *J*=8.0 Hz, 1H), 7.18 (d, *J*=8.6 Hz, 2H), 7.13 (s, 1H), 7.07 (d, *J*=5.2 Hz, 1H), 6.99 (d, *J*=8.8 Hz, 4H), 4.85 (dd, *J*=7.6, 4.4 Hz, 1H), 4.45 (d, *J*=5.6 Hz, 2H), 4.38-4.33 (m, 1H), 4.22 (t, *J*=5.4 Hz, 1H), 4.13 (t, *J*=7.6 Hz, 2H), 3.87 (dd, *J*=8.8, 4.7 Hz, 2H), 6.37-3.58 (m, 3H), 3.05-3.00 (m, 1H), 2.98-2.92 (m, 1H), 2.71-2.63 (m, 3H), 2.54-2.46 (m, 1H), 2.42 (s, 3H), 2.34-2.26 (m, 4H), 2.07-2.20 (m, 2H), 1.91 (d, *J*=12.6 Hz, 3H), 1.84-1.76 (m, 5H), 1.47-1.38 (m, 2H), 1.16 (d, *J*=6.1 Hz, 6H). m/z 815.47 [M+H]⁺. |
| 120 | ¹H NMR (400 MHz, CDCl₃) δ 8.41 (d, *J*=5.2 Hz, 1H), 7.90 (s, 1H), 7.86-7.84 (m, 2H), 7.55 (d, *J*=8.9 Hz, 2H), 7.37 (d, *J*=7.7 Hz, 1H), 7.12 (t, *J*=8.1 Hz, 1H), 7.07 (d, *J*=5.2 Hz, 1H), 7.03 (s, 1H), 6.99 (d, *J*=8.9 Hz, 2H), 6.44 (dd, *J*=8.2, 2.0 Hz, 1H), 6.27 (t, *J*=2.0 Hz, 1H), 6.18 (dd, *J*=8.2, 2.0 Hz, 1H), 4.68 (d, *J*=3.6 Hz, 1H), 4.45 (d, *J*=5.6 Hz, 2H), 4.38-4.33 (m, 1H), 4.22 (t, *J*=5.4 Hz, 1H), 4.15-4.07 (m, 3H), 3.87 (dd, *J*=8.7, 4.7 Hz, 2H), 3.66-3.58 (m, 3H), 3.19 (t, *J*=4.8 Hz, 4H), 2.91-2.85 (m, 1H), 2.80-2.75 (m, 1H), 2.69 (td, *J*=12.1, 2.3 Hz, 2H), 2.58 (t, *J*=4.8 Hz, 4H), 2.42 (s, 3H), 2.29 (d, 7.1 Hz, 2H), 1.97-1.86 (m, 3H), 1.74-1.63 (m, 1H), 1.45-1.36 (m, 2H), 1.16 (d, *J*=6.1 Hz, 6H). m/z 815.50 [M+H]⁺. |
| 121 | ¹H NMR (400 MHz, CDCl₃) δ 8.41 (d, *J*=5.2 Hz, 1H), 7.97 (s, 1H), 7.86-7.84 (m, 2H), 7.55 (d, J=9.0 Hz, 2H), 7.37 (d, *J*=7.9 Hz, 1H), 7.09 (s, 1H), 7.07 (d, *J*=5.2 Hz, 1H), 6.99 (d, *J*=9.1 Hz, 2H), 6.89 (d, *J*=8.9 Hz, 2H), 6.68 (d, *J*=8.9 Hz, 2H), 4.45 (d, *J*=5.6 Hz, 2H), 4.37-4.32 (m, 1H), 4.22 (t, *J*=5.6 Hz, 1H), 4.13 (t, *J*=7.6 Hz, 2H), 4.01 (dd, *J*=12.3, 4.8 Hz, 1H), 3.87 (dd, *J*=8.7, 4.7 Hz, 2H), 3.65-3.57 (m, 3H), 3.09 (t, *J*=4.6 Hz, 4H), 2.89-2.83 (m, 1H), 2.79-2.73 (m, 1H), 2.71-2.66 (m, 2H), 2.60 (t, *J*=4.2 Hz, 4H), 2.56-2.52 (m, 1H), 2.42 (s, 3H), 2.30 (d, *J*=7.1 Hz, 2H), 1.97-1.86 (m, 3H), 1.45-1.35 (m, 3H), 1.16 (d, *J*=6.1 Hz, 6H). m/z 815.49 [M+H]⁺. |
| 122 | ¹H NMR(400 MHz, CDCl₃) δ 8.41 (d, *J*=5.2 Hz, 1H), 7.89 (brs, 1H), 7.86-7.84 (m, 2H), 7.55(d, *J*=8.8 Hz, 2H), 7.37 (d, *J*=8.0 Hz, 1H), 7.16 (t, *J*=7.9 Hz, 1H), 7.08 (d, *J*=5.2 Hz, 1H), 7.02 (s, 1H), 6.98 (d, *J*=8.4 Hz, 2H), 6.73-6.51 (m, 3H), 4.72 (d, *J*=3.0 Hz, 1H), 4.45 (d, *J*=5.6 Hz, 2H), 4.39-4.33 (m, 1H), 4.22 (t, *J*=5.6 Hz, 1H), 4.15-4.05 (m, 3H), 3.87 (dd, *J*=8.7, 4.7 Hz, 2H), 3.82-3.73 (m, 1H), 3.66-3.58 (m, 3H), 3.50 (d, *J*=5.0 Hz, 1H), 3.06-2.84 (m, 4H), 2.70 (t, *J*=12.1 Hz, 3H), 2.62-2.54 (m, 2H), 2.42 (s, 3H), 2.28-2.23 (m, 1H), 2.05-1.75 (m, 9H), 1.16 (d, *J*=6.1 Hz, 6H). m/z 814.51 [M+H]⁻. |
| 123 | ¹H NMR (400 MHz, CDCl₃) δ 8.41 (d, *J*=5.2 Hz, 1H), 7.86-7.84 (m, 3H), 7.55 (d, *J*=8.8 Hz, 2H), 7.37 (d, *J*=7.5 Hz, 1H), 7.11 (d, *J*=8.3 Hz, 2H), 7.07 (d, *J*=5.2 Hz, 1H), 7.01-6.97 (m, 3H), 6.65 (d, *J*=8.5 Hz, 2H), 4.67 (d, *J*=2.3 Hz, 1H), 4.45 (d, *J*=5.5 Hz, 2H), 4.39-4.33 (m, 1H), 4.22 (t, *J*=5.6 Hz, 1H), 4.13 (t, *J*=7.6 Hz, 2H), 4.09-4.03 (m, 1H), 3.87 (dd, *J*=8.5, 4.6 Hz, 2H), 3.66-3.58 (m, 4H), 3.02-2.98 (m, 2H), 2.92-2.85 (m, 1H), 2.81-2.75 (m, 1H), 2.72-2.66 (m, 3H), 2.61-2.54 (m, 1H), 2.42 (s, 3H), 2.28-2.23 (m, 2H), 2.05-1.97 (m, 3H), 1.93-1.87 (m, 3H), 1.81-1.71 (m, 4H), 1.16 (d, *J*=6.1 Hz, 6H). m/z 814.50 [M+H]⁺. |
| 124 | ¹H NMR (400 MHz, CDCl₃) δ 8.41 (d, *J*=5.2 Hz, 1H), 8.34 (d, *J*=2.9 Hz, 1H), 7.96 (s, 1H), 7.86-7.84 (m, 2H), 7.55 (d, *J*=8.9 Hz, 2H), 7.37 (d, *J*=8.3 Hz, 2H), 7.17 (d, *J*=8.9 Hz, 1H), 7.08-7.06 (m, 2H), 6.98 (d, *J*=8.7 Hz, 2H), 4.85 (dd, *J*=7.2, 5.4 Hz, 1H), 4.45 (d, *J*=5.6 Hz, 2H), 4.39-4.33 (m, 1H), 4.22 (t, *J*=5.1 Hz, 1H), 4.13 (t, *J*=7.6 Hz, 2H), 3.87 (dd, *J*=8.7, 4.7 Hz, 2H), 3.88-5.58 (m, 4H), 3.03-2.94 (m, 3H), 2.75-2.67 (m, 4H), 2.42 (s, 3H), 3.39-2.33 (m, 2H), 2.30-2.23 (m, 1H), 2.11-1.82 (m, 8H), 1.48-1.36 (m, 3H), 1.16 (d, *J*=6.1 Hz, 6H). m/z 816.44 [M+H]⁺. |
| 125 | ¹H NMR (400 MHz, CDCl₃) δ 8.41 (d, *J*=5.2 Hz, 1H), 8.04 (d, *J*=3.0 Hz, 1H), 7.93 (s, 1H), 7.85 (d, *J*=7.4 Hz, 2H), 7.55 (d, *J*=9.0 Hz, 2H), 7.37 (d, *J*=7.9 Hz, 1H), 7.31 (dd, *J*=9.0, 2.9 Hz, 1H), 7.07 (d, J=5.2 Hz, 2H), 6.99 (d, *J*=9.1 Hz, 2H), 6.63 (d, *J*=9.2 Hz, 1H), 4.65 (dd, *J*=7.6, 4.6 Hz, 1H), 4.45 (d, *J*=5.6 Hz, 2H), 4.38-4.33 (m, 1H), 4.22 (t, *J*=5.5 Hz, 1H), 4.13 (t, *J*=7.6 Hz, 2H), 3.87 (dd, *J*=8.8, 4.7 Hz, 2H), 3.66-3.58 (m, 3H), 3.47 (t, *J*=4.6 Hz, 4H), 2.99-2.91 (m, 1H), 2.72-2.63 (m, 3H), 2.55-2.53 (m, 4H), 2.42 (s, 3H), 2.36-2.28 (4H), 1.91 (d, *J*=12.6 Hz, 2H), 1.75-1.66 (m, 1H), 1.45-1.36 (m, 2H), 1.16 (d, *J*=6.1 Hz, 6H). m/z 817.47 [M+H]⁺. |
| 126 | ¹H NMR (400 MHz, CDCl₃) δ 8.41 (d, *J*=5.2 Hz, 1H), 8.05 (d, *J*=2.8 Hz, 2H), 7.85 (m, 2H), 7.55 (d, *J*=8.9 Hz, 2H), 7.37 (d, *J*=7.7 Hz, 1H), 7.07 (m, 3H), 6.97 (dd, *J*=11.1, 7.5 Hz, 3H), 4.66 (d, *J*=3.7 Hz, 1H), 4.45 (d, *J*=5.5 Hz, 2H), 4.36 (m, 1H), 4.23 (m, 1H), 4.17-4.04 (m, 3H), 3.87 (dd, *J*=8.6, 4.7 Hz, 2H), 3.71-3.56 (m, 3H), 3.02 (d, *J*=10.7 Hz, 2H), 2.89 (m, 1H), 2.70 (m, 6H), 2.42 (s, 3H), 2.26 (d, *J*=6.7 Hz, 2H), 2.05 (t, *J*=10.8 Hz, 2H), 1.93 (dd, *J*=21.3, 8.4 Hz, 5H), 1.80 (d, *J*=12.1 Hz, 2H), 1.40 (d, *J*=12.6 Hz, 2H), 1.19-1.11 (m, 6H). m/z 815.49 [M+H]⁺. |
| 127 | ¹H NMR (400 MHz, CDCl₃) δ 8.41 (d, *J*=5.2 Hz, 1H), 7.90 (s, 1H), 7.85 (m, 2H), 7.81 (d, *J*=2.9 Hz, 1H), 7.55 (d, *J*=8.9 Hz, 2H), 7.37 (d, *J*=7.7 Hz, 1H), 7.06 (m, 3H), 6.99 (d, *J*=8.8 Hz, 2H), 6.65 (d, *J*=9.0 Hz, 1H), 4.45 (d, *J*=5.6 Hz, 2H), 4.39-4.30 (m, 2H), 4.22 (m, 1H), 4.17-4.08 (m, 2H), 3.97 (d, *J*=9.7 Hz, 1H), 3.87 (dd, *J*=8.6, 4.7 Hz, 2H), 3.68-3.58 (m, 3H), 3.42 (d, *J*=5.0 Hz, 4H), 2.91-2.82 (m, 1H), 2.69 (dd, *J*=14.0, 9.2 Hz, 3H), 2.59-2.48 (m, 5H), 2.42 (s, 3H), 2.29 (d, *J*=7.1 Hz, 2H), 1.97-1.86 (m, 3H), 1.69 (s, 1H), 1.40 (d, *J*=14.1 Hz, 2H), 1.16 (d, *J*=6.1 Hz, 6H). m/z 816.49 [M+H]⁺. |
| 128 | ¹H NMR (400 MHz, CDCl₃) δ 8.40 (d, *J*=5.2 Hz, 1H), 8.04 (d, *J*=2.8 Hz, 1H), 7.93 (s, 1H), 7.91 (brs, 1H), 7.88 (s, 1H), 7.86 (d, *J*=8.0 Hz, 1H), 7.72 (s, 1H), 7.53 (d, *J*=8.9 Hz, 2H), 7.39 (d, *J*=7.8 Hz, 1H), 7.07-7.04 (m, 2H), 7.02 (s, 1H), 6.99-4.93 (m, 3H), 5.86 (t, *J*=5.0 Hz, 1H), 4.65 (d, *J*=5.6 Hz, 2H), 4.54-4.47 (m, 1H), 4.09-4.04 (m, 1H), 3.62 (d, *J*=12.0 Hz, 2H), 3.03-2.98 (m, 2H), 2.92-2.86 (m, 1H), 2.79-2.74 (m, 1H), 2.70-2.63 (m, 3H), 2.58-2.52 (m, 1H), 2.44 (s, 3H), 2.27-2.22 (m, 2H), 2.09-2.01 (m, 2H), 1.95-1.85 (m, 6H), 1.82-1.75 (m, 1H), 1.69-1.64 (m, 1H), 1.51 (d, *J*=6.7 Hz, 6H), 1.42-1.32 (m, 2H). m/z 810.47 [M+H]⁺. |
| 129 | ¹H NMR (400 MHz, CDCl₃) δ 8.41 (d, *J*=5.2 Hz, 1H), 8.16 (brs, 1H), 7.95 (s, 1H), 7.89 (s, 1H), 7.87 (d, *J*=8.0 Hz, 1H), 7.81 (d, *J*=2.9 Hz, 1H), 7.74 (s, 1H), 7.56 (d, *J*=8.9 Hz, 2H), 7.40 (d, *J*=7.9 Hz, 1H), 7.18 (s, 1H), 7.08-7.04 (m, 2H), 6.98 (d, *J*=9.0 Hz, 2H), 6.64 (d, *J*=9.0 Hz, 1H), 5.92 (t, *J*=5.5 Hz, 1H), 4.66 (d, *J*=5.5 Hz, 2H), 4.55-4.48 (m, 1H), 3.96 (dd, *J*=12.3, 4.8 Hz, 1H), 3.64 (d, *J*=12.1 Hz, 2H), 3.47-3.39 (m, 4H), 2.90-2.83 (m, 1H), 2.74-2.66 (m, 3H), 2.57-2.50 (m, 5H), 2.45 (s, 3H), 2.35-2.29 (m, 2H), 1.94-1.90 (m, 3H), 1.53 (d, *J*=6.7 Hz, 6H), 1.46-1.36 (m, 3H). m/z 811.49 [M+H]⁺. |
| 130 | ¹H NMR (400 MHz, DMSO-*d₆*) δ 10.82 (s, 1H), 9.38 (s, 1H), 8.53-8.41 (m, 2H), 8.33 (s, 1H), 7.98-7.90 (m, 4H), 7.64 (d, *J*=9.0 Hz, 2H), 7.37 (d, *J*=8.0 Hz, 1H), 7.29 (d, *J*=5.2 Hz, 1H), 6.98 (s, 2H), 6.91 (d, *J*=9.1 Hz, 2H), 5.94 (d, *J*=7.9 Hz, 1H), 4.46 (d, *J*=5.6 Hz, 2H), 4.34 (m, 1H), 3.59 (d, *J*=11.5 Hz, 2H), 2.93 (d, *J*=11.0 Hz, 2H), 2.80-2.64 (m, 2H), 2.58 (m, 4H), 2.41 (s, 3H), 2.18 (d, *J*=7.2 Hz, 2H), 2.10 (m, 1H), 1.99-1.87 (m, 2H), 1.84-1.63 (m, 7H), 1.53 (s, 9H), 1.23 (d, *J*=7.8 Hz, 2H). m/z 824.53 [M+H]⁺. |
| 131 | ¹H NMR (400 MHz, CDCl₃) δ 8.42 (d, *J*=5.2 Hz, 1H), 8.06 (m, 2H), 7.92-7.85 (m, 2H), 7.81 (d, *J*=2.9 Hz, 1H), 7.75 (s, 1H), 7.55 (d, *J*=8.9 Hz, 2H), 7.41 (d, *J*=7.9 Hz, 1H), 7.12-7.04 (m, 3H), 6.98 (d, *J*=9.0 Hz, 2H), 6.64 (d, *J*=8.9 Hz, 1H), 5.90 (t, *J*=5.4 Hz, 1H), 4.66 (d, *J*=5.6 Hz, 2H), 4.32 (d, *J*=3.0 Hz, 1H), 4.02-3.91 (m, 1H), 3.64 (d, *J*=12.1 Hz, 2H), 3.44-3.36 (m, 4H), 2.86 (dt, *J*=7.1, 3.9 Hz, 1H), 2.70 (m, 3H), 2.58-2.48 (m, 5H), 2.45 (s, 3H), 2.28 (d, *J*=7.1 Hz, 2H), 1.95-1.85 (m, 3H), 1.60 (s, 9H), 1.40 (m, 2H). m/z 825.51 [M+H]⁺. |
| 132 | ¹H NMR (400 MHz, CDCl₃) δ 8.41 (d, *J*=5.2 Hz, 1H), 8.19 (s, 1H), 8.07-7.98 (m, 2H), 7.90-7.84 (m, 2H), 7.55 (d, *J*=8.8 Hz, 2H), 7.43 (d, *J*=7.9 Hz, 2H), 7.07 (m, 3H), 6.97 (m, 3H), 4.71 (d, *J*=5.9 Hz, 2H), 4.65 (d, *J*=3.8 Hz, 1H), 4.08 (m, 1H), 3.63 (d, *J*=12.0 Hz, 2H), 3.01 (d, *J*=10.6 Hz, 2H), 2.90 (d, *J*=17.2 Hz, 1H), 2.81-2.50 (m, 5H), 2.46 (s, 3H), 2.26 (d, *J*=6.6 Hz, 2H), 2.05 (t, *J*=11.5 Hz, 2H), 1.93 (dd, *J*=22.1, 8.7 Hz, 5H), 1.85-1.77 (m, 2H), 1.71 (s, 9H), 1.45-1.33 (m, 2H). m/z 825.50 [M+H]⁺. |
| 133 | ¹H NMR (400 MHz, CDCl₃) δ 8.41 (d, *J*=5.2 Hz, 1H), 8.18 (d, *J*=0.9 Hz, 1H), 7.92-7.84 (m, 3H), 7.81 (d, *J*=2.9 Hz, 1H), 7.55 (d, *J*=8.8 Hz, 2H), 7.45-7.37 (m, 2H), 7.09-7.03 (m, 3H), 6.98 (d, J=8.9 Hz, 2H), 6.64 (d, *J*=9.0 Hz, 1H), 4.71 (d, *J*=6.0 Hz, 2H), 4.32 (s, 1H), 3.96 (d, *J*=12.2 Hz, 1H), 3.64 (d, *J*=12.1 Hz, 2H), 3.42 (m, 4H), 2.87 (d, *J*=18.2 Hz, 1H), 2.72 (dd, *J*=15.0, 7.9 Hz, 3H), 2.55 (m, 4H), 2.51 (d, *J*=4.6 Hz, 1H), 2.46 (s, 3H), 2.31-2.20 (m, 2H), 1.91 (m, 3H), 1.71 (d, *J*=0.8 Hz, 9H), 1.43 (d, *J*=11.5 Hz, 2H). m/z 826.51 [M+H]⁺. |
| 134 | ¹H NMR (400 MHz, DMSO-*d₆*) δ 10.77 (s, 1H), 9.36 (s, 1H), 8.50 (t, *J*=5.7 Hz, 1H), 8.45 (d, *J*=5.2 Hz, 1H), 8.27 (s, 1H), 7.99-7.89 (m, 3H), 7.65 (d, *J*=8.8 Hz, 2H), 7.37 (d, *J*=8.0 Hz, 1H), 7.29 (d, *J*=5.2 Hz, 1H), 6.98-6.92 (m, 4H), 6.64 (d, *J*=8.2 Hz, 2H), 5.80-5.60 (m, 1H), 4.55-4.48 (m, 1H), 4.46 (d, *J*=5.6 Hz, 2H), 4.33-4.22 (m, 1H), 3.65-3.57 (m, 3H), 3.10-2.89 (m, 3H), 2.80-2.55 (m, 6H), 2.41 (s, 3H), 2.13-2.07 (m, 1H), 2.03-1.77 (m, 7H), 1.77-1.52 (m, 2H), 1.42 (d, *J*=6.7 Hz, 6H), 1.35-1.23 (m, 2H). m/z 809.45 [M+H]⁺. |
| 135 | ¹H NMR (400 MHz, CDCl₃) δ 8.41 (d, *J*=5.2 Hz, 1H), 8.23 (brs, 1H), 8.04 (s, 1H), 7.89 (s, 1H), 7.87 (d, *J*=7.9 Hz, 1H), 7.75 (s, 1H), 7.56 (d, *J*=8.9 Hz, 2H), 7.41 (d, *J*=7.9 Hz, 1H), 7.24 (s, 1H), 7.11 (d, *J*=8.5 Hz, 2H), 7.07 (d, *J*=5.2 Hz, 1H), 6.98 (d, *J*=9.0 Hz, 2H), 6.65 (d, *J*=8.5 Hz, 2H), 5.93 (t, *J*=5.4 Hz, 1H), 4.66 (d, *J*=5.6 Hz, 2H), 4.06 (dd, *J*=12.4, 4.7 Hz, 1H), 3.63 (d, *J*=12.3 Hz, 2H), 3.12 (brs, 1H), 2.91-2.84 (m, 1H), 2.78 (dd, *J*=13.2, 5.1 Hz, 1H), 2.73-2.64 (m, 2H), 2.60-2.59 (m, 1H), 2.45 (s, 3H), 2.37 (brs, 1H), 2.20-2.06 (m, 2H), 1.96-1.83 (m, 11H), 1.60 (s, 9H), 1.49-1.36 (m, 2H). m/z 823.56 [M+H]⁺. |
| 136 | ¹H NMR (400 MHz, CDCl₃) δ 8.41 (d, *J*=5.2 Hz, 1H), 8.19 (s, 1H), 8.00 (s, 1H), 7.91-7.83 (m, 2H), 7.55 (d, *J*=8.9 Hz, 2H), 7.43 (d, *J*=8.0 Hz, 2H), 7.12 (d, *J*=8.4 Hz, 2H), 7.08 (d, *J*=5.1 Hz, 2H), 6.98 (d, *J*=9.0 Hz, 2H), 6.65 (d, *J*=8.5 Hz, 2H), 4.71 (d, *J*=5.9 Hz, 2H), 4.67 (s, 1H), 4.06 (d, *J*=11.8 Hz, 1H), 3.64 (d, *J*=12.1 Hz, 2H), 3.03 (s, 1H), 2.88 (m, 1H), 2.73 (m, 3H), 2.61-2.53 (m, 1H), 2.46 (s, 3H), 2.43-2.38 (m, 1H), 2.28 (m, 2H), 2.06-1.84 (m, 5H), 1.79 (s, 3H), 1.73 (m, 12H), 1.43-1.40 (m, 2H). m/z 824.55 [M+H]⁺. |
| 137 | ¹H NMR (400 MHz, CDCl₃) δ 9.09 (s, 1H), 8.83 (s, 1H), 8.45 (d, *J*=5.1 Hz, 1H), 8.20 (d, *J*=1.5 Hz, 1H), 8.14 (s, 1H), 7.98 (s, 1H), 7.55 (d, *J*=8.8 Hz, 2H), 7.12-7.09 (m, 4H), 7.00 (d, *J*=7.6 Hz, 2H), 6.65 (d, *J*=8.6 Hz, 2H), 4.78 (d, *J*=4.3 Hz, 2H), 4.66 (m, 1H), 4.06 (m, 1H), 3.65 (d, *J*=11.7 Hz, 2H), 3.04 (m, 2H), 2.86 (m, 1H), 2.81-2.66 (m, 3H), 2.56 (m, 1H), 2.44 (s, 3H), 2.28 (m, 2H), 2.04 (m, 2H), 1.91 (m, 3H), 1.79 (m, 4H), 1.72 (m, 10H), 1.42 (m, 3H). m/z 825.55 [M+H]⁺. |
| 138 | ¹H NMR (400 MHz, DMSO-*d*₆) δ 10.31 (s, 1H), 9.32 (s, 1H), 8.97 (t, *J*=6.1 Hz, 1H), 8.70 (s, 1H), 8.44 (d, J=5.2 Hz, 1H), 7.96 (s, 1H), 7.92 (d, *J*=8.0 Hz, 1H), 7.63 (d, *J*=9.0 Hz, 2H), 7.37 (d, J=8.0 Hz, 1H), 7.32-7.25 (m, 2H), 7.22 (s, 1H), 7.18-7.10 (m, 2H), 6.91 (d, *J*=9.1 Hz, 2H), 4.51 (d, *J*=6.0 Hz, 2H), 3.78 (t, *J*=6.7 Hz, 2H), 3.59 (d, *J*=12.1 Hz, 2H), 2.96 (d, *J*=11.1 Hz, 2H), 2.68 (dt, *J*=7.7, 6.3 Hz, 2H), 2.59 (t, *J*=11.0 Hz, 3H), 2.43 (s, 3H), 2.20 (d, *J*=7.1 Hz, 2H), 1.99 (t, *J*=10.5 Hz, 2H), 1.77 (m, 6H), 1.64 (s, 9H), 1.33-1.15 (m, 3H). m/z 810.50 [M+H]⁺. |
| 139 | ¹H NMR (400 MHz, DMSO-*d*₆) δ 10.31 (s, 1H), 9.32 (s, 1H), 8.97 (t, *J*=6.1 Hz, 1H), 8.70 (s, 1H), 8.44 (d, *J*=5.2 Hz, 1H), 7.99-7.87 (m, 2H), 7.63 (d, *J*=9.0 Hz, 2H), 7.37 (d, *J*=8.0 Hz, 1H), 7.32-7.21 (m, 5H), 6.91 (d, *J*=9.1 Hz, 2H), 4.51 (d, *J*=6.0 Hz, 2H), 3.76 (t, *J*=6.7 Hz, 2H), 3.59 (d, *J*=12.1 Hz, 2H), 2.96 (d, *J*=11.2 Hz, 2H), 2.69 (t, *J*=6.6 Hz, 2H), 2.59 (m, 3H), 2.43 (s, 3H), 2.20 (d, *J*=7.1 Hz, 2H), 1.99 (t, *J*=10.5 Hz, 2H), 1.78-1.70 (m, 4H), 1.66 (m, 11H), 1.30-1.17 (m, 3H). m/z 810.49 [M+H]⁺. |
| 140 | ¹H NMR (400 MHz, CDCl₃) δ 8.45 (d, *J*=5.2 Hz, 1H), 8.19 (s, 1H), 7.93-7.84 (m, 2H), 7.71-7.62 (m, 3H), 7.47-7.40 (m, 2H), 7.35-7.28 (m, 4H), 7.14 (dd, *J*=10.7, 3.4 Hz, 4H), 4.71 (d, *J*=5.9 Hz, 2H), 3.87 (t, *J*=6.7 Hz, 2H), 3.51 (m,2H), 3.02-2.89 (m, 4H), 2.82 (t, *J*=6.7 Hz, 2H), 2.47 (m, 4H), 2.20 (d, *J*=7.0 Hz, 2H), 2.04-1.90 (m, 4H), 1.85-1.75 (m, 6H), 1.71 (s, 9H), 1.28 (m, 3H). m/z 824.60 [M+H]⁺. |
| 141 | ¹H NMR (400 MHz, DMSO-*d*₆) δ 10.34 (s, 1H), 9.62 (s, 1H), 9.03 (t, *J*=6.1 Hz, 1H), 8.72 (s, 1H), 8.51 (d, *J*=5.2 Hz, 1H), 7.99 (s, 1H), 7.95 (d, *J*=8.0 Hz, 1H), 7.77 (d, *J*=8.5 Hz, 2H), 7.37 (m, 2H), 7.23 (m, 6H), 4.51 (d, *J*=6.0 Hz, 2H), 3.75 (t, *J*=6.7 Hz, 2H), 2.91 (d, *J*=10.8 Hz, 2H), 2.80 (d, *J*=10.6 Hz, 2H), 2.72-2.65 (m, 3H), 2.43 (s, 3H), 2.13 (d, *J*=7.0 Hz, 2H), 1.89 (m, 4H), 1.76-1.58 (m, 17H), 1.48 (m, 1H), 1.10 (m, 2H). m/z 824.58 [M+H]⁺. |
| 142 | ¹H NMR (400 MHz, CDCl₃) δ 8.45 (d, *J*=5.2 Hz, 1H), 8.19 (s, 1H), 8.02 (s, 1H), 7.91-7.83 (m, 2H), 7.66 (d, *J*=8.4 Hz, 2H), 7.45 (m, 2H), 7.30 (d, *J*=8.4 Hz, 2H), 7.24 (s, 1H), 7.13 (d, *J*=5.2 Hz, 1H), 7.09 (d, *J*=8.5 Hz, 2H), 6.63 (d, *J*=8.5 Hz, 2H), 4.71 (d, *J*=5.9 Hz, 2H), 4.65 (d, *J*=3.5 Hz, 1H), 4.10-4.00 (m, 1H), 3.51 (m, 2H), 2.93 (m, 4H), 2.85 (m, 1H), 2.75 (m, 1H), 2.63-2.52 (m, 1H), 2.47 (s, 3H), 2.38 (m, 1H), 2.20 (d, *J*=6.8 Hz, 2H), 2.04-1.82 (m, 5H), 1.74 (d, J=3.8 Hz, 6H), 1.71 (s, 9H), 1.29 (s, 3H). m/z 838.61 [M+H]⁺. |
| 143 | ¹H NMR (400 MHz, CDCl₃) δ 8.41 (d, *J*=5.2 Hz, 1H), 8.14 (s, 1H), 8.06 (d, *J*=1.7 Hz, 1H), 8.03-7.93 (m, 2H), 7.89 (t, *J*=6.2 Hz, 1H), 7.53 (dd, *J*=8.5, 3.9 Hz, 3H), 7.14-7.06 (m, 4H), 6.99 (t, *J*=6.1 Hz, 2H), 6.65 (d, *J*=8.5 Hz, 2H), 4.89 (s, 2H), 4.79 (d, *J*=6.3 Hz, 2H), 4.66 (s, 1H), 4.06 (dd, *J*=12.6, 4.7 Hz, 1H), 3.64 (d, *J*=12.1 Hz, 2H), 3.03 (m, 2H), 2.87 (m, 1H), 2.82-2.74 (m, 1H), 2.70 (m, 2H), 2.60-2.51 (m, 1H), 2.43 (m, 1H), 2.28 (m, 2H), 2.05 (m, 2H), 1.91 (m, 4H), 1.79 (m, 4H), 1.68 (s, 9H), 1.41 (dd, *J*=21.5, 8.9 Hz, 3H). m/z 840.54 [M+H]⁺. |
| 144 | ¹H NMR (400 MHz, CDCl₃) δ 8.38 (d, *J*=5.2 Hz, 1H), 8.28 (d, *J*=2.6 Hz, 1H), 7.85-7.82 (m, 4H), 7.35 (d, *J*=8.4 Hz, 1H), 7.17 (d, J=8.7 Hz, 2H), 7.06 (d, *J*=5.2 Hz, 1H), 6.99 (d, *J*=8.4 Hz, 2H), 6.83 (s, 1H), 6.41 (d, *J*=9.0 Hz, 1H), 4.84 (dd, *J*=7.7, 4.4 Hz, 1H), 4.44 (d, *J*=5.6 Hz, 2H), 4.35 (tt, *J*=6.5, 4.7 Hz, 1H), 4.22 (t, *J*=5.5 Hz, 1H), 4.16-4.10 (m, 2H), 3.86 (dd, *J*=8.6, 4.7 Hz, 2H), 3.76-3.70 (m, 1H), 3.69-3.56 (m, *J*=8.0, 4.2 Hz, 2H), 3.45-3.39 (m, 1H), 3.12-3.06 (m, 2H), 2.99-2.91 (m, 1H), 2.71-2.63 (m, 1H), 2.54-2.44 (m, 3H), 2.41 (s, 3H), 2.37-2.21 (m, 4H), 2.11-1.99 (m, 2H), 1.88-1.67 (m, 8H), 1.16 (d, *J*=6.1 Hz, 6H). m/z 816.53 [M+H]⁺. |
| 145 | ¹H NMR (400 MHz, CDCl₃) δ 8.38 (d, *J*=5.2 Hz, 1H), 8.28 (d, *J*=2.6 Hz, 1H), 8.05 (brs, 1H), 7.86-7.82 (m, 3H), 7.35 (d, *J*=8.5 Hz, 1H), 7.11 (d, *J*=8.5 Hz, 2H), 7.06 (d, *J*=5.2 Hz, 1H), 6.89 (s, 1H), 6.64 (d, *J*=8.5 Hz, 2H), 6.41 (d, *J*=9.0 Hz, 1H), 4.67 (d, *J*=3.3 Hz, 1H), 4.44 (d, *J*=5.5 Hz, 2H), 4.40-4.32 (m, 1H), 4.23 (t, *J*=5.5 Hz, 1H), 4.13 (t, *J*=7.6 Hz, 2H), 4.09-4.02 (m, 1H), 3.86 (dd, *J*=8.6, 4.7 Hz, 2H), 3.76-3.72 (m, 1H), 3.64-3.56 (m, 2H), 3.42 (q, *J*=8.7 Hz, 1H), 3.13-3.07 (m, 3H), 2.93-2.83 (m, 1H), 2.75 (ddd, *J*=18.1, 13.3, 5.1 Hz, 1H), 2.60-2.48 (m, 3H), 2.41 (s, 3H), 2.36-2.27 (m, 1H), 2.25-2.17 (m, 1H), 2.16-2.05 (m, 2H), 1.96-1.74 (m, 8H), 1.16 (d, *J*=6.1 Hz, 6H). m/z 408.47 [M+H]²⁺. |
| 146 | ¹H NMR (400 MHz, CDCl₃) δ 8.38 (d, *J*=5.2 Hz, 1H), 8.26 (d, *J*=2.6 Hz, 1H), 8.19 (s, 1H), 7.94 (brs, 1H), 7.87-7.83 (m, 3H), 7.42 (d, *J*=7.9 Hz, 2H), 7.11 (d, *J*=8.5 Hz, 2H), 7.07 (d, *J*=5.2 Hz, 1H), 6.89 (s, 1H), 6.64 (d, *J*=8.5 Hz, 2H), 6.41 (d, *J*=9.0 Hz, 1H), 4.70 (d, *J*=5.9 Hz, 2H), 4.67 (s, 1H), 4.06 (dd, *J*=12.5, 4.9 Hz, 1H), 3.73 (dd, *J*=9.7, 7.3 Hz, 1H), 3.61-3.55 (m, 1H), 3.44-3.38 (m, 1H), 3.30-3.06 (m, 3H), 2.91-2.84 (m, 1H), 2.80-2.72 (m, 1H), 2.62-2.47 (m, 3H), 2.45 (m, 4H), 2.38-2.26 (m, 2H), 2.24-2.21 (m, 1H), 1.97-1.74 (m, 8H), 1.71 (s, 9H). m/z 825.62 [M+H]⁺. |

### Evaluation of Compounds of the Present Invention

### Experimental Example 1: Measurement of BTK protein degradation ability

TMD-8, RAMOS, or 293T BTK MUT (C481S) cells were treated with the synthesized compounds, and then the amount of BTK protein present in the cells was measured using Western blotting detection. The protocol for the experiment conducted using TMD-8 cells was as follows:
[Culture] TMD-8 cells were resuspended in RPMI1640 MEDIUM (Hyclone, SH30027.01), 10% FBS (Hyclone, SV30207.02), and 1% Penicillin-streptomycin (Welgene, LS 202-02) media, and 1 mL of this suspension was seeded in a 12-well plate at a cell concentration of 1 × 10⁶/mL.
[Compound treatment] The 12-well plate was treated as follows: The 10 mM stock was serially diluted 1/10 with DMSO (3 µL + 27 µL DMSO), and cells were treated with compounds at final concentrations of 0.1, 1, 10 µM or 0.0001, 0.001, 0.01, 0.1 µM, and harvested after 24 hours. In the negative control group, DMSO diluted 1/10 was added to the media. (3 µL DMSO + 27 µL media)
[Harvest] Each well was pipetted with a 1 mL pipette, collected in a 1.5 mL microtube, and centrifuged (500 g, 5 min, 4 °C). After removing the supernatant, it was washed with PBS and centrifuged again (500 g, 5 min, 4 °C). The supernatant was removed and the pellet was collected.
[Cell lysis and Sample preparation] lysis buffer was prepared as follows: RIPA buffer (Biosesang, RC2038-050-00), 0.5 mM PMSF (SIGMA, P7626) and 1× Protease/Phosphatase Inhibitor (Cell signaling, 5872S) were added at 60-70 µL per well and left on ice for 30 minutes. (Vortexing at 0 and 30 minutes, respectively), followed by sonication (10 seconds pulse, 30 seconds rest, 5 cycles, 70% amplification) and centrifuge (15000 g, 15 min, 4 °C). Only the supernatant was collected and transferred to a new microtube. The samples were diluted by 1/2 with RIPA buffer in a 96-well plate. 200 µL of the mixture A:B = 50:1 of the BCA Protein Assay Kit (iNtRON, 21071) was added each, stayed at 37 °C for 30 minutes, and cooled for 10 minutes. Afterwards, absorbance was measured at 562 nm using BioTek's SYNERGY H1 microplate reader. After making a sample by quantifying the protein using the measured value, it was boiled at 70 °C for 10 minutes. The sample buffer used at this time was a mixture of NuPAGE or Bolt 4x sample buffer (Invitrogen) and each 10× sample reducing agent according to the gel to be used. RIPA buffer was used for protein dilution.
[Western blotting detection method] The same amount of sample was loaded on NuPAGE or Bolt Bis-tris 4-12% gradient gel and run (200 V, 35 minutes). It was transferred to a 0.2 mm NC membrane using Trans-blot Turbo (BIO-RAD) (1.3 A constant, 25 V limit, 15 minutes). For blocking, skim milk or Intercept Blocking Buffer (LI-COR, 927-60001):0.1% TBST = 1:1 was used at room temperature for 1 hour. Anti BTK Rabbit (1:1,000 in 5% skim milk/0.2% TBST, size: 77 kDa, Cell signaling Technology) was reacted at 4 °C for 16 hours or at room temperature for 2 hours. Anti GAPDH Rabbit (1:10,000 in 5% BSA/0.2% TBST, size: 36 kDa, GENETEX) and Anti β-actin mouse (1:10,000 in 5% BSA/0.2% TBST, RT, size: 43 kDa, GENETEX) were reacted at room temperature for 1 hour or 3 hours. The samples were washed 3 times with 0.5% TBST for 5 minutes each. Secondary antibodies, Anti-Rabbit IgG (1:5,000 in 5% Skim milk/TBST, CST), IRDye^{®} 800CW Goat anti-Rabbit IgG Secondary Antibody (1:10,000 in 5% skim/TBST, RT 45 min), IRDye^{®} 680RD Goat anti-Mouse IgG Secondary Antibody (1:10,000 in 5% skim/TBST, RT 45 min) were added depending on each sample and reacted for 45 minutes on a rocker at room temperature. The samples were washed 5 times with 0.5% TBST for 5 minutes each and detected with LI-COR's Odyssey. At this time, BTK was detected using SuperSignal West Pico PLUS Chemiluminescent Substrate or SuperSignal West femto Maximum Sensitivity Substrate, and house-keeping genes were detected as is.

The results of evaluating the BTK protein degradation ability of the compounds of the present invention in TMD-8 cells are summarized in Table 3 below.

**[Table 3]**

| Example | BTK degradation (%) | | |
|---|---|---|---|
| | 1 nM | 10 nM | 100 nM |
| 1 | | 92 | 97.5 |
| 2 | | 84.5 | 94 |
| 3 | | 84 | 93 |
| 4 | | -3.5 | 29 |
| 5 | | -12 | -7 |
| 6 | | -2.5 | 10 |
| 7 | | 90 | 98.5 |
| 8 | | 89.5 | 97.5 |
| 9 | | 76.5 | 96.5 |
| 10 | 56 | 94.5 | 98.5 |
| 11 | 60 | 93.5 | 98.5 |
| 12 | 23.5 | 84.5 | 98.5 |
| 13 | 38 | 91 | 99 |
| 14 | 20 | 85.5 | 98 |
| 15 | 56.5 | 95.5 | 99 |
| 16 | 59 | 93 | 98.5 |
| 17 | 37 | 91 | 98 |
| 18 | 46 | 92 | 98.5 |
| 19 | 48 | 93 | 99 |
| 20 | 49 | 92 | 98.5 |
| 21 | 70 | 96.5 | 99 |
| 22 | 75 | 97 | 99 |
| 23 | | 72 | 91.5 |
| 24 | | 6.5 | 20 |
| 25 | | 8.5 | 24 |
| 26 | 15.5 | 76 | 93 |
| 27 | 26.5 | 71.5 | 94.5 |
| 28 | 18 | 12 | 4.5 |
| 29 | 7 | 40.5 | 81.5 |
| 30 | -1 | 20.5 | 91.5 |
| 31 | 61.5 | 94.5 | 98.5 |
| 32 | 58.5 | 92 | 98.5 |
| 33 | 49.5 | 93.5 | 99.5 |
| 34 | 41 | 90 | 99.5 |
| 35 | 50.5 | 93.5 | 99.5 |
| 36 | 60 | 94 | 99 |
| 37 | 54.5 | 92.5 | 99 |
| 38 | 72.5 | 96 | 98.5 |
| 39 | 65.5 | 93.5 | 98.5 |
| 40 | 69.5 | 95.5 | 98 |
| 41 | 81.5 | 100.0 | 100.0 |
| 42 | 60.5 | 97.5 | 100.0 |
| 43 | 83.0 | 100.0 | 100.0 |
| 44 | 66.0 | 95.0 | 99.0 |
| 45 | 45.5 | 89.5 | 98.0 |
| 46 | 46.5 | 91.0 | 98.0 |
| 47 | 22.5 | 93.5 | 99.5 |
| 48 | 53.5 | 94.5 | 99.0 |
| 49 | 23.0 | 87.0 | 90.5 |
| 50 | 19.7 | 82.0 | 99.3 |
| 51 | 24.0 | 86.7 | 99.3 |
| 52 | 24.0 | 92.0 | 99.3 |
| 53 | 13.3 | 86.7 | 99.3 |
| 54 | 38.7 | 95.0 | 99.7 |
| 55 | 37.7 | 94.7 | 99.7 |
| 56 | 13.7 | 91.3 | 99.7 |
| 57 | 54.0 | 95.0 | 99.5 |
| 58 | 62.0 | 97.5 | 99.0 |
| 59 | 72.0 | 97.0 | 99.0 |
| 60 | 58.0 | 96.5 | 99.5 |
| 61 | 66.5 | 95.5 | 99.0 |
| 62 | 57.0 | 97.5 | 100.0 |
| 63 | 29.0 | 83.0 | 97.5 |
| 64 | 43.0 | 92.5 | 98.5 |
| 65 | 32.5 | 92.0 | 99.0 |
| 66 | 28.0 | 91.5 | 98.5 |
| 67 | 13.5 | 77.0 | 98.0 |
| 68 | 22.5 | 88.0 | 99.0 |
| 69 | 61.5 | 95.0 | 99.5 |
| 70 | 67.0 | 97.5 | 99.5 |
| 71 | 80.5 | 98.0 | 99.5 |
| 72 | 62.5 | 95.5 | 99.0 |
| 73 | 43.0 | 91.5 | 97.0 |
| 74 | 43.0 | 91.5 | 96.5 |
| 75 | 14.0 | 87.0 | 98.0 |
| 76 | 55.0 | 96.5 | 100.0 |
| 77 | 57.5 | 96.0 | 99.5 |
| 78 | 43.5 | 93.5 | 99.0 |
| 79 | 26.0 | 92.5 | 98.0 |
| 80 | 73.5 | 98.0 | 99.5 |
| 81 | 65.0 | 97.0 | 99.5 |
| 82 | 32.0 | 87.5 | 99.5 |
| 83 | 51.0 | 96.5 | 99.5 |
| 84 | 36.0 | 92.0 | 99.0 |
| 85 | 45.5 | 93.5 | 98.5 |
| 86 | 22 | 90 | 97.5 |
| 87 | 36.5 | 94.5 | 98.5 |
| 88 | 35.5 | 94.5 | 98.5 |
| 89 | -0.5 | 33 | 87 |
| 90 | 4 | 10 | 37.5 |
| 91 | 4.5 | 68.5 | 95.0 |
| 92 | 7.0 | 33.5 | 89.0 |
| 93 | 62.0 | 94.5 | 98.5 |
| 94 | 59.5 | 93.5 | 98.5 |
| 95 | 67.5 | 94.5 | 98.0 |
| 96 | 70.0 | 95.5 | 98.0 |
| 97 | 67.5 | 94.0 | 98.5 |
| 98 | 71.0 | 95.5 | 98.5 |
| 99 | 46.5 | 92.0 | 98.5 |
| 100 | 40.5 | 91.0 | 98.0 |
| 101 | 50.5 | 98.0 | 100.0 |
| 102 | 19.0 | 82.5 | 94.0 |
| 103 | -12.5 | 69.0 | 97.0 |
| 104 | -9.0 | 39.0 | 95.5 |
| 105 | 35 | 91.5 | 98 |
| 106 | 1 | 20 | 76 |
| 107 | 63.5 | 95 | 98 |
| 108 | 21.5 | 73 | 94.5 |
| 109 | 41.5 | 94.0 | 99.5 |
| 110 | 28.0 | 92.0 | 99.5 |
| 111 | 44 | 93.5 | 99.5 |
| 112 | 44.0 | 97.0 | 100.0 |
| 113 | 39.5 | 97.0 | 100.0 |
| 114 | 59.0 | 99.5 | 99.5 |
| 115 | 48.0 | 97.0 | 100.0 |
| 116 | 1.0 | 56.0 | 96.5 |
| 117 | 24.0 | 87.5 | 99.0 |
| 118 | 15.0 | 68.0 | 97.5 |
| 119 | 41.5 | 91.0 | 99.5 |
| 120 | 18.5 | 91.5 | 99.5 |
| 121 | 43.0 | 97.0 | 100.0 |
| 122 | -9.0 | 63.0 | 98.5 |
| 123 | 17.5 | 92.5 | 100.0 |
| 124 | -1.3 | 63.7 | 97.7 |
| 125 | 14.7 | 85.0 | 99.3 |
| 126 | 39.7 | 95.7 | 100.0 |
| 127 | 43.3 | 94.0 | 99.3 |
| 128 | 51.5 | 94.5 | 99.0 |
| 129 | 49.0 | 95.5 | 100.0 |
| 130 | 56.5 | 95.0 | 99.5 |
| 131 | 59.5 | 96.0 | 100.0 |
| 132 | 54.5 | 95.5 | 100.0 |
| 133 | 60.5 | 96.5 | 100.0 |
| 134 | 58.5 | 93.5 | 98.5 |
| 135 | 59.0 | 94.5 | 99.0 |
| 136 | 71.0 | 95.5 | 98.5 |
| 137 | 25.0 | 81.5 | 97.0 |
| 138 | 15.5 | 71.5 | 90.0 |
| 139 | 15.5 | 68.0 | 95.0 |
| 140 | 0.5 | 10.5 | 77.0 |
| 141 | 15.0 | 70.5 | 96.5 |
| 142 | 44.5 | 88.5 | 99.0 |
| 143 | 74.0 | 99.5 | 100.0 |
| 144 | 28.0 | 77.0 | 98.5 |
| 145 | 27.5 | 85.0 | 99.0 |
| 146 | 26.5 | 88.0 | 99.0 |

As shown in Table 3, the compounds according to the present invention showed excellent activity in degrading BTK protein.

## Claims

1. A compound of Chemical Formula 1: 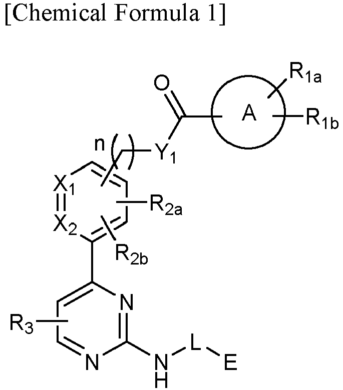
or a pharmaceutically acceptable salt thereof,
in the Chemical Formula 1,
A is carbocycle, heterocycle, aryl, or heteroaryl,
R₁ₐ and R_{1b} are each independently H, halogen, C₁₋₆ alkyl, C₁₋₆ alkoxy, or carbocycle, wherein one or more hydrogens in the alkyl, alkoxy, or carbocycle are optionally substituted with one or more selected from the group consisting of halogen, C₁₋₆ alkyl, C₁₋₆ haloalkyl, hydroxy, and C₁₋₆ hydroxyalkyl,
R₂ₐ and R_{2b} are each independently H, halogen, C₁₋₆ alkyl, C₁₋₆ haloalkyl, C₁₋₆ alkoxy, C₁₋₆ haloalkoxy, hydroxy, or C₁₋₆ hydroxyalkyl,
R₃ is H, halogen, C₁₋₆ alkyl, C₁₋₆ haloalkyl, or -NH₂,
X₁ and X₂ are each independently CH or N,
Yi is NH or O,
n is an integer of 0, 1 or 2,
L is the following Chemical Formula 2, 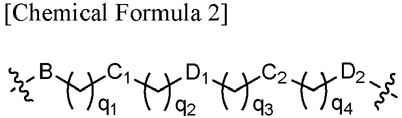
in the Chemical Formula 2,
B is aryl or heteroaryl, wherein one or more hydrogens in the aryl or heteroaryl are optionally substituted with one or more selected from the group consisting of C₁₋₆ alkyl, halogen, and C₁₋₆ haloalkyl,
C₁ and C₂ are each independently direct bond, carbocycle, or heterocycle, wherein one or more hydrogens in the carbocycle or heterocycle are optionally substituted with one or more selected from the group consisting of C₁₋₆ alkyl, halogen, C₁₋₆ haloalkyl, and hydroxy,
D₁ and D₂ are each independently direct bond, -O-, -N(R₄)- -C(O)-, -CC-, -C(O)NH-, or -NHC(O)-, wherein R₄ is H, C₁₋₆ alkyl, or C₁₋₆ haloalkyl,
q₁, q₂, q₃ and q₄ are each independently an integer of from 0 to 3,
E is the following Chemical Formula 3 or Chemical Formula 4, 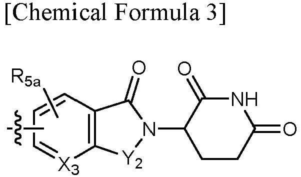 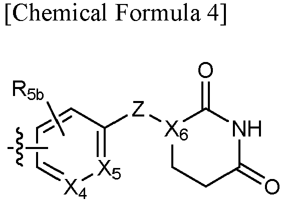
in the Chemical Formula 3 and 4,
X₃, X₄, X₅, and X₆ are each independently CH or N,
Y₂ is -C(R₆ₐ)(R_{6b})-, -C(O)-, -C(R₆ₐ)(R_{6b})-C(R₆ₐ)(R_{6b})-, -C(R₆ₐ)=C(R_{6b})-, -C(R₆ₐ)=N-, - N=C(R₆ₐ)-, or -N=N-,
Z is direct bond, -C(R₆ₐ)(R_{6b})-, -N(R₆ₐ)-, -O-, or -C(O)NH-,
R₅ₐ and R_{5b} are each independently H, halogen, C₁₋₆ alkyl, C₁₋₆ alkoxy, C₁₋₆ haloalkyl, or C₁₋₆ haloalkoxy,
R₆ₐ and R_{6b} are each independently H, halogen, C₁₋₆ alkyl, C₁₋₆ haloalkyl, C₁₋₆ alkoxy, or C₁₋₆ haloalkoxy.

2. The compound of Claim 1 or a pharmaceutically acceptable salt thereof, in the Chemical Formula 1,
A is carbocycle, heterocycle, aryl, or heteroaryl,
R₁ₐ and R_{1b} are each independently H, halogen, C₁₋₆ alkyl, or C₁₋₆ alkoxy, wherein one or more hydrogens in the alkyl or alkoxy are optionally substituted with one or more selected from the group consisting of halogen, C₁₋₃ alkyl, C₁₋₃ haloalkyl, hydroxy, and C₁₋₃ hydroxyalkyl,
R₂ₐ and R_{2b} are each independently H, halogen, C₁₋₆ alkyl, C₁₋₆ haloalkyl, C₁₋₆ alkoxy, C₁₋₆ haloalkoxy, hydroxy, or C₁₋₆ hydroxyalkyl,
R₃ is H, halogen, C₁₋₃ alkyl, C₁₋₃ haloalkyl, or -NH₂,
X₁ and X₂ are each independently CH or N,
Yi is NH or O,
n is an integer of 0, 1 or 2,
L is the following Chemical Formula 2, 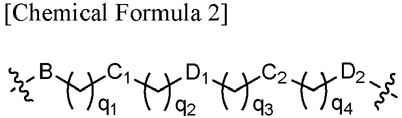
in the Chemical Formula 2,
B is aryl or heteroaryl, wherein one or more hydrogens in the aryl or heteroaryl are optionally substituted with one or more selected from the group consisting of C₁₋₃ alkyl, halogen, and C₁₋₃ haloalkyl,
C₁ and C₂ are each independently direct bond, carbocycle, or heterocycle, wherein one or more hydrogens in the carbocycle or heterocycle are optionally substituted with one or more selected from the group consisting of C₁₋₃ alkyl, halogen, and hydroxy,
D₁ and D₂ are each independently direct bond, -O-, -N(R₄)- -C(O)-, -CC-, -C(O)NH-, or -NHC(O)-, wherein R₄ is H, C₁₋₃ alkyl, or C₁₋₃ haloalkyl,
q₁, q₂, q₃ and q₄ are each independently an integer of from 0 to 3,
E is the following Chemical Formula 3 or Chemical Formula 4, 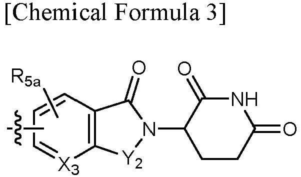 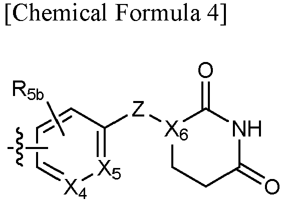
in the Chemical Formula 3 and 4,
X₃, X₄, X₅, and X₆ are each independently CH or N,
Y₂ is -C(R₆ₐ)(R_{6b})-, -C(O)-, -C(R₆ₐ)=C(R_{6b})-, -C(R₆ₐ)=N-, -N=C(R₆ₐ)-, or -N=N-,
Z is direct bond, -C(R₆ₐ)(R_{6b})-, -N(R₆ₐ)-, -O-, or -C(O)NH-,
R₅ₐ and R_{5b} are each independently H, halogen, C₁₋₃ alkyl, C₁₋₃ alkoxy, C₁₋₃ haloalkyl, or C₁₋₆ haloalkoxy,
R₆ₐ and R_{6b} are each independently H, halogen, C₁₋₃ alkyl, or C₁₋₃ haloalkyl.

3. The compound of Claim 1 or a pharmaceutically acceptable salt thereof, in the Chemical Formula 1,
A is azetidine, pyrrolidine, piperidine, piperazine, benzene, pyridine, pyrimidine, pyrazole, triazole, oxazole, thiazole, oxadiazole, or thiadiazole,
R₁ₐ and R_{1b} are each independently H, halogen, C₁₋₆ alkyl, or C₁₋₆ alkoxy, wherein one or more hydrogens in the alkyl or alkoxy are optionally substituted with one or more selected from the group consisting of halogen, C₁₋₃ alkyl, and hydroxy,
R₂ₐ and R_{2b} are each independently H, halogen, C₁₋₃ alkyl, C₁₋₃ haloalkyl, or C₁₋₃ hydroxyalkyl,
R₃ is H or halogen,
X₁ is CH or N,
X₂ is CH,
Yi is NH or O,
n is an integer of 0, 1 or 2,
L is the following Chemical Formula 2, 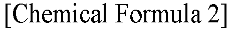 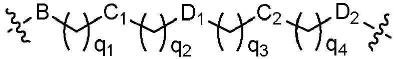
in the Chemical Formula 2,
B is benzene, pyridine, pyrimidine, pyrazine, pyridazine, tetrahydroisoquinoline, pyrazole, triazole, or tetrahydropyrazolopyrazine,
C₁ and C₂ are each independently direct bond, cyclobutane, cyclohexane, azetidine, pyrrolidine, piperidine, or piperazine, wherein one or more hydrogens in the cyclobutane, cyclohexane, azetidine, pyrrolidine, piperidine, or piperazine are optionally substituted with one or more selected from the group consisting of C₁₋₃ alkyl, halogen, and hydroxy,
D₁ and D₂ are direct bond, -O-, -NH-, -C(O)-, -C(O)NH-, or -NHC(O)-,
q₁, q₂, q₃ and q₄ are each independently an integer of from 0 to 3,
E is the following Chemical Formula 3 or Chemical Formula 4, 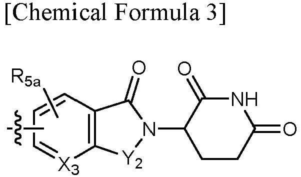 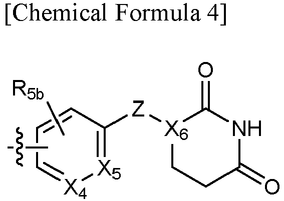
in the Chemical Formula 3 and 4,
X₃, X₄, X₅, and X₆ are each independently CH or N,
Y₂ is -CH₂- or -C(O)-,
Z is direct bond, -NH-, or -O-,
R₅ₐ and R_{5b} are each independently H, halogen, C₁₋₃ alkyl, or C₁₋₃ alkoxy.

4. The compound of Claim 1 or a pharmaceutically acceptable salt thereof, wherein L is any one of the followings:

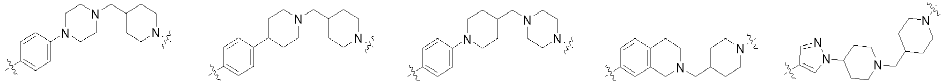

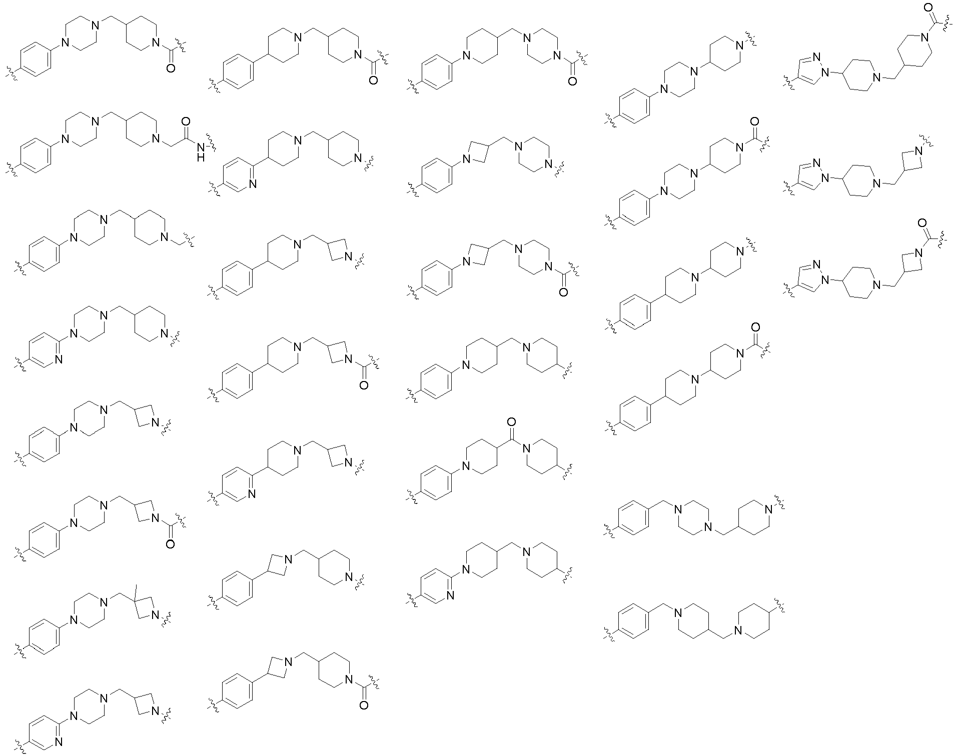

5. The compound of Claim 1 or a pharmaceutically acceptable salt thereof, wherein the compound is any one of the following compounds:
| Comp ound no. | IUPAC Name |
|---|---|
| 1 | N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-3-isopropoxyazetidine-1-carboxamide |
| 2 | 1-(tert-butyl)-N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-1H-pyrazole-4-carboxamide |
| 3 | 1-(tert-butyl)-N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-1H-1,2,3-triazole-4-carboxamide |
| 4 | N-(3-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-5-fluoro-2-methylphenyl)-3-isopropoxyazetidine-1-carboxamide |
| 5 | 1-(tert-butyl)-N-(3-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-5-fluoro-2-methylphenyl)-1H-pyrazole-4-carboxamide |
| 6 | 1-(tert-butyl)-N-(3-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-5-fluoro-2-methylphenyl)-1H-1,2,3-triazole-4-carboxamide |
| 7 | N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-3-isopropoxyazetidine-1-carboxamide |
| 8 | 1-(tert-butyl)-N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1H-pyrazole-4-carboxamide |
| 9 | 1-(tert-butyl)-N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1H-1,2,3-triazole-4-carboxamide |
| 10 | N-(4-(2-((4-(4-((1-(6-(2,6-dioxopiperidin-3-yl)-5-oxo-6,7-dihydro-5H-pyrrolo[3,4-b]pyridin-2-yl)piperidin-4-yl)methyl)piperazin-1-yl)phenyl)amino)pyrimidin-4-yl)-2-methylbenzyl)-3-isopropoxyazetidine-1-carboxamide |
| 11 | N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-3-isopropoxyazetidine-1-carboxamide |
| 12 | N-(4-(2-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)pyrimidin-4-yl)-2-methylbenzyl)-3-isopropoxyazetidine-1-carboxamide |
| 13 | N-(4-(2-((4-(4-((1-(2-(2,6-dioxopiperidin-3-yl)-6-fluoro-1,3-dioxoisoindolin-5-yl)azetidin-3-yl)methyl)piperazin-1-yl)phenyl)amino)pyrimidin-4-yl)-2-methylbenzyl)-3-isopropoxyazetidine-1-carboxamide |
| 14 | N-(4-(2-((4-(4-((1-(2-(2,6-dioxopiperidin-3-yl)-1,3-dioxoisoindolin-5-yl)-3-methylazetidin-3-yl)methyl)piperazin-1-yl)phenyl)amino)pyrimidin-4-yl)-2-methylbenzyl)-3-isopropoxyazetidine-1-carboxamide |
| 15 | N-(4-(2-((4-(1-((1-(2-(2,6-dioxopiperidin-3-yl)-1,3-dioxoisoindolin-5-yl)piperidin-4-yl)methyl)piperidin-4-yl)phenyl)amino)pyrimidin-4-yl)-2-methylbenzyl)-3-isopropoxyazetidine-1-carboxamide |
| 16 | N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-3-isopropoxyazetidine-1-carboxamide |
| 17 | N-(4-(2-((6-(4-((1-(2-(2,6-dioxopiperidin-3-yl)-1,3-dioxoisoindolin-5-yl)piperidin-4-yl)methyl)piperazin-1-yl)pyridin-3-yl)amino)pyrimidin-4-yl)-2-methylbenzyl)-3-isopropoxyazetidine-1-carboxamide |
| 18 | N-(4-(2-((6-(4-((1-(2-(2,6-dioxopiperidin-3-yl)-1,3-dioxoisoindolin-5-yl)azetidin-3-yl)methyl)piperazin-1-yl)pyridin-3-yl)amino)pyrimidin-4-yl)-2-methylbenzyl)-3-isopropoxyazetidine-1-carboxamide |
| 19 | N-(4-(2-((6-(1-((1-(2-(2,6-dioxopiperidin-3-yl)-1,3-dioxoisoindolin-5-yl)piperidin-4-yl)methyl)piperidin-4-yl)pyridin-3-yl)amino)pyrimidin-4-yl)-2-methylbenzyl)-3-isopropoxyazetidine-1-carboxamide |
| 20 | N-(4-(2-((6-(1-((1-(2-(2,6-dioxopiperidin-3-yl)-1,3-dioxoisoindolin-5-yl)azetidin-3-yl)methyl)piperidin-4-yl)pyridin-3-yl)amino)pyrimidin-4-yl)-2-methylbenzyl)-3-isopropoxyazetidine-1-carboxamide |
| 21 | N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-3-isopropoxyazetidine-1-carboxamide |
| 22 | N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-3-isopropoxyazetidine-1-carboxamide |
| 23 | N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-fluoro-2-methylphenyl)-3-isopropoxyazetidine-1-carboxamide |
| 24 | 1-(tert-butyl)-N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-fluoro-2-methylphenyl)-1H-pyrazole-4-carboxamide |
| 25 | 1-(tert-butyl)-N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-fluoro-2-methylphenyl)-1H-1,2,3-triazole-4-carboxamide |
| 26 | N-(3-(2-((4-(4-((1-(6-(2,6-dioxopiperidin-3-yl)-5-oxo-6,7-dihydro-5H-pyrrolo[3,4-b]pyridin-2-yl)piperidin-4-yl)methyl)piperazin-1-yl)phenyl)amino)pyrimidin-4-yl)-5-fluoro-2-methylphenyl)-3-isopropoxyazetidine-1-carboxamide |
| 27 | N-(3-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-5-fluoro-2-methylphenyl)-3-isopropoxyazetidine-1-carboxamide |
| 28 | N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-methylbenzyl)-3-isopropoxyazetidine-1-carboxamide |
| 29 | N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-methylphenyl)-3-isopropoxyazetidine-1-carboxamide |
| 30 | 4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl3-isopropoxyazetidine-1-carboxylate |
| 31 | 1-(tert-butyl)-N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1H-pyrazole-4-carboxamide |
| 32 | 1-(tert-butyl)-N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1H-1,2,3-triazole-4-carboxamide |
| 33 | N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1-isopropyl-1H-pyrazole-4-carboxamide |
| 34 | N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1-isopropyl-1H-pyrazole-4-carboxamide |
| 35 | N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1-isopropyl-1H-pyrazole-4-carboxamide |
| 36 | N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1-isopropyl-1H-pyrazole-4-carboxamide |
| 37 | 1-(tert-butyl)-N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1H-pyrazole-4-carboxamide |
| 38 | 1-(tert-butyl)-N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1H-pyrazole-4-carboxamide |
| 39 | 1-(tert-butyl)-N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1H-1,2,3-triazole-4-carboxamide |
| 40 | 1-(tert-butyl)-N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1H-1,2,3-triazole-4-carboxamide |
| 41 | N-(4-(2-((4-(1-((1-(2-(2,6-dioxopiperidin-3-yl)-1,3-dioxoisoindolin-5-yl)piperidin-4-yl)methyl)azetidin-3-yl)phenyl)amino)pyrimidin-4-yl)-2-methylbenzyl)-3-isopropoxyazetidine-1-carboxamide |
| 42 | N-(4-(2-((4-(4-((1-(3-((2,6-dioxopiperidin-3-yl)oxy)phenyl)piperidin-4-yl)methyl)piperazin-1-yl)phenyl)amino)pyrimidin-4-yl)-2-methylbenzyl)-3-isopropoxyazetidine-1-carboxamide |
| 43 | N-(4-(2-((4-(4-((1-(4-((2,6-dioxopiperidin-3-yl)oxy)phenyl)piperidin-4-yl)methyl)piperazin-1-yl)phenyl)amino)pyrimidin-4-yl)-2-methylbenzyl)-3-isopropoxyazetidine-1-carboxamide |
| 44 | N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1-isopropyl-1H-pyrazole-4-carboxamide |
| 45 | 1-(tert-butyl)-N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1H-pyrazole-4-carboxamide |
| 46 | 1-(tert-butyl)-N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1H-1,2,3-triazole-4-carboxamide |
| 47 | N-(4-(2-((4-(1-((1-(4-((2,6-dioxopiperidin-3-yl)oxy)phenyl)piperidin-4-yl)methyl)piperidin-4-yl)phenyl)amino)pyrimidin-4-yl)-2-methylbenzyl)-3-isopropoxyazetidine-1-carboxamide |
| 48 | N-(4-(2-((4-(4-((1-(4-((2,6-dioxopiperidin-3-yl)oxy)phenyl)piperidin-4-yl)methyl)piperazin-1-yl)phenyl)amino)pyrimidin-4-yl)-2-methylbenzyl)-1-isopropyl-1H-pyrazole-4-carboxamide |
| 49 | N-(4-(2-((4-(1-((1-(4-((2,6-dioxopiperidin-3-yl)oxy)phenyl)piperidin-4-yl)methyl)piperidin-4-yl)phenyl)amino)pyrimidin-4-yl)-2-methylbenzyl)-1-isopropyl-1H-pyrazole-4-carboxamide |
| 50 | N-(4-(2-((4-(4-((1-(5-((2,6-dioxopiperidin-3-yl)oxy)pyridin-2-yl)piperidin-4-yl)methyl)piperazin-1-yl)phenyl)amino)pyrimidin-4-yl)-2-methylbenzyl)-3-isopropoxyazetidine-1-carboxamide |
| 51 | N-(4-(2-((4-(1-((1-(5-((2,6-dioxopiperidin-3-yl)oxy)pyridin-2-yl)piperidin-4-yl)methyl)piperidin-4-yl)phenyl)amino)pyrimidin-4-yl)-2-methylbenzyl)-3-isopropoxyazetidine-1-carboxamide |
| 52 | N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-3-isopropoxyazetidine-1-carboxamide |
| 53 | N-(4-(2-((4-(1-((1-(5-((2,6-dioxopiperidin-3-yl)amino)pyridin-2-yl)piperidin-4-yl)methyl)piperidin-4-yl)phenyl)amino)pyrimidin-4-yl)-2-methylbenzyl)-3-isopropoxyazetidine-1-carboxamide |
| 54 | N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-3-isopropoxyazetidine-1-carboxamide |
| 55 | N-(4-(2-((4-(4-((1-(4-((2,6-dioxopiperidin-3-yl)amino)phenyl)piperidin-4-yl)methyl)piperazin-1-yl)phenyl)amino)pyrimidin-4-yl)-2-methylbenzyl)-3-isopropoxyazetidine-1-carboxamide |
| 56 | N-(4-(2-((4-(1-((1-(4-((2,6-dioxopiperidin-3-yl)amino)phenyl)piperidin-4-yl)methyl)piperidin-4-yl)phenyl)amino)pyrimidin-4-yl)-2-methylbenzyl)-3-isopropoxyazetidine-1-carboxamide |
| 57 | N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1-isopropyl-1H-pyrazole-4-carboxamide |
| 58 | N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1-isopropyl-1H-pyrazole-4-carboxamide |
| 59 | 1-(tert-butyl)-N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1H-pyrazole-4-carboxamide |
| 60 | 1-(tert-butyl)-N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1H-pyrazole-4-carboxamide |
| 61 | 1-(tert-butyl)-N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1H-1,2,3-triazole-4-carboxamide |
| 62 | 1-(tert-butyl)-N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1H-1,2,3-triazole-4-carboxamide |
| 63 | N-(4-(2-((4-((4-((1-(3-((2,6-dioxopiperidin-3-yl)oxy)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 64 | N-(4-(2-((4-((4-((1-(3-((2,6-dioxopiperidin-3-yl)amino)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 65 | N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-3-isopropoxyazetidine-1-carboxamide |
| 66 | N-(4-(2-((4-(4-((1-(4-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-3-isopropoxyazetidine-1-carboxamide |
| 67 | N-(4-(2-((4-((4-((1-(3-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 68 | N-(4-(2-((4-((4-((1-(4-(2,4-dioxotetrahydropyrimidin-1 (2H)-yl)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 69 | 1-(tert-butyl)-N-(4-(2-((4-((4-((1-(3-((2,6-dioxopiperidin-3-yl)amino)phenyl)piperidin-4-yl)methyl)piperazin-1-yl)methyl)phenyl)amino)pyrimidin-4-yl)-2-methylbenzyl)-1H-1,2,3-triazole-4-carboxamide |
| 70 | 1-(tert-butyl)-N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1H-1,2,3-triazole-4-carboxamide |
| 71 | 1-(tert-butyl)-N-(4-(2-((4-(4-((1-(4-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1H-1,2,3-triazole-4-carboxamide |
| 72 | 1-(tert-butyl)-N-(4-(2-((4-(4-((1-(4-((2,6-dioxopiperidin-3-yl)amino)-2-fluorophenyl)piperidin-4-yl)methyl)piperazin-1-yl)phenyl)amino)pyrimidin-4-yl)-2-methylbenzyl)-1H-1,2,3-triazole-4-carboxamide |
| 73 | 1-(tert-butyl)-N-(4-(2-((4-(4-((1-(4-(2,4-dioxotetrahydropyrimidin-1(2H)-yl)-2-fluorophenyl)piperidin-4-yl)methyl)piperazin-1-yl)phenyl)amino)pyrimidin-4-yl)-2-methylbenzyl)-1H-1,2,3-triazole-4-carboxamide |
| 74 | 1-(tert-butyl)-N-(4-(2-((4-((4-((1-(3-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-1H-1,2,3-triazole-4-carboxamide |
| 75 | 1-(tert-butyl)-N-(4-(2-((4-((4-((1-(4-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-1H-1,2,3-triazole-4-carboxamide |
| 76 | 1-(tert-butyl)-N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1H-1,2,3-triazole-4-carboxamide |
| 77 | 1-(tert-butyl)-N-(4-(2-((6-(4-((1-(4-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1H-1,2,3-triazole-4-carboxamide |
| 78 | 1-(tert-butyl)-N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1H-1,2,3-triazole-4-carboxamide |
| 79 | 1-(tert-butyl)-N-(4-(2-((4-(1-((1-(4-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1H-1,2,3-triazole-4-carboxamide |
| 80 | N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1-isopropyl-1H-pyrazole-4-carboxamide |
| 81 | 1-(tert-butyl)-N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1H-pyrazole-4-carboxamide |
| 82 | N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1-isopropyl-1H-pyrazole-4-carboxamide |
| 83 | 1-(tert-butyl)-N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1H-pyrazole-4-carboxamide |
| 84 | N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1-isopropyl-1H-pyrazole-4-carboxamide |
| 85 | 1-(tert-butyl)-N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1H-pyrazole-4-carboxamide |
| 86 | N-(4-(2-((4-(4-((1-(3-(2,4-dioxotetrahydropyrimidin-1(2H)-yl)-4-methoxybenzoyl)piperidin-4-yl)methyl)piperazin-1-yl)phenyl)amino)pyrimidin-4-yl)-2-methylbenzyl)-3-isopropoxyazetidine-1-carboxamide |
| 87 | N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-3-isopropoxyazetidine-1-carboxamide |
| 88 | N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-3-isopropoxyazetidine-1-carboxamide |
| 89 | N-(4-(2-((4-(4-((1-(3-(2,4-dioxotetrahydropyrimidin-1(2H)-yl)benzoyl)piperidin-4-yl)methyl)piperazin-1-yl)phenyl)amino)pyrimidin-4-yl)-2-methylbenzyl)-3-isopropoxyazetidine-1-carboxamide |
| 90 | N-(3-(2-((4-(4-((1-(3-(2,4-dioxotetrahydropyrimidin-1(2H)-yl)-4-methoxybenzoyl)piperidin-4-yl)methyl)piperazin-1-yl)phenyl)amino)pyrimidin-4-yl)-5-fluoro-2-methylphenyl)-3-isopropoxyazetidine-1-carboxamide |
| 91 | N-(4-(2-((1-(1-((1-(3-((2,6-dioxopiperidin-3-yl)amino)benzoyl)piperidin-4-yl)methyl)piperidin-4-yl)-1 H-pyrazol-4-yl)amino)pyrimidin-4-yl)-2-methylbenzyl)-3-isopropoxyazetidine-1-carboxamide |
| 92 | N-(4-(2-((1-(1-((1-(3-((2,6-dioxopiperidin-3-yl)amino)benzoyl)azetidin-3-yl)methyl)piperidin-4-yl)-1 H-pyrazol-4-yl)amino)pyrimidin-4-yl)-2-methylbenzyl)-3-isopropoxyazetidine-1-carboxamide |
| 93 | N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1-isopropyl-1H-pyrazole-4-carboxamide |
| 94 | N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1-isopropyl-1H-pyrazole-4-carboxamide |
| 95 | 1-(tert-butyl)-N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1H-pyrazole-4-carboxamide |
| 96 | 1-(tert-butyl)-N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1H-pyrazole-4-carboxamide |
| 97 | 1-(tert-butyl)-N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1H-1,2,3-triazole-4-carboxamide |
| 98 | 1-(tert-butyl)-N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1H-1,2,3-triazole-4-carboxamide |
| 99 | N-(4-(2-((4-(1-((1-(3-((2,6-dioxopiperidin-3-yl)amino)benzoyl)piperidin-4-yl)methyl)piperidin-4-yl)phenyl)amino)pyrimidin-4-yl)-2-methylbenzyl)-3-isopropoxyazetidine-1-carboxamide |
| 100 | N-(4-(2-((4-(1-((1-(3-((2,6-dioxopiperidin-3-yl)amino)benzoyl)azetidin-3-yl)methyl)piperidin-4-yl)phenyl)amino)pyrimidin-4-yl)-2-methylbenzyl)-3-isopropoxyazetidine-1-carboxamide |
| 101 | N-(4-(2-((4-(1-((1-(3-((2,6-dioxopiperidin-3-yl)amino)benzoyl)piperidin-4-yl)methyl)azetidin-3-yl)phenyl)amino)pyrimidin-4-yl)-2-methylbenzyl)-3-isopropoxyazetidine-1-carboxamide |
| 102 | N-(4-(2-((4-(4-((1-(3-((2,6-dioxopiperidin-3-yl)oxy)benzoyl)piperidin-4-yl)methyl)piperazin-1-yl)phenyl)amino)pyrimidin-4-yl)-2-methylbenzyl)-3-isopropoxyazetidine-1-carboxamide |
| 103 | N-(4-(2-((4-(4-(1-(3-((2,6-dioxopiperidin-3-yl)amino)benzoyl)piperidin-4-yl)piperazin-1-yl)phenyl)amino)pyrimidin-4-yl)-2-methylbenzyl)-3-isopropoxyazetidine-1-carboxamide |
| 104 | N-(4-(2-((4-(1'-(3-((2,6-dioxopiperidin-3-yl)amino)benzoyl)-[1,4'-bipiperidin]-4-yl)phenyl)amino)pyrimidin-4-yl)-2-methylbenzyl)-3-isopropoxyazetidine-1-carboxamide |
| 105 | N-(4-(2-((4-(4-((1-(2-((3-((2,6-dioxopiperidin-3-yl)amino)phenyl)amino)-2-oxoethyl)piperidin-4-yl)methyl)piperazin-1-yl)phenyl)amino)pyrimidin-4-yl)-2-methylbenzyl)-3-isopropoxyazetidine-1-carboxamide |
| 106 | N-(3-(2-((4-(4-((1-(2-((3-((2,6-dioxopiperidin-3-yl)amino)phenyl)amino)-2-oxoethyl)piperidin-4-yl)methyl)piperazin-1-yl)phenyl)amino)pyrimidin-4-yl)-5-fluoro-2-methylphenyl)-3-isopropoxyazetidine-1-carboxamide |
| 107 | N-(4-(2-((4-(4-((1-(6-(2,6-dioxopiperidin-3-yl)-5-oxo-6,7-dihydro-5H-pyrrolo[3,4-b]pyridin-2-yl)azetidin-3-yl)methyl)piperazin-1-yl)phenyl)amino)pyrimidin-4-yl)-2-methylbenzyl)-3-isopropoxyazetidine-1-carboxamide |
| 108 | N-(3-(2-((4-(4-((1-(6-(2,6-dioxopiperidin-3-yl)-5-oxo-6,7-dihydro-5H-pyrrolo[3,4-b]pyridin-2-yl)azetidin-3-yl)methyl)piperazin-1-yl)phenyl)amino)pyrimidin-4-yl)-5-fluoro-2-methylphenyl)-3-isopropoxyazetidine-1-carboxamide |
| 109 | N-(4-(2-((4-(4-(1-(2-(2,6-dioxopiperidin-3-yl)-1,3-dioxoisoindolin-5-yl)piperidin-4-yl)piperazin-1-yl)phenyl)amino)pyrimidin-4-yl)-2-methylbenzyl)-3-isopropoxyazetidine-1-carboxamide |
| 110 | N-(4-(2-((4-(1'-(2-(2,6-dioxopiperidin-3-yl)-1,3-dioxoisoindolin-5-yl)-[1,4'-bipiperidin]-4-yl)phenyl)amino)pyrimidin-4-yl)-2-methylbenzyl)-3-isopropoxyazetidine-1-carboxamide |
| 111 | N-(4-(2-((4-(4-((4-(2-(2,6-dioxopiperidin-3-yl)-1,3-dioxoisoindolin-5-yl)piperazin-1-yl)methyl)piperidin-1-yl)phenyl)amino)pyrimidin-4-yl)-2-methylbenzyl)-3-isopropoxyazetidine-1-carboxamide |
| 112 | N-(4-(2-((4-(3-((4-(2-(2,6-dioxopiperidin-3-yl)-1,3-dioxoisoindolin-5-yl)piperazin-1-yl)methyl)azetidin-1-yl)phenyl)amino)pyrimidin-4-yl)-2-methylbenzyl)-3-isopropoxyazetidine-1-carboxamide |
| 113 | N-(4-(2-((4-(3-((4-(3-((2,6-dioxopiperidin-3-yl)amino)benzoyl)piperazin-1-yl)methyl)azetidin-1-yl)phenyl)amino)pyrimidin-4-yl)-2-methylbenzyl)-3-isopropoxyazetidine-1-carboxamide |
| 114 | N-(4-(2-((4-(4-((4-(3-((2,6-dioxopiperidin-3-yl)amino)benzoyl)piperazin-1-yl)methyl)piperidin-1-yl)phenyl)amino)pyrimidin-4-yl)-2-methylbenzyl)-3-isopropoxyazetidine-1-carboxamide |
| 115 | N-(4-(2-((4-(4-((1-(3-((2,6-dioxopiperidin-3-yl)amino)benzyl)piperidin-4-yl)methyl)piperazin-1-yl)phenyl)amino)pyrimidin-4-yl)-2-methylbenzyl)-3-isopropoxyazetidine-1-carboxamide |
| 116 | N-(4-(2-((4-(4-(4-(3-((2,6-dioxopiperidin-3-yl)amino)phenyl)piperidine-1-carbonyl)piperidin-1-yl)phenyl)amino)pyrimidin-4-yl)-2-methylbenzyl)-3-isopropoxyazetidine-1-carboxamide |
| 117 | N-(4-(2-((4-(4-(4-(4-((2,6-dioxopiperidin-3-yl)amino)phenyl)piperidine-1-carbonyl)piperidin-1-yl)phenyl)amino)pyrimidin-4-yl)-2-methylbenzyl)-3-isopropoxyazetidine-1-carboxamide |
| 118 | N-(4-(2-((4-(4-(4-(4-((2,6-dioxopiperidin-3-yl)oxy)phenyl)piperidine-1-carbonyl)piperidin-1-yl)phenyl)amino)pyrimidin-4-yl)-2-methylbenzyl)-3-isopropoxyazetidine-1-carboxamide |
| 119 | N-(4-(2-((4-(4-((4-(4-((2,6-dioxopiperidin-3-yl)oxy)phenyl)piperidin-1-yl)methyl)piperidin-1-yl)phenyl)amino)pyrimidin-4-yl)-2-methylbenzyl)-3-isopropoxyazetidine-1-carboxamide |
| 120 | N-(4-(2-((4-(4-((4-(3-((2,6-dioxopiperidin-3-yl)amino)phenyl)piperazin-1-yl)methyl)piperidin-1-yl)phenyl)amino)pyrimidin-4-yl)-2-methylbenzyl)-3-isopropoxyazetidine-1-carboxamide |
| 121 | N-(4-(2-((4-(4-((4-(4-((2,6-dioxopiperidin-3-yl)amino)phenyl)piperazin-1-yl)methyl)piperidin-1-yl)phenyl)amino)pyrimidin-4-yl)-2-methylbenzyl)-3-isopropoxyazetidine-1-carboxamide |
| 122 | N-(4-(2-((4-(4-((4-(3-((2,6-dioxopiperidin-3-yl)amino)phenyl)piperidin-1-yl)methyl)piperidin-1-yl)phenyl)amino)pyrimidin-4-yl)-2-methylbenzyl)-3-isopropoxyazetidine-1-carboxamide |
| 123 | N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-3-isopropoxyazetidine-1-carboxamide |
| 124 | N-(4-(2-((4-(4-((4-(5-((2,6-dioxopiperidin-3-yl)oxy)pyridin-2-yl)piperidin-1-yl)methyl)piperidin-1-yl)phenyl)amino)pyrimidin-4-yl)-2-methylbenzyl)-3-isopropoxyazetidine-1-carboxamide |
| 125 | N-(4-(2-((4-(4-((4-(5-((2,6-dioxopiperidin-3-yl)oxy)pyridin-2-yl)piperazin-1-yl)methyl)piperidin-1-yl)phenyl)amino)pyrimidin-4-yl)-2-methylbenzyl)-3-isopropoxyazetidine-1-carboxamide |
| 126 | N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-3-isopropoxyazetidine-1-carboxamide |
| 127 | N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-3-isopropoxyazetidine-1-carboxamide |
| 128 | N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1-isopropyl-1H-pyrazole-4-carboxamide |
| 129 | N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1-isopropyl-1H-pyrazole-4-carboxamide |
| 130 | 1-(tert-butyl)-N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1H-pyrazole-4-carboxamide |
| 131 | 1-(tert-butyl)-N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1H-pyrazole-4-carboxamide |
| 132 | 1-(tert-butyl)-N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1H-1,2,3-triazole-4-carboxamide |
| 133 | 1-(tert-butyl)-N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1H-1,2,3-triazole-4-carboxamide |
| 134 | N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1-isopropyl-1H-pyrazole-4-carboxamide |
| 135 | 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1H-pyrazole-4-carboxamide |
| 136 | 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1H-1,2,3-triazole-4-carboxamide |
| 137 | 1-(tert-butyl)-N-((5-(2-((4-(4-((4-(4-((2,6-dioxopiperidin-3-yl)amino)phenyl)piperidin-1-yl)methyl)piperidin-1-yl)phenyl)amino)pyrimidin-4-yl)-3-methylpyridin-2-yl)methyl)-1H-1,2,3-triazole-4-carboxamide |
| 138 | 1-(tert-butyl)-N-(4-(2-((4-(4-((4-(3-(2,4-dioxotetrahydropyrimidin-1(2H)-yl)phenyl)piperidin-1-yl)methyl)piperidin-1-yl)phenyl)amino)pyrimidin-4-yl)-2-methylbenzyl)-1H-1,2,3-triazole-4-carboxamide |
| 139 | 1-(tert-butyl)-N-(4-(2-((4-(4-((4-(4-(2,4-dioxotetrahydropyrimidin-1(2H)-yl)phenyl)piperidin-1-yl)methyl)piperidin-1-yl)phenyl)amino)pyrimidin-4-yl)-2-methylbenzyl)-1H-1,2,3-triazole-4-carboxamide |
| 140 | 1-(tert-butyl)-N-(4-(2-((4-((4-((4-(3-(2,4-dioxotetrahydropyrimidin-1(2H)-yl)phenyl)piperidin-1-yl)methyl)piperidin-1-yl)methyl)phenyl)amino)pyrimidin-4-yl)-2-methylbenzyl)-1H-1,2,3-triazole-4-carboxamide |
| 141 | 1-(tert-butyl)-N-(4-(2-((4-((4-((4-(4-(2,4-dioxotetrahydropyrimidin-1(2H)-yl)phenyl)piperidin-1-yl)methyl)piperidi n-1-yl)methyl)phenyl)amino)pyrimidin-4-yl)-2-methylbenzyl)-1H-1,2,3-triazole-4-carboxamide |
| 142 | 1-(tert-butyl)-N-(4-(2-((4-((4-((4-(4-((2,6-dioxopiperidin-3-yl)amino)phenyl)piperidin-1-yl)methyl)piperidin-1-yl)methyl)phenyl)amino)pyrimidin-4-yl)-2-methylbenzyl)-1H-1,2,3-triazole-4-carboxamide |
| 143 | 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-(hydroxymethyl)benzyl)-1H-1,2,3-triazole-4-carboxamide |
| 144 | N-(4-(2-((6-(4-((4-(4-((2,6-dioxopiperidin-3-yl)oxy)phenyl)piperidin-1-yl)methyl)piperidin-1-yl)pyridin-3-yl)amino)pyrimidin-4-yl)-2-methylbenzyl)-3-isopropoxyazetidine-1-carboxamide |
| 145 | N-(4-(2-((6-(4-((4-(4-((2,6-dioxopiperidin-3-yl)amino)phenyl)piperidin-1-yl)methyl)piperidin-1-yl)pyridin-3-yl)amino)pyrimidin-4-yl)-2-methylbenzyl)-3-isopropoxyazetidine-1-carboxamide |
| 146 | 1-(tert-butyl)-N-(4-(2-((6-(4-((4-(4-((2,6-dioxopiperidin-3-yl)amino)phenyl)piperidin-1-yl)methyl)piperidin-1-yl)pyridin-3-yl)amino)pyrimidin-4-yl)-2-methylbenzyl)-1H-1,2,3-triazole-4-carboxamide |

6. The compound of Claim 5 or a pharmaceutically acceptable salt thereof, wherein the compound is any one of the following compounds:
| Comp ound no. | IUPAC Name |
|---|---|
| 1 | N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-3-isopropoxyazetidine-1-carboxamide |
| 2 | 1-(tert-butyl)-N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-1H-pyrazole-4-carboxamide |
| 3 | 1-(tert-butyl)-N-(4-(2-((2-((1-(2-(2,6-dioxopiperidin-3-yl)-1,3-dioxoisoindolin-5-yl)piperidin-4-yl)methyl)-1,2,3,4-tetrahydroisoquinolin-7-yl)amino)pyrimidin-4-yl)-2-methylbenzyl)-1H-1,2,3-triazole-4-carboxamide |
| 7 | N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-3-isopropoxyazetidine-1-carboxamide |
| 8 | 1-(tert-butyl)-N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1H-pyrazole-4-carboxamide |
| 9 | 1-(tert-butyl)-N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1H-1,2,3-triazole-4-carboxamide |
| 10 | N-(4-(2-((4-(4-((1-(6-(2,6-dioxopiperidin-3-yl)-5-oxo-6,7-dihydro-5H-pyrrolo[3,4-b]pyridin-2-yl)piperidin-4-yl)methyl)piperazin-1-yl)phenyl)amino)pyrimidin-4-yl)-2-methylbenzyl)-3-isopropoxyazetidine-1-carboxamide |
| 11 | N-(4-(2-((4-(4-((1-(2-(2,6-dioxopiperidi n-3-yl)-1,3-dioxoisoindolin-5-yl)azetidin-3-yl)methyl)piperazin-1-yl)phenyl)amino)pyrimidin-4-yl)-2-methylbenzyl)-3-isopropoxyazetidine-1-carboxamide |
| 12 | N-(4-(2-((4-(4-((1-(2-(2,6-dioxopiperidin-3-yl)-6-fluoro-1,3-dioxoisoindolin-5-yl)piperidin-4-yl)methyl)piperazin-1-yl)phenyl)amino)pyrimidin-4-yl)-2-methylbenzyl)-3-isopropoxyazetidine-1-carboxamide |
| 13 | N-(4-(2-((4-(4-((1-(2-(2,6-dioxopiperidin-3-yl)-6-fluoro-1,3-dioxoisoindolin-5-yl)azetidin-3-yl)methyl)piperazin-1-yl)phenyl)amino)pyrimidin-4-yl)-2-methylbenzyl)-3-isopropoxyazetidine-1-carboxamide |
| 14 | N-(4-(2-((4-(4-((1-(2-(2,6-dioxopiperidin-3-yl)-1,3-dioxoisoindolin-5-yl)-3-methylazetidin-3-yl)methyl)piperazin-1-yl)phenyl)amino)pyrimidin-4-yl)-2-methylbenzyl)-3-isopropoxyazetidine-1-carboxamide |
| 15 | N-(4-(2-((4-(1-((1-(2-(2,6-dioxopiperidin-3-yl)-1,3-dioxoisoindolin-5-yl)piperidin-4-yl)methyl)piperidin-4-yl)phenyl)amino)pyrimidin-4-yl)-2-methylbenzyl)-3-isopropoxyazetidine-1-carboxamide |
| 16 | N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-3-isopropoxyazetidine-1-carboxamide |
| 17 | N-(4-(2-((6-(4-((1-(2-(2,6-dioxopiperidin-3-yl)-1,3-dioxoisoindolin-5-yl)piperidin-4-yl)methyl)piperazin-1-yl)pyridin-3-yl)amino)pyrimidin-4-yl)-2-methylbenzyl)-3-isopropoxyazetidine-1-carboxamide |
| 18 | N-(4-(2-((6-(4-((1-(2-(2,6-dioxopiperidin-3-yl)-1,3-dioxoisoindolin-5-yl)azetidin-3-yl)methyl)piperazin-1-yl)pyridin-3-yl)amino)pyrimidin-4-yl)-2-methylbenzyl)-3-isopropoxyazetidine-1-carboxamide |
| 19 | N-(4-(2-((6-(1-((1-(2-(2,6-dioxopiperidin-3-yl)-1,3-dioxoisoindolin-5-yl)piperidin-4-yl)methyl)piperidin-4-yl)pyridin-3-yl)amino)pyrimidin-4-yl)-2-methylbenzyl)-3-isopropoxyazetidine-1-carboxamide |
| 20 | N-(4-(2-((6-(1-((1-(2-(2,6-dioxopiperidin-3-yl)-1,3-dioxoisoindolin-5-yl)azetidin-3-yl)methyl)piperidin-4-yl)pyridin-3-yl)amino)pyrimidin-4-yl)-2-methylbenzyl)-3-isopropoxyazetidine-1-carboxamide |
| 21 | N-(4-(2-((1-(1-((1-(2-(2,6-dioxopiperidin-3-yl)-1,3-dioxoisoindolin-5-yl)piperidin-4-yl)methyl)piperidin-4-yl)-1 H-pyrazol-4-yl)amino)pyrimidin-4-yl)-2-methylbenzyl)-3-isopropoxyazetidine-1-carboxamide |
| 22 | N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-3-isopropoxyazetidine-1-carboxamide |
| 23 | N-(3-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-5-fluoro-2-methylphenyl)-3-isopropoxyazetidine-1-carboxamide |
| 26 | N-(3-(2-((4-(4-((1-(6-(2,6-dioxopiperidin-3-yl)-5-oxo-6,7-dihydro-5H-pyrrolo[3,4-b]pyridin-2-yl)piperidin-4-yl)methyl)piperazin-1-yl)phenyl)amino)pyrimidin-4-yl)-5-fluoro-2-methylphenyl)-3-isopropoxyazetidine-1-carboxamide |
| 27 | N-(3-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-5-fluoro-2-methylphenyl)-3-isopropoxyazetidine-1-carboxamide |
| 31 | 1-(tert-butyl)-N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1H-pyrazole-4-carboxamide |
| 32 | 1-(tert-butyl)-N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1H-1,2,3-triazole-4-carboxamide |
| 33 | N-(4-(2-((4-(4-((1-(2-(2,6-dioxopiperidin-3-yl)-1,3-dioxoisoindolin-5-yl)piperidin-4-yl)methyl)piperazin-1-yl)phenyl)amino)pyrimidin-4-yl)-2-methylbenzyl)-1-isopropyl-1H-pyrazole-4-carboxamide |
| 34 | N-(4-(2-((4-(4-((1-(2-(2,6-dioxopiperidin-3-yl)-1,3-dioxoisoindolin-5-yl)azetidin-3-yl)methyl)piperazin-1-yl)phenyl)amino)pyrimidin-4-yl)-2-methylbenzyl)-1-isopropyl-1H-pyrazole-4-carboxamide |
| 35 | N-(4-(2-((1-(1-((1-(2-(2,6-dioxopiperidin-3-yl)-1,3-dioxoisoindolin-5-yl)piperidin-4-yl)methyl)piperidin-4-yl)-1 H-pyrazol-4-yl)amino)pyrimidin-4-yl)-2-methylbenzyl)-1-isopropyl-1H-pyrazole-4-carboxamide |
| 36 | N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1-isopropyl-1H-pyrazole-4-carboxamide |
| 37 | 1-(tert-butyl)-N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1H-pyrazole-4-carboxamide |
| 38 | 1-(tert-butyl)-N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1H-pyrazole-4-carboxamide |
| 39 | 1-(tert-butyl)-N-(4-(2-((1-(1-((1-(2-(2,6-dioxopiperidin-3-yl)-1,3-dioxoisoindolin-5-yl)piperidin-4-yl)methyl)piperidin-4-yl)-1H-pyrazol-4-yl)amino)pyrimidin-4-yl)-2-methylbenzyl)-1H-1,2,3-triazole-4-carboxamide |
| 40 | 1-(tert-butyl)-N-(4-(2-((1-(1-((1-(2-(2,6-dioxopiperidin-3-yl)-1,3-dioxoisoindolin-5-yl)azetidin-3-yl)methyl)piperidin-4-yl)-1H-pyrazol-4-yl)amino)pyrimidin-4-yl)-2-methylbenzyl)-1H-1,2,3-triazole-4-carboxamide |
| 41 | N-(4-(2-((4-(1-((1-(2-(2,6-dioxopiperidin-3-yl)-1,3-dioxoisoindolin-5-yl)piperidin-4-yl)methyl)azetidin-3-yl)phenyl)amino)pyrimidin-4-yl)-2-methylbenzyl)-3-isopropoxyazetidine-1-carboxamide |
| 42 | N-(4-(2-((4-(4-((1-(3-((2,6-dioxopiperidin-3-yl)oxy)phenyl)piperidin-4-yl)methyl)piperazin-1-yl)phenyl)amino)pyrimidin-4-yl)-2-methylbenzyl)-3-isopropoxyazetidine-1-carboxamide |
| 43 | N-(4-(2-((4-(4-((1-(4-((2,6-dioxopiperidin-3-yl)oxy)phenyl)piperidin-4-yl)methyl)piperazin-1-yl)phenyl)amino)pyrimidin-4-yl)-2-methylbenzyl)-3-isopropoxyazetidine-1-carboxamide |
| 44 | N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1-isopropyl-1H-pyrazole-4-carboxamide |
| 45 | 1-(tert-butyl)-N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1H-pyrazole-4-carboxamide |
| 46 | 1-(tert-butyl)-N-(4-(2-((4-(1-((1-(2-(2,6-dioxopiperidin-3-yl)-1,3-dioxoisoindolin-5-yl)azetidin-3-yl)methyl)piperidin-4-yl)phenyl)amino)pyrimidin-4-yl)-2-methylbenzyl)-1H-1,2,3-triazole-4-carboxamide |
| 47 | N-(4-(2-((4-(1-((1-(4-((2,6-dioxopiperidin-3-yl)oxy)phenyl)piperidin-4-yl)methyl)piperidin-4-yl)phenyl)amino)pyrimidin-4-yl)-2-methylbenzyl)-3-isopropoxyazetidine-1-carboxamide |
| 48 | N-(4-(2-((4-(4-((1-(4-((2,6-dioxopiperidin-3-yl)oxy)phenyl)piperidin-4-yl)methyl)piperazin-1-yl)phenyl)amino)pyrimidin-4-yl)-2-methylbenzyl)-1-isopropyl-1H-pyrazole-4-carboxamide |
| 49 | N-(4-(2-((4-(1-((1-(4-((2,6-dioxopiperidin-3-yl)oxy)phenyl)piperidin-4-yl)methyl)piperidin-4-yl)phenyl)amino)pyrimidin-4-yl)-2-methylbenzyl)-1-isopropyl-1H-pyrazole-4-carboxamide |
| 50 | N-(4-(2-((4-(4-((1-(5-((2,6-dioxopiperidin-3-yl)oxy)pyridin-2-yl)piperidin-4-yl)methyl)piperazin-1-yl)phenyl)amino)pyrimidin-4-yl)-2-methylbenzyl)-3-isopropoxyazetidine-1-carboxamide |
| 51 | N-(4-(2-((4-(1-((1-(5-((2,6-dioxopiperidin-3-yl)oxy)pyridin-2-yl)piperidin-4-yl)methyl)piperidin-4-yl)phenyl)amino)pyrimidin-4-yl)-2-methylbenzyl)-3-isopropoxyazetidine-1-carboxamide |
| 52 | N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-3-isopropoxyazetidine-1-carboxamide |
| 53 | N-(4-(2-((4-(1-((1-(5-((2,6-dioxopiperidin-3-yl)amino)pyridin-2-yl)piperidin-4-yl)methyl)piperidin-4-yl)phenyl)amino)pyrimidin-4-yl)-2-methylbenzyl)-3-isopropoxyazetidine-1-carboxamide |
| 54 | N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-3-isopropoxyazetidine-1-carboxamide |
| 55 | N-(4-(2-((4-(4-((1-(4-((2,6-dioxopiperidin-3-yl)amino)phenyl)piperidin-4-yl)methyl)piperazin-1-yl)phenyl)amino)pyrimidin-4-yl)-2-methylbenzyl)-3-isopropoxyazetidine-1-carboxamide |
| 56 | N-(4-(2-((4-(1-((1-(4-((2,6-dioxopiperidin-3-yl)amino)phenyl)piperidin-4-yl)methyl)piperidin-4-yl)phenyl)amino)pyrimidin-4-yl)-2-methylbenzyl)-3-isopropoxyazetidine-1-carboxamide |
| 57 | N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1-isopropyl-1H-pyrazole-4-carboxamide |
| 58 | N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1-isopropyl-1H-pyrazole-4-carboxamide |
| 59 | 1-(tert-butyl)-N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1H-pyrazole-4-carboxamide |
| 60 | 1-(tert-butyl)-N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1H-pyrazole-4-carboxamide |
| 61 | 1-(tert-butyl)-N-(4-(2-((4-(4-((1-(3-((2,6-dioxopiperidin-3-yl)amino)phenyl)piperidin-4-yl)methyl)piperazin-1-yl)phenyl)amino)pyrimidin-4-yl)-2-methylbenzyl)-1H-1,2,3-triazole-4-carboxamide |
| 62 | 1-(tert-butyl)-N-(4-(2-((4-(4-((1-(5-((2,6-dioxopiperidin-3-yl)amino)pyridin-2-yl)piperidin-4-yl)methyl)piperazin-1-yl)phenyl)amino)pyrimidin-4-yl)-2-methylbenzyl)-1H-1,2,3-triazole-4-carboxamide |
| 63 | N-(4-(2-((4-((4-((1-(3-((2,6-dioxopiperidin-3-yl)oxy)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 64 | N-(4-(2-((4-((4-((1-(3-((2,6-dioxopiperidin-3-yl)amino)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 65 | N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-3-isopropoxyazetidine-1-carboxamide |
| 66 | N-(4-(2-((4-(4-((1-(4-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-3-isopropoxyazetidine-1-carboxamide |
| 67 | N-(4-(2-((4-((4-((1-(3-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 68 | N-(4-(2-((4-((4-((1-(4-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-3-isopropoxyazetidine-1-carboxamide |
| 69 | 1-(tert-butyl)-N-(4-(2-((4-((4-((1-(3-((2,6-dioxopiperidin-3-yl)amino)phenyl)piperidin-4-yl)methyl)piperazin-1-yl)methyl)phenyl)amino)pyrimidin-4-yl)-2-methylbenzyl)-1H-1,2,3-triazole-4-carboxamide |
| 70 | 1-(tert-butyl)-N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1H-1,2,3-triazole-4-carboxamide |
| 71 | 1-(tert-butyl)-N-(4-(2-((4-(4-((1-(4-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1H-1,2,3-triazole-4-carboxamide |
| 72 | 1-(tert-butyl)-N-(4-(2-((4-(4-((1-(4-((2,6-dioxopiperidin-3-yl)amino)-2-fluorophenyl)piperidin-4-yl)methyl)piperazin-1-yl)phenyl)amino)pyrimidin-4-yl)-2-methylbenzyl)-1H-1,2,3-triazole-4-carboxamide |
| 73 | 1-(tert-butyl)-N-(4-(2-((4-(4-((1-(4-(2,4-dioxotetrahydropyrimidin-1(2H)-yl)-2-fluorophenyl)piperidin-4-yl)methyl)piperazin-1-yl)phenyl)amino)pyrimidin-4-yl)-2-methylbenzyl)-1H-1,2,3-triazole-4-carboxamide |
| 74 | 1-(tert-butyl)-N-(4-(2-((4-((4-((1-(3-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-1H-1,2,3-triazole-4-carboxamide |
| 75 | 1-(tert-butyl)-N-(4-(2-((4-((4-((1-(4-(2,4-dioxotetrahydropyrimidin-1(2H)-yl)phenyl)piperidin-4-yl)methyl)piperazin-1-yl)methyl)phenyl)amino)pyrimidin-4-yl)-2-methylbenzyl)-1H-1,2,3-triazole-4-carboxamide |
| 76 | 1-(tert-butyl)-N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1H-1,2,3-triazole-4-carboxamide |
| 77 | 1-(tert-butyl)-N-(4-(2-((6-(4-((1-(4-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1H-1,2,3-triazole-4-carboxamide |
| 78 | 1-(tert-butyl)-N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1H-1,2,3-triazole-4-carboxamide |
| 79 | 1-(tert-butyl)-N-(4-(2-((4-(1-((1-(4-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1H-1,2,3-triazole-4-carboxamide |
| 80 | N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1-isopropyl-1H-pyrazole-4-carboxamide |
| 81 | 1-(tert-butyl)-N-(4-(2-((4-(4-((1-(3-(2,4-dioxotetrahydropyrimidin-1(2H)-yl)phenyl)piperidin-4-yl)methyl)piperazin-1-yl)phenyl)amino)pyrimidin-4-yl)-2-methylbenzyl)-1H-pyrazole-4-carboxamide |
| 82 | N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1-isopropyl-1H-pyrazole-4-carboxamide |
| 83 | 1-(tert-butyl)-N-(4-(2-((6-(4-((1-(3-(2,4-dioxotetrahydropyrimidin-1(2H)-yl)phenyl)piperidin-4-yl)methyl)piperazin-1-yl)pyridin-3-yl)amino)pyrimidin-4-yl)-2-methylbenzyl)-1H-pyrazole-4-carboxamide |
| 84 | N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1-isopropyl-1H-pyrazole-4-carboxamide |
| 85 | 1-(tert-butyl)-N-(4-(2-((4-(1-((1-(3-(2,4-dioxotetrahydropyrimidin-1(2H)-yl)phenyl)piperidin-4-yl)methyl)piperidin-4-yl)phenyl)amino)pyrimidin-4-yl)-2-methylbenzyl)-1H-pyrazole-4-carboxamide |
| 86 | N-(4-(2-((4-(4-((1-(3-(2,4-dioxotetrahydropyrimidin-1(2H)-yl)-4-methoxybenzoyl)piperidin-4-yl)methyl)piperazin-1-yl)phenyl)amino)pyrimidin-4-yl)-2-methylbenzyl)-3-isopropoxyazetidine-1-carboxamide |
| 87 | N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-3-isopropoxyazetidine-1-carboxamide |
| 88 | N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-3-isopropoxyazetidine-1-carboxamide |
| 91 | N-(4-(2-((1-(1-((1-(3-((2,6-dioxopiperidin-3-yl)amino)benzoyl)piperidin-4-yl)methyl)piperidin-4-yl)-1H-pyrazol-4-yl)amino)pyrimidin-4-yl)-2-methylbenzyl)-3-isopropoxyazetidine-1-carboxamide |
| 93 | N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1-isopropyl-1H-pyrazole-4-carboxamide |
| 94 | N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1-isopropyl-1H-pyrazole-4-carboxamide |
| 95 | 1-(tert-butyl)-N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1H-pyrazole-4-carboxamide |
| 96 | 1-(tert-butyl)-N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1H-pyrazole-4-carboxamide |
| 97 | 1-(tert-butyl)-N-(4-(2-((4-(4-((1-(3-((2,6-dioxopiperidin-3-yl)amino)benzoyl)piperidin-4-yl)methyl)piperazin-1-yl)phenyl)amino)pyrimidin-4-yl)-2-methylbenzyl)-1H-1,2,3-triazole-4-carboxamide |
| 98 | 1-(tert-butyl)-N-(4-(2-((4-(4-((1-(3-((2,6-dioxopiperidin-3-yl)amino)benzoyl)azetidin-3-yl)methyl)piperazin-1-yl)phenyl)amino)pyrimidin-4-yl)-2-methylbenzyl)-1H-1,2,3-triazole-4-carboxamide |
| 99 | N-(4-(2-((4-(1-((1-(3-((2,6-dioxopiperidin-3-yl)amino)benzoyl)piperidin-4-yl)methyl)piperidin-4-yl)phenyl)amino)pyrimidin-4-yl)-2-methylbenzyl)-3-isopropoxyazetidine-1-carboxamide |
| 100 | N-(4-(2-((4-(1-((1-(3-((2,6-dioxopiperidin-3-yl)amino)benzoyl)azetidin-3-yl)methyl)piperidin-4-yl)phenyl)amino)pyrimidin-4-yl)-2-methylbenzyl)-3-isopropoxyazetidine-1-carboxamide |
| 101 | N-(4-(2-((4-(1-((1-(3-((2,6-dioxopiperidin-3-yl)amino)benzoyl)piperidin-4-yl)methyl)azetidin-3-yl)phenyl)amino)pyrimidin-4-yl)-2-methylbenzyl)-3-isopropoxyazetidine-1-carboxamide |
| 102 | N-(4-(2-((4-(4-((1-(3-((2,6-dioxopiperidin-3-yl)oxy)benzoyl)piperidin-4-yl)methyl)piperazin-1-yl)phenyl)amino)pyrimidin-4-yl)-2-methylbenzyl)-3-isopropoxyazetidine-1-carboxamide |
| 103 | N-(4-(2-((4-(4-(1-(3-((2,6-dioxopiperidin-3-yl)amino)benzoyl)piperidin-4-yl)piperazin-1-yl)phenyl)amino)pyrimidin-4-yl)-2-methylbenzyl)-3-isopropoxyazetidine-1-carboxamide |
| 105 | N-(4-(2-((4-(4-((1-(2-((3-((2,6-dioxopiperidin-3-yl)amino)phenyl)amino)-2-oxoethyl)piperidin-4-yl)methyl)piperazin-1-yl)phenyl)amino)pyrimidin-4-yl)-2-methylbenzyl)-3-isopropoxyazetidine-1-carboxamide |
| 107 | N-(4-(2-((4-(4-((1-(6-(2,6-dioxopiperidin-3-yl)-5-oxo-6,7-dihydro-5H-pyrrolo[3,4-b]pyridin-2-yl)azetidin-3-yl)methyl)piperazin-1-yl)phenyl)amino)pyrimidin-4-yl)-2-methylbenzyl)-3-isopropoxyazetidine-1-carboxamide |
| 108 | N-(3-(2-((4-(4-((1-(6-(2,6-dioxopiperidin-3-yl)-5-oxo-6,7-dihydro-5H-pyrrolo[3,4-b]pyridin-2-yl)azetidin-3-yl)methyl)piperazin-1-yl)phenyl)amino)pyrimidin-4-yl)-5-fluoro-2-methylphenyl)-3-isopropoxyazetidine-1-carboxamide |
| 109 | N-(4-(2-((4-(4-(1-(2-(2,6-dioxopiperidin-3-yl)-1,3-dioxoisoindolin-5-yl)piperidin-4-yl)piperazin-1-yl)phenyl)amino)pyrimidin-4-yl)-2-methylbenzyl)-3-isopropoxyazetidine-1-carboxamide |
| 110 | N-(4-(2-((4-(1'-(2-(2,6-dioxopiperidin-3-yl)-1,3-dioxoisoindolin-5-yl)-[1,4'-bipiperidin]-4-yl)phenyl)amino)pyrimidin-4-yl)-2-methylbenzyl)-3-isopropoxyazetidine-1-carboxamide |
| 111 | N-(4-(2-((4-(4-((4-(2-(2,6-dioxopiperidin-3-yl)-1,3-dioxoisoindolin-5-yl)piperazin-1-yl)methyl)piperidin-1-yl)phenyl)amino)pyrimidin-4-yl)-2-methylbenzyl)-3-isopropoxyazetidine-1-carboxamide |
| 112 | N-(4-(2-((4-(3-((4-(2-(2,6-dioxopiperidin-3-yl)-1,3-dioxoisoindolin-5-yl)piperazin-1-yl)methyl)azetidin-1-yl)phenyl)amino)pyrimidin-4-yl)-2-methylbenzyl)-3-isopropoxyazetidine-1-carboxamide |
| 113 | N-(4-(2-((4-(3-((4-(3-((2,6-dioxopiperidin-3-yl)amino)benzoyl)piperazin-1-yl)methyl)azetidin-1-yl)phenyl)amino)pyrimidin-4-yl)-2-methylbenzyl)-3-isopropoxyazetidine-1-carboxamide |
| 114 | N-(4-(2-((4-(4-((4-(3-((2,6-dioxopiperidin-3-yl)amino)benzoyl)piperazin-1-yl)methyl)piperidin-1-yl)phenyl)amino)pyrimidin-4-yl)-2-methylbenzyl)-3-isopropoxyazetidine-1-carboxamide |
| 115 | N-(4-(2-((4-(4-((1-(3-((2,6-dioxopiperidin-3-yl)amino)benzyl)piperidin-4-yl)methyl)piperazin-1-yl)phenyl)amino)pyrimidin-4-yl)-2-methylbenzyl)-3-isopropoxyazetidine-1-carboxamide |
| 116 | N-(4-(2-((4-(4-(4-(3-((2,6-dioxopiperidin-3-yl)amino)phenyl)piperidine-1-carbonyl)piperidin-1-yl)phenyl)amino)pyrimidin-4-yl)-2-methylbenzyl)-3-isopropoxyazetidine-1-carboxamide |
| 117 | N-(4-(2-((4-(4-(4-(4-((2,6-dioxopiperidin-3-yl)amino)phenyl)piperidine-1-carbonyl)piperidin-1-yl)phenyl)amino)pyrimidin-4-yl)-2-methylbenzyl)-3-isopropoxyazetidine-1-carboxamide |
| 118 | N-(4-(2-((4-(4-(4-(4-((2,6-dioxopiperidin-3-yl)oxy)phenyl)piperidine-1-carbonyl)piperidin-1-yl)phenyl)amino)pyrimidin-4-yl)-2-methylbenzyl)-3-isopropoxyazetidine-1-carboxamide |
| 119 | N-(4-(2-((4-(4-((4-(4-((2,6-dioxopiperidin-3-yl)oxy)phenyl)piperidin-1-yl)methyl)piperidin-1-yl)phenyl)amino)pyrimidin-4-yl)-2-methylbenzyl)-3-isopropoxyazetidine-1-carboxamide |
| 120 | N-(4-(2-((4-(4-((4-(3-((2,6-dioxopiperidin-3-yl)amino)phenyl)piperazin-1-yl)methyl)piperidin-1-yl)phenyl)amino)pyrimidin-4-yl)-2-methylbenzyl)-3-isopropoxyazetidine-1-carboxamide |
| 121 | N-(4-(2-((4-(4-((4-(4-((2,6-dioxopiperidin-3-yl)amino)phenyl)piperazin-1-yl)methyl)piperidin-1-yl)phenyl)amino)pyrimidin-4-yl)-2-methylbenzyl)-3-isopropoxyazetidine-1-carboxamide |
| 122 | N-(4-(2-((4-(4-((4-(3-((2,6-dioxopiperidin-3-yl)amino)phenyl)piperidin-1-yl)methyl)piperidin-1-yl)phenyl)amino)pyrimidin-4-yl)-2-methylbenzyl)-3-isopropoxyazetidine-1-carboxamide |
| 123 | N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-3-isopropoxyazetidine-1-carboxamide |
| 124 | N-(4-(2-((4-(4-((4-(5-((2,6-dioxopiperidin-3-yl)oxy)pyridin-2-yl)piperidin-1-yl)methyl)piperidin-1-yl)phenyl)amino)pyrimidin-4-yl)-2-methylbenzyl)-3-isopropoxyazetidine-1-carboxamide |
| 125 | N-(4-(2-((4-(4-((4-(5-((2,6-dioxopiperidin-3-yl)oxy)pyridin-2-yl)piperazin-1-yl)methyl)piperidin-1-yl)phenyl)amino)pyrimidin-4-yl)-2-methylbenzyl)-3-isopropoxyazetidine-1-carboxamide |
| 126 | N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-3-isopropoxyazetidine-1-carboxamide |
| 127 | N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-3-isopropoxyazetidine-1-carboxamide |
| 128 | N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1-isopropyl-1H-pyrazole-4-carboxamide |
| 129 | N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1-isopropyl-1H-pyrazole-4-carboxamide |
| 130 | 1-(tert-butyl)-N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1H-pyrazole-4-carboxamide |
| 131 | 1-(tert-butyl)-N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1H-pyrazole-4-carboxamide |
| 132 | 1-(tert-butyl)-N-(4-(2-((4-(4-((4-(5-((2,6-dioxopiperidin-3-yl)amino)pyridin-2-yl)piperidin-1-yl)methyl)piperidin-1-yl)phenyl)amino)pyrimidin-4-yl)-2-methylbenzyl)-1H-1,2,3-triazole-4-carboxamide |
| 133 | 1-(tert-butyl)-N-(4-(2-((4-(4-((4-(5-((2,6-dioxopiperidin-3-yl)amino)pyridin-2-yl)piperazin-1-yl)methyl)piperidin-1-yl)phenyl)amino)pyrimidin-4-yl)-2-methylbenzyl)-1H-1,2,3-triazole-4-carboxamide |
| 134 | N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1-isopropyl-1H-pyrazole-4-carboxamide |
| 135 | 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1H-pyrazole-4-carboxamide |
| 136 | 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-methylbenzyl)-1H-1,2,3-triazole-4-carboxamide |
| 137 | 1-(tert-butyl)-N-((5-(2-((4-(4-((4-(4-((2,6-dioxopiperidin-3-yl)amino)phenyl)piperidin-1-yl)methyl)piperidin-1-yl)phenyl)amino)pyrimidin-4-yl)-3-methylpyridin-2-yl)methyl)-1H-1,2,3-triazole-4-carboxamide |
| 138 | 1-(tert-butyl)-N-(4-(2-((4-(4-((4-(3-(2,4-dioxotetrahydropyrimidin-1(2H)-yl)phenyl)piperidin-1-yl)methyl)piperidin-1-yl)phenyl)amino)pyrimidin-4-yl)-2-methylbenzyl)-1H-1,2,3-triazole-4-carboxamide |
| 139 | 1-(tert-butyl)-N-(4-(2-((4-(4-((4-(4-(2,4-dioxotetrahydropyrimidin-1(2H)-yl)phenyl)piperidin-1-yl)methyl)piperidin-1-yl)phenyl)amino)pyrimidin-4-yl)-2-methylbenzyl)-1H-1,2,3-triazole-4-carboxamide |
| 141 | 1-(tert-butyl)-N-(4-(2-((4-((4-((4-(4-(2,4-dioxotetrahydropyrimidin-1(2H)-yl)phenyl)piperidin-1-yl)methyl)piperidin-1-yl)methyl)phenyl)amino)pyrimidin-4-yl)-2-methylbenzyl)-1H-1,2,3-triazole-4-carboxamide |
| 142 | 1-(tert-butyl)-N-(4-(2-((4-((4-((4-(4-((2,6-dioxopiperidin-3-yl)amino)phenyl)piperidin-1-yl)methyl)piperidin-1-yl)methyl)phenyl)amino)pyrimidin-4-yl)-2-methylbenzyl)-1H-1,2,3-triazole-4-carboxamide |
| 143 | 1-(tert-butyl)-N-(4-(2-((4-(4-((4-(4-((2,6-dioxopiperidin-3-yl)amino)phenyl)piperidin-1-yl)methyl)piperidin-1-yl)phenyl)amino)pyrimidin-4-yl)-2-(hydroxymethyl)benzyl)-1H-1,2,3-triazole-4-carboxamide |
| 144 | N-(4-(2-((6-(4-((4-(4-((2,6-dioxopiperidin-3-yl)oxy)phenyl)piperidin-1-yl)methyl)piperidin-1-yl)pyridin-3-yl)amino)pyrimidin-4-yl)-2-methylbenzyl)-3-isopropoxyazetidine-1-carboxamide |
| 145 | N-(4-(2-((6-(4-((4-(4-((2,6-dioxopiperidin-3-yl)amino)phenyl)piperidin-1-yl)methyl)piperidin-1-yl)pyridin-3-yl)amino)pyrimidin-4-yl)-2-methylbenzyl)-3-isopropoxyazetidine-1-carboxamide |
| 146 | 1-(tert-butyl)-N-(4-(2-((6-(4-((4-(4-((2,6-dioxopiperidin-3-yl)amino)phenyl)piperidin-1-yl)methyl)piperidin-1-yl)pyridin-3-yl)amino)pyrimidin-4-yl)-2-methylbenzyl)-1H-1,2,3-triazole-4-carboxamide |

7. A composition comprising a compound of any one of Claims 1 to 6 or a pharmaceutically acceptable salt thereof, and a pharmaceutically acceptable carrier.

8. A pharmaceutical composition for degrading or inhibiting Bruton's tyrosine kinase (BTK), comprising as an effective agent a compound of any one of Claims 1 to 6 or a pharmaceutically acceptable salt thereof.

9. The pharmaceutical composition of Claim 8, wherein the pharmaceutical composition is for treating or preventing an autoimmune disease or cancer.

10. The pharmaceutical composition of Claim 8, wherein the pharmaceutical composition is for treating or preventing leukemia, lymphoma, solid tumor, autoimmune disease, Waldenstrom macroglobulinemia, B-cell malignancy, recurrent mature B-cell neoplasm, multiple myeloma, multiple sclerosis, graft-versus-host-disease, agammaglobulinemia, hepatitis, neuromyelitis optica, myelodysplastic syndrome (MDS), plasmacytoma, asthma, chronic obstructive pulmonary disease (COPD), transplant rejection, gout, atherosclerosis, inflammatory bowel disease, pancreatitis, B-cell-mediated hyperacute disease, thromboembolic disorder, pulmonary embolism, polycythemia vera, essential thrombocythemia, myelofibrosis with myeloid metaplasia, or acute inflammatory disease.

11. A method for treating or preventing a Bruton's tyrosine kinase (BTK)-related disease, comprising administering to a subject in need thereof a therapeutically effective amount of a compound of any one of Claims 1 to 6 or pharmaceutically acceptable salt thereof.

12. The method of Claim 11, wherein the Bruton's tyrosine kinase (BTK)-related disease is an autoimmune disease or cancer.

13. The method of Claim 11, wherein the Bruton's tyrosine kinase (BTK)-related disease is leukemia, lymphoma, solid tumor, autoimmune disease, Waldenstrom macroglobulinemia, B-cell malignancy, recurrent mature B-cell neoplasm, multiple myeloma, multiple sclerosis, graft-versus-host-disease, agammaglobulinemia, hepatitis, neuromyelitis optica, myelodysplastic syndrome (MDS), plasmacytoma, asthma, chronic obstructive pulmonary disease (COPD), transplant rejection, gout, atherosclerosis, inflammatory bowel disease, pancreatitis, B-cell-mediated hyperacute disease, thromboembolic disorder, pulmonary embolism, polycythemia vera, essential thrombocythemia, myelofibrosis with myeloid metaplasia, or acute inflammatory disease.
